(19) Europäisches Patentamt
European Patent Office
Office européen des brevets

(11) EP 3 053 577 A1

(12) **EUROPEAN PATENT APPLICATION**

(43) Date of publication:
**10.08.2016 Bulletin 2016/32**

(21) Application number: **15154342.8**

(22) Date of filing: **09.02.2015**

(51) Int Cl.:
***A61K 31/433*** (2006.01) ***A61K 31/435*** (2006.01)
***A61K 31/4353*** (2006.01) ***A61K 31/437*** (2006.01)
***A61K 31/438*** (2006.01) ***A61K 31/4436*** (2006.01)
***A61K 31/454*** (2006.01) ***A61K 31/4545*** (2006.01)
***A61K 31/4709*** (2006.01) ***A61K 31/506*** (2006.01)
***A61K 31/5377*** (2006.01) ***A61P 35/00*** (2006.01)
***A61K 45/06*** (2006.01) ***A61K 31/501*** (2006.01)
***A61K 31/502*** (2006.01) ***A61K 31/551*** (2006.01)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **F. Hoffmann-La Roche AG**
**4070 Basel (CH)**

(72) Inventor: **The designation of the inventor has not yet been filed**

(74) Representative: **Haefele Racin, Thomas**
**F. Hoffmann-La Roche AG**
**Patent Department**
**Grenzacherstrasse 124**
**4070 Basel (CH)**

Remarks:
Claims 16-25 are deemed to be abandoned due to non-payment of the claims fees (Rule 45(3) EPC).

(54) **Compounds for the treatment of cancer**

(57) The present invention relates to a FoxM1 gene splicing modifier selected from a compound of formula (I) or of formula (VI)

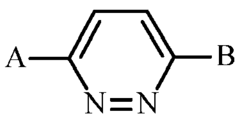
(I)

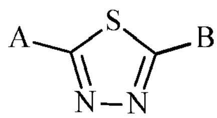
(VI),

wherein A and B are as defined herein, or a pharmaceutically acceptable salt thereof, for use in the treatment, prevention and/or delay of progression of cancer.

EP 3 053 577 A1

SEQUENCE LISTING

<110> F. Hoffmann-La Roche AG

<120> Compounds for the treatment of cancer

<130> 32500

<160> 6

<170> PatentIn version 3.5

<210> 1
<211> 801
<212> PRT
<213> Homo sapiens

<400> 1

Met Lys Thr Ser Pro Arg Arg Pro Leu Ile Leu Lys Arg Arg Arg Leu
1 5 10 15

Pro Leu Pro Val Gln Asn Ala Pro Ser Glu Thr Ser Glu Glu Glu Pro
20 25 30

Lys Arg Ser Pro Ala Gln Gln Glu Ser Asn Gln Ala Glu Ala Ser Lys
35 40 45

Glu Val Ala Glu Ser Asn Ser Cys Lys Phe Pro Ala Gly Ile Lys Ile
50 55 60

Ile Asn His Pro Thr Met Pro Asn Thr Gln Val Val Ala Ile Pro Asn
65 70 75 80

Asn Ala Asn Ile His Ser Ile Ile Thr Ala Leu Thr Ala Lys Gly Lys
85 90 95

Glu Ser Gly Ser Ser Gly Pro Asn Lys Phe Ile Leu Ile Ser Cys Gly
100 105 110

Gly Ala Pro Thr Gln Pro Pro Gly Leu Arg Pro Gln Thr Gln Thr Ser
115 120 125

Tyr Asp Ala Lys Arg Thr Glu Val Thr Leu Glu Thr Leu Gly Pro Lys
130 135 140

Pro Ala Ala Arg Asp Val Asn Leu Pro Arg Pro Pro Gly Ala Leu Cys
145 150 155 160

Glu Gln Lys Arg Glu Thr Cys Ala Asp Gly Glu Ala Ala Gly Cys Thr
165 170 175

```
Ile Asn Asn Ser Leu Ser Asn Ile Gln Trp Leu Arg Lys Met Ser Ser
            180                 185                 190

Asp Gly Leu Gly Ser Arg Ser Ile Lys Gln Glu Met Glu Glu Lys Glu
        195                 200                 205

Asn Cys His Leu Glu Gln Arg Gln Val Lys Val Glu Glu Pro Ser Arg
    210                 215                 220

Pro Ser Ala Ser Trp Gln Asn Ser Val Ser Glu Arg Pro Pro Tyr Ser
225                 230                 235                 240

Tyr Met Ala Met Ile Gln Phe Ala Ile Asn Ser Thr Glu Arg Lys Arg
                245                 250                 255

Met Thr Leu Lys Asp Ile Tyr Thr Trp Ile Glu Asp His Phe Pro Tyr
            260                 265                 270

Phe Lys His Ile Ala Lys Pro Gly Trp Lys Asn Ser Ile Arg His Asn
        275                 280                 285

Leu Ser Leu His Asp Met Phe Val Arg Glu Thr Ser Ala Asn Gly Lys
    290                 295                 300

Val Ser Phe Trp Thr Ile His Pro Ser Ala Asn Arg Tyr Leu Thr Leu
305                 310                 315                 320

Asp Gln Val Phe Lys Pro Leu Asp Pro Gly Ser Pro Gln Leu Pro Glu
                325                 330                 335

His Leu Glu Ser Gln Gln Lys Arg Pro Asn Pro Glu Leu Arg Arg Asn
            340                 345                 350

Met Thr Ile Lys Thr Glu Leu Pro Leu Gly Ala Arg Arg Lys Met Lys
        355                 360                 365

Pro Leu Leu Pro Arg Val Ser Ser Tyr Leu Val Pro Ile Gln Phe Pro
    370                 375                 380

Val Asn Gln Ser Leu Val Leu Gln Pro Ser Val Lys Val Pro Leu Pro
385                 390                 395                 400

Leu Ala Ala Ser Leu Met Ser Ser Glu Leu Ala Arg His Ser Lys Arg
                405                 410                 415

Val Arg Ile Ala Pro Lys Val Phe Gly Glu Gln Val Val Phe Gly Tyr
            420                 425                 430
```

```
Met Ser Lys Phe Phe Ser Gly Asp Leu Arg Asp Phe Gly Thr Pro Ile
        435                 440                 445

Thr Ser Leu Phe Asn Phe Ile Phe Leu Cys Leu Ser Val Leu Leu Ala
    450                 455                 460

Glu Glu Gly Ile Ala Pro Leu Ser Ser Ala Gly Pro Gly Lys Glu Glu
465                 470                 475                 480

Lys Leu Leu Phe Gly Glu Gly Phe Ser Pro Leu Leu Pro Val Gln Thr
                485                 490                 495

Ile Lys Glu Glu Glu Ile Gln Pro Gly Glu Glu Met Pro His Leu Ala
            500                 505                 510

Arg Pro Ile Lys Val Glu Ser Pro Pro Leu Glu Glu Trp Pro Ser Pro
        515                 520                 525

Ala Pro Ser Phe Lys Glu Glu Ser Ser His Ser Trp Glu Asp Ser Ser
    530                 535                 540

Gln Ser Pro Thr Pro Arg Pro Lys Lys Ser Tyr Ser Gly Leu Arg Ser
545                 550                 555                 560

Pro Thr Arg Cys Val Ser Glu Met Leu Val Ile Gln His Arg Glu Arg
                565                 570                 575

Arg Glu Arg Ser Arg Ser Arg Arg Lys Gln His Leu Leu Pro Pro Cys
            580                 585                 590

Val Asp Glu Pro Glu Leu Leu Phe Ser Glu Gly Pro Ser Thr Ser Arg
        595                 600                 605

Trp Ala Ala Glu Leu Pro Phe Pro Ala Asp Ser Ser Asp Pro Ala Ser
    610                 615                 620

Gln Leu Ser Tyr Ser Gln Glu Val Gly Gly Pro Phe Lys Thr Pro Ile
625                 630                 635                 640

Lys Glu Thr Leu Pro Ile Ser Ser Thr Pro Ser Lys Ser Val Leu Pro
                645                 650                 655

Arg Thr Pro Glu Ser Trp Arg Leu Thr Pro Pro Ala Lys Val Gly Gly
            660                 665                 670

Leu Asp Phe Ser Pro Val Gln Thr Ser Gln Gly Ala Ser Asp Pro Leu
        675                 680                 685
```

```
Pro Asp Pro Leu Gly Leu Met Asp Leu Ser Thr Thr Pro Leu Gln Ser
    690                 695                 700

Ala Pro Pro Leu Glu Ser Pro Gln Arg Leu Leu Ser Ser Glu Pro Leu
705                 710                 715                 720

Asp Leu Ile Ser Val Pro Phe Gly Asn Ser Ser Pro Ser Asp Ile Asp
                725                 730                 735

Val Pro Lys Pro Gly Ser Pro Glu Pro Gln Val Ser Gly Leu Ala Ala
            740                 745                 750

Asn Arg Ser Leu Thr Glu Gly Leu Val Leu Asp Thr Met Asn Asp Ser
        755                 760                 765

Leu Ser Lys Ile Leu Leu Asp Ile Ser Phe Pro Gly Leu Asp Glu Asp
    770                 775                 780

Pro Leu Gly Pro Asp Asn Ile Asn Trp Ser Gln Phe Ile Pro Glu Leu
785                 790                 795                 800

Gln



<210>  2
<211>  748
<212>  PRT
<213>  Homo sapiens

<400>  2

Met Lys Thr Ser Pro Arg Arg Pro Leu Ile Leu Lys Arg Arg Arg Leu
1               5                   10                  15

Pro Leu Pro Val Gln Asn Ala Pro Ser Glu Thr Ser Glu Glu Glu Pro
            20                  25                  30

Lys Arg Ser Pro Ala Gln Gln Glu Ser Asn Gln Ala Glu Ala Ser Lys
        35                  40                  45

Glu Val Ala Glu Ser Asn Ser Cys Lys Phe Pro Ala Gly Ile Lys Ile
    50                  55                  60

Ile Asn His Pro Thr Met Pro Asn Thr Gln Val Val Ala Ile Pro Asn
65                  70                  75                  80

Asn Ala Asn Ile His Ser Ile Ile Thr Ala Leu Thr Ala Lys Gly Lys
                85                  90                  95
```

Glu Ser Gly Ser Ser Gly Pro Asn Lys Phe Ile Leu Ile Ser Cys Gly
100 105 110

Gly Ala Pro Thr Gln Pro Pro Gly Leu Arg Pro Gln Thr Gln Thr Ser
115 120 125

Tyr Asp Ala Lys Arg Thr Glu Val Thr Leu Glu Thr Leu Gly Pro Lys
130 135 140

Pro Ala Ala Arg Asp Val Asn Leu Pro Arg Pro Pro Gly Ala Leu Cys
145 150 155 160

Glu Gln Lys Arg Glu Thr Cys Asp Gly Glu Ala Ala Gly Cys Thr Ile
165 170 175

Asn Asn Ser Leu Ser Asn Ile Gln Trp Leu Arg Lys Met Ser Ser Asp
180 185 190

Gly Leu Gly Ser Arg Ser Ile Lys Gln Glu Met Glu Glu Lys Glu Asn
195 200 205

Cys His Leu Glu Gln Arg Gln Val Lys Val Glu Glu Pro Ser Arg Pro
210 215 220

Ser Ala Ser Trp Gln Asn Ser Val Ser Glu Arg Pro Pro Tyr Ser Tyr
225 230 235 240

Met Ala Met Ile Gln Phe Ala Ile Asn Ser Thr Glu Arg Lys Arg Met
245 250 255

Thr Leu Lys Asp Ile Tyr Thr Trp Ile Glu Asp His Phe Pro Tyr Phe
260 265 270

Lys His Ile Ala Lys Pro Gly Trp Lys Asn Ser Ile Arg His Asn Leu
275 280 285

Ser Leu His Asp Met Phe Val Arg Glu Thr Ser Ala Asn Gly Lys Val
290 295 300

Ser Phe Trp Thr Ile His Pro Ser Ala Asn Arg Tyr Leu Thr Leu Asp
305 310 315 320

Gln Val Phe Lys Gln Gln Gln Lys Arg Pro Asn Pro Glu Leu Arg Arg
325 330 335

Asn Met Thr Ile Lys Thr Glu Leu Pro Leu Gly Ala Arg Arg Lys Met

340 345 350

Lys Pro Leu Leu Pro Arg Val Ser Ser Tyr Leu Val Pro Ile Gln Phe
355 360 365

Pro Val Asn Gln Ser Leu Val Leu Gln Pro Ser Val Lys Val Pro Leu
370 375 380

Pro Leu Ala Ala Ser Leu Met Ser Ser Glu Leu Ala Arg His Ser Lys
385 390 395 400

Arg Val Arg Ile Ala Pro Lys Val Leu Leu Ala Glu Glu Gly Ile Ala
405 410 415

Pro Leu Ser Ser Ala Gly Pro Gly Lys Glu Glu Lys Leu Leu Phe Gly
420 425 430

Glu Gly Phe Ser Pro Leu Leu Pro Val Gln Thr Ile Lys Glu Glu Glu
435 440 445

Ile Gln Pro Gly Glu Glu Met Pro His Leu Ala Arg Pro Ile Lys Val
450 455 460

Glu Ser Pro Pro Leu Glu Glu Trp Pro Ser Pro Ala Pro Ser Phe Lys
465 470 475 480

Glu Glu Ser Ser His Ser Trp Glu Asp Ser Ser Gln Ser Pro Thr Pro
485 490 495

Arg Pro Lys Lys Ser Tyr Ser Gly Leu Arg Ser Pro Thr Arg Cys Val
500 505 510

Ser Glu Met Leu Val Ile Gln His Arg Glu Arg Arg Glu Arg Ser Arg
515 520 525

Ser Arg Arg Lys Gln His Leu Leu Pro Pro Cys Val Asp Glu Pro Glu
530 535 540

Leu Leu Phe Ser Glu Gly Pro Ser Thr Ser Arg Trp Ala Ala Glu Leu
545 550 555 560

Pro Phe Pro Ala Asp Ser Ser Asp Pro Ala Ser Gln Leu Ser Tyr Ser
565 570 575

Gln Glu Val Gly Gly Pro Phe Lys Thr Pro Ile Lys Glu Thr Leu Pro
580 585 590

```
Ile Ser Ser Thr Pro Ser Lys Ser Val Leu Pro Arg Thr Pro Glu Ser
        595                 600                 605

Trp Arg Leu Thr Pro Pro Ala Lys Val Gly Gly Leu Asp Phe Ser Pro
    610                 615                 620

Val Gln Thr Ser Gln Gly Ala Ser Asp Pro Leu Pro Asp Pro Leu Gly
625                 630                 635                 640

Leu Met Asp Leu Ser Thr Thr Pro Leu Gln Ser Ala Pro Pro Leu Glu
                645                 650                 655

Ser Pro Gln Arg Leu Leu Ser Ser Glu Pro Leu Asp Leu Ile Ser Val
            660                 665                 670

Pro Phe Gly Asn Ser Ser Pro Ser Asp Ile Asp Val Pro Lys Pro Gly
        675                 680                 685

Ser Pro Glu Pro Gln Val Ser Gly Leu Ala Ala Asn Arg Ser Leu Thr
    690                 695                 700

Glu Gly Leu Val Leu Asp Thr Met Asn Asp Ser Leu Ser Lys Ile Leu
705                 710                 715                 720

Leu Asp Ile Ser Phe Pro Gly Leu Asp Glu Asp Pro Leu Gly Pro Asp
                725                 730                 735

Asn Ile Asn Trp Ser Gln Phe Ile Pro Glu Leu Gln
            740                 745


<210>  3
<211>  763
<212>  PRT
<213>  Homo sapiens

<400>  3

Met Lys Thr Ser Pro Arg Arg Pro Leu Ile Leu Lys Arg Arg Arg Leu
1               5                   10                  15

Pro Leu Pro Val Gln Asn Ala Pro Ser Glu Thr Ser Glu Glu Glu Pro
            20                  25                  30

Lys Arg Ser Pro Ala Gln Gln Glu Ser Asn Gln Ala Glu Ala Ser Lys
        35                  40                  45

Glu Val Ala Glu Ser Asn Ser Cys Lys Phe Pro Ala Gly Ile Lys Ile
    50                  55                  60
```

```
Ile Asn His Pro Thr Met Pro Asn Thr Gln Val Val Ala Ile Pro Asn
65                  70                  75                  80

Asn Ala Asn Ile His Ser Ile Ile Thr Ala Leu Thr Ala Lys Gly Lys
                85                  90                  95

Glu Ser Gly Ser Ser Gly Pro Asn Lys Phe Ile Leu Ile Ser Cys Gly
            100                 105                 110

Gly Ala Pro Thr Gln Pro Pro Gly Leu Arg Pro Gln Thr Gln Thr Ser
        115                 120                 125

Tyr Asp Ala Lys Arg Thr Glu Val Thr Leu Glu Thr Leu Gly Pro Lys
    130                 135                 140

Pro Ala Ala Arg Asp Val Asn Leu Pro Arg Pro Pro Gly Ala Leu Cys
145                 150                 155                 160

Glu Gln Lys Arg Glu Thr Cys Ala Asp Gly Glu Ala Ala Gly Cys Thr
                165                 170                 175

Ile Asn Asn Ser Leu Ser Asn Ile Gln Trp Leu Arg Lys Met Ser Ser
            180                 185                 190

Asp Gly Leu Gly Ser Arg Ser Ile Lys Gln Glu Met Glu Glu Lys Glu
        195                 200                 205

Asn Cys His Leu Glu Gln Arg Gln Val Lys Val Glu Glu Pro Ser Arg
    210                 215                 220

Pro Ser Ala Ser Trp Gln Asn Ser Val Ser Glu Arg Pro Pro Tyr Ser
225                 230                 235                 240

Tyr Met Ala Met Ile Gln Phe Ala Ile Asn Ser Thr Glu Arg Lys Arg
                245                 250                 255

Met Thr Leu Lys Asp Ile Tyr Thr Trp Ile Glu Asp His Phe Pro Tyr
            260                 265                 270

Phe Lys His Ile Ala Lys Pro Gly Trp Lys Asn Ser Ile Arg His Asn
        275                 280                 285

Leu Ser Leu His Asp Met Phe Val Arg Glu Thr Ser Ala Asn Gly Lys
    290                 295                 300

Val Ser Phe Trp Thr Ile His Pro Ser Ala Asn Arg Tyr Leu Thr Leu
305                 310                 315                 320
```

Asp Gln Val Phe Lys Pro Leu Asp Pro Gly Ser Pro Gln Leu Pro Glu
325 330 335

His Leu Glu Ser Gln Gln Lys Arg Pro Asn Pro Glu Leu Arg Arg Asn
340 345 350

Met Thr Ile Lys Thr Glu Leu Pro Leu Gly Ala Arg Arg Lys Met Lys
355 360 365

Pro Leu Leu Pro Arg Val Ser Ser Tyr Leu Val Pro Ile Gln Phe Pro
370 375 380

Val Asn Gln Ser Leu Val Leu Gln Pro Ser Val Lys Val Pro Leu Pro
385 390 395 400

Leu Ala Ala Ser Leu Met Ser Ser Glu Leu Ala Arg His Ser Lys Arg
405 410 415

Val Arg Ile Ala Pro Lys Val Leu Leu Ala Glu Glu Gly Ile Ala Pro
420 425 430

Leu Ser Ser Ala Gly Pro Gly Lys Glu Glu Lys Leu Leu Phe Gly Glu
435 440 445

Gly Phe Ser Pro Leu Leu Pro Val Gln Thr Ile Lys Glu Glu Glu Ile
450 455 460

Gln Pro Gly Glu Glu Met Pro His Leu Ala Arg Pro Ile Lys Val Glu
465 470 475 480

Ser Pro Pro Leu Glu Glu Trp Pro Ser Pro Ala Pro Ser Phe Lys Glu
485 490 495

Glu Ser Ser His Ser Trp Glu Asp Ser Ser Gln Ser Pro Thr Pro Arg
500 505 510

Pro Lys Lys Ser Tyr Ser Gly Leu Arg Ser Pro Thr Arg Cys Val Ser
515 520 525

Glu Met Leu Val Ile Gln His Arg Glu Arg Arg Glu Arg Ser Arg Ser
530 535 540

Arg Arg Lys Gln His Leu Leu Pro Pro Cys Val Asp Glu Pro Glu Leu
545 550 555 560

Leu Phe Ser Glu Gly Pro Ser Thr Ser Arg Trp Ala Ala Glu Leu Pro
565 570 575

```
Phe Pro Ala Asp Ser Ser Asp Pro Ala Ser Gln Leu Ser Tyr Ser Gln
            580                 585                 590

Glu Val Gly Gly Pro Phe Lys Thr Pro Ile Lys Glu Thr Leu Pro Ile
        595                 600                 605

Ser Ser Thr Pro Ser Lys Ser Val Leu Pro Arg Thr Pro Glu Ser Trp
    610                 615                 620

Arg Leu Thr Pro Pro Ala Lys Val Gly Gly Leu Asp Phe Ser Pro Val
625                 630                 635                 640

Gln Thr Ser Gln Gly Ala Ser Asp Pro Leu Pro Asp Pro Leu Gly Leu
                645                 650                 655

Met Asp Leu Ser Thr Thr Pro Leu Gln Ser Ala Pro Pro Leu Glu Ser
            660                 665                 670

Pro Gln Arg Leu Leu Ser Ser Glu Pro Leu Asp Leu Ile Ser Val Pro
        675                 680                 685

Phe Gly Asn Ser Ser Pro Ser Asp Ile Asp Val Pro Lys Pro Gly Ser
    690                 695                 700

Pro Glu Pro Gln Val Ser Gly Leu Ala Ala Asn Arg Ser Leu Thr Glu
705                 710                 715                 720

Gly Leu Val Leu Asp Thr Met Asn Asp Ser Leu Ser Lys Ile Leu Leu
                725                 730                 735

Asp Ile Ser Phe Pro Gly Leu Asp Glu Asp Pro Leu Gly Pro Asp Asn
            740                 745                 750

Ile Asn Trp Ser Gln Phe Ile Pro Glu Leu Gln
        755                 760


<210>  4
<211>  3665
<212>  DNA
<213>  Homo sapiens


<220>
<221>  CDS
<222>  (284)..(2689)

<400>  4
tttcaaacag cggaacaaac tgaaagctcc ggtgccagac cccacccccg gccccggccc        60
```

```
gggaccccct cccctcccgg gatcccccgg ggttcccacc ccgcccgcac cgccggggac      120

ccggccggtc cggcgcgagc cccgtccgg ggccctggct cggcccccag gttggaggag       180

cccggagccc gccttcggag ctacggccta acggcggcgg cgactgcagt ctggagggtc      240

cacacttgtg attctcaatg gagagtgaaa acgcagattc ata atg aaa act agc        295
                                                Met Lys Thr Ser
                                                1

ccc cgt cgg cca ctg att ctc aaa aga cgg agg ctg ccc ctt cct gtt       343
Pro Arg Arg Pro Leu Ile Leu Lys Arg Arg Arg Leu Pro Leu Pro Val
5                   10                  15                  20

caa aat gcc cca agt gaa aca tca gag gag gaa cct aag aga tcc cct       391
Gln Asn Ala Pro Ser Glu Thr Ser Glu Glu Glu Pro Lys Arg Ser Pro
                25                  30                  35

gcc caa cag gag tct aat caa gca gag gcc tcc aag gaa gtg gca gag       439
Ala Gln Gln Glu Ser Asn Gln Ala Glu Ala Ser Lys Glu Val Ala Glu
            40                  45                  50

tcc aac tct tgc aag ttt cca gct ggg atc aag att att aac cac ccc       487
Ser Asn Ser Cys Lys Phe Pro Ala Gly Ile Lys Ile Ile Asn His Pro
        55                  60                  65

acc atg ccc aac acg caa gta gtg gcc atc ccc aac aat gct aat att       535
Thr Met Pro Asn Thr Gln Val Val Ala Ile Pro Asn Asn Ala Asn Ile
    70                  75                  80

cac agc atc atc aca gca ctg act gcc aag gga aaa gag agt ggc agt       583
His Ser Ile Ile Thr Ala Leu Thr Ala Lys Gly Lys Glu Ser Gly Ser
85                  90                  95                  100

agt ggg ccc aac aaa ttc atc ctc atc agc tgt ggg gga gcc cca act       631
Ser Gly Pro Asn Lys Phe Ile Leu Ile Ser Cys Gly Gly Ala Pro Thr
                105                 110                 115

cag cct cca gga ctc cgg cct caa acc caa acc agc tat gat gcc aaa       679
Gln Pro Pro Gly Leu Arg Pro Gln Thr Gln Thr Ser Tyr Asp Ala Lys
            120                 125                 130

agg aca gaa gtg acc ctg gag acc ttg gga cca aaa cct gca gct agg       727
Arg Thr Glu Val Thr Leu Glu Thr Leu Gly Pro Lys Pro Ala Ala Arg
        135                 140                 145

gat gtg aat ctt cct aga cca cct gga gcc ctt tgc gag cag aaa cgg       775
Asp Val Asn Leu Pro Arg Pro Pro Gly Ala Leu Cys Glu Gln Lys Arg
    150                 155                 160

gag acc tgt gca gat ggt gag gca gca ggc tgc act atc aac aat agc       823
Glu Thr Cys Ala Asp Gly Glu Ala Ala Gly Cys Thr Ile Asn Asn Ser
165                 170                 175                 180

cta tcc aac atc cag tgg ctt cga aag atg agt tct gat gga ctg ggc       871
Leu Ser Asn Ile Gln Trp Leu Arg Lys Met Ser Ser Asp Gly Leu Gly
                185                 190                 195

tcc cgc agc atc aag caa gag atg gag gaa aag gag aat tgt cac ctg       919
Ser Arg Ser Ile Lys Gln Glu Met Glu Glu Lys Glu Asn Cys His Leu
            200                 205                 210

gag cag cga cag gtt aag gtt gag gag cct tcg aga cca tca gcg tcc       967
```

```
Glu Gln Arg Gln Val Lys Val Glu Glu Pro Ser Arg Pro Ser Ala Ser
        215                 220                 225

tgg cag aac tct gtg tct gag cgg cca ccc tac tct tac atg gcc atg        1015
Trp Gln Asn Ser Val Ser Glu Arg Pro Pro Tyr Ser Tyr Met Ala Met
    230                 235                 240

ata caa ttc gcc atc aac agc act gag agg aag cgc atg act ttg aaa        1063
Ile Gln Phe Ala Ile Asn Ser Thr Glu Arg Lys Arg Met Thr Leu Lys
245                 250                 255                 260

gac atc tat acg tgg att gag gac cac ttt ccc tac ttt aag cac att        1111
Asp Ile Tyr Thr Trp Ile Glu Asp His Phe Pro Tyr Phe Lys His Ile
                265                 270                 275

gcc aag cca ggc tgg aag aac tcc atc cgc cac aac ctt tcc ctg cac        1159
Ala Lys Pro Gly Trp Lys Asn Ser Ile Arg His Asn Leu Ser Leu His
            280                 285                 290

gac atg ttt gtc cgg gag acg tct gcc aat ggc aag gtc tcc ttc tgg        1207
Asp Met Phe Val Arg Glu Thr Ser Ala Asn Gly Lys Val Ser Phe Trp
        295                 300                 305

acc att cac ccc agt gcc aac cgc tac ttg aca ttg gac cag gtg ttt        1255
Thr Ile His Pro Ser Ala Asn Arg Tyr Leu Thr Leu Asp Gln Val Phe
    310                 315                 320

aag cca ctg gac cca ggg tct cca caa ttg ccc gag cac ttg gaa tca        1303
Lys Pro Leu Asp Pro Gly Ser Pro Gln Leu Pro Glu His Leu Glu Ser
325                 330                 335                 340

cag cag aaa cga ccg aat cca gag ctc cgc cgg aac atg acc atc aaa        1351
Gln Gln Lys Arg Pro Asn Pro Glu Leu Arg Arg Asn Met Thr Ile Lys
                345                 350                 355

acc gaa ctc ccc ctg ggc gca cgg cgg aag atg aag cca ctg cta cca        1399
Thr Glu Leu Pro Leu Gly Ala Arg Arg Lys Met Lys Pro Leu Leu Pro
            360                 365                 370

cgg gtc agc tca tac ctg gta cct atc cag ttc ccg gtg aac cag tca        1447
Arg Val Ser Ser Tyr Leu Val Pro Ile Gln Phe Pro Val Asn Gln Ser
        375                 380                 385

ctg gtg ttg cag ccc tcg gtg aag gtg cca ttg ccc ctg gcg gct tcc        1495
Leu Val Leu Gln Pro Ser Val Lys Val Pro Leu Pro Leu Ala Ala Ser
    390                 395                 400

ctc atg agc tca gag ctt gcc cgc cat agc aag cga gtc cgc att gcc        1543
Leu Met Ser Ser Glu Leu Ala Arg His Ser Lys Arg Val Arg Ile Ala
405                 410                 415                 420

ccc aag gtt ttt ggg gaa cag gtg gtg ttt ggt tac atg agt aag ttc        1591
Pro Lys Val Phe Gly Glu Gln Val Val Phe Gly Tyr Met Ser Lys Phe
                425                 430                 435

ttt agt ggc gat ctg cga gat ttt ggt aca ccc atc acc agc ttg ttt        1639
Phe Ser Gly Asp Leu Arg Asp Phe Gly Thr Pro Ile Thr Ser Leu Phe
            440                 445                 450

aat ttt atc ttt ctt tgt tta tca gtg ctg cta gct gag gag ggg ata        1687
Asn Phe Ile Phe Leu Cys Leu Ser Val Leu Leu Ala Glu Glu Gly Ile
        455                 460                 465
```

```
gct cct ctt tct tct gca gga cca ggg aaa gag gag aaa ctc ctg ttt      1735
Ala Pro Leu Ser Ser Ala Gly Pro Gly Lys Glu Glu Lys Leu Leu Phe
    470                 475                 480

gga gaa ggg ttt tct cct ttg ctt cca gtt cag act atc aag gag gaa      1783
Gly Glu Gly Phe Ser Pro Leu Leu Pro Val Gln Thr Ile Lys Glu Glu
485                 490                 495                 500

gaa atc cag cct ggg gag gaa atg cca cac tta gcg aga ccc atc aaa      1831
Glu Ile Gln Pro Gly Glu Glu Met Pro His Leu Ala Arg Pro Ile Lys
                505                 510                 515

gtg gag agc cct ccc ttg gaa gag tgg ccc tcc ccg gcc cca tct ttc      1879
Val Glu Ser Pro Pro Leu Glu Glu Trp Pro Ser Pro Ala Pro Ser Phe
            520                 525                 530

aaa gag gaa tca tct cac tcc tgg gag gat tcg tcc caa tct ccc acc      1927
Lys Glu Glu Ser Ser His Ser Trp Glu Asp Ser Ser Gln Ser Pro Thr
        535                 540                 545

cca aga ccc aag aag tcc tac agt ggg ctt agg tcc cca acc cgg tgt      1975
Pro Arg Pro Lys Lys Ser Tyr Ser Gly Leu Arg Ser Pro Thr Arg Cys
    550                 555                 560

gtc tcg gaa atg ctt gtg att caa cac agg gag agg agg gag agg agc      2023
Val Ser Glu Met Leu Val Ile Gln His Arg Glu Arg Arg Glu Arg Ser
565                 570                 575                 580

cgg tct cgg agg aaa cag cat cta ctg cct ccc tgt gtg gat gag ccg      2071
Arg Ser Arg Arg Lys Gln His Leu Leu Pro Pro Cys Val Asp Glu Pro
                585                 590                 595

gag ctg ctc ttc tca gag ggg ccc agt act tcc cgc tgg gcc gca gag      2119
Glu Leu Leu Phe Ser Glu Gly Pro Ser Thr Ser Arg Trp Ala Ala Glu
            600                 605                 610

ctc ccg ttc cca gca gac tcc tct gac cct gcc tcc cag ctc agc tac      2167
Leu Pro Phe Pro Ala Asp Ser Ser Asp Pro Ala Ser Gln Leu Ser Tyr
        615                 620                 625

tcc cag gaa gtg gga gga cct ttt aag aca ccc att aag gaa acg ctg      2215
Ser Gln Glu Val Gly Gly Pro Phe Lys Thr Pro Ile Lys Glu Thr Leu
    630                 635                 640

ccc atc tcc tcc acc ccg agc aaa tct gtc ctc ccc aga acc cct gaa      2263
Pro Ile Ser Ser Thr Pro Ser Lys Ser Val Leu Pro Arg Thr Pro Glu
645                 650                 655                 660

tcc tgg agg ctc acg ccc cca gcc aaa gta ggg gga ctg gat ttc agc      2311
Ser Trp Arg Leu Thr Pro Pro Ala Lys Val Gly Gly Leu Asp Phe Ser
                665                 670                 675

cca gta caa acc tcc cag ggt gcc tct gac ccc ttg cct gac ccc ctg      2359
Pro Val Gln Thr Ser Gln Gly Ala Ser Asp Pro Leu Pro Asp Pro Leu
            680                 685                 690

ggg ctg atg gat ctc agc acc act ccc ttg caa agt gct ccc ccc ctt      2407
Gly Leu Met Asp Leu Ser Thr Thr Pro Leu Gln Ser Ala Pro Pro Leu
        695                 700                 705

gaa tca ccg caa agg ctc ctc agt tca gaa ccc tta gac ctc atc tcc      2455
Glu Ser Pro Gln Arg Leu Leu Ser Ser Glu Pro Leu Asp Leu Ile Ser
    710                 715                 720
```

gtc ccc ttt ggc aac tct tct ccc tca gat ata gac gtc ccc aag cca 2503
Val Pro Phe Gly Asn Ser Ser Pro Ser Asp Ile Asp Val Pro Lys Pro
725 730 735 740

ggc tcc ccg gag cca cag gtt tct ggc ctt gca gcc aat cgt tct ctg 2551
Gly Ser Pro Glu Pro Gln Val Ser Gly Leu Ala Ala Asn Arg Ser Leu
745 750 755

aca gaa ggc ctg gtc ctg gac aca atg aat gac agc ctc agc aag atc 2599
Thr Glu Gly Leu Val Leu Asp Thr Met Asn Asp Ser Leu Ser Lys Ile
760 765 770

ctg ctg gac atc agc ttt cct ggc ctg gac gag gac cca ctg ggc cct 2647
Leu Leu Asp Ile Ser Phe Pro Gly Leu Asp Glu Asp Pro Leu Gly Pro
775 780 785

gac aac atc aac tgg tcc cag ttt att cct gag cta cag tag 2689
Asp Asn Ile Asn Trp Ser Gln Phe Ile Pro Glu Leu Gln
790 795 800

agccctgccc ttgcccctgt gctcaagctg tccaccatcc cgggcactcc aaggctcagt 2749

gcaccccaag cctctgagtg aggacagcag gcagggactg ttctgctcct catagctccc 2809

tgctgcctga ttatgcaaaa gtagcagtca caccctagcc actgctggga ccttgtgttc 2869

cccaagagta tctgattcct ctgctgtccc tgccaggagc tgaagggtgg gaacaacaaa 2929

ggcaatggtg aaaagagatt aggaaccccc cagcctgttt ccattctctg cccagcagtc 2989

tcttaccttc cctgatcttt gcagggtggt ccgtgtaaat agtataaatt ctccaaatta 3049

tcctctaatt ataaatgtaa gcttatttcc ttagatcatt atccagagac tgccagaagg 3109

tgggtaggat gacctggggt ttcaattgac ttctgttcct tgcttttagt tttgatagaa 3169

gggaagacct gcagtgcacg gtttcttcca ggctgaggta cctggatctt gggttcttca 3229

ctgcagggac ccagacaagt ggatctgctt gccagagtcc tttttgcccc tccctgccac 3289

ctccccgtgt ttccaagtca gctttcctgc aagaagaaat cctggttaaa aaagtctttt 3349

gtattgggtc aggagttgaa tttggggtgg gaggatggat gcaactgaag cagagtgtgg 3409

gtgcccagat gtgcgctatt agatgtttct ctgataatgt ccccaatcat accagggaga 3469

ctggcattga cgagaactca ggtggaggct tgagaaggcc gaaagggccc ctgacctgcc 3529

tggcttcctt agcttgcccc tcagctttgc aaagagccac cctaggcccc agctgaccgc 3589

atgggtgtga gccagcttga gaacactaac tactcaataa aagcgaaggt ggacatgaaa 3649

aaaaaaaaaa aaaaaa 3665

<210> 5
<211> 801
<212> PRT
<213> Homo sapiens

<400> 5

```
Met Lys Thr Ser Pro Arg Arg Pro Leu Ile Leu Lys Arg Arg Arg Leu
1               5                   10                  15

Pro Leu Pro Val Gln Asn Ala Pro Ser Glu Thr Ser Glu Glu Glu Pro
            20                  25                  30

Lys Arg Ser Pro Ala Gln Gln Glu Ser Asn Gln Ala Glu Ala Ser Lys
        35                  40                  45

Glu Val Ala Glu Ser Asn Ser Cys Lys Phe Pro Ala Gly Ile Lys Ile
    50                  55                  60

Ile Asn His Pro Thr Met Pro Asn Thr Gln Val Val Ala Ile Pro Asn
65                  70                  75                  80

Asn Ala Asn Ile His Ser Ile Ile Thr Ala Leu Thr Ala Lys Gly Lys
                85                  90                  95

Glu Ser Gly Ser Ser Gly Pro Asn Lys Phe Ile Leu Ile Ser Cys Gly
            100                 105                 110

Gly Ala Pro Thr Gln Pro Pro Gly Leu Arg Pro Gln Thr Gln Thr Ser
        115                 120                 125

Tyr Asp Ala Lys Arg Thr Glu Val Thr Leu Glu Thr Leu Gly Pro Lys
    130                 135                 140

Pro Ala Ala Arg Asp Val Asn Leu Pro Arg Pro Pro Gly Ala Leu Cys
145                 150                 155                 160

Glu Gln Lys Arg Glu Thr Cys Ala Asp Gly Glu Ala Ala Gly Cys Thr
                165                 170                 175

Ile Asn Asn Ser Leu Ser Asn Ile Gln Trp Leu Arg Lys Met Ser Ser
            180                 185                 190

Asp Gly Leu Gly Ser Arg Ser Ile Lys Gln Glu Met Glu Glu Lys Glu
        195                 200                 205

Asn Cys His Leu Glu Gln Arg Gln Val Lys Val Glu Glu Pro Ser Arg
    210                 215                 220

Pro Ser Ala Ser Trp Gln Asn Ser Val Ser Glu Arg Pro Pro Tyr Ser
225                 230                 235                 240

Tyr Met Ala Met Ile Gln Phe Ala Ile Asn Ser Thr Glu Arg Lys Arg
                245                 250                 255
```

Met Thr Leu Lys Asp Ile Tyr Thr Trp Ile Glu Asp His Phe Pro Tyr
260 265 270

Phe Lys His Ile Ala Lys Pro Gly Trp Lys Asn Ser Ile Arg His Asn
275 280 285

Leu Ser Leu His Asp Met Phe Val Arg Glu Thr Ser Ala Asn Gly Lys
290 295 300

Val Ser Phe Trp Thr Ile His Pro Ser Ala Asn Arg Tyr Leu Thr Leu
305 310 315 320

Asp Gln Val Phe Lys Pro Leu Asp Pro Gly Ser Pro Gln Leu Pro Glu
325 330 335

His Leu Glu Ser Gln Gln Lys Arg Pro Asn Pro Glu Leu Arg Arg Asn
340 345 350

Met Thr Ile Lys Thr Glu Leu Pro Leu Gly Ala Arg Arg Lys Met Lys
355 360 365

Pro Leu Leu Pro Arg Val Ser Ser Tyr Leu Val Pro Ile Gln Phe Pro
370 375 380

Val Asn Gln Ser Leu Val Leu Gln Pro Ser Val Lys Val Pro Leu Pro
385 390 395 400

Leu Ala Ala Ser Leu Met Ser Ser Glu Leu Ala Arg His Ser Lys Arg
405 410 415

Val Arg Ile Ala Pro Lys Val Phe Gly Glu Gln Val Val Phe Gly Tyr
420 425 430

Met Ser Lys Phe Phe Ser Gly Asp Leu Arg Asp Phe Gly Thr Pro Ile
435 440 445

Thr Ser Leu Phe Asn Phe Ile Phe Leu Cys Leu Ser Val Leu Leu Ala
450 455 460

Glu Glu Gly Ile Ala Pro Leu Ser Ser Ala Gly Pro Gly Lys Glu Glu
465 470 475 480

Lys Leu Leu Phe Gly Glu Gly Phe Ser Pro Leu Leu Pro Val Gln Thr
485 490 495

Ile Lys Glu Glu Glu Ile Gln Pro Gly Glu Glu Met Pro His Leu Ala
500 505 510

```
Arg Pro Ile Lys Val Glu Ser Pro Pro Leu Glu Glu Trp Pro Ser Pro
        515                 520                 525

Ala Pro Ser Phe Lys Glu Glu Ser Ser His Ser Trp Glu Asp Ser Ser
    530                 535                 540

Gln Ser Pro Thr Pro Arg Pro Lys Lys Ser Tyr Ser Gly Leu Arg Ser
545                 550                 555                 560

Pro Thr Arg Cys Val Ser Glu Met Leu Val Ile Gln His Arg Glu Arg
                565                 570                 575

Arg Glu Arg Ser Arg Ser Arg Arg Lys Gln His Leu Leu Pro Pro Cys
            580                 585                 590

Val Asp Glu Pro Glu Leu Leu Phe Ser Glu Gly Pro Ser Thr Ser Arg
        595                 600                 605

Trp Ala Ala Glu Leu Pro Phe Pro Ala Asp Ser Ser Asp Pro Ala Ser
    610                 615                 620

Gln Leu Ser Tyr Ser Gln Glu Val Gly Gly Pro Phe Lys Thr Pro Ile
625                 630                 635                 640

Lys Glu Thr Leu Pro Ile Ser Ser Thr Pro Ser Lys Ser Val Leu Pro
                645                 650                 655

Arg Thr Pro Glu Ser Trp Arg Leu Thr Pro Pro Ala Lys Val Gly Gly
            660                 665                 670

Leu Asp Phe Ser Pro Val Gln Thr Ser Gln Gly Ala Ser Asp Pro Leu
        675                 680                 685

Pro Asp Pro Leu Gly Leu Met Asp Leu Ser Thr Thr Pro Leu Gln Ser
    690                 695                 700

Ala Pro Pro Leu Glu Ser Pro Gln Arg Leu Leu Ser Ser Glu Pro Leu
705                 710                 715                 720

Asp Leu Ile Ser Val Pro Phe Gly Asn Ser Ser Pro Ser Asp Ile Asp
                725                 730                 735

Val Pro Lys Pro Gly Ser Pro Glu Pro Gln Val Ser Gly Leu Ala Ala
            740                 745                 750

Asn Arg Ser Leu Thr Glu Gly Leu Val Leu Asp Thr Met Asn Asp Ser
```

```
        755                 760                 765

Leu Ser Lys Ile Leu Leu Asp Ile Ser Phe Pro Gly Leu Asp Glu Asp
    770                 775                 780

Pro Leu Gly Pro Asp Asn Ile Asn Trp Ser Gln Phe Ile Pro Glu Leu
785                 790                 795                 800

Gln


<210>  6
<211>  3506
<212>  DNA
<213>  Homo sapiens


<220>
<221>  CDS
<222>  (284)..(2530)

<400>  6
tttcaaacag cggaacaaac tgaaagctcc ggtgccagac cccacccccg gccccggccc      60

gggacccccct cccctcccgg gatcccccgg ggttcccacc ccgcccgcac cgccggggac     120

ccggccggtc cggcgcgagc ccccgtccgg ggccctggct cggcccccag gttggaggag     180

cccggagccc gccttcggag ctacggccta acggcggcgg cgactgcagt ctggagggtc     240

cacacttgtg attctcaatg gagagtgaaa acgcagattc ata atg aaa act agc       295
                                                Met Lys Thr Ser
                                                1

ccc cgt cgg cca ctg att ctc aaa aga cgg agg ctg ccc ctt cct gtt       343
Pro Arg Arg Pro Leu Ile Leu Lys Arg Arg Arg Leu Pro Leu Pro Val
5                   10                  15                  20

caa aat gcc cca agt gaa aca tca gag gag gaa cct aag aga tcc cct       391
Gln Asn Ala Pro Ser Glu Thr Ser Glu Glu Glu Pro Lys Arg Ser Pro
                25                  30                  35

gcc caa cag gag tct aat caa gca gag gcc tcc aag gaa gtg gca gag       439
Ala Gln Gln Glu Ser Asn Gln Ala Glu Ala Ser Lys Glu Val Ala Glu
            40                  45                  50

tcc aac tct tgc aag ttt cca gct ggg atc aag att att aac cac ccc       487
Ser Asn Ser Cys Lys Phe Pro Ala Gly Ile Lys Ile Ile Asn His Pro
        55                  60                  65

acc atg ccc aac acg caa gta gtg gcc atc ccc aac aat gct aat att       535
Thr Met Pro Asn Thr Gln Val Val Ala Ile Pro Asn Asn Ala Asn Ile
    70                  75                  80

cac agc atc atc aca gca ctg act gcc aag gga aaa gag agt ggc agt       583
His Ser Ile Ile Thr Ala Leu Thr Ala Lys Gly Lys Glu Ser Gly Ser
85                  90                  95                  100

agt ggg ccc aac aaa ttc atc ctc atc agc tgt ggg gga gcc cca act       631
```

```
Ser Gly Pro Asn Lys Phe Ile Leu Ile Ser Cys Gly Gly Ala Pro Thr
                105                 110                 115

cag cct cca gga ctc cgg cct caa acc caa acc agc tat gat gcc aaa        679
Gln Pro Pro Gly Leu Arg Pro Gln Thr Gln Thr Ser Tyr Asp Ala Lys
            120                 125                 130

agg aca gaa gtg acc ctg gag acc ttg gga cca aaa cct gca gct agg        727
Arg Thr Glu Val Thr Leu Glu Thr Leu Gly Pro Lys Pro Ala Ala Arg
        135                 140                 145

gat gtg aat ctt cct aga cca cct gga gcc ctt tgc gag cag aaa cgg        775
Asp Val Asn Leu Pro Arg Pro Pro Gly Ala Leu Cys Glu Gln Lys Arg
    150                 155                 160

gag acc tgt gca gat ggt gag gca gca ggc tgc act atc aac aat agc        823
Glu Thr Cys Ala Asp Gly Glu Ala Ala Gly Cys Thr Ile Asn Asn Ser
165                 170                 175                 180

cta tcc aac atc cag tgg ctt cga aag atg agt tct gat gga ctg ggc        871
Leu Ser Asn Ile Gln Trp Leu Arg Lys Met Ser Ser Asp Gly Leu Gly
                185                 190                 195

tcc cgc agc atc aag caa gag atg gag gaa aag gag aat tgt cac ctg        919
Ser Arg Ser Ile Lys Gln Glu Met Glu Glu Lys Glu Asn Cys His Leu
            200                 205                 210

gag cag cga cag gtt aag gtt gag gag cct tcg aga cca tca gcg tcc        967
Glu Gln Arg Gln Val Lys Val Glu Glu Pro Ser Arg Pro Ser Ala Ser
        215                 220                 225

tgg cag aac tct gtg tct gag cgg cca ccc tac tct tac atg gcc atg       1015
Trp Gln Asn Ser Val Ser Glu Arg Pro Pro Tyr Ser Tyr Met Ala Met
    230                 235                 240

ata caa ttc gcc atc aac agc act gag agg aag cgc atg act ttg aaa       1063
Ile Gln Phe Ala Ile Asn Ser Thr Glu Arg Lys Arg Met Thr Leu Lys
245                 250                 255                 260

gac atc tat acg tgg att gag gac cac ttt ccc tac ttt aag cac att       1111
Asp Ile Tyr Thr Trp Ile Glu Asp His Phe Pro Tyr Phe Lys His Ile
                265                 270                 275

gcc aag cca ggc tgg aag aac tcc atc cgc cac aac ctt tcc ctg cac       1159
Ala Lys Pro Gly Trp Lys Asn Ser Ile Arg His Asn Leu Ser Leu His
            280                 285                 290

gac atg ttt gtc cgg gag acg tct gcc aat ggc aag gtc tcc ttc tgg       1207
Asp Met Phe Val Arg Glu Thr Ser Ala Asn Gly Lys Val Ser Phe Trp
        295                 300                 305

acc att cac ccc agt gcc aac cgc tac ttg aca ttg gac cag gtg ttt       1255
Thr Ile His Pro Ser Ala Asn Arg Tyr Leu Thr Leu Asp Gln Val Phe
    310                 315                 320

aag cag cag aaa cga ccg aat cca gag ctc cgc cgg aac atg acc atc       1303
Lys Gln Gln Lys Arg Pro Asn Pro Glu Leu Arg Arg Asn Met Thr Ile
325                 330                 335                 340

aaa acc gaa ctc ccc ctg ggc gca cgg cgg aag atg aag cca ctg cta       1351
Lys Thr Glu Leu Pro Leu Gly Ala Arg Arg Lys Met Lys Pro Leu Leu
                345                 350                 355
```

```
cca cgg gtc agc tca tac ctg gta cct atc cag ttc ccg gtg aac cag        1399
Pro Arg Val Ser Ser Tyr Leu Val Pro Ile Gln Phe Pro Val Asn Gln
                360                 365                 370

tca ctg gtg ttg cag ccc tcg gtg aag gtg cca ttg ccc ctg gcg gct        1447
Ser Leu Val Leu Gln Pro Ser Val Lys Val Pro Leu Pro Leu Ala Ala
            375                 380                 385

tcc ctc atg agc tca gag ctt gcc cgc cat agc aag cga gtc cgc att        1495
Ser Leu Met Ser Ser Glu Leu Ala Arg His Ser Lys Arg Val Arg Ile
        390                 395                 400

gcc ccc aag gtg ctg cta gct gag gag ggg ata gct cct ctt tct tct        1543
Ala Pro Lys Val Leu Leu Ala Glu Glu Gly Ile Ala Pro Leu Ser Ser
405                 410                 415                 420

gca gga cca ggg aaa gag gag aaa ctc ctg ttt gga gaa ggg ttt tct        1591
Ala Gly Pro Gly Lys Glu Glu Lys Leu Leu Phe Gly Glu Gly Phe Ser
                    425                 430                 435

cct ttg ctt cca gtt cag act atc aag gag gaa gaa atc cag cct ggg        1639
Pro Leu Leu Pro Val Gln Thr Ile Lys Glu Glu Glu Ile Gln Pro Gly
                440                 445                 450

gag gaa atg cca cac tta gcg aga ccc atc aaa gtg gag agc cct ccc        1687
Glu Glu Met Pro His Leu Ala Arg Pro Ile Lys Val Glu Ser Pro Pro
            455                 460                 465

ttg gaa gag tgg ccc tcc ccg gcc cca tct ttc aaa gag gaa tca tct        1735
Leu Glu Glu Trp Pro Ser Pro Ala Pro Ser Phe Lys Glu Glu Ser Ser
        470                 475                 480

cac tcc tgg gag gat tcg tcc caa tct ccc acc cca aga ccc aag aag        1783
His Ser Trp Glu Asp Ser Ser Gln Ser Pro Thr Pro Arg Pro Lys Lys
485                 490                 495                 500

tcc tac agt ggg ctt agg tcc cca acc cgg tgt gtc tcg gaa atg ctt        1831
Ser Tyr Ser Gly Leu Arg Ser Pro Thr Arg Cys Val Ser Glu Met Leu
                    505                 510                 515

gtg att caa cac agg gag agg agg gag agg agc cgg tct cgg agg aaa        1879
Val Ile Gln His Arg Glu Arg Arg Glu Arg Ser Arg Ser Arg Arg Lys
                520                 525                 530

cag cat cta ctg cct ccc tgt gtg gat gag ccg gag ctg ctc ttc tca        1927
Gln His Leu Leu Pro Pro Cys Val Asp Glu Pro Glu Leu Leu Phe Ser
            535                 540                 545

gag ggg ccc agt act tcc cgc tgg gcc gca gag ctc ccg ttc cca gca        1975
Glu Gly Pro Ser Thr Ser Arg Trp Ala Ala Glu Leu Pro Phe Pro Ala
        550                 555                 560

gac tcc tct gac cct gcc tcc cag ctc agc tac tcc cag gaa gtg gga        2023
Asp Ser Ser Asp Pro Ala Ser Gln Leu Ser Tyr Ser Gln Glu Val Gly
565                 570                 575                 580

gga cct ttt aag aca ccc att aag gaa acg ctg ccc atc tcc tcc acc        2071
Gly Pro Phe Lys Thr Pro Ile Lys Glu Thr Leu Pro Ile Ser Ser Thr
                    585                 590                 595

ccg agc aaa tct gtc ctc ccc aga acc cct gaa tcc tgg agg ctc acg        2119
Pro Ser Lys Ser Val Leu Pro Arg Thr Pro Glu Ser Trp Arg Leu Thr
                600                 605                 610
```

```
ccc cca gcc aaa gta ggg gga ctg gat ttc agc cca gta caa acc tcc        2167
Pro Pro Ala Lys Val Gly Gly Leu Asp Phe Ser Pro Val Gln Thr Ser
        615                 620                 625

cag ggt gcc tct gac ccc ttg cct gac ccc ctg ggg ctg atg gat ctc        2215
Gln Gly Ala Ser Asp Pro Leu Pro Asp Pro Leu Gly Leu Met Asp Leu
    630                 635                 640

agc acc act ccc ttg caa agt gct ccc ccc ctt gaa tca ccg caa agg        2263
Ser Thr Thr Pro Leu Gln Ser Ala Pro Pro Leu Glu Ser Pro Gln Arg
645                 650                 655                 660

ctc ctc agt tca gaa ccc tta gac ctc atc tcc gtc ccc ttt ggc aac        2311
Leu Leu Ser Ser Glu Pro Leu Asp Leu Ile Ser Val Pro Phe Gly Asn
                665                 670                 675

tct tct ccc tca gat ata gac gtc ccc aag cca ggc tcc ccg gag cca        2359
Ser Ser Pro Ser Asp Ile Asp Val Pro Lys Pro Gly Ser Pro Glu Pro
            680                 685                 690

cag gtt tct ggc ctt gca gcc aat cgt tct ctg aca gaa ggc ctg gtc        2407
Gln Val Ser Gly Leu Ala Ala Asn Arg Ser Leu Thr Glu Gly Leu Val
        695                 700                 705

ctg gac aca atg aat gac agc ctc agc aag atc ctg ctg gac atc agc        2455
Leu Asp Thr Met Asn Asp Ser Leu Ser Lys Ile Leu Leu Asp Ile Ser
    710                 715                 720

ttt cct ggc ctg gac gag gac cca ctg ggc cct gac aac atc aac tgg        2503
Phe Pro Gly Leu Asp Glu Asp Pro Leu Gly Pro Asp Asn Ile Asn Trp
725                 730                 735                 740

tcc cag ttt att cct gag cta cag tag agccctgccc ttgcccctgt             2550
Ser Gln Phe Ile Pro Glu Leu Gln
                745

gctcaagctg tccaccatcc cgggcactcc aaggctcagt gcaccccaag cctctgagtg      2610

aggacagcag gcagggactg ttctgctcct catagctccc tgctgcctga ttatgcaaaa      2670

gtagcagtca caccctagcc actgctggga ccttgtgttc cccaagagta tctgattcct      2730

ctgctgtccc tgccaggagc tgaagggtgg gaacaacaaa ggcaatggtg aaaagagatt      2790

aggaaccccc cagcctgttt ccattctctg cccagcagtc tcttaccttc cctgatcttt      2850

gcagggtggt ccgtgtaaat agtataaatt ctccaaatta tcctctaatt ataaatgtaa      2910

gcttatttcc ttagatcatt atccagagac tgccagaagg tgggtaggat gacctggggt      2970

ttcaattgac ttctgttcct tgcttttagt tttgatagaa gggaagacct gcagtgcacg      3030

gtttcttcca ggctgaggta cctggatctt gggttcttca ctgcagggac ccagacaagt      3090

ggatctgctt gccagagtcc tttttgcccc tccctgccac ctccccgtgt ttccaagtca      3150

gctttcctgc aagaagaaat cctggttaaa aaagtctttt gtattgggtc aggagttgaa      3210

tttggggtgg gaggatggat gcaactgaag cagagtgtgg gtgcccagat gtgcgctatt      3270

agatgtttct ctgataatgt ccccaatcat accagggaga ctggcattga cgagaactca      3330
```

ggtggaggct tgagaaggcc gaaagggccc ctgacctgcc tggcttcctt agcttgcccc 3390

tcagctttgc aaagagccac cctaggcccc agctgaccgc atgggtgtga gccagcttga 3450

gaacactaac tactcaataa aagcgaaggt ggacatgaaa aaaaaaaaaa aaaaaa 3506

**Description**

**FIELD OF THE INVENTION**

The present invention relates to a FoxM1 gene splicing modifier selected from a compound of formula (I) or of formula (VI)

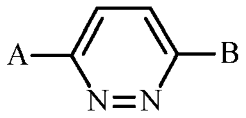
(I)
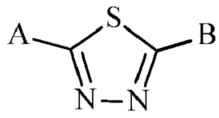
(VI),

wherein A and B are as defined herein, or a pharmaceutically acceptable salt thereof, for use in the treatment, prevention and/or delay of progression of cancer.

**BACKGROUND**

FoxM1 is a transcription factor of the Forkhead family. It is also known in the literature as Trident (in mouse), HFH-11 (in human), WIN or INS-1 (in rat), MPP-2 (partial human cDNA) or FKHL-16. The Forkhead family comprises a large number of transcription factors defined by a conserved DNA binding domain called Forkhead or winged-helix domain. The FoxM1 gene was cloned by screening cDNA libraries with degenerate primers for homologues with a conserved Forkhead DNAbinding domain (W. Korver, J. Roose, H. Clevers, Nucleic Acids Res. 25 (1997) 1715-1719). The FoxM1 gene was revealed to encode a Forkhead transcription factor family member that exhibits 45% identity in the DNA-binding domain with five of its closest related Forkhead members, namely FoxA3 (HNF-3γ, FoxC1 (fkh-1), FoxF2 (FREAC-2), FoxK1 (ILF) and FoxN2 (HTLF). The FoxM1 C-terminal region was found to have homology (76% identity) with a human partial cDNA encoding an open reading-frame of 221 amino acids, termed MPP-2. MPP-2 stands for MPM-2-reactive phosphoprotein-2 and was identified after screening a lymphoblast-derived cDNA library with the MPM-2 monoclonal antibody, which binds specifically to epitopes on mitotic proteins that are phosphorylated in a phosphoseline-proline dependent manner. FoxM1 binds DNA in vitro through the consensus site TAAACA. This motif shares the core sequence recognized by other members of the forkhead family. In particular, repeats of these motifs, in alternating orientation, were often characterized within the selected binding sequences for FoxM1.

The human FoxM1 gene is a 10-exon structure spanning approximately 25 kb on the 12p13-3 chromosomal band (telomeric position) (W. Korver, J. Roose, H. Clevers, Nucleic Acids Res. 25 (1997) 1715-1719). Two exons, named exons Va and VIIa, also referred to as exon A1 (or rat exon 6) and A2 respectively, are alternatively spliced (H. Ye, T.F. Kelly, U. Samadani, L. Lim, S. Rubio, D.G. Overdier, K.A. Roebuck, R.H. Costa, Mol. Cell Biol. 17 (1997) 1626-1641). Exon Va encodes a 15 amino-acidinsertion within the C-terminal part of the DNA binding-domain, and is not seen in any of the other Forkhead transcription factor family members. Exon VIIa represents a 38 amino-acid insertion within the C-terminus of the protein. Differential splicing of exons Va and VIIa in human FoxM1, gives rise to three classes of transcripts, class A containing both alternative exons, class B containing none of the alternative exons, and class C in which exon Va only is retained (H. Ye, T.F. Kelly, U. Samadani, L. Lim, S. Rubio, D.G. Overdier, K.A. Roebuck, R.H. Costa, Mol. Cell Biol. 17 (1997) 1626-1641). Both FoxM1B and FoxM1C are transcriptionally active, whereas FoxM1A is transcriptionally inactive, due to the insertion of exon VIIa in the C-terminal transactivation domain. This disruption of the transactivation domain in FoxM1A not only leads to transcriptional inactivation, it might also cause this variant to act as a dominant-negative variant as it has retained normal DNA binding activity in the absence of a functional transactivation domain (H. Ye, T.F. Kelly, U. Samadani, L. Lim, S. Rubio, D.G. Overdier, K.A. Roebuck, R.H. Costa, Mol. Cell Biol. 17 (1997) 1626-1641).

FoxM1 is overexpressed in a broad range of tumor types, including those of neural, gastrointestinal, and reproductive origin (see Bektas et al., supra; Nakamura et al., 2004, Oncogene 23: 2385-400; Pilarsky et al., 2004, Neoplasia .Q: 744-50; Liu et al., 2006, Cancer Res 66: 3593-602). This expression pattern of FoxM1 is attributed to the ability of FoxM1 to transactivate genes required for cell cycle progression (Wang et al.,2002, Proc Nat. Acad Sci US A 99:16881-6). Increased nuclear staining of FoxM1B found in human basal cell carcinomas suggests that FoxM1 is required for cellular proliferation in human cancers (Teh et al., 2002, Cancer Res. 62: 4773-80). The detailed role of FoxM1 in establishing or facilitating tumor progression and disease management has not been fully elucidated, however.

EP 2 298 896 (A1) discloses siRNA molecules inhibiting expression of FoxM1B protein and the use of the siRNA molecules for inhibiting tumor growth.

WO 2011/127297 (A1) discloses a composition comprising a siRNA FoxM1 inhibitor and Herceptin for the treatment of breast cancer.

WO 2014/028459 (A1) discloses 1,4-disubstituted pyridazine analogs and methods for treating SMN-deficiency related conditions.

WO 2014/116845 (A1) discloses thiadiazole analogs and methods for treating SMN-deficiency related conditions.

The problem to be solved by the present invention was to provide new compounds suitable for modifying splicing of the FoxM1 gene for use in the treatment of cancer.

## BRIEF DESCRIPTION OF THE FIGURES

**Figure 1.** Induction of alternative splicing of FoxM1 towards full-length FoxM1 in fibroblasts. Human fibroblasts were incubated with compounds of present invention at different concentrations for 24 hours, and changes in FoxM1_FL (containing exon VIIa) and FoxM1_ΔVIIa (lacking exon VIIa) mRNA expression were assessed by RT-qPCR. **Fig. 1A** Compound 1; **Fig. 1B** Compound 2; **Fig. 1C** Compound 3; **Fig. 1D** Compound 4; **Fig. IE** Compound 6; **Fig. 1F** Compound 7; **Fig. 1G** Compound 8; **Fig. 1H** Compound 9; **Fig. 1I** Compound 11. Data represent means ± standard error of the mean (SEM) of 3-9 independent observations. Data was generated as described in Example 1.

**Figure 2.** Correlation of in vitro potency of the compounds of the invention for modulation of the FoxM1 splicing vs. splicing of the survival of motoneuron 2 (SMN2) gene. Half-maximal effects for the FoxM_ΔVIIa splice variant and for the SMN protein are shown. Data was obtained as described in Example 2.

## DETAILED DESCRIPTION OF THE INVENTION

Unless otherwise defined, all technical and scientific terms used herein have the same meaning as commonly understood by one of ordinary skill in the art to which this invention belongs. Although methods and materials similar or equivalent to those described herein can be used in the practice or testing of the invention, suitable methods and materials are described below.

All publications, patent applications, patents, and other references mentioned herein are incorporated by reference in their entirety.

The nomenclature used in this Application is based on IUPAC systematic nomenclature, unless indicated otherwise.

Any open valency appearing on a carbon, oxygen, sulfur or nitrogen atom in the structures herein indicates the presence of a hydrogen, unless indicated otherwise.

The definitions described herein apply irrespective of whether the terms in question appear alone or in combination. It is contemplated that the definitions described herein can be appended to form chemically-relevant combinations, such as e.g. "heterocycloalkylaryl", "haloalkylheteroaryl", "arylalkylheterocycloalkyl", or "alkoxyalkyl". The last member of the combination is the radical which is binding to the rest of the molecule. The other members of the combination are attached to the binding radical in reversed order in respect of the literal sequence, e.g. the combination arylalkylheterocycloalkyl refers to a heterocycloalkyl-radical which is substituted by an alkyl which is substituted by an aryl.

When indicating the number of substituents, the term "one or more" refers to the range from one substituent to the highest possible number of substitution, i.e. replacement of one hydrogen up to replacement of all hydrogens by substituents.

The term "optional" or "optionally" denotes that a subsequently described event or circumstance can but need not occur, and that the description includes instances where the event or circumstance occurs and instances in which it does not.

The term "substituent" denotes an atom or a group of atoms replacing a hydrogen atom on the parent molecule.

The term "substituted" denotes that a specified group bears one or more substituents. Where any group can carry multiple substituents and a variety of possible substituents is provided, the substituents are independently selected and need not to be the same. The term "unsubstituted" means that the specified group bears no substituents. The term "optionally substituted" means that the specified group is unsubstituted or substituted by one or more substituents, independently chosen from the group of possible substituents. When indicating the number of substituents, the term "one or more" means from one substituent to the highest possible number of substitution, i.e. replacement of one hydrogen up to replacement of all hydrogens by substituents.

The terms "compound(s) of this invention", "compound(s) of the present invention", "FoxM1 gene splicing modifier", "FoxM1 splicing modifier", and "compounds modifying splicing of the FoxM1 gene" are interchangeably used herein and refer to compounds as disclosed herein and stereoisomers, tautomers, solvates, and salts (e.g., pharmaceutically acceptable salts) thereof.

When the compounds of the invention are solids, it is understood by those skilled in the art that these compounds, and their solvates and salts, may exist in different solid forms, particularly different crystal forms, all of which are intended to be within the scope of the present invention and specified formulas.

The term "pharmaceutically acceptable salts" denotes salts which are not biologically or otherwise undesirable. Pharmaceutically acceptable salts include both acid and base addition salts.

The term "pharmaceutically acceptable acid addition salt" denotes those pharmaceutically acceptable salts formed with inorganic acids such as hydrochloric acid, hydrobromic acid, sulfuric acid, nitric acid, carbonic acid, phosphoric acid, and organic acids selected from aliphatic, cycloaliphatic, aromatic, araliphatic, heterocyclic, carboxylic, and sulfonic classes of organic acids such as formic acid, acetic acid, propionic acid, glycolic acid, gluconic acid, lactic acid, pyruvic acid, oxalic acid, malic acid, maleic acid, maloneic acid, succinic acid, fumaric acid, tartaric acid, citric acid, aspartic acid, ascorbic acid, glutamic acid, anthranilic acid, benzoic acid, cinnamic acid, mandelic acid, embonic acid, phenylacetic acid, methanesulfonic acid, ethanesulfonic acid, p-toluenesulfonic acid, and salicyclic acid.

The term "pharmaceutically acceptable base addition salt" denotes those pharmaceutically acceptable salts formed with an organic or inorganic base. Examples of acceptable inorganic bases include sodium, potassium, ammonium, calcium, magnesium, iron, zinc, copper, manganese, and aluminum salts. Salts derived from pharmaceutically acceptable organic nontoxic bases includes salts of primary, secondary, and tertiary amines, substituted amines including naturally occurring substituted amines, cyclic amines and basic ion exchange resins, such as isopropylamine, trimethylamine, diethylamine, triethylamine, tripropylamine, ethanolamine, 2-diethylaminoethanol, trimethamine, dicyclohexylamine, lysine, arginine, histidine, caffeine, procaine, hydrabamine, choline, betaine, ethylenediamine, glucosamine, methylglucamine, theobromine, purines, piperizine, piperidine, N-ethylpiperidine, and polyamine resins.

Stereochemical definitions and conventions used herein generally follow S. P. Parker, Ed., McGraw-Hill Dictionary of Chemical Terms (1984) McGraw-Hill Book Company, New York; and Eliel, E. and Wilen, S., "Stereochemistry of Organic Compounds", John Wiley & Sons, Inc., New York, 1994. In describing an optically active compound, the prefixes D and L, or R and S, are used to denote the absolute configuration of the molecule about its chiral center(s). The substituents attached to the chiral center under consideration are ranked in accordance with the Sequence Rule of Cahn, Ingold and Prelog. (Cahn et al. Angew. Chem. Inter. Edit. 1966, 5, 385; errata 511). The prefixes D and L or (+) and (-) are employed to designate the sign of rotation of plane-polarized light by the compound, with (-) or L designating that the compound is levorotatory. A compound prefixed with (+) or D is dextrorotatory.

The term "halo", "halogen", and "halide" are used interchangeably herein and denote fluoro, chloro, bromo, or iodo, most particularly fluoro or chloro.

The term "alkyl" denotes a monovalent linear or branched saturated hydrocarbon group of 1 to 12 carbon atoms. In particular embodiments, alkyl has 1 to 7 carbon atoms, and in more particular embodiments 1 to 4 carbon atoms. Examples of alkyl include methyl, ethyl, propyl, isopropyl, n-butyl, iso-butyl, sec-butyl, or tert-butyl, particularly methyl.

The term "alkenyl" denotes a monovalent linear or branched hydrocarbon group of 2 to 7 carbon atoms with at least one double bond. In particular embodimets, alkenyl has 2 to 4 carbon atoms with at least one double bond. Examples of alkenyl include ethenyl, propenyl, prop-2-enyl, isopropenyl, n-butenyl, and iso-butenyl.

The term "alkynyl" denotes a monovalent linear or branched saturated hydrocarbon group of 2 to 7 carbon atoms comprising one, two or three triple bonds. In particular embodiments alkynyl has from 2 to 4 carbon atoms comprising one or two triple bonds. Examples of alkynyl include ethynyl, propynyl, prop-2-ynyl, isopropynyl, and n-butynyl.

The term "alkoxy" denotes a group of the formula -O-R', wherein R' is an alkyl group. Examples of alkoxy moieties include methoxy, ethoxy, isopropoxy, and tert-butoxy, particularly methoxy.

The term "haloalkyl" denotes an alkyl group wherein at least one of the hydrogen atoms of the alkyl group has been replaced by same or different halogen atoms, particularly fluoro atoms. Examples of haloalkyl include monofluoro-, difluoro- or trifluoro-methyl, -ethyl or -propyl, for example 3,3,3-trifluoropropyl, 2-fluoroethyl, 2,2,2-trifluoroethyl, fluormethyl, or trifluoromethyl. The term "perhaloalkyl" denotes an alkyl group where all hydrogen atoms of the alkyl group have been replaced by the same or different halogen atoms.

The term "haloalkoxy" denotes an alkoxy group wherein at least one of the hydrogen atoms of the alkoxy group has been replaced by same or different halogen atoms, particularly fluoro atoms. Examples of haloalkoxyl include monofluoro-, difluoro- or trifluoro-methoxy,-ethoxy or -propoxy, for example 3,3,3-trifluoropropoxy, 2-fluoroethoxy, 2,2,2-trifluoroethoxy, fluoromethoxy, or trifluoromethoxy. The term "perhaloalkoxy" denotes an alkoxy group where all hydrogen atoms of the alkoxy group have been replaced by the same or different halogen atoms.

The term "hydroxyalkyl" denotes an alkyl group wherein at least one of the hydrogen atoms of the alkyl group has been replaced by a hydroxy group. Examples of hydroxyalky include hydroxymethyl, 2-hydroxyethyl, 2-hydroxypropyl, 3-hydroxypropyl, 1-(hydroxymethyl)-2-methylpropyl, 2-hydroxybutyl, 3-hydroxybutyl, 4-hydroxybutyl, 2,3-dihydroxypropyl, 2-hydroxy-1-hydroxymethylethyl, 2,3-dihydroxybutyl, 3,4-dihydroxybutyl or 2-(hydroxymethyl)-3-hydroxypropyl.

The term "bicyclic ring system" denotes two rings which are fused to each other via a common single or double bond (annelated bicyclic ring system), via a sequence of three or more common atoms (bridged bicyclic ring system) or via a common single atom (spiro bicyclic ring system). Bicyclic ring systems can be saturated, partially unsaturated,

unsaturated or aromatic. Bicyclic ring systems can comprise heteroatoms selected from N, O and S.

The term "cycloalkyl" denotes a monovalent saturated monocyclic or bicyclic hydrocarbon group of 3 to 10 ring carbon atoms. In particular embodiments cycloalkyl denotes a monovalent saturated monocyclic hydrocarbon group of 3 to 8 ring carbon atoms. Bicyclic means consisting of two saturated carbocycles having one or more carbon atoms in common. Particular cycloalkyl groups are monocyclic. Examples for monocyclic cycloalkyl are cyclopropyl, cyclobutanyl, cyclopentyl, cyclohexyl or cycloheptyl. Examples for bicyclic cycloalkyl are bicyclo[2.2.1]heptanyl, or bicyclo[2.2.2]octanyl.

The term "cycloalkenyl" denotes a monovalent unsaturated non-aromatic monocyclic or bicyclic hydrocarbon group of 3 to 8 ring carbon atoms. Particular cycloalkenyl groups are monocyclic. Examples of cycloalkenyl groups include cyclobuten-1-yl, and cyclopenten-1-yl.

The term "heterocycloalkyl" denotes a monovalent saturated or partly unsaturated mono- or bicyclic ring system of 3 to 9 ring atoms, comprising 1, 2, or 3 ring heteroatoms selected from N, O and S, the remaining ring atoms being carbon. In particular embodiments, heterocycloalkyl is a monovalent saturated monocyclic ring system of 4 to 7 ring atoms, comprising 1, 2, or 3 ring heteroatoms selected from N, O and S, the remaining ring atoms being carbon. Examples for monocyclic saturated heterocycloalkyl are aziridinyl, oxiranyl, azetidinyl, oxetanyl, pyrrolidinyl, tetrahydrofuranyl, tetrahydro-thienyl, pyrazolidinyl, imidazolidinyl, oxazolidinyl, isoxazolidinyl, thiazolidinyl, piperidinyl, tetrahydropyranyl, tetrahydrothiopyranyl, piperazinyl, morpholinyl, thiomorpholinyl, 1,1-dioxo-thiomorpholin-4-yl, azepanyl, diazepanyl, homopiperazinyl, or oxazepanyl. Examples for bicyclic saturated heterocycloalkyl are 8-aza-bicyclo[3.2.1]octyl, quinuclidinyl, 8-oxa-3-aza-bicyclo[3.2.1]octyl, 9-aza-bicyclo[3.3.1]nonyl, 3-oxa-9-aza-bicyclo[3.3.1]nonyl, or 3-thia-9-aza-bicyclo[3.3.1]nonyl. Examples for partly unsaturated heterocycloalkyl are dihydrofuryl, imidazolinyl, dihydro-oxazolyl, tetrahydro-pyridinyl, or dihydropyranyl.

The term "aromatic" denotes the conventional idea of aromaticity as defined in the literature, in particular in IUPAC - Compendium of Chemical Terminology, 2nd, A. D. McNaught & A. Wilkinson (Eds). Blackwell Scientific Publications, Oxford (1997).

The term "aryl" denotes a monovalent aromatic carbocyclic mono- or bicyclic ring system comprising 6 to 10 carbon ring atoms. Examples of aryl moieties include phenyl and naphthyl.

The term "aryloxy" denotes a group of the formula -O-R', wherein R' is aryl. An example of aryloxy is phenoxy.

The term "heteroaryl" denotes a monovalent aromatic heterocyclic mono- or bicyclic ring system of 5 to 12 ring atoms, comprising 1, 2, 3 or 4 heteroatoms selected from N, O and S, the remaining ring atoms being carbon. Examples of heteroaryl moieties include pyrrolyl, furanyl, thienyl, imidazolyl, oxazolyl, thiazolyl, triazolyl, oxadiazolyl, thiadiazolyl, tetrazolyl, pyridinyl, pyrazinyl, pyrazolyl, pyridazinyl, pyrimidinyl, triazinyl, azepinyl, diazepinyl, isoxazolyl, benzofuranyl, isothiazolyl, benzothienyl, indolyl, isoindolyl, isobenzofuranyl, benzimidazolyl, benzoxazolyl, benzoisoxazolyl, benzothiazolyl, benzoisothiazolyl, benzooxadiazolyl, benzothiadiazolyl, benzotriazolyl, purinyl, quinolinyl, isoquinolinyl, quinazolinyl, or quinoxalinyl, most particularly pyrazolyl or pyridinyl.

The term "pyridinyl substituted with hydroxy" equally refers to its tautomeric form pyridine-one, such as for example "pyridin-2-ol" and its tautomer "3H-pyridin-2-one"

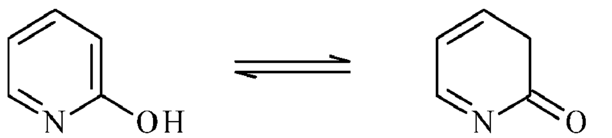

The term "alkylene" denotes a linear saturated divalent hydrocarbon group of 1 to 7 carbon atoms or a divalent branched saturated divalent hydrocarbon group of 3 to 7 carbon atoms. Examples of alkylene groups include methylene, ethylene, propylene, 2-methylpropylene, butylene, 2-ethylbutylene, pentylene, hexylene.

The term "alkylamino" denotes a group -NR'R", wherein R' is hydrogen and R" is a alkyl. The term "dialkylamino" as used herein denotes a group -NR'R", wherein R' and R" are both alkyl. Examples of alkylamino groups include methylamino and ethylamino. Examples of alkylamino groups include dimethylamino, methylethylamino, diethylamino and di(1-methylethyl)amino.

The term "active pharmaceutical ingredient" (or "API") denotes the compound or molecule in a pharmaceutical composition that has a particular biological activity.

The terms "pharmaceutical composition" and "pharmaceutical formulation" (or "formulation") are used interchangeably and denote a mixture or solution comprising a therapeutically effective amount of an active pharmaceutical ingredient together with one or more pharmaceutically acceptable excipients to be administered to a mammal, e.g., a human in need thereof.

The term "pharmaceutically acceptable" denotes an attribute of a material which is useful in preparing a pharmaceutical composition that is generally safe, non-toxic, and neither biologically nor otherwise undesirable and is acceptable for veterinary as well as human pharmaceutical use.

The terms "pharmaceutically acceptable excipient", "pharmaceutically acceptable carrier" and "therapeutically inert excipient" can be used interchangeably and denote any pharmaceutically acceptable ingredient in a pharmaceutical composition having no therapeutic activity and being non-toxic to the subject administered, such as disintegrators, binders, fillers, solvents, buffers, tonicity agents, stabilizers, antioxidants, surfactants, carriers, diluents or lubricants used in formulating pharmaceutical products.

A "pharmaceutically acceptable carrier" refers to an ingredient in a pharmaceutical composition, other than an active ingredient, which is nontoxic to a subject. A pharmaceutically acceptable carrier includes, but is not limited to, a buffer, excipient, stabilizer, or preservative.

An "individual" or "subject" is a mammal. Mammals include, but are not limited to, domesticated animals (e.g., cows, sheep, cats, dogs, and horses), primates (e.g., humans and non-human primates such as monkeys), rabbits, and rodents (e.g., mice and rats). In certain embodiments, the individual or subject is a human.

The term "animal" as used herein comprises human beings and non-human animals. In one embodiment, a "non-human animal" is a mammal, for example a rodent such as rat or a mouse. In one embodiment, a non-human animal is a mouse.

The term "treating" or "treatment" of a disease state includes inhibiting the disease state, i.e., arresting the development of the disease state or its clinical symptoms, or relieving the disease state, i.e., causing temporary or permanent regression of the disease state or its clinical symptoms.

The term "preventing" or "prevention" of a disease state denotes causing the clinical symptoms of the disease state not to develop in a subject that can be exposed to or predisposed to the disease state, but does not yet experience or display symptoms of the disease state.

The term "FoxM1 polypeptide" is used herein to refer to native FoxM1 polypeptide from any animal, e.g. mammalian, species, including humans, and FoxM1 variants. The amino acid sequence of human FoxM1A polypeptide is given in Seq. Id. No. 1, the amino acid sequence of human FoxM1B is given in Seq. Id. No. 2 and the amino acid sequence of FoxM1C polypeptide is given in Seq. Id. No. 3.

The nucleotide sequences of the three FoxM1 variants are set forth in Seq. Id. No. 4 (FoxM1A), Seq. Id. No. 5 (FoxM1B) and Seq. Id. No. 6 (FoxM1C).

The term "compound modifying splicing of the FoxM1 gene" is used herein to refer to compounds which lead to the production of transcriptionally inactive forms of the FoxM1 polypeptide, in particular to the production of FoxM1A variant, by modifying the FoxM1 splicing such that transcriptionally inactive forms are generated, in particular FoxM1A, and by suppressing the production of transcriptionally active FoxM1 variants, in particular FoxM1B and FoxM1C.

Methods for detection and/or measurement of polypeptides in biological material are well known in the art and include, but are not limited to, Western-blotting, Flow cytometry, ELISAs or RIAs, or various proteomics techniques. An example for a method to measure a polypeptide is an ELISA. This type of protein quantitation is based on an antibody capable of capturing a specific antigen, and a second antibody capable of detecting the captured antigen. The assays mentioned hereinbefore are described in Harlow, E. and Lane, D. Antibodies: A Laboratory Manual, (1988), Cold Spring Harbor Laboratory Press.

Methods for detection and/or measurement of RNA in biological material are well known in the art and include, but are not limited to, Northern-blotting, RNA protection assay, RT PCR. Suitable methods are described in Molecular Cloning: A Laboratory Manual(Fourth Edition) By Michael R. Green, Joseph Sambrook, Peter MacCallum 2012, 2,028 pp, ISBN 978-1-936113-42-2.

In a first aspect, the present invention provides compounds modifying splicing of the FoxM1 gene for use in the treatment, prevention and/or delay of progression of cancer, wherein the compounds induce a transcriptionally inactive FoxM1 variant. In a particular embodiment of the present invention, the transcriptionally inactive FoxM1 variant is FoxM1A.

In a particular embodiment, the present invention relates to a FoxM1 gene splicing modifier for use in the treatment, prevention and/or delay of progression of cancer, wherein the FoxM1 gene splicing modifier induces a transcriptionally inactive FoxM1 variant.

In a particular embodiment, the present invention relates to a FoxM1 gene splicing modifier for use in the treatment, prevention and/or delay of progression of cancer, wherein the FoxM1 gene splicing modifier induces the transcriptionally inactive FoxM1A variant.

In a particular embodiment of the present invention the FoxM1 gene is the human FoxM1 gene.

In a particular embodiment, the present invention relates to a FoxM1 gene splicing modifier for use in the treatment, prevention and/or delay of progression of cancer, wherein the FoxM1 gene is the human FoxM1 gene.

In a particular embodiment of the present invention the cancer is selected from the group consisting of cancer of the liver, prostate, brain, breast, lung, colon, pancreas, skin, cervix, ovary, mouth, blood and nervous system.

In a particular embodiment, the present invention relates to a FoxM1 gene splicing modifier for use in the treatment, prevention and/or delay of progression of cancer, wherein the cancer is selected from the group consisting

of cancer of the liver, prostate, brain, breast, lung, colon, pancreas, skin, cervix, ovary, mouth, blood and nervous system.

In more detail, the present invention relates to a FoxM1 gene splicing modifier selected from a compound of formula (I) or a compound of formula (VI)

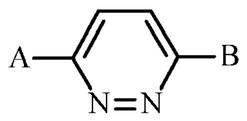
(I)

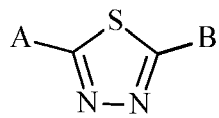
(VI),

wherein

A is 2-hydroxy-phenyl which is substituted with:

0, 1,2, or 3 substituents independently selected from $C_{1-4}$ alkyl, oxo, oxime, hydroxy, halo-$C_{1-4}$, alkyl, dihalo-$C_{1-4}$ alkyl, trihalo-$C_{1-4}$ alkyl, $C_{1-4}$, alkoxy, $C_{1-4}$, alkoxy-$C_{3-7}$ cycloalkyl, halo-$C_{1-4}$, alkoxy, dihalo-$C_{1-4}$ alkoxy, trihalo-$C_{1-4}$ alkoxy, hydroxy, cyano, halogen, amino, mono-$C_{1-4}$ alkylamino, di-$C_{1-4}$ alkylamino, heteroaryl, $C_{1-4}$ alkyl substituted with hydroxy, $C_{1-4}$ alkoxy substituted with aryl, amino, -C(O)NH-$C_{1-4}$alkyl-heteroaryl, -NHC(O)-$C_{1-4}$ alkylheteroaryl, $C_{1-4}$alkyl-C(O)NH-heteroaryl, $C_{1-4}$alkyl-NHC(O)-heteroaryl, $C_{3-7}$ cycloalkyl, 5-7 membered cycloalkenyl, or 5, 6 or 9 membered heterocycle containing 1 or 2 heteroatoms independently, selected from S, O and N,

wherein two $C_{1-4}$ alkyl groups can combine with the atoms to which they are bound to form a 5-6 membered ring; wherein heteroaryl has 5, 6 or 9 ring atoms, 1, 2 or 3 ring heteroatoms selected from N, O and S, and is substituted with 0, 1, or 2 substituents independently selected from oxo, hydroxy, nitro, halogen, $C_{1-4}$ alkyl, $C_{1-4}$ alkenyl, $C_{1-4}$ alkoxy, $C_{3-7}$ cycloalkyl, $C_{1-4}$ alkyl-OH, trihalo-$C_{1-4}$ alkyl, mono-$C_{1-4}$ alkylamino, di-$C_{1-4}$ alkylamino, -C(O)$NH_2$, -$NH_2$, $NO_2$, hydroxy-$C_{1-4}$alkylamino, hydroxy-$C_{1-4}$alkyl, 4-7 membered heterocycle-$C_{1-4}$ alkyl, amino-$C_{1-4}$ alkyl, mono-$C_{1-4}$alkylamino-$C_{1-4}$ alkyl, and di-$C_{1-4}$alkylamino-$C_{1-4}$ alkyl; or

A is 2-naphthyl optionally substituted at the 3 position with hydroxy and additionally substituted with 0, 1, or 2 substituents selected from hydroxy, cyano, halogen, $C_{1-4}$ alkyl, $C_{2-4}$ alkenyl, $C_{1-5}$ alkoxy, wherein the alkoxy is unsubstituted or substituted with hydroxy, $C_{1-4}$ alkoxy, amino, -NHC(O)-$C_{1-4}$ alkyl, -NHC(O)O-$C_{1-4}$ alkyl, $C_{1-4}$ alkylene-4-7 membered heterocycle, 4-7 membered heterocycle, mono-$C_{1-4}$ alkylamino, and di- $C_{1-4}$ alkylamino; or

A is 6 membered heteroaryl having 1-3 ring nitrogen atoms and which is substituted by phenyl or a heteroaryl having 5 or 6 ring atoms, 1 or 2 ring heteroatoms independently selected from N, O and S and is substituted with 0, 1, or 2 substituents independently selected from $C_{1-4}$ alkyl, mono-$C_{1-4}$ alkylamino, di-$C_{1-4}$alkylamino, hydroxy-$C_{1-4}$ alkylamino, hydroxy-$C_{1-4}$ alkyl, amino-$C_{1-4}$ alkyl and mono-$C_{1-4}$ alkylamino-$C_{1-4}$ alkyl, and di-$C_{1-4}$alkylamino-$C_{1-4}$ alkyl; or

A is bicyclic heteroaryl having 9 to 10 ring atoms, 1, 2, or 3 ring heteroatoms independently selected from N, O or S, and which is substituted with 0, 1, or 2 substituents independently selected from cyano, oxime, halogen, hydroxy, $C_{1-4}$ alkyl, $C_{2-4}$ alkenyl, $C_{2-4}$ alkynyl, $C_{1-4}$ alkoxy, $C_{1-4}$ alkoxy substituted with hydroxy, amino, mono-$C_{1-4}$ alkylamino, and di-$C_{1-4}$ alkylamino; or

A is tricyclic heteroaryl having 12 or 13 ring atoms, 1, 2, or 3 ring heteroatoms independently selected from N, O or S, and which is substituted with 0, 1, or 2 substituents independently selected from cyano, halogen, hydroxy, $C_{1-4}$ alkyl, $C_{1-4}$ alkenyl, $C_{2-4}$ alkynyl, $C_{1-4}$ alkoxy, $C_{1-4}$ alkoxy substituted with hydroxy, amino, mono-$C_{1-4}$ alkylamino, di-$C_{1-4}$ alkylamino, and heteroaryl having 5, 6 or 9 ring atoms, 1, 2 or 3 ring heteroatoms selected from N, O and S, and which is substituted with 0, 1, or 2 substituents independently selected from oxo, hydroxy, nitro, halogen, $C_{1-4}$ alkyl, $C_{1-4}$ alkenyl, $C_{1-4}$ alkoxy, $C_{3-7}$ cycloalkyl, $C_{1-4}$ alkyl-OH, trihalo-$C_{1-4}$ alkyl, mono-$C_{1-4}$ alkylamino, di-$C_{1-4}$ alkylamino, -C(O)$NH_2$, -$NH_2$,-$NO_2$, hydroxy-$C_{1-4}$ alkylamino, hydroxy-$C_{1-4}$ alkyl, 4-7 membered heterocycle-$C_{1-4}$ alkyl, amino-$C_{1-4}$ alkyl, mono-$C_{1-4}$ alkylamino-$C_{1-4}$ alkyl and di-$C_{1-4}$ alkylamino-$C_{1-4}$ alkyl; or

A is phenyl which is substituted with 0, 1,2, or 3 substituents independently selected from $C_{1-4}$ alkyl, oxo, oxime, hydroxy, halo-$C_{1-4}$ alkyl, dihalo-$C_{1-4}$ alkyl, trihalo-$C_{1-4}$ alkyl, $C_{1-4}$ alkoxy, $C_{1-4}$ alkoxy-$C_{3-7}$ cycloalkyl, halo-$C_{1-4}$ alkoxy, dihalo-$C_{1-4}$ alkoxy, trihalo-$C_{1-4}$ alkoxy, cyano, halogen, amino, mono-$C_{1-4}$ alkylamino, di-$C_{1-4}$ alkylamino, heteroaryl, $C_{1-4}$ alkyl substituted with hydroxy, $C_{1-4}$ alkoxy substituted with aryl, -C(O)NH-$C_{1-4}$, alkyl- heteroaryl, -NHC(O)-$C_{1-4}$ alkylheteroaryl, $C_{1-4}$ alkyl-C(O)NH-heteroaryl, $C_{1-4}$ alkyl-NHC(O)-heteroaryl, $C_{3-7}$ cycloalkyl, 5-7 membered cycloalkenyl or 5, 6 or 9 membered heterocycle containing 1 or 2 heteroatoms, independently, selected from S, 0 and N; wherein two $C_{1-4}$ alkyl groups can combine with the atoms to which they are bound to form a 5-6 membered ring; wherein heteroaryl has 5, 6 or 9 ring atoms, 1, 2 or 3 ring heteroatoms selected from N, 0 and S and is substituted with 0, 1, or 2 substituents independently selected from oxo, hydroxy, nitro, halogen, $C_{1-4}$ alkyl, $C_{1-4}$ alkenyl, $C_{1-4}$ alkoxy, $C_{3-7}$ cycloalkyl, $C_{1-4}$ alkyl-OH, trihalo-$C_{1-4}$ alkyl, mono-$C_{1-4}$ alkylamino, di-$C_{1-4}$ alkylamino, -C(O)$NH_2$, -$NH_2$,-$NO_2$, hydroxy-$C_{1-4}$ alkylamino, hydroxy-$C_{1-4}$ alkyl, 4-7 memberered heterocycle-$C_{1-4}$ alkyl, amino-$C_{1-4}$ alkyl, mono-$C_{1-4}$ alkylamino-$C_{1-4}$ alkyl, and di-$C_{1-4}$ alkylamino-$C_{1-4}$ alkyl;

B is a group of the formula

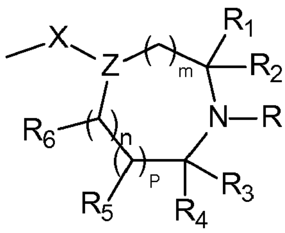

,

wherein

m, n and p are independently selected from 0 or 1;

R, $R_1$, $R_2$, $R_3$, and $R_4$ are independently selected from the group consisting of hydrogen and $C_{1-4}$ alkyl, wherein alkyl is optionally substituted with hydroxy, amino, mono-$C_{1-4}$ akylamino or di-$C_{1-4}$ akylamino;

$R_5$ and $R_6$ are independently selected from hydrogen and fluorine; or

R and $R_3$, taken in combination form a fused 5 or 6 membered heterocyclic ring having 0 or 1 additional ring heteroatoms selected from N, O or S; or

$R_1$ and $R_3$, taken in combination form a $C_{1-3}$ alkylene group; or

$R_1$ and $R_5$, taken in combination form a $C_{1-3}$ alkylene group; or

$R_3$ and $R_4$, taken in combination with the carbon atom to which they attach, form a spirocyclic $C_{3-6}$ cycloalkyl;

X is $CR_AR_B$, O, $NR_7$ or a bond;

$R_7$ is hydrogen, or $C_{1-4}$ alkyl;

$R_A$ and $R_B$ are independently selected from hydrogen and $C_{1-4}$ alkyl, or $R_A$ and $R_B$, taken in combination, form a divalent $C_{2-5}$ alkylene group;

Z is $CR_8$ or N; with the proviso that when Z is N, X is a bond;

$R_8$ is hydrogen or taken in combination with $R_6$ form a double bond; or

B is a group of the formula

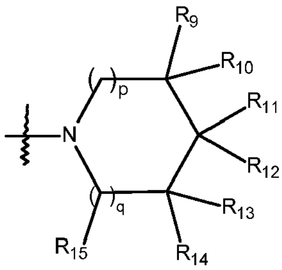

,

wherein

p and q are independently selected from the group consisting of 0, 1, and 2;

$R_9$ and $R_{13}$ are independently selected from hydrogen and $C_{1-4}$ alkyl;

$R_{10}$ and $R_{14}$ are independently selected from hydrogen, amino, mono-$C_{1-4}$ alkylamino, di-$C_{1-4}$ alkylamino, and $C_{1-4}$ alkyl optionally substituted with hydroxy, amino, mono-$C_{1-4}$ alkylamino or di-$C_{1-4}$ alkylamino;

$R_{11}$ is hydrogen, $C_{1-4}$ alkyl, amino, mono-$C_{1-4}$ alkylamino, or di-$C_{1-4}$ alkylamino;

$R_{12}$ is hydrogen or $C_{1-4}$ alkyl; or

$R_9$ and $R_{11}$ taken in combination form a saturated azacycle having 4 to 7 ring atoms which is optionally substituted with one to three $C_{1-4}$ alkyl groups; or

$R_{11}$ and $R_{12}$, taken in combination form a saturated azacycle having 4 to 7 ring atoms which is optionally substituted with one to three $C_{1-4}$ alkyl groups.

or a pharmaceutically acceptable salt thereof;
for use in the treatment, prevention and/or delay of progression of cancer.

In a particular embodiment of the present invention, the FoxM1 gene splicing modifier is selected from a compound of formula (I):

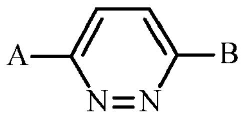

(I),

wherein

A is 2-hydroxy-phenyl which is substituted with:

0, 1,2, or 3 substituents independently selected from $C_{1-4}$ alkyl, oxo, oxime, hydroxy, halo-$C_{1-4}$ alkyl, dihalo-$C_{1-4}$alkyl, trihalo-$C_{1-4}$ alkyl, $C_{1-4}$, alkoxy, $C_{1-4}$, alkoxy-$C_{3-7}$ cycloalkyl, halo-$C_{1-4}$alkoxy, dihalo-$C_{1-4}$alkoxy, trihalo-$C_{1-4}$ alkoxy, hydroxy, cyano, halogen, amino, mono-$C_{1-4}$ alkylamino, di-$C_{1-4}$ alkylamino, heteroaryl, $C_{1-4}$ alkyl substituted with hydroxy, $C_{1-4}$ alkoxy substituted with aryl, amino, -C(O)NH-$C_{1-4}$alkyl-heteroaryl, -NHC(O)-$C_{1-4}$ alkylheteroaryl, $C_{1-4}$ alkyl-C(O)NH-heteroaryl, $C_{1-4}$alkyl-NHC(O)-heteroaryl, $C_{3-7}$ cycloalkyl, 5-7 membered cycloalkenyl, or 5, 6 or 9 membered heterocycle containing 1 or 2 heteroatoms independently, selected from S, O and N,
wherein two $C_{1-4}$ alkyl groups can combine with the atoms to which they are bound to form a 5-6 membered ring; wherein heteroaryl has 5, 6 or 9 ring atoms, 1, 2 or 3 ring heteroatoms selected from N, O and S, and is substituted with 0, 1, or 2 substituents independently selected from oxo, hydroxy, nitro, halogen, $C_{1-4}$ alkyl, $C_{1-4}$ alkenyl, $C_{1-4}$ alkoxy, $C_{3-7}$ cycloalkyl, $C_{1-4}$ alkyl-OH, trihalo-$C_{1-4}$ alkyl, mono-$C_{1-4}$ alkylamino, di-$C_{1-4}$ alkylamino, -C(O)$NH_2$, -$NH_2$, $NO_2$, hydroxy-$C_{1-4}$alkylamino, hydroxy-$C_{1-4}$ alkyl, 4-7 membered heterocycle-$C_{1-4}$ alkyl, amino-$C_{1-4}$ alkyl, mono-$C_{1-4}$alkylamino-$C_{1-4}$ alkyl, and di-$C_{1-4}$alkylamino-$C_{1-4}$ alkyl; or

A is 2-naphthyl optionally substituted at the 3 position with hydroxy and additionally substituted with 0, 1, or 2 substituents selected from hydroxy, cyano, halogen, $C_{1-4}$ alkyl, $C_{2-4}$ alkenyl, $C_{1-5}$ alkoxy, wherein the alkoxy is unsubstituted or substituted with hydroxy, $C_{1-4}$ alkoxy, amino, -NHC(O)-$C_{1-4}$ alkyl, -NHC(O)O-$C_{1-4}$ alkyl, $C_{1-4}$ alkylene-4-7 membered heterocycle, 4-7 membered heterocycle, mono-$C_{1-4}$ alkylamino, and di- $C_{1-4}$ alkylamino; or

A is 6 membered heteroaryl having 1-3 ring nitrogen atoms and which is substituted by phenyl or a heteroaryl having 5 or 6 ring atoms, 1 or 2 ring heteroatoms independently selected from N, 0 and S and is substituted with 0, 1, or 2 substituents independently selected from $C_{1-4}$ alkyl, mono-$C_{1-4}$ alkylamino, di-$C_{1-4}$alkylamino, hydroxy-$C_{1-4}$ alkylamino, hydroxy-$C_{1-4}$ alkyl, amino-$C_{1-4}$ alkyl and mono-$C_{1-4}$ alkylamino-$C_{1-4}$ alkyl, and di-$C_{1-4}$alkylamino-$C_{1-4}$ alkyl; or

A is bicyclic heteroaryl having 9 to 10 ring atoms, 1, 2, or 3 ring heteroatoms independently selected from N, 0 or S, and which is substituted with 0, 1, or 2 substituents independently selected from cyano, oxime, halogen, hydroxy, $C_{1-4}$ alkyl, $C_{2-4}$ alkenyl, $C_{2-4}$ alkynyl, $C_{1-4}$ alkoxy, $C_{1-4}$ alkoxy substituted with hydroxy, amino, mono-$C_{1-4}$ alkylamino, and di-$C_{1-4}$ alkylamino; or

A is tricyclic heteroaryl having 12 or 13 ring atoms, 1, 2, or 3 ring heteroatoms independently selected from N, O or S, and which is substituted with 0, 1, or 2 substituents independently selected from cyano, halogen, hydroxy, $C_{1-4}$ alkyl, $C_{1-4}$ alkenyl, $C_{2-4}$ alkynyl, $C_{1-4}$ alkoxy, $C_{1-4}$ alkoxy substituted with hydroxy, amino, mono-$C_{1-4}$ alkylamino, di-$C_{1-4}$ alkylamino, and heteroaryl having 5, 6 or 9 ring atoms, 1, 2 or 3 ring heteroatoms selected from N, O and S, and which is substituted with 0, 1, or 2 substituents independently selected from oxo, hydroxy, nitro, halogen, $C_{1-4}$ alkyl, $C_{1-4}$ alkenyl, $C_{1-4}$ alkoxy, $C_{3-7}$ cycloalkyl, $C_{1-4}$ alkyl-OH, trihalo-$C_{1-4}$ alkyl, mono-$C_{1-4}$ alkylamino, di-$C_{1-4}$ alkylamino, -C(O)$NH_2$, -$NH_2$,-$NO_2$, hydroxy-$C_{1-4}$ alkylamino, hydroxy-$C_{1-4}$ alkyl, 4-7 membered heterocycle-$C_{1-4}$ alkyl, amino-$C_{1-4}$ alkyl, mono-$C_{1-4}$ alkylamino-$C_{1-4}$ alkyl and di-$C_{1-4}$ alkylamino-$C_{1-4}$ alkyl;

B is a group of the formula

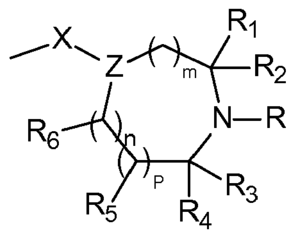

,

wherein

m, n and p are independently selected from 0 or 1;

R, $R_1$, $R_2$, $R_3$, and $R_4$ are independently selected from the group consisting of hydrogen and $C_{1-4}$ alkyl, wherein alkyl is optionally substituted with hydroxy, amino, mono-$C_{1-4}$ akylamino or di-$C_{1-4}$ akylamino;

$R_5$ and $R_6$ are independently selected from hydrogen and fluorine; or

R and $R_3$, taken in combination form a fused 5 or 6 membered heterocyclic ring having 0 or 1 additional ring heteroatoms selected from N, O or S; or

$R_1$ and $R_3$, taken in combination form a $C_{1-3}$ alkylene group; or

$R_1$ and $R_5$, taken in combination form a $C_{1-3}$ alkylene group; or

$R_3$ and $R_4$, taken in combination with the carbon atom to which they attach, form a spirocyclic $C_{3-6}$ cycloalkyl;

X is $CR_AR_B$, O, $NR_7$ or a bond;

$R_7$ is hydrogen, or $C_{1-4}$ alkyl;

$R_A$ and $R_B$ are independently selected from hydrogen and $C_{1-4}$ alkyl, or $R_A$ and $R_B$, taken in combination, form a divalent $C_{2-5}$ alkylene group;

Z is $CR_8$ or N; with the proviso that when Z is N, X is a bond;

$R_8$ is hydrogen or taken in combination with $R_6$ form a double bond; or

B is a group of the formula

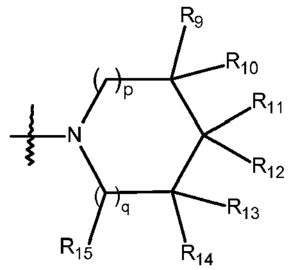

,

wherein

p and q are independently selected from the group consisting of 0, 1, and 2;

$R_9$ and $R_{13}$ are independently selected from hydrogen and $C_{1-4}$ alkyl;

$R_{10}$ and $R_{14}$ are independently selected from hydrogen, amino, mono-$C_{1-4}$ alkylamino, di-$C_{1-4}$ alkylamino, and $C_{1-4}$ alkyl optionally substituted with hydroxy, amino, mono-$C_{1-4}$ alkylamino or di-$C_{1-4}$ alkylamino;

$R_{11}$ is hydrogen, $C_{1-4}$ alkyl, amino, mono-$C_{1-4}$ alkylamino, or di-$C_{1-4}$ alkylamino;

$R_{12}$ is hydrogen or $C_{1-4}$ alkyl; or

$R_9$ and $R_{11}$ taken in combination form a saturated azacycle having 4 to 7 ring atoms which is optionally substituted with one to three $C_{1-4}$ alkyl groups; or

$R_{11}$ and $R_{12}$, taken in combination form a saturated azacycle having 4 to 7 ring atoms which is optionally substituted with one to three $C_{1-4}$ alkyl group;

or a pharmaceutically acceptable salt thereof;
for use in the treatment, prevention and/or delay of progression of cancer.

In a particular embodiment of the present invention, the FoxM1 gene splicing modifier is selected from a compound of formula (VI)

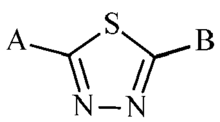

(VI),

wherein

A is 6 membered heteroaryl having 1-3 ring nitrogen atoms and which is substituted by phenyl or a heteroaryl having 5 or 6 ring atoms, 1 or 2 ring heteroatoms independently selected from N, O and S and is substituted with 0, 1, or 2 substituents independently selected from $C_{1-4}$ alkyl, mono-$C_{1-4}$ alkylamino, di-$C_{1-4}$alkylamino, hydroxy-$C_{1-4}$ alkylamino, hydroxy-$C_{1-4}$ alkyl, amino-$C_{1-4}$ alkyl and mono-$C_{1-4}$ alkylamino-$C_{1-4}$ alkyl, and di-$C_{1-4}$alkylamino-$C_{1-4}$ alkyl; or

A is bicyclic heteroaryl having 9 to 10 ring atoms, 1, 2, or 3 ring heteroatoms independently selected from N, O or S, and which is substituted with 0, 1, or 2 substituents independently selected from cyano, oxime, halogen, hydroxy, $C_{1-4}$ alkyl, $C_{2-4}$ alkenyl, $C_{2-4}$ alkynyl, $C_{1-4}$ alkoxy, $C_{1-4}$ alkoxy substituted with hydroxy, amino, mono-$C_{1-4}$ alkylamino, and di-$C_{1-4}$ alkylamino; or

A is phenyl which is substituted with 0, 1,2, or 3 substituents independently selected from $C_{1-4}$ alkyl, oxo, oxime, hydroxy, halo-$C_{1-4}$ alkyl, dihalo-$C_{1-4}$ alkyl, trihalo-$C_{1-4}$ alkyl, $C_{1-4}$ alkoxy, $C_{1-4}$ alkoxy-$C_{3-7}$ cycloalkyl, halo-$C_{1-4}$ alkoxy, dihalo-$C_{1-4}$ alkoxy, trihalo-$C_{1-4}$ alkoxy, cyano, halogen, amino, mono-$C_{1-4}$ alkylamino, di-$C_{1-4}$ alkylamino, heteroaryl, $C_{1-4}$ alkyl substituted with hydroxy, $C_{1-4}$ alkoxy substituted with aryl, -C(O)NH-$C_{1-4}$, alkyl- heteroaryl, -NHC(O)-$C_{1-4}$ alkylheteroaryl, $C_{1-4}$ alkyl-C(O)NH-heteroaryl, $C_{1-4}$ alkyl-NHC(O)-heteroaryl, $C_{3-7}$ cycloalkyl, 5-7 membered cycloalkenyl or 5, 6 or 9 membered heterocycle containing 1 or 2 heteroatoms, independently, selected from S, 0 and N; wherein two $C_{1-4}$ alkyl groups can combine with the atoms to which they are bound to form a 5-6 membered ring; wherein heteroaryl has 5, 6 or 9 ring atoms, 1, 2 or 3 ring heteroatoms selected from N, 0 and S and is substituted with 0, 1, or 2 substituents independently selected from oxo, hydroxy, nitro, halogen, $C_{1-4}$ alkyl, $C_{1-4}$ alkenyl, $C_{1-4}$ alkoxy, $C_{3-7}$ cycloalkyl, $C_{1-4}$ alkyl-OH, trihalo-$C_{1-4}$ alkyl, mono-$C_{1-4}$ alkylamino, di-$C_{1-4}$ alkylamino, -C(O)$NH_2$, -$NH_2$,-$NO_2$, hydroxy-$C_{1-4}$ alkylamino, hydroxy-$C_{1-4}$ alkyl, 4-7 memberered heterocycle-$C_{1-4}$ alkyl, amino-$C_{1-4}$ alkyl, mono-$C_{1-4}$ alkylamino-$C_{1-4}$ alkyl, and di-$C_{1-4}$ alkylamino-$C_{1-4}$ alkyl;

B is a group of the formula

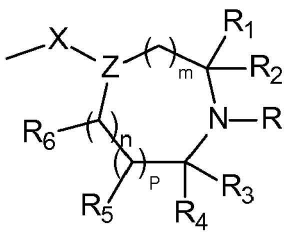

,

wherein

m, n and p are independently selected from 0 or 1;

R, $R_1$, $R_2$, $R_3$, and $R_4$ are independently selected from the group consisting of hydrogen and $C_{1-4}$ alkyl, wherein alkyl is optionally substituted with hydroxy, amino, mono-$C_{1-4}$ akylamino or di-$C_{1-4}$ akylamino;

$R_5$ and $R_6$ are independently selected from hydrogen and fluorine; or

R and $R_3$, taken in combination form a fused 5 or 6 membered heterocyclic ring having 0 or 1 additional ring heteroatoms selected from N, O or S; or

$R_1$ and $R_3$, taken in combination form a $C_{1-3}$ alkylene group; or

$R_1$ and $R_5$, taken in combination form a $C_{1-3}$ alkylene group; or

$R_3$ and $R_4$, taken in combination with the carbon atom to which they attach, form a spirocyclic $C_{3-6}$ cycloalkyl;

X is $CR_AR_B$, O, $NR_7$ or a bond;

$R_7$ is hydrogen, or $C_{1-4}$ alkyl;

$R_A$ and $R_B$ are independently selected from hydrogen and $C_{1-4}$ alkyl, or $R_A$ and $R_B$, taken in combination, form a divalent $C_{2-5}$ alkylene group;

Z is $CR_8$ or N; with the proviso that when Z is N, X is a bond;

$R_8$ is hydrogen or taken in combination with $R_6$ form a double bond; or

B is a group of the formula

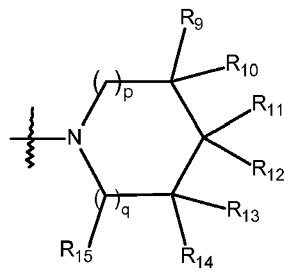

,

wherein

p and q are independently selected from the group consisting of 0, 1, and 2;

$R_9$ and $R_{13}$ are independently selected from hydrogen and $C_{1-4}$ alkyl;

$R_{10}$ and $R_{14}$ are independently selected from hydrogen, amino, mono-$C_{1-4}$ alkylamino, di-$C_{1-4}$ alkylamino, and $C_{1-4}$ alkyl optionally substituted with hydroxy, amino, mono-$C_{1-4}$ alkylamino or di-$C_{1-4}$ alkylamino;

$R_{11}$ is hydrogen, $C_{1-4}$ alkyl, amino, mono-$C_{1-4}$ alkylamino, or di-$C_{1-4}$ alkylamino;

$R_{12}$ is hydrogen or $C_{1-4}$ alkyl; or

$R_9$ and $R_{11}$ taken in combination form a saturated azacycle having 4 to 7 ring atoms which is optionally substituted with one to three $C_{1-4}$ alkyl groups; or

$R_{11}$ and $R_{12}$, taken in combination form a saturated azacycle having 4 to 7 ring atoms which is optionally substituted with one to three $C_{1-4}$ alkyl groups.

or a pharmaceutically acceptable salt thereof;
for use in the treatment, prevention and/or delay of progression of cancer.

In a particular embodiment, the present invention relates to a FoxM1 gene splicing modifier selected from a compound of formula (I) or of formula (VI), wherein

A is 2-hydroxy-phenyl which is substituted with:

0, 1,2, or 3 substituents independently selected from $C_{1-4}$ alkyl, oxo, oxime, hydroxy, halo-$C_{1-4}$ alkyl, dihalo-$C_{1-4}$ alkyl, trihalo-$C_{1-4}$ alkyl, $C_{1-4}$ alkoxy, $C_{1-4}$ alkoxy-$C_{3-7}$ cycloalkyl, halo-$C_{1-4}$ alkoxy, dihalo-$C_{1-4}$ alkoxy, trihalo-$C_{1-4}$ alkoxy, hydroxy, cyano, halogen, amino, mono-$C_{1-4}$ alkylamino, di-$C_{1-4}$ alkylamino, heteroaryl, $C_{1-4}$ alkyl substituted with hydroxy, $C_{1-4}$ alkoxy substituted with aryl, amino, -C(O)NH-$C_{1-4}$alkyl-heteroaryl, -NHC(O)-$C_{1-4}$ alkylheteroaryl, $C_{1-4}$ alkyl-C(O)NH-heteroaryl, $C_{1-4}$lkyl-NHC(O)-heteroaryl, $C_{3-7}$ cycloalkyl, 5-7 membered cycloalkenyl, or 5, 6 or 9 membered heterocycle containing 1 or 2 heteroatoms independently, selected from S, O and N,
wherein two $C_{1-4}$ alkyl groups can combine with the atoms to which they are bound to form a 5-6 membered ring; wherein heteroaryl has 5, 6 or 9 ring atoms, 1, 2 or 3 ring heteroatoms selected from N, O and S, and is substituted with 0, 1, or 2 substituents independently selected from oxo, hydroxy, nitro, halogen, $C_{1-4}$ alkyl, $C_{1-4}$ alkenyl, $C_{1-4}$ alkoxy, $C_{3-7}$ cycloalkyl, $C_{1-4}$ alkyl-OH, trihalo-$C_{1-4}$ alkyl, mono-$C_{1-4}$ alkylamino, di-$C_{1-4}$ alkylamino, -C(O)$NH_2$, -$NH_2$, $NO_2$, hydroxy-$C_{1-4}$alkylamino, hydroxy-$C_{1-4}$ alkyl, 4-7 membered heterocycle-$C_{1-4}$ alkyl, amino-$C_{1-4}$ alkyl, mono-$C_{1-4}$alkylamino-$C_{1-4}$ alkyl, and di-$C_{1-4}$alkylamino-$C_{1-4}$ alkyl; or

A is 2-naphthyl optionally substituted at the 3 position with hydroxy and additionally substituted with 0, 1, or 2 substituents selected from hydroxy, cyano, halogen, $C_{1-4}$ alkyl, $C_{2-4}$ alkenyl, $C_{1-5}$ alkoxy, wherein the alkoxy is unsubstituted or substituted with hydroxy, $C_{1-4}$ alkoxy, amino, -NHC(O)-$C_{1-4}$ alkyl, -NHC(O)O-$C_{1-4}$ alkyl, $C_{1-4}$ alkylene-4-7 membered heterocycle, 4-7 membered heterocycle, mono-$C_{1-4}$ alkylamino, and di- $C_{1-4}$ alkylamino; or

A is phenyl which is substituted with 0, 1,2, or 3 substituents independently selected from $C_{1-4}$ alkyl, oxo, oxime, hydroxy, halo-$C_{1-4}$ alkyl, dihalo-$C_{1-4}$ alkyl, trihalo-$C_{1-4}$ alkyl, $C_{1-4}$ alkoxy, $C_{1-4}$ alkoxy-$C_{3-7}$ cycloalkyl, halo-$C_{1-4}$ alkoxy, dihalo-$C_{1-4}$ alkoxy, trihalo-$C_{1-4}$ alkoxy, cyano, halogen, amino, mono-$C_{1-4}$ alkylamino, di-$C_{1-4}$ alkylamino, heteroaryl, $C_{1-4}$ alkyl substituted with hydroxy, $C_{1-4}$ alkoxy substituted with aryl, -C(O)NH-$C_{1-4}$, alkyl- heteroaryl, -NHC(O)-$C_{1-4}$ alkylheteroaryl, $C_{1-4}$ alkyl-C(O)NH-heteroaryl, $C_{1-4}$ alkyl-NHC(O)-heteroaryl, $C_{3-7}$ cycloalkyl, 5-7 membered cycloalkenyl or 5, 6 or 9 membered heterocycle containing 1 or 2 heteroatoms, independently, selected from S, 0 and N; wherein two $C_{1-4}$ alkyl groups can combine with the atoms to which they are bound to form a 5-6 membered ring; wherein heteroaryl has 5, 6 or 9 ring atoms, 1, 2 or 3 ring heteroatoms selected from N, 0 and S and is substituted with 0, 1, or 2 substituents independently selected from oxo, hydroxy, nitro, halogen, $C_{1-4}$ alkyl, $C_{1-4}$ alkenyl, $C_{1-4}$ alkoxy, $C_{3-7}$ cycloalkyl, $C_{1-4}$ alkyl-OH, trihalo-$C_{1-4}$ alkyl, mono-$C_{1-4}$ alkylamino, di-$C_{1-4}$ alkylamino, -C(O)$NH_2$, -$NH_2$,-$NO_2$, hydroxy-$C_{1-4}$ alkylamino, hydroxy-$C_{1-4}$ alkyl, 4-7 memberered heterocycle-$C_{1-4}$ alkyl, amino-$C_{1-4}$ alkyl, mono-$C_{1-4}$ alkylamino-$C_{1-4}$ alkyl, and di-$C_{1-4}$ alkylamino-$C_{1-4}$ alkyl;

or a pharmaceutically acceptable salt thereof;
for use in the treatment, prevention and/or delay of progression of cancer.

In a particular embodiment, the present invention relates to a FoxM1 gene splicing modifier selected from a compound of formula (I) or a compound of formula (VI), wherein A is 2-hydroxy-phenyl substituted with 0, 1,2, or 3 substituents independently selected from $C_{1-4}$ alkyl, halo-$C_{1-4}$ alkyl, $C_{1-4}$ alkoxy, hydroxy, cyano, halogen, amino, mono-$C_{1-4}$ alkylamino, di-$C_{1-4}$ alkylamino, heteroaryl, and $C_{1-4}$ alkyl substituted with hydroxy or amino, wherein heteroaryl has 5 or 6 ring atoms, 1 or 2 ring heteroatoms selected from N, 0 and S and is substituted with 0, 1, or 2 substituents independently selected from $C_{1-4}$ alkyl, mono-$C_{1-4}$ alkylamino, di-$C_{1-4}$ alkylamino, hydroxy-$C_{1-4}$ alkylamino, hydroxy-$C_{1-4}$ alkyl, 4-7 membered heterocycle-$C_{1-4}$ alkyl, amino-$C_{1-4}$ alkyl, mono- $C_{1-4}$ alkylamino-$C_{1-4}$ alkyl, and di-$C_{1-4}$ alkylamino-$C_{1-4}$ alkyl; or a pharmaceutically acceptable salt thereof; for use in the treatment, prevention and/or delay of progression of cancer.

In a particular embodiment, the present invention relates to a FoxM1 gene splicing modifier selected from a compound of formula (I) or a compound of formula (VI), wherein A is 2-naphthyl optionally substituted at the 3 position with hydroxy and additionally substituted with 0, 1, or 2 substituents selected from hydroxy, cyano, halogen, $C_{1-4}$ alkyl, $C_{2-4}$ alkenyl, $C_{1-4}$ alkoxy, wherein alkoxy is unsubstituted or substituted with hydroxy, $C_{1-4}$ alkoxy, amino, -N(H)C(O)-$C_{1-4}$ alkyl, -N(H)C(O)O-$C_{1-4}$ alkyl, 4 to 7 membered heterocycle, mono-$C_{1-4}$ alkylamino and di-$C_{1-4}$ alkylamino; or a pharmaceutically acceptable salt thereof; for use in the treatment, prevention and/or delay of progression of cancer.

In a particular embodiment, the present invention relates to a FoxM1 gene splicing modifier selected from a compound of formula (I) or a compound of formula (VI), wherein A is phenyl substituted with 0, 1,2, or 3 substituents independently selected from $C_{1-4}$ alkyl, halo-$C_{1-4}$ alkyl, $C_{1-4}$ alkoxy, hydroxy, cyano, halogen, amino, mono-$C_{1-4}$ alkylamino, di-$C_{1-4}$ alkylamino, heteroaryl, and $C_{1-4}$ alkyl substituted with hydroxy or amino, wherein heteroaryl has 5 or 6 ring atoms, 1 or 2 ring heteroatoms selected from N, 0 and S and is substituted with 0, 1, or 2 substituents independently selected from $C_{1-4}$ alkyl, mono-$C_{1-4}$ alkylamino, di-$C_{1-4}$ alkylamino, hydroxy-$C_{1-4}$ alkylamino, hydroxy-$C_{1-4}$ alkyl, 4-7 membered heterocycle-$C_{1-4}$ alkyl, amino-$C_{1-4}$ alkyl, mono- $C_{1-4}$ alkylamino-$C_{1-4}$ alkyl, and di-$C_{1-4}$ alkylamino-$C_{1-4}$ alkyl; or a pharmaceutically acceptable salt thereof; for use in the treatment, prevention and/or delay of progression of cancer.

In a particular embodiment, the present invention relates to a FoxM1 gene splicing modifier selected from a compound of formula (I) or a compound of formula (VI), wherein
A is 2-hydroxy-phenyl substituted with one additional substituent selected from cyano and heteroaryl; or
A is 2-naphthyl optionally substituted at the 3 position with hydroxy and additionally substituted with 0 or 1 substituents selected from hydroxy and $C_{1-4}$ alkoxy; or
A is phenyl which is substituted with two or three substituents independently selected from halogen and heteroaryl; wherein heteroaryl has 5 or 6 ring atoms of which 1 or 2 are nitrogen and is substituted with 0, 1, or 2 substituents independently selected from $C_{1-4}$ alkyl and hydroxy;
or a pharmaceutically acceptable salt thereof;
for use in the treatment, prevention and/or delay of progression of cancer.

In a particular embodiment, the present invention relates to a FoxM1 gene splicing modifier selected from a compound of formula (I) or a compound of formula (VI), wherein
A is 2-hydroxy-phenyl substituted with one additional substituent selected from cyano and heteroaryl; or
A is 2-naphthyl optionally substituted at the 3 position with hydroxy and additionally substituted with 0 or 1 substituents selected from hydroxy and methoxy; or
A is phenyl which is substituted with two or three substituents independently selected from chloro, fluoro and heteroaryl; wherein heteroaryl is pyrazolyl or pyridinyl substituted with 0, 1, or 2 substituents independently selected from methyl and hydroxy;
or a pharmaceutically acceptable salt thereof;
for use in the treatment, prevention and/or delay of progression of cancer.

In a particular embodiment, the present invention relates to a FoxM1 gene splicing modifier selected from a compound of formula (I) or a compound of formula (VI), wherein B is a group of the formula

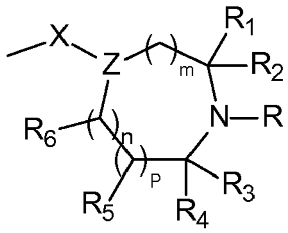

,

wherein m, n and p are independently selected from 0 or 1; R, $R_1$, $R_2$, $R_3$, and $R_4$ are independently selected from the group consisting of hydrogen, and $C_{1-4}$ alkyl, which alkyl is optionally substituted with hydroxy, amino, mono-$C_{1-4}$ alkylamino or di-$C_{1-4}$ alkylamino; $R_5$ and $R_6$ are hydrogen; or R and $R_3$ taken in combination form a fused 5 or 6 membered heterocyclic ring having 0 or 1 additional ring heteroatoms selected from N, O or S; $R_1$ and $R_3$, taken in combination form a $C_{1-3}$ alkylene group; $R_1$ and $R_5$ taken in combination form a $C_{1-3}$ alkylene group; $R_3$ and $R_4$ taken in combination with the carbon atom to which they attach, form a spirocyclic $C_{3-6}$ cycloalkyl; X is $CR_AR_B$, O, $NR_7$ or a bond; $R_A$ and $R_B$ are independently selected from hydrogen and $C_{1-4}$ alkyl, or $R_A$ and $R_B$ taken in combination, form a divalent $C_{2-5}$ alkylene group; Z is $CR_8$ or N; when Z is N, X is a bond; $R_8$ is hydrogen or taken in combination with $R_6$ form a double bond; or a pharmaceutically acceptable salt thereof;
for use in the treatment, prevention and/or delay of progression of cancer.

In a particular embodiment, the present invention relates to a FoxM1 gene splicing modifier selected from a compound of formula (I) or a compound of formula (VI), wherein B is a group of the formula

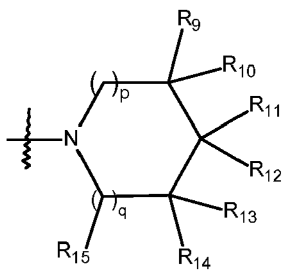

,

wherein p and q are independently selected from the group consisting of 0, 1, and 2; $R_9$ and $R_1$; are independently selected from hydrogen and $C_{1-4}$ alkyl; $R_{10}$ and $R_{14}$ are independently selected from hydrogen, amino, mono-$C_{1-4}$ alkylamino, di-$C_{1-4}$ alkylamino and $C_{1-4}$ alkyl, which alkyl is optionally substituted with hydroxy, amino, mono-$C_{1-4}$ alkylamino or di-$C_{1-4}$ alkylamino; $R_{11}$ is hydrogen, $C_{1-4}$ alkyl, amino, mono-$C_{1-4}$ alkylamino or di-$C_{1-4}$ alkylamino; $R_{12}$ is hydrogen or $C_{1-4}$ alkyl; or $R_9$ and $R_{11}$ taken in combination form a saturated azacycle having 4 to 7 ring atoms which is optionally substituted with one to three $C_{1-4}$ alkyl groups; or $R_{11}$ and $R_{12}$ taken in combination form a saturated azacycle having 4 to 7 ring atoms which is optionally substituted with one to three $C_{1-4}$ alkyl groups;
or a pharmaceutically acceptable salt thereof;
for use in the treatment, prevention and/or delay of progression of cancer.

In a particular embodiment, the present invention relates to a FoxM1 gene splicing modifier selected from a compound of formula (I) or a compound of formula (VI), wherein B is selected from the group consisting of

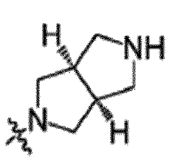 , 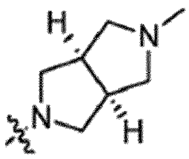 , 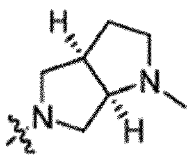 , 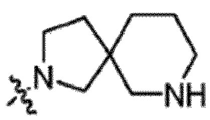 ,

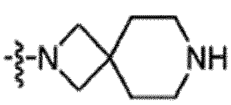 , 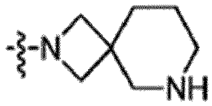 , 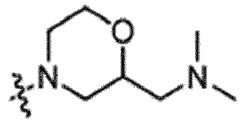 ,

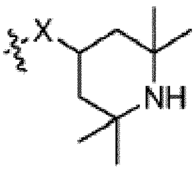 , 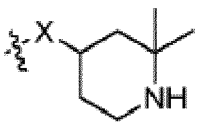 , 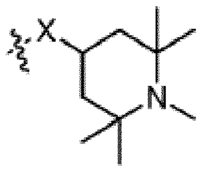 , 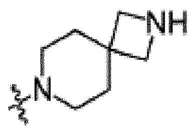 ,

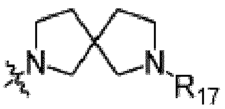 , 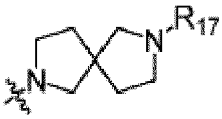 , 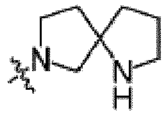 , 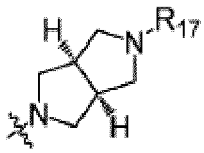 ,

and

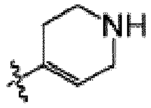

wherein X is O or -N($CH_3$)-; and $R_{17}$ is hydrogen or methyl;
or a pharmaceutically acceptable salt thereof;
for use in the treatment, prevention and/or delay of progression of cancer.

In a particular embodiment, the present invention relates to a FoxM1 gene splicing modifier selected from a compound of formula (I) or a compound of formula (VI), wherein B is

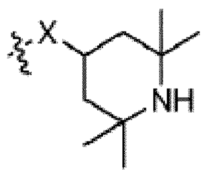

and X is O or -N($CH_3$)-; or a pharmaceutically acceptable salt thereof; for use in the treatment, prevention and/or delay of progression of cancer.

In a particular embodiment, the present invention relates to a FoxM1 gene splicing modifier selected from a compound of formula (I) or a compound of formula (VI), wherein X is O; or a pharmaceutically acceptable salt thereof; for use in the treatment, prevention and/or delay of progression of cancer.

In a particular embodiment, the present invention relates to a FoxM1 gene splicing modifier selected from a compound of formula (I) or a compound of formula (VI), wherein X is -N($CH_3$)-; or a pharmaceutically acceptable salt thereof;
for use in the treatment, prevention and/or delay of progression of cancer.

In a particular embodiment, the present invention relates to a FoxM1 gene splicing modifier selected from a compound of formula (I) or a compound of formula (VI), wherein B is

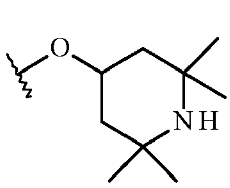
or
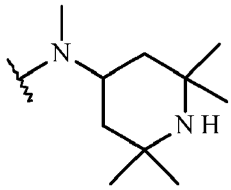
;

or a pharmaceutically acceptable salt thereof; for use in the treatment, prevention and/or delay of progression of cancer.

In a particular embodiment, the present invention relates to a FoxM1 gene splicing modifier selected from a compound of formula (I) or a compound of formula (VI), wherein B is

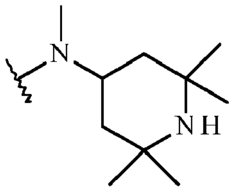
;

or a pharmaceutically acceptable salt thereof; for use in the treatment, prevention and/or delay of progression of cancer.

In a particular embodiment, the present invention relates to a FoxM1 gene splicing modifier selected from a compound of formula (I) or a compound of formula (VI), wherein B is

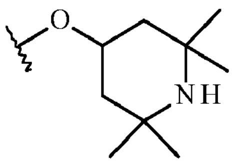
;

or a pharmaceutically acceptable salt thereof;
for use in the treatment, prevention and/or delay of progression of cancer.

In a particular embodiment, the present invention relates to a FoxM1 gene splicing modifier selected from a compound of formula (II):

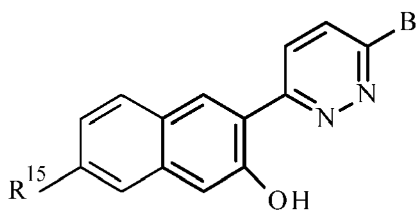
(II),

wherein $R^{15}$ is hydrogen, hydroxy, or $C_{1-4}$ alkoxy, wherein alkoxy is optionally substituted with hydroxy, methoxy, amino, mono-methylamino, dimethylamino or morpholine; or a pharmaceutically acceptable salt thereof; for use in the treatment, prevention and/or delay of progression of cancer.

In a particular embodiment, the present invention relates to a FoxM1 gene splicing modifier selected from a compound of formula (II), wherein $R^{15}$ is hydrogen, hydroxy or methoxy; or a pharmaceutically acceptable salt thereof; for use in the treatment, prevention and/or delay of progression of cancer.

In a particular embodiment, the present invention relates to a FoxM1 gene splicing modifier selected from a compound of formula (III):

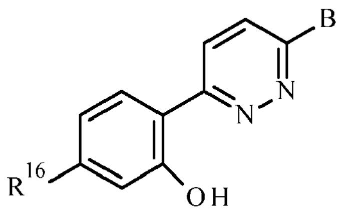

(III),

wherein $R^{16}$ is cyano, 5-membered heteroaryl having two ring nitrogen atoms, or 6-membered heteroaryl having one ring nitrogen atom; wherein the 5-membered heteroaryl is optionally substituted with $C_{1-4}$ alkyl; wherein the 6-membered heteroaryl is optionally substituted with one or two substituents selected from $C_{1-4}$ alkyl and hydroxy; or a pharmaceutically acceptable salt thereof; for use in the treatment, prevention and/or delay of progression of cancer.

In a particular embodiment, the present invention relates to a FoxM1 gene splicing modifier selected from a compound of formula (III), wherein $R^{16}$ is cyano, pyrazolyl or pyridinyl, wherein pyrazolyl is optionally substituted with methyl and wherein pyridinyl is optionally substituted with one or two substituents selected from methyl and hydroxy; or a pharmaceutically acceptable salt thereof; for use in the treatment, prevention and/or delay of progression of cancer.

In a particular embodiment, the present invention relates to a FoxM1 gene splicing modifier selected from a compound of formula (III), wherein $R^{16}$ is

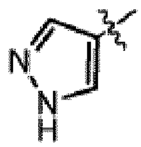

;

or a pharmaceutically acceptable salt thereof; for use in the treatment, prevention and/or delay of progression of cancer.

In a particular embodiment, the present invention relates to a FoxM1 gene splicing modifier selected from a compound of formula (VII):

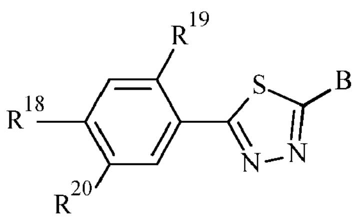

(VII),

wherein $R^{18}$ is 5-membered heteroaryl having two ring nitrogen atoms or 6-membered heteroaryl having one ring nitrogen atom; wherein the 5-membered heteroaryl is optionally substituted with $C_{1-4}$ alkyl; wherein the 6-membered heteroaryl is optionally substituted with one or two substituents selected from $C_{1-4}$ alkyl and hydroxy; $R^{19}$ is hydrogen or halogen; and $R^{20}$ is hydrogen or halogen; or a pharmaceutically acceptable salt thereof; for use in the treatment, prevention and/or delay of progression of cancer.

In a particular embodiment, the present invention relates to a FoxM1 gene splicing modifier selected from a compound of formula (VII), wherein $R^{18}$ is pyrazolyl optionally substituted with methyl, or pyridinyl substituted with methyl and hydroxy; $R^{19}$ is hydrogen, chloro or fluoro; and $R^{20}$ is hydrogen, chloro or fluoro; or a pharmaceutically acceptable salt thereof; for use in the treatment, prevention and/or delay of progression of cancer.

In a particular embodiment, the present invention relates to a FoxM1 gene splicing modifier selected from a compound of formula (IV) or of formula (V) or of formula (VIII):

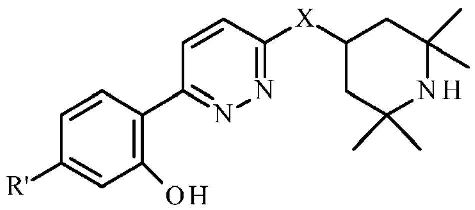

(IV)

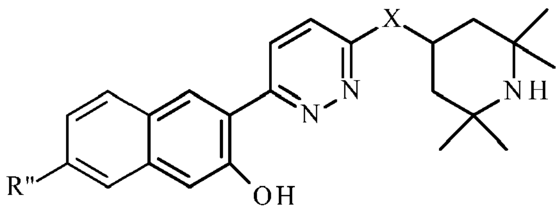

(V)

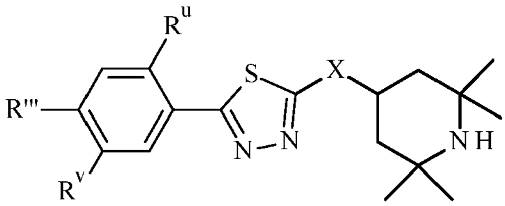

(VIII),

wherein

X is -O- or $-N(CH_3)-$; R' is cyano, pyrazolyl optionally substituted with methyl, or pyridinyl substituted with methyl and hydroxy; R" is hydrogen, methyl or methoxy; R'" is pyrazolyl optionally substituted with methyl, or pyridinyl substituted with methyl and hydroxy; $R^u$ is hydrogen, chloro or fluoro; $R^v$ is hydrogen, chloro or fluoro; or a pharmaceutically acceptable salt thereof; for use in the treatment, prevention and/or delay of progression of cancer.

In a particular embodiment, the present invention relates to a FoxM1 gene splicing modifier selected from a compound of formula (IV), wherein X is -O- or $-N(CH_3)-$; R' is cyano, pyrazolyl optionally substituted with methyl, or pyridinyl substituted with methyl and hydroxy; or a pharmaceutically acceptable salt thereof; for use in the treatment, prevention and/or delay of progression of cancer.

In a particular embodiment, the present invention relates to a FoxM1 gene splicing modifier selected from a compound of formula (V), wherein X is -O- or $-N(CH_3)-$; R" is hydrogen, methyl or methoxy; or a pharmaceutically acceptable salt thereof; for use in the treatment, prevention and/or delay of progression of cancer.

In a particular embodiment, the present invention relates to a FoxM1 gene splicing modifier selected from a compound of formula (VIII), wherein X is -O- or $-N(CH_3)-$; R"' is pyrazolyl optionally substituted with methyl, or pyridinyl substituted with methyl and hydroxy; $R^u$ is hydrogen, chloro or fluoro; $R^v$ is hydrogen, chloro or fluoro; or a pharmaceutically acceptable salt thereof; for use in the treatment, prevention and/or delay of progression of cancer.

In a particular embodiment, the present invention relates to a FoxM1 gene splicing modifier as described herein selected from the group consisting of:

6-(naphthalen-2-yl)-N-(2,2,6,6-tetramethylpiperidin-4-yl)pyridazin-3-amine;
6-(benzo[b]thio-phen-2-yl)-N-methyl-N-(2,2,6,6-tetra-methylpiperidin-4-yl)pyridazin-3-amine;
2-(6-(2,2,6,6-tetra methylpiperidin-4-ylamino)-pyridazin-3-yl)phenol;
2-(6-(methyl-(2,2,6,6-tetra-methylpiperidin-4-yl)amino)pyridazin-3-yl)benzo[b]-thiophene-5-carbonitrile;
6-(quinolin-3-yl)-N-(2,2,6,6-tetramethyl-piperidin-4-yl)pyridazin-3-amine;
3-(benzo[b]-thiophen-2-yl)-6-(2,2,6,6-tetra-methylpiperidin-4-yloxy)pyridazine;
2-(6-(methyl-(2,2,6,6-tetra-methylpiperidin-4-yl)amino)-pyridazin-3-yl)phenol;
6-(6-(methyl-(2,2,6,6-tetra-methylpiperidin-4-yl)amino)-pyridazin-3-yl)naphthalen-2-ol;
6-(benzo[b]-thiophen-2-yl)-N-(2,2,6,6-tetra-methylpiperidin-4-yl)pyridazin-3-amine;
7-(6-((2,2,6,6-tetramethylpiperidin-4-yl)oxy)pyridazin-3-yl)isoquinoline;
6-(6-((2,2,6,6-tetramethylpiperidin-4-yl)oxy)pyridazin-3-yl)isoquinoline;
N-methyl-6-(quinolin-7-yl)-N-(2,2,6,6-tetramethyl-piperidin-4-yl)pyridazin-3-amine;
N-methyl-6-(quinolin-6-yl)-N-(2,2,6,6-tetramethylpiperidin-4-yl)pyridazin-3-amine;
6-(isoquinolin-7-yl)-N-methyl-N-(2,2,6,6-tetramethylpiperidin-4-yl)pyridazin-3-amine;
6-(isoquinolin-6-yl)-N-methyl-N-(2,2,6,6-tetramethylpiperidin-4-yl)pyridazin-3-amine;
6-(imidazo[1,2-a]pyridin-6-yl-pyridazin-3-yl)-methyl-(2,2,6,6-tetramethyl-piperidin-4-yl)-amine methyl-[6-(6-phenyl-pyridin-3-yl)-pyridazin-3-yl]-(2,2,6,6-tetramethyl-piperidin-4-yl)-amine;
methyl-[6-(6-pyrrol-1-yl-pyridin-3-yl)-pyridazin-3-yl]-(2,2,6,6-tetramethyl-piperidin-4-yl)-amine;
methyl-(6-quinoxalin-2-yl-pyridazin-3-yl)-(2,2,6,6-tetramethyl-piperidin-4-yl)-amine;
methyl-(6-quinolin-3-yl-pyridazin-3-yl)-(2,2,6,6-tetramethyl-piperidin-4-yl)-amine;
N-methyl-6-(phthalazin-6-yl)-N-(2,2,6,6-tetramethylpiperidin-4-yl)pyridazin-3-amine;

6-(benzo[c][1,2,5]oxa-diazol-5-yl)-N-(2,2,6,6-tetramethyl-piperidin-4-yl)pyridazin-3-amine;
6-(benzo[d]thiazol-5-yl)-N-(2,2,6,6-tetramethyl-piperidin-4-yl)pyridazin-3-amine;
6-(2-methylbenzo-[d]oxazol-6-yl)-N-(2,2,6,6-tetramethyl-piperidin-4-yl)pyridazin-3-amine;
3-(6-(methyl(2,2,6,6-tetramethylpiperidin-4-yl)amino)pyridazin-3-yl)naphthalen-2-ol;
5-chloro-2-(6-(methyl(1,2,2,6,6-pentamethylpiperidin-4-yl)amino)pyridazin-3-yl)phenol;
3-(6-(2,2,6,6-tetramethylpiperidin-4-ylamino)pyridazin-3-yl)naphthalen-2-ol;
5-chloro-2-(6-(1,2,2,6,6-pentamethylpiperidin-4-ylamino)pyridazin-3-yl)phenol;
4-hydroxy-3-(6-(methyl(2,2,6,6-tetramethylpiperidin-4-yl)amino)pyridazin-3-yl)benzonitrile;
3-[6-(2,2,6,6-tetramethyl-piperidin-4-yloxy)-pyridazin-3-yl]-naphthalen-2-ol;
2-{6-[methyl-(2,2,6,6-tetramethyl-piperidin-4-yl)-amino]-pyridazin-3-yl}-4-trifluoromethylphenol;
2-fluoro-6-{6-[methyl-(2,2,6,6-tetramethyl-piperidin-4-yl)-amino]-pyridazin-3-yl}-phenol;
3,5-dimethoxy-2-{6-[methyl-(2,2,6,6-tetramethyl-piperidin-4-yl)-amino]-pyridazin-3-yl}-phenol;
4,5-dimethoxy-2-{6-[methyl-(2,2,6,6-tetramethyl-piperidin-4-yl)-amino]-pyridazin-3-yl}-phenol;
5-methoxy-2-{6-[methyl-(2,2,6,6-tetramethyl-piperidin-4-yl)-amino]-pyridazin-3-yl}-phenol;
4,5-difluoro-2-{6-[methyl-(2,2,6,6-tetramethyl-piperidin-4-yl)-amino]-pyridazin-3-yl}-phenol;
5-fluoro-2-{6-[methyl-(2,2,6,6-tetramethyl-piperidin-4-yl)-amino]-pyridazin-3-yl}-phenol;
3-hydroxy-4-(6-(methyl(2,2,6,6-tetramethylpiperidin-4-yl)amino)pyridazin-3-yl)benzonitrile;
1-allyl-6-(6-(methyl(2,2,6,6-tetramethylpiperidin-4-yl)amino)pyridazin-3-yl)naphthalen-2-ol;
6-(benzo[b]thiophen-2-yl)-N-(1,2,2,6,6-pentamethylpiperidin-4-yl)pyridazin-3-amine;
N-allyl-3-hydroxy-4-(6-(methyl(2,2,6,6-tetramethylpiperidin-4-yl)amino)pyridazin-3-yl)benzamide;
2-(6-(methyl(2,2,6,6-tetramethylpiperidin-4-yl)amino)pyridazin-3-yl)-5-(1H-pyrazol-1-yl)phenol;
5-(5-methyl-oxazol-2-yl)-2-{6-[methyl-(2,2,6,6-tetramethyl-piperidin-4-yl)-amino]-pyridazin-3-yl}-phenol;
5-(4-hydroxymethyl)-1H-pyrazole-1-yl)-2-(6-(methyl(2,2,6,6-tetramethylpiperidin-4 yl)amino)pyridazin-3-yl)phenol;
5-(1H-imidazole-1-yl)-2-(6-(methyl(2,2,6,6-tetramethyl-piperidin-4-yl)amino)pyridazin-3-yl)phenol;
5-(4-amino-1H-pyrazole-1-yl)-2-(6-(methyl(2,2,6,6-tetramethylpiperidin-4-yl)amino)pyridazin-3-yl)phenol;
5-(4-amino-1H-pyrazol-1-yl)-2-(6-(methyl(2,2,6,6-tetramethylpiperidin-4-yl)amino)pyridazin-3-yl)phenol;
5-(3-amino-pyrazol-1-yl)-2-{6-[methyl-(2,2,6,6-tetramethyl-piperidin-4-yl)-amino]-pyridazin-3-yl}-phenol;
2-(6-(methyl-(2,2,6,6-tetramethylpiperidin-4-yl)amino)pyridazin-3-yl)-5-(1-(2-morpholinoethyl)-1H-pyrazol-4-yl)phenol;
2-(6-(methyl-(2,2,6,6-tetramethylpiperidin-4-yl)amino)pyridazin-3-yl)-5-(1-methyl-1H-pyrazol-4-yl)phenol;
5-(5-amino-1H-pyrazol-1-yl)-2-(6-(methyl-(2,2,6,6-tetramethyl-piperidin-4-yl) amino)pyridazin-3-yl)phenol;
2-(6-(methyl-(2,2,6,6-tetramethylpiperidin-4-yl)amino)pyridazin-3-yl)-4-(1H-pyrazol-1-yl)phenol;
2-{6-[(2-hydroxy-ethyl)-(2,2,6,6-tetramethyl-piperidn-4-yl)-amino]-pyridazin-3-yl}-5-pyrazol-1-yl-phenol;
2-(6-(piperidin-4-yloxy)pyridazin-3-yl)-5-(1H-pyrazol-1-yl)phenol;
2-(6-(((2S,4R,6R)-2,6-dimethylpiperidin-4-yl)oxy)pyridazin-3-yl)-5-(1H-pyrazol-1-yl)phenol;
5 2-(6-((2,6-di-methylpiperidin-4-yl)oxy)pyridazin-3-yl)-5-(1H-pyrazol-1-yl)phenol;
2-(6-((2,6-di-methylpiperidin-4-yl)oxy)pyridazin-3-yl)-5-(1H-pyrazol-1-yl)phenol;
5-(1H-pyrazol-1-yl)-2-(6-(pyrrolidin-3-yloxy)pyridazin-3-yl)phenol;
2-(6-((-2-methylpiperidin-4-yl)oxy)pyridazin-3-yl)-5-(1H-pyrazol-1-yl)phenol;
(S)-5-(1H-pyrazol-1-yl)-2-(6-(pyrrolidin-3-ylmethoxy)pyridazin-3-yl)phenol;
(R)-5-(1H-pyrazol-1-yl)-2-(6-(pyrrolidin-3-yl methoxy)pyridazin-3-yl)phenol;
2-(6-((3-fluoropiperidin-4-yl)oxy)pyridazin-3-yl)-5-(1H-pyrazol-1-yl)-phenol;
2-[6-(1,2,2,6,6-pentamethyl-piperidin-4-yloxy)-pyridazin-3-yl]-5-pyrazol-1-yl-phenol;
5-pyrazol-1-yl-2-[6-((2,2,6,6-tetramethylpiperidin-4-yloxy)-pyridazin-3-yl]-phenol;
5-(1H-pyrazol-4-yl)-2-(6-((2,2,6,6-tetramethylpiperidin-4-yl)oxy)pyridazin-3-yl)phenol;
2-(6-piperazin-1-yl-pyridazin-3-yl)-5-pyrazol-1-yl-phenol;
3-[6-(azetidin-3-yl-amino)-pyridazin-3-yl]-naphthalen-2-ol;
2-[6-(azetidin-3-ylamino)-pyridazin-3-yl]-5-pyrazol-1-yl-phenol;
2-[6-(3, 5-dimethyl-piperazin-1-yl)-pyridazin-3-yl]-5-pyrazol-1-yl-phenol;
2-[6-(7-methyl-2,7-diaza-spiro[4.4]non-2-yl)-pyridazin-3-yl]-5-pyrazol-1-yl-phenol;
2-(6-[1,4]diazepan-1-yl-pyridazin-3-yl)-5-pyrazol-1-yl-phenol;
2-{6-[4-(2-hydroxy-ethyl)-piperazin-1-yl]-pyridazin-3-yl}-5-pyrazol-1-yl-phenol;
2-[6-(3,6-diaza-bicyclo[3.2.1]oct-3-yl)-pyridazin-3-yl]-5-pyrazol-1-yl-phenol;
2-[6-(2, 7-diaza-spiro[3.5]non-7-yl)-pyridazin-3-yl]-5-pyrazol-1-yl-phenol;
2-[6-(3-hydroxy-methyl-piperazin-1-yl)-pyridazin-3-yl]-5-pyrazol-1-yl-phenol;
2-[6-(1,7-diaza-spiro[4.4]non-7-yl)-pyridazin-3-yl]-5-pyrazol-1-yl-phenol;
2-[6-(4-amino-4-methyl-piperidin-1-yl)-pyridazin-3-yl]-5-pyrazol-1-yl-phenol;
2-[6-(3-dimethyl-amino-piperidin-1-yl)-pyridazin-3-yl]-5-pyrazol-1-yl-phenol;

2-[6-(12,2,6,6-pentamethyl-piperidin-4-ylamino)-pyridazin-3-yl]-5-pyrazol-1-yl-phenol;
2-[6-(3,3-dimethyl-piperazin-1-yl)-pyridazin-3-yl]-5-pyrazol-1-yl-phenol;
2-(6-(7-(2-hydroxyethyl)-2,7-diazaspiro[4.4]-nonan-2-yl)pyridazin-3-yl)-5-(1H-pyrazol-1-yl)phenol;
2-(6-((3aR,6aS)-hexahydropyrrolo[3,4-c]pyrrol-2(1H)-yl)pyridazin-3-yl)-5-(1H-pyrazol-1-yl)phenol;
3-(6-(piperazin-1-yl)pyridazin-3-yl)naphthalene-2,7-diol;
5-pyrazol-1-yl-2-[6-(1,2,3,6-tetrahydro-pyridin-4-yl)-pyridazin-3-yl]-phenol;
2-(6-piperidin-4-yl-pyridazin-3-yl)-5-pyrazol-1-yl-phenol;
3-(6-(1,2,3,6-tetra-hydropyridin-4-yl)pyridazin-3-yl)naphthalen-2-ol;
3-(6-(1,2,3,6-tetrahydropyridin-4-yl)pyridazin-3-yl)naphthalene-2,7-diol;
3-(6-(2,2,6,6-tetramethyl-1,2,3,6-tetrahydropyridin-4-yl)pyridazin-3-yl)naphthalene-2,7-diol;
3-(6-(1-methyl-1,2,3,6-tetrahydropyridin-4-yl)pyridazin-3-yl)naphthalene-2,7-diol;
3-(6-(piperidin-4-yl)pyridazin-3-yl)naphthalene-2,7-diol;
3-(6-((2,2,6,6-tetramethylpiperidin-4-yl)oxy)pyridazin-3-yl)naphthalene-2,7-diol;
3-(6-(methyl-(2,2,6,6-tetramethylpiperidin-4-yl)amino)pyridazin-3-yl)naphthalene-2,7-diol;
3-(6-((2,2,6,6-tetramethylpiperidin-4-yl)amino)pyridazin-3-yl)naphthalene-2,7-diol;
[3-(7-hydroxy-6-{6-[methyl-(2,2,6,6-tetramethyl-piperidin-4-yl)-amino]-pyridazin-3-yl}-naphthalen-2-yloxy)-propyl]-carbamic acid tert-butyl ester;
7-(3-amino-propoxy)-3-{6-[methyl-(2,2,6,6-tetramethyl-piperidin-4-yl)-amino]-pyridazin-3-yl}naphthalen-2-ol;
N-[3-(7-hydroxy-6-{6-[methyl-(2,2,6,6-tetramethyl-piperidin-4-yl)-amino]-pyridazin-3-yl}naphthalen-2-yloxy)-propyl]-acetamide;
7-(3-hydroxypropoxy)-3-(6-(methyl(2,2,6,6-tetramethylpiperidin-4-yl)amino)pyridazin-3-yl)naphthalen-2-ol;
7-(3-methoxypropoxy)-3-(6-(methyl(2,2,6,6-tetramethylpiperidin-4-yl)amino)pyridazin-3-yl)naphthalen-2-ol;
7-(2-morpholinoethoxy)-3-(6-((2,2,6,6-tetramethylpiperidin-4-yl)oxy)pyridazin-3-yl)naphthalen-2-ol;
3-(6-(piperidin-4-ylmethyl)pyridazin-3-yl)naphthalen-2-ol;
5-(1H-pyrazol-1-yl)-2-(6-((2,2,6,6-tetramethylpiperidin-4-yl)methyl)pyridazin-3-yl)phenol;
3-methoxy-2-(6-(methyl(2,2,6-trimethylpiperidin-4-yl)amino)pyridazin-3-yl)-5-(5-methyloxazol-2-yl)phenol;
2-(6-((6S)-6-((S)-1-hydroxyethyl)-2,2-dimethylpiperidin-4-yloxy)pyridazin-3-yl)-5-(1H pyrazol-1-yl)phenol;
7-hydroxy-6-(6-(methyl(2,2,6,6-tetramethylpiperidin-4-yl)amino)pyridazin-3-yl)-2-naphthonitrile;
3-(6-(methyl(2,2,6,6-tetramethylpiperidin-4-yl)amino)pyridazin-3-yl)-7-(piperidin-1-ylmethyl)naphthalen-2-ol;
3-(6-(methyl(2,2,6,6-tetramethylpiperidin-4-yl)amino)pyridazin-3-yl)-7-(pyrrolidin-1-ylmethyl)naphthalen-2-ol;
1-bromo-6-(6-(methyl(2,2,6,6-tetramethylpiperidin-4-yl)amino)pyridazin-3-yl)naphthalene-2,7-diol;
1-chloro-6-(6-(methyl(2,2,6,6-tetramethylpiperidin-4-yl)amino)pyridazin-3-yl)naphthalene-2,7-diol;
7-methoxy-3-(6-(methyl(2,2,6,6-tetramethylpiperidin-4-yl)amino)pyridazin-3-yl)naphthalen-2-ol;
7-methoxy-3-(6-(methyl(1,2,2,6,6-pentamethylpiperidin-4-yl)amino)pyridazin-3-yl)naphthalen-2-ol;
7-(3,6-dihydro-2H-pyran-4-yl)-3-(6-(methyl(2,2,6,6-tetramethylpiperidin-4-yl)amino)pyridazin-3-yl)naphthalen-2-ol;
3-(6-(methyl-(2,2,6,6-tetramethylpiperidin-4-yl)amino)pyridazin-3-yl)-7-(tetrahydro-2H-pyran-4-yl)naphthalen-2-ol;
7-(difluoromethyl)-3-(6-(methyl-(2,2,6,6-tetramethylpiperidin-4-yl)amino)pyridazin-3-yl)naphthalen-2-ol;
7-((4-hydroxy-2-methylbutan-2-yl)oxy)-3-(6-(methyl(2,2,6,6-tetramethylpiperidin-4-yl)amino)pyridazin-3-yl)naphthalen-2-ol;
7-(3-hydroxy-3-methylbutoxy)-3-(6-(methyl-(2,2,6,6-tetramethylpiperidin-4-yl)amino)pyridazin-3-yl)naphthalen-2-ol;
2-(6-(methyl(2,2,6,6-tetramethylpiperidin-4-yl)amino)pyridazin-3-yl)-5-(1H-pyrazol-4-yl)benzene-1,3-diol;
3-methoxy-2-(6-(methyl-(2,2,6,6-tetramethylpiperidin-4-yl)amino)pyridazin-3-yl)-5-(1H pyrazol-4-yl)phenol;
5-(1H-pyrazol-4-yl)-2-(6-((2,2,6,6-tetramethylpiperidin-4-yl)amino)pyridazin-3-yl)-3-(trifluoromethoxy)phenol;
2-(6-(methyl(2,2,6,6-tetramethylpiperidin-4-yl)amino)pyridazin-3-yl)-5-(1-methyl-1H-pyrazol-4-yl)-3-(trifluoromethoxy)phenol;
2-(6-(methyl(2,2,6,6-tetramethylpiperidin-4-yl)amino)pyridazin-3-yl)-5-(1H-pyrazol-4-yl)-3-(trifluoromethoxy)phenol;
4-(3-hydroxy-4-(6-(methyl-(2,2,6,6-tetramethylpiperidin-4-yl)amino)pyridazin-3-yl)-5-(trifluoromethoxy)phenyl)-1-methylpyridin-2(1H)-one;
3-methoxy-2-(6-(methyl-(2,2,6,6-tetramethylpiperidin-4-yl)amino)pyridazin-3-yl)-5-(1-methyl-1H-pyrazol-4-yl)phenol;
3-methoxy-2-(6-(methyl-(2,2,6,6-tetramethylpiperidin-4-yl)amino)pyridazin-3-yl)-5-(5,6,7,8-tetrahydroimidazo[1,2-a]pyridin-3-yl)phenol;
3-methoxy-2-(6-(methyl-(2,2,6,6-tetramethylpiperidin-4-yl)amino)pyridazn-3-yl)-5-(pyridin-3-yl)phenol;
5-(1-cyclopentyl-1H-pyrazol-4-yl)-3-methoxy-2-(6-(methyl(2,2,6,6-tetra methylpiperidin-4-yl)amino)pyridazin-3-yl)phenol;
3'5-dimethoxy-4-(6-(methyl-(2,2,6,6-tetramethylpiperidin-4-yl)amino)pyridazin-3-yl)-[1,1'-biphenyl]-3-ol;

3-(benzyloxy)-2-(6-(methyl-(2,2,6,6-tetramethylpiperidin-4-yl)amino)pyridazin-3-yl)-5-(5-methyloxazol-2-yl)phenol;
3-ethoxy-2-(6-(methyl-(2,2,6,6-tetramethylpiperidin-4-yl)amino)pyridazin-3-yl)-5-(5-methyloxazol-2-yl)phenol;
5 3-(cyclopropylmethoxy)-2-(6-(methyl-(2,2,6,6-tetramethylpiperidin-4-yl)amino)-pyridazin-3-yl)-5-(5-methyloxazol-2-yl)phenol;
2-methyl-5-(6-(methyl-(2,2,6,6-tetramethylpiperidin-4-yl)amino)pyridazin-3-yl)-1H benzo[d]imidazol-6-ol;
5-chloro-2-(6-(methyl-(2,2,6,6-tetramethylpiperidin-4-yl)amino)pyridazin-3-yl)phenol;
5-(1H-pyrazol-1-yl)-2-(6-((2,2,6,6-tetramethylpiperidin-4-yl)amino)pyridazin-3-yl)phenol;
3-hydroxy-4-(6-((2,2,6,6-tetramethylpiperidin-4-yl)amino)pyridazin-3-yl)benzonitrile;
2-(6-((2,2-dimethylpiperidin-4-yl)oxy)pyridazin-3-yl)-5-(1H-pyrazol-1-yl)phenol;
2-(6-(methyl-(2,2,6,6-tetramethylpiperidin-4-yl)amino)pyridazin-3-yl)-4-(1H-pyrazol-4-yl)phenol;
2-(6-(methyl-(2,2,6,6-tetramethylpiperidin-4-yl)amino)pyridazin-3-yl)-4-(4,5,6,7-tetrahydropyrazolo[1,5-a]pyridin-3-yl)phenol;
2-(6-(methyl-(2,2,6,6-tetramethylpiperidin-4-yl)amino)pyridazin-3-yl)-4-(4,5,6,7-tetrahydropyrazolo[1,5-a] pyrazin-3-yl)phenol;
4-(1H-indol-2-yl)-2-(6-(methyl-(2,2,6,6-tetramethylpiperidin-4-yl)amino)pyridazin-3-yl)phenol;
4-(cyclopent-1-en-1-yl)-2-(6-(methyl(2,2,6,6-tetramethylpiperidin-4-yl)amino)pyridazin-3-yl)phenol;
2-(6-(methyl-(2,2,6,6-tetramethylpiperidin-4-yl)amino)pyridazin-3-yl)-4-(1H-pyrazol-3-yl)phenol;
4-(4-hydroxy-3-(6-(methyl-(2,2,6,6-tetramethylpiperidin-4-yl)amino)pyridazin-3 yl)phenyl)pyridin-2-ol;
4-(4-hydroxy-3-(6-((2,2,6,6-tetramethylpiperidin-4-yl)oxy)pyridazin-3-yl)phenyl)-1-methylpyridin-2-(1H)-one;
4-(4-hydroxy-3-(6-((2,2,6,6-tetramethylpiperidin-4-yl)oxy)pyridazin-3-yl)phenyl)pyridin-2-ol;
5-(1H-indazol-7-yl)-2-(6-(methyl-(2,2,6,6-tetramethylpiperidin-4-yl)amino)pyridazin-3-yl)phenol;
4-chloro-2-(6-(methyl-(2,2,6,6-tetramethylpiperidin-4-yl)amino)pyridazin-3-yl)-5-(1H-pyrazol-4-yl)phenol;
4-fluoro-2-(6-(methyl-(2,2,6,6-tetramethylpiperidin-4-yl)amino)pyridazin-3-yl)-5-(1H-pyrazol-4-yl)phenol;
5-fluoro-4-(1H-imidazol-4-yl)-2-(6-(methyl-(2,2,6,6-tetramethylpiperidin-4-yl)amino)pyridazin-3-yl)phenol;
5-fluoro-2-(6-(methyl-(2,2,6,6-tetramethylpiperidin-4-yl)amino)pyridazin-3-yl)-4-(1H-pyrazol-4-yl)phenol;
5-fluoro-2-(6-(methyl-(2,2,6,6-tetramethylpiperidin-4-yl)amino)pyridazin-3-yl)-4-(1H-pyrazol-5-yl)phenol;
5,6-hydroxy-5-(6-(methyl-(2,2,6,6-tetramethylpiperidin-4-yl)amino)pyridazin-3-yl)-2,3-dihydro-1H-inden-1-one;
6-(6-(methyl-(2,2,6,6-tetramethylpiperidin-4-yl)amino)pyridazin-3-yl)-1,4-dihydroindeno[1,2-c]pyrazol-7-ol;
6-hydroxy-5-(6-(methyl-(2,2,6,6-tetramethylpiperidin-4-yl)amino)pyridazin-3-yl)-2,3-dihydro-1H-inden-1-one oxime hydrochloride salt;
5-(6-(methyl-(2,2,6,6-tetramethylpiperidin-4-yl)amino)pyridazin-3-yl)-2,3-dihydro-1H-indene-1,6-diol;
2-amino-6-(6-(methyl-(2,2,6,6-tetramethylpiperidin-4-yl)amino)pyridazin-3-yl)-8H-indeno[1,2-d]thiazol-5-ol hydrochloride salt;
15 9-(6-(methyl-(2,2,6,6-tetramethylpiperidin-4-yl)amino)pyridazin-3-yl)-5,6-dihydroimidazo[5, 1-a]isoquinolin-8-ol hydrochloride salt;
4-hydroxy-3-(6-(methyl-(2,2,6,6-tetramethylpiperidin-4-yl)amino)pyridazin-3-yl)-N-((1-methyl-1H-pyrazol-4-yl)methyl)benzamide;
4-(4-(hydroxymethyl)-1H-pyrazol-1-yl)-2-(6-(methyl(2,2,6,6-tetramethylpiperidin-4-yl)amino)pyridazin-3-yl)phenol;
5-(1H-pyrazol-4-yl)-2-(6-((2,2,6,6-tetramethylpiperidin-4-yl)methyl)pyridazin-3-yl)phenol;
6-(3-(benzyloxy)isoquinolin-6-yl)-N-methyl-N-(2,2,6,6-tetramethylpiperidin-4-yl)pyridazin-3-amine;
6-(1-(benzyloxy)isoquinolin-7-yl)-N-methyl-N-(2,2,6,6-tetramethylpiperidin-4-yl)pyridazin-3-amine;
3-fluoro-5-(2-methoxypyridin-4-yl)-2-(6-(methyl-(2,2,6,6-tetramethylpiperidin-4-yl)amino)pyridazin-3-yl)phenol hydrochloride salt;
4-(3-fluoro-5-hydroxy-4-(6-(methyl-(2,2,6,6-tetramethylpiperidin-4-yl)amino)pyridazin-3-yl)phenyl)pyridin-2(1H)-one hydrochloride salt;
4-(3-fluoro-5-hydroxy-4-(6-(methyl-(2,2,6,6-tetramethylpiperidin-4-yl)amino)pyridazin-3-yl)phenyl)-1-methylpyridin-2(1H)-one hydrochloride salt;
5-(3-fluoro-5-hydroxy-4-(6-(methyl-(2,2,6,6-tetramethylpiperidin-4-yl)amino)pyridazin-3-yl)phenyl)-1-methylpyridin-2(1H)-one hydrochloride salt;
3-fluoro-5-(1H-pyrazol-4-yl)-2-(6-((2,2,6,6-tetramethylpiperidin-4-yl)oxy)pyridazin-3-yl)phenol hydrochloride salt;
5-chloro-3-fluoro-2-(6-(methyl-(2,2,6,6-tetramethylpiperidin-4-yl)amino)pyridazin-3-yl)phenol hydrochloride salt;
3-fluoro-2-(6-(methyl-(2,2,6,6-tetramethylpiperidin-4-yl)amino)pyridazin-3-yl)-5-(1H-pyrazol-4-yl)phenol hydrochloride salt;
3-fluoro-2-(6-(methyl-(2,2,6,6-tetramethylpiperidin-4-yl)amino)pyridazin-3-yl)-5-(1-methyl-1H pyrazol-4-yl)phenol hydrochloride salt;
5-(5-methoxypyridin-3-yl)-2-(6-(methyl-(2,2,6,6-tetramethylpiperidin-4-yl)amino)pyridazin-3-yl)phenol;
5-(3-hydroxy-4-(6-methyl-(2,2,6,6-tetramethylpiperidin-4-yl)amino)pyridazin-3-yl) phenyl)pyridin-2-ol;
4-(3-hydroxy-4-(6-methyl-(2,2,6,6-tetramethylpiperidin-4-yl)amino)pyridazin-3-yl)phenyl)pyridin-2-ol;

5-(6-methoxypyridin-3-yl)-2-(6-(methyl-(2,2,6,6-tetramethylpiperidin-4-yl)amino)pyridazin-3-yl)phenol;
5-(3-hydroxy-4-(6-(methyl-(2,2,6,6-tetramethylpiperidin-4-yl)amino)pyridazin-3-yl)phenyl)-3-(trifluoromethyl)pyridin-2-ol;
5-(3-hydroxy-4-(6-(methyl-(2,2,6,6-tetramethylpiperidin-4-yl)amino)pyridazin-3-yl)phenyl)-1-methylpyridin-2(1H)-one;
4-(3-hydroxy-4-(6-(methyl-(2,2,6,6-tetramethylpiperidin-4-yl)amino)pyridazin-3-yl)phenyl)-1-methylpyridin-2(1H)-one;
5-(2-methoxypyridin-4-yl)-2-(6-(methyl-(2,2,6,6-tetramethylpiperidin-4-yl)amino)pyridazin-3-yl)phenol;
4-(3-hydroxy-4-(6-((2,2,6,6-tetramethylpiperidin-4-yl)oxy)pyridazin-3-yl)phenyl)pyridin-2-ol;
5-(6-(dimethylamino)pyridin-3-yl)-2-(6-(methyl(2,2,6,6-tetramethylpiperidin-4-yl)amino)pyridazin-3-yl)phenol;
4-(3-hydroxy-4-(6-((2,2,6,6-tetramethylpiperidin-4-yl)oxy)pyridazin-3-yl)phenyl)-1-methylpyridin-2(1H)-one;
2-(6-(methyl-(2,2,6,6-tetramethylpiperidin-4-yl)amino)pyridazin-3-yl)-5-(pyrimidin-5-yl)phenol;
5-(3-hydroxy-4-(6-(methyl-(2,2,6,6-tetramethylpiperidin-4-yl)amino)pyridazin-3-yl) phenyl)pyridin-3-ol;
1-cyclopropyl-4-(3-hydroxy-4-(6-(methyl(2,2,6,6-tetra methylpiperidin-4-yl)amino)pyridazin-3-yl)phenyl)pyridin-2(1H)-one;
2-(6-(methyl-(2,2,6,6-tetramethylpiperidin-4-yl)amino)pyridazin-3-yl)-5-(1,2,3,6-tetrahydropyridin-4-yl)phenol;
5-(cyclopent-1-en-1-yl)-2-(6-(methyl(2,2,6,6-tetramethylpiperidin-4-yl)amino)pyridazin-3-yl)phenol;
5-(3,6-dihydro-2H-pyran-4-yl)-2-(6-(methyl(2,2,6,6-tetramethylpiperidin-4-yl)amino)pyridazin-3-yl)phenol;
5-(imidazo[1,5-a]pyridin-7-yl)-2-(6-(methyl(2,2,6,6-tetramethylpiperidin-4-yl)amino)pyridazin-3-yl)phenol;
5-(imidazo[1,2-a]pyridin-7-yl)-2-(6-(methyl(2,2,6,6-tetramethylpiperidin-4-yl)amino)pyridazin-3-yl)phenol;
2-(6-(methyl(2,2,6,6-tetramethylpiperidin-4-yl)amino)pyridazin-3-yl)-5-(2-methylpyridin-4-yl)phenol;
5-(1H-imidazol-2-yl)-2-(6-(methyl-(2,2,6,6-tetramethylpiperidin-4-yl)amino)pyridazin-3-yl)phenol;
5-(1H-imidazol-4-yl)-2-(6-(methyl-(2,2,6,6-tetramethylpiperidin-4-yl)amino)pyridazin-3-yl)phenol;
5-(imidazo[1,2-a]pyrazin-3-yl)-2-(6-(methyl(2,2,6,6-tetramethylpiperidin-4-yl)amino)pyridazin-3-yl)phenol;
2-(6-(methyl-(2,2,6,6-tetramethylpiperidin-4-yl)amino)pyridazin-3-yl)-5-(5,6,7,8-tetrahydroimidazo[1,2-a]pyrazin-3-yl)phenol;
2-(6-(methyl-(2,2,6,6-tetramethylpiperidin-4-yl)amino)pyridazin-3-yl)-5-(4-methyl-1H-imidazol-2-yl)phenol;
2-(6-(methyl-(2,2,6,6-tetramethylpiperidin-4-yl)amino)pyridazin-3-yl)-5-(1-methyl-1H-imidazol-4-yl)phenol;
2-(6-(methyl-(2,2,6,6-tetramethylpiperidin-4-yl)amino)pyridazin-3-yl)-5-(1-methyl-1H imidazol-5-yl)phenol;
2-(6-(methyl-(2,2,6,6-tetramethylpiperidin-4-yl)amino)pyridazin-3-yl)-5-(4-nitro-1H-imidazol-2-yl)phenol;
2-(6-(methyl-(2,2,6,6-tetramethylpiperidin-4-yl)amino)pyridazin-3-yl)-5-(2-methyl-1H imidazol-4-yl)phenol;
5-(1,2-dimethyl-1H-imidazol-4-yl)-2-(6-(methyl(2,2,6,6-tetramethylpiperidin-4-yl)amino)pyridazin-3-yl)phenol;
1-(3-hydroxy-4-(6-(methyl-(2,2,6,6-tetramethylpiperidin-4-yl)amino)pyridazin-3-yl)phenyl)-1H-pyrazole-4-carboxamide;
2-(6-((3aR,6aS)-5-(2-hydroxyethyl)hexahydropyrrolo[3,4-c]pyrrol-2(1H)-yl)pyridazin-3-yl)-5-(1H-pyrazol-4-yl)phenol;
2-(6-((3aR,6aS)-hexahydropyrrolo[3,4-c]pyrrol-2(1H)-yl)pyridazin-3-yl)-5-(1H-pyrazol-4-yl)phenol;
2-(6-((3aR,6aS)-5-methylhexahydropyrrolo[3,4-c]pyrrol-2(1H)-yl)pyridazin-3-yl)-5-(1 Hpyrazol-4-yl)phenol;
4-(3-hydroxy-4-(6-(5-methylhexahydropyrrolo[3,4-c]pyrrol-2(1H)-yl)pyridazin-3-yl)phenyl)-1-methylpyridin-2(1H)-one;
4-(3-hydroxy-4-(6-((3aR,6aR)-1-methylhexahydropyrrolo[3,4-b]pyrrol-5(1H)-yl)pyridazin-3-yl)phenyl)-1-methylpyridin-2(1H)-one;
2-(6-(2,7-diazaspiro[4.5]decan-2-yl)pyridazin-3-yl)-5-(1H-pyrazol-4-yl)phenol; and
4-(4-(6-(2,7-diazaspiro[4.5]decan-2-yl)pyridazin-3-yl)-3-hydroxyphenyl)-1-methylpyridin-2(1H)-one;
5-(2-Methoxy-4-(1H-pyrazol-1-yl)phenyl)-N-methyl-N-(2,2,6,6-tetramethylpiperidin-4-yl)-1,3,4-thiadiazol-2-amine;
6-(5-(Methyl(2,2,6,6-tetramethylpiperidin-4-yl)amino)-1,3,4-thiadiazol-2-yl)naphthalen-2-ol;
5-(2-Methoxyquinolin-3-yl)-N-methyl-N-(2,2,6,6-tetramethylpiperidin-4-yl)-1,3,4-thiadiazol-2-amine;
5-(3-Methoxynaphthalen-2-yl)-N-methyl-N-(2,2,6,6-tetramethylpiperidin-4-yl)-1,3,4-thiadiazol-2-amine;
5-(2-Methoxy-4-(1H-pyrazol-1-yl)phenyl)-N-(1,2,2,6,6-pentamethylpiperidin-4-yl)-1,3,4-thiadiazol-2-amine;
5-(2-Methoxy-4-(1-methyl-1H-pyrazol-4-yl)phenyl)-N-methyl-N-(2,2,6,6-tetramethylpiperidin-4-yl)-1,3,4-thiadiazol-2-amine;
5-(2-Methoxy-4-(1H-pyrazol-4-yl)phenyl)-N-methyl-N-(2,2,6,6-tetramethylpiperidin-4-yl)-1,3,4-thiadiazol-2-amine;
4-(3-Methoxy-4-(5-(methyl(2,2,6,6-tetramethylpiperidin-4-yl)amino)-1,3,4-thiadiazol-2-yl)phenyl)-1-methylpyridin-2(1H)-one;
5-(3-Methoxy-4-(5-(methyl(2,2,6,6-tetramethylpiperidin-4-yl)amino)-1,3,4-thiadiazol-2-yl)phenyl)pyridin-2-ol;
5-(3-Methoxy-4-(5-(methyl(2,2,6,6-tetramethylpiperidin-4-yl)amino)-1,3,4-thiadiazol-2-yl)phenyl)-1-methylpyridin-2(1H)-one;
N-Methyl-5-(2-methyl-4-(1-methyl-1H-pyrazol-4-yl)phenyl)-N-(2,2,6,6-tetramethylpiperidin-4-yl)-1,3,4-thiadiazol-2-amine;
1-Methyl-4-(4-(5-(methyl(2,2,6,6-tetramethylpiperidin-4-yl)amino)-1,3,4-thiadiazol-2-yl)-3-(trifluoromethoxy)phenyl)pyridin-2(1H)-one;
5-(4-(3,5-Dimethyl-1H-pyrazol-4-yl)-2-methoxyphenyl)-N-methyl-N-(2,2,6,6-tetramethylpiperidin-4-yl)-1,3,4-thiadiazol-2-amine;
5-(2-Methoxy-4-(1-methyl-1H-pyrazol-4-yl)phenyl)-N-methyl-N-(2,2,6,6-tetramethylpiperidin-4-yl)-1,3,4-thiadiazol-2-amine;
2-(5-(Methyl(2,2,6,6-tetramethylpiperidin-4-yl)amino)-1,3,4-thiadiazol-2-yl)-5-(1-methyl-1H-pyrazol-4-yl)phenol;
2-(5-(Methyl(2,2,6,6-tetramethylpiperidin-4-yl)amino)-1,3,4-thiadiazol-2-yl)-5-(1H-pyrazol-1-yl)phenol;
5-(3-Hydroxy-4-(5-(methyl(2,2,6,6-tetramethylpiperidin-4-yl)amino)-1,3,4-thiadiazol-2-yl)phenyl)-1-methylpyridin-2(1H)-one;
4-(3-Hydroxy-4-(5-(methyl(2,2,6,6-tetramethylpiperidin-4-yl)amino)-1,3,4-thiadiazol-2-yl)phenyl)-1-methylpyridin-2(1H)-one;
5-(3-Hydroxy-4-(5-(methyl(2,2,6,6-tetramethylpiperidin-4-yl)amino)-1,3,4-thiadiazol-2-yl)phenyl)pyridin-2-ol;
3-(5-(Methyl(2,2,6,6-tetramethylpiperidin-4-yl)amino)-1,3,4-thiadiazol-2-yl)naphthalene-2,7-diol;
3-(5-((3aR,6aS)-Hexahydropyrrolo[3,4-c]pyrrol-2(1H)-yl)-1,3,4-thiadiazol-2-yl)naphthalene-2,7-diol;
3-(5-(Methyl(2,2,6,6-tetramethylpiperidin-4-yl)amino)-1,3,4-thiadiazol-2-yl)naphthalen-2-ol hydrobromide salt;
3-(5-(Methyl(2,2,6,6-tetramethylpiperidin-4-yl)amino)-1,3,4-thiadiazol-2-yl)quinolin-2-ol; 2-(5-(Methyl(2,2,6,6-tetramethylpiperidin-4-yl)amino)-1,3,4-thiadiazol-2-yl)-4-(1H-pyrazol-1-yl)phenol;
5-(2-Chloro-4-(1-methyl-1H-pyrazol-4-yl)phenyl)-N-methyl-N-(2,2,6,6-tetramethylpiperidin-4-yl)-1,3,4-thiadiazol-2-amine;
3-Chloro-2-(5-(methyl(2,2,6,6-tetramethylpiperidin-4-yl)amino)-1,3,4-thiadiazol-2-yl)-5-(1-methyl-1H-pyrazol-4-yl)phenol;
5-(2-Chloro-4-(1-methyl-1H-pyrazol-4-yl)phenyl)-N-(2,2,6,6-tetramethylpiperidin-4-yl)-1,3,4-thiadiazol-2-amine;
3-Methoxy-2-(5-(methyl(2,2,6,6-tetramethylpiperidin-4-yl)amino)-1,3,4-thiadiazol-2-yl)-5-(5-methyloxazol-2-yl)phenol;
2-(2-Methoxy-4-(1H-pyrazol-1-yl)phenyl)-5-(1,2,3,6-tetrahydropyridin-4-yl)-1,3,4-thiadiazole;
2-(5-(piperazin-1-yl)-1,3,4-thiadiazol-2-yl)-5-(1H-pyrazol-1-yl)phenol;
5-(7-Methoxyquinolin-6-yl)-N-methyl-N-(2,2,6,6-tetramethylpiperidin-4-yl)-1,3,4-thiadiazol-2-amine;
6-(5-(Methyl(2,2,6,6-tetramethylpiperidin-4-yl)amino)-1,3,4-thiadiazol-2-yl)quinolin-7-ol; 3-methoxy-4-(5-(methyl(2,2,6,6-tetramethylpiperidin-4-yl)amino)-1,3,4-thiadiazol-2-yl)benzonitrile;
3-fluoro-4-(5-(methyl(2,2,6,6-tetramethylpiperidin-4-yl)amino)-1,3,4-thiadiazol-2-yl)benzonitrile;
methyl 3-fluoro-4-(5-(methyl(2,2,6,6-tetramethylpiperidin-4-yl)amino)-1,3,4-thiadiazol-2-yl)benzoate;
5-(2-methoxy-4-(3-(methylamino)-1H-pyrazol-1-yl)phenyl)-N-methyl-N-(2,2,6,6-tetramethylpiperidin-4-yl)-1,3,4-thiadiazol-2-amine;
7-methoxy-6-(5-(methyl(2,2,6,6-tetramethylpiperidin-4-yl)amino)-1,3,4-thiadiazol-2-yl)quinoline-2-carbonitrile;
4-(3-methoxy-4-(5-((2,2,6,6-tetramethylpiperidin-4-yl)oxy)-1,3,4-thiadiazol-2-yl)phenyl)-1-methylpyridin-2(1H)-one;
4-(3-chloro-4-(5-(methyl(2,2,6,6-tetramethylpiperidin-4-yl)amino)-1,3,4-thiadiazol-2-yl)phenyl)-1-methylpyridin-2(1H)-one;
5-(2-Chloro-4-(1H-pyrazol-4-yl)phenyl)-N-methyl-N-(2,2,6,6-tetramethylpiperidin-4-yl)-1,3,4-thiadiazol-2-amine;
5-(2-Chloro-4-(4,5,6,7-tetrahydropyrazolo[1,5-a]pyridin-3-yl)phenyl)-N-methyl-N-(2,2,6,6-tetramethylpiperidin-4-yl)-1,3,4-thiadiazol-2-amine;
N-methyl-5-(5-(1-methyl-1H-pyrazol-4-yl)pyridin-2-yl)-N-(2,2,6,6-tetramethylpiperidin-4-yl)-1,3,4-thiadiazol-2-amine Hydrochloride salt;
2-(2-chloro-4-(1-methyl-1H-pyrazol-4-yl)phenyl)-5-((2,2,6,6-tetramethylpiperidin-4-yl)oxy)-1,3,4-thiadiazole;
5-(2-chloro-4-(6-methoxypyridin-3-yl)phenyl)-N-methyl-N-(2,2,6,6-tetramethylpiperidin-4-yl)-1,3,4-thiadiazol-2-amine;
5-(4-(6-aminopyridin-3-yl)-2-fluorophenyl)-N-methyl-N-(2,2,6,6-tetramethylpiperidin-4-yl)-1,3,4-thiadiazol-2-amine;
5-(2-fluoro-4-(3-methyl-1H-pyrazol-5-yl)phenyl)-N-methyl-N-(2,2,6,6-tetramethylpiperidin-4-yl)-1,3,4-thiadiazol-2-amine;
5-(2-fluoro-4-(1H-pyrazol-5-yl)phenyl)-N-methyl-N-(2,2,6,6-tetramethylpiperidin-4-yl)-1,3,4-thiadiazol-2-amine;
5-(2,3-difluoro-4-(1H-pyrazol-4-yl)phenyl)-N-methyl-N-(2,2,6,6-tetramethylpiperidin-4-yl)-1,3,4-thiadiazol-2-amine;
5-(2,3-difluoro-4-(1H-pyrazol-5-yl)phenyl)-N-methyl-N-(2,2,6,6-tetramethylpiperidin-4-yl)-1,3,4-thiadiazol-2-amine;
5-(2,5-difluoro-4-(1H-pyrazol-4-yl)phenyl)-N-methyl-N-(2,2,6,6-tetramethylpiperidin-4-yl)-1,3,4-thiadiazol-2-amine;
5-(2,5-difluoro-4-(1H-pyrazol-5-yl)phenyl)-N-methyl-N-(2,2,6,6-tetramethylpiperidin-4-yl)-1,3,4-thiadiazol-2-amine;
5-(2,6-difluoro-4-(1H-pyrazol-4-yl)phenyl)-N-methyl-N-(2,2,6,6-tetramethylpiperidin-4-yl)-1,3,4-thiadiazol-2-amine;
2-(2,5-difluoro-4-(1H-pyrazol-4-yl)phenyl)-5-((3aR,6aS)-hexahydropyrrolo[3,4-c]pyrrol-2(1H)-yl)-1,3,4-thiadiazole;

5-(2-chloro-5-fluoro-4-(1H-pyrazol-4-yl)phenyl)-N-methyl-N-(2,2,6,6-tetramethylpiperidin-4-yl)-1,3,4-thiadiazol-2-amine;
5-(3-fluoro-5-(1H-pyrazol-4-yl)pyridin-2-yl)-N-methyl-N-(2,2,6,6-tetramethylpiperidin-4-yl)-1,3,4-thiadiazol-2-amine;
5-(4-(2-aminopyrimidin-4-yl)-2-chlorophenyl)-N-methyl-N-(2,2,6,6-tetramethylpiperidin-4-yl)-1,3,4-thiadiazol-2-amine;
5-(5-(2-aminopyrimidin-4-yl)-2-chlorophenyl)-N-methyl-N-(2,2,6,6-tetramethylpiperidin-4-yl)-1,3,4-thiadiazol-2-amine;
5-(4-(2,4-dimethylthiazol-5-yl)-2,5-difluorophenyl)-N-methyl-N-(2,2,6,6-tetramethylpiperidin-4-yl)-1,3,4-thiadiazol-2-amine;
5-(4-(2,4-dimethylthiazol-5-yl)-2,3-difluorophenyl)-N-methyl-N-(2,2,6,6-tetramethylpiperidin-4-yl)-1,3,4-thiadiazol-2-amine;
4-(3-hydroxy-4-(5-(methyl(2,2,6,6-tetramethylpiperidin-4-yl)amino)-1,3,4-thiadiazol-2-yl)-5-(trifluoromethoxy)phenyl)-1-methylpyridin-2(1H)-one;
5-(2-fluoro-6-methoxy-4-(1H-pyrazol-4-yl)phenyl)-N-methyl-N-(2,2,6,6-tetramethylpiperidin-4-yl)-1,3,4-thiadiazol-2-amine;
2-(2-fluoro-6-methoxy-4-(1H-pyrazol-4-yl)phenyl)-5-((3aR,6aS)-5-methylhexahydropyrrolo[3,4-c]pyrrol-2(1H)-yl)-1,3,4-thiadiazole;
5-(2,3-difluoro-6-methoxy-4-(1H-pyrazol-4-yl)phenyl)-N-methyl-N-(2,2,6,6-tetramethylpiperidin-4-yl)-1,3,4-thiadiazol-2-amine;
6-methoxy-2-(5-(methyl(2,2,6,6-tetramethylpiperidin-4-yl)amino)-1,3,4-thiadiazol-2-yl)-3,4-dihydroisoquinolin-1(2H)-one;
5-(2-Chloro-4-(1H-pyrazol-1-yl)phenyl)-N-methyl-N-(2,2,6,6-tetramethylpiperidin-4-yl)-1,3,4-thiadiazol-2-amine;
5-(2-Chloro-4-(1H-1,2,3-triazol-1-yl)phenyl)-N-methyl-N-(2,2,6,6-tetramethylpiperidin-4-yl)-1,3,4-thiadiazol-2-amine;
5-(2-chloro-4-(2H-1,2,3-triazol-2-yl)phenyl)-N-methyl-N-(2,2,6,6-tetramethylpiperidin-4-yl)-1,3,4-thiadiazol-2-amine;
5-(2-chloro-4-(1H-1,2,4-triazol-1-yl)phenyl)-N-methyl-N-(2,2,6,6-tetramethylpiperidin-4-yl)-1,3,4-thiadiazol-2-amine;
5-(4-(3-amino-1H-pyrazol-1-yl)-2-chlorophenyl)-N-methyl-N-(2,2,6,6-tetramethylpiperidin-4-yl)-1,3,4-thiadiazol-2-amine;
2-(2-chloro-4-(1H-imidazol-1-yl)phenyl)-5-((3aR,6aS)-5-methylhexahydropyrrolo[3,4-c]pyrrol-2(1H)-yl)-1,3,4-thiadiazole;
5-(2-Chloro-4-(1H-imidazol-1-yl)phenyl)-N-methyl-N-(2,2,6,6-tetramethylpiperidin-4-yl)-1,3,4-thiadiazol-2-amine;
5-(2-fluoro-4-(1H-imidazol-1-yl)phenyl)-N-methyl-N-(2,2,6,6-tetramethylpiperidin-4-yl)-1,3,4-thiadiazol-2-amine;
5-(2-methoxy-4-(1H-pyrazol-5-yl)phenyl)-N-methyl-N-(2,2,6,6-tetramethylpiperidin-4-yl)-1,3,4-thiadiazol-2-amine;
5-(4-(2,4-dimethylthiazol-5-yl)-2-methoxyphenyl)-N-methyl-N-(2,2,6,6-tetramethylpiperidin-4-yl)-1,3,4-thiadiazol-2-amine;
5-(2-methoxy-4-(pyridin-3-yl)phenyl)-N-methyl-N-(2,2,6,6-tetramethylpiperidin-4-yl)-1,3,4-thiadiazol-2-amine;
5-(2-fluoro-4-(1H-pyrazol-4-yl)phenyl)-N-methyl-N-(2,2,6,6-tetramethylpiperidin-4-yl)-1,3,4-thiadiazol-2-amine;
5-(2-methoxy-4-(2-methoxypyridin-4-yl)phenyl)-N-methyl-N-(2,2,6,6-tetramethylpiperidin-4-yl)-1,3,4-thiadiazol-2-amine;
5-(2-methoxy-4-(6-methoxypyridin-3-yl)phenyl)-N-methyl-N-(2,2,6,6-tetramethylpiperidin-4-yl)-1,3,4-thiadiazol-2-amine;
2-(2-Chloro-4-(1-methyl-1H-pyrazol-4-yl)phenyl)-5-((3aR,6aS)-5-methylhexahydropyrrolo[3,4-c]pyrrol-2(1H)-yl)-1,3,4-thiadiazole;
2-(2-chloro-4-(1H-pyrazol-4-yl)phenyl)-5-((3aR,6aS)-5-methylhexahydropyrrolo[3,4-c]pyrrol-2(1H)-yl)-1,3,4-thiadiazole;
2-(2-Chloro-4-(1H-pyrazol-4-yl)phenyl)-5-((3aR, 6a R)-1-methylhexahyd ropyrrolo[3,4-b]pyrrol-5(1H)-yl)-1,3,4-thiadiazole;
1-(4-(5-(2-chloro-4-(1H-pyrazol-4-yl)phenyl)-1,3,4-thiadiazol-2-yl)morpholin-2-yl)-N,N-dimethylmethanamine;
2-(2-chloro-4-(1H-pyrazol-4-yl)phenyl)-5-(2-methyl-2,7-diazaspiro[4.5]decan-7-yl)-1,3,4-thiadiazole;
2-(2-fluoro-4-(1H-pyrazol-4-yl)phenyl)-5-((3aR,6aS)-5-methylhexahydropyrrolo[3,4-c]pyrrol-2(1H)-yl)-1,3,4-thiadiazole;
2-(2-methoxy-4-(1-methyl-1H-pyrazol-4-yl)phenyl)-5-(2,6-diazaspiro[3.5]nonan-2-yl)-1,3,4-thiadiazole;
2-(2-methoxy-4-(1-methyl-1H-pyrazol-4-yl)phenyl)-5-(2,7-diazaspiro[3.5]nonan-2-yl)-1,3,4-thiadiazole;
2-(5-(Methyl(2,2,6,6-tetramethylpiperidin-4-yl)amino)-1,3,4-thiadiazol-2-yl)-5-(1H-pyrazol-1-yl)phenol;
5-(3-chloro-4-(5-(methyl(2,2,6,6-tetramethylpiperidin-4-yl)amino)-1,3,4-thiadiazol-2-yl)phenyl)pyridin-2(1H)-one;

2-(5-(methyl(2,2,6,6-tetramethylpiperidin-4-yl)amino)-1,3,4-thiadiazol-2-yl)-5-(3-(methylamino)-1H-pyrazol-1-yl)phenol;
3-fluoro-2-(5-(methyl(2,2,6,6-tetramethylpiperidin-4-yl)amino)-1,3,4-thiadiazol-2-yl)-5-(1H-pyrazol-4-yl)phenol;
3,4-d ifluoro-2-(5-(methyl(2,2,6,6-tetramethylpiperidin-4-yl)amino)-1,3,4-thiadiazol-2-yl)-5-(1H-pyrazol-4-yl)phenol;
6-hydroxy-5-(5-(methyl(2,2,6,6-tetramethylpiperidin-4-yl)amino)-1,3,4-thiadiazol-2-yl)-2,3-dihydro-1H-inden-1-one;
2-(5-(methyl(2,2,6,6-tetramethylpiperidin-4-yl)amino)-1,3,4-thiadiazol-2-yl)-5-(1H-pyrazol-4-yl)phenol;
2-(5-(2,6-diazaspiro[3.5]nonan-2-yl)-1,3,4-thiadiazol-2-yl)-5-(1-methyl-1H-pyrazol-4-yl)phenol;
2-(5-(2,7-diazaspiro[3.5]nonan-2-yl)-1,3,4-thiadiazol-2-yl)-5-(1-methyl-1H-pyrazol-4-yl)phenol;
3-fluoro-2-(5-((3aR,6aS)-hexahydropyrrolo[3,4-c]pyrrol-2(1H)-yl)-1,3,4-thiadiazol-2-yl)-5-(1H-pyrazol-4-yl)phenol Di-hydrochloride salt;
3-Chloro-2-(5-(methyl(2,2,6,6-tetramethylpiperidin-4-yl)amino)-1,3,4-thiadiazol-2-yl)-5-(1H-pyrazol-4-yl)phenol;
2-(2-methoxy-4-(1H-pyrazol-1-yl)phenyl)-5-((2,2,6,6-tetramethylpiperidin-4-yl)methyl)-1,3,4-thiadiazole;
2-(2,3-difluoro-4-(1H-pyrazol-4-yl)phenyl)-5-(2,7-diazaspiro[3.5]nonan-2-yl)-1,3,4-thiadiazole;
2-(5-(2,7-diazaspiro[3.5]nonan-2-yl)-1,3,4-thiadiazol-2-yl)-3-fluoro-5-(1H-pyrazol-4-yl)phenol; 4-methoxy-1-methyl-3-(5-(methyl(2,2,6,6-tetramethylpiperidin-4-yl)amino)-1,3,4-thiadiazol-2-yl)quinolin-2(1H)-one;
4-hydroxy-1-methyl-3-(5-(methyl(2,2,6,6-tetramethylpiperidin-4-yl)amino)-1,3,4-thiadiazol-2-yl)quinolin-2(1H)-one;
3-(5-(methyl(2,2,6,6-tetramethylpiperidin-4-yl)amino)-1,3,4-thiadiazol-2-yl)quinolin-2(1H)-one;
1-methyl-3-(5-(methyl(2,2,6,6-tetramethylpiperidin-4-yl)amino)-1,3,4-thiadiazol-2-yl)quinolin-2(1H)-one;
2-(2-Chloro-4-(1H-pyrazol-4-yl)phenyl)-5-((3aR,6aS)-hexahydropyrrolo[3,4-c]pyrrol-2(1H)yl)-1,3,4-thiadiazole Hydrochloride Salt;
2-(2-Chloro-4-(1H-pyrazol-4-yl)phenyl)-5-(2,7-diazaspiro[4.5]decan-2-yl)-1,3,4-thiadiazole Hydrochloride Salt;
(R)-(4-(5-(2-chloro-4-(1H-pyrazol-4-yl)phenyl)-1,3,4-thiadiazol-2-yl)piperazin-2-yl)methanol Hydrochloride Salt;
2-(5-(methyl(2,2,6,6-tetramethylpiperidin-4-yl)amino)-1,3,4-thiadiazol-2-yl)benzo[b]thiophene-5-carbonitrile; and
5-(3-chlorobenzo[b]thiophen-2-yl)-N-methyl-N-(2,2,6,6-tetramethylpiperidin-4-yl)-1,3,4-thiadiazol-2-amine;

or a pharmaceutically acceptable salt thereof;
for use in the treatment, prevention and/or delay of progression of cancer.

In a particular embodiment, the present invention relates to a FoxM1 gene splicing modifier as described herein selected from the group consisting of:

6-(naphthalen-2-yl)-N-(2,2,6,6-tetramethylpiperidin-4-yl)pyridazin-3-amine;
6-(benzo[b]thio-phen-2-yl)-N-methyl-N-(2,2,6,6-tetra-methylpiperidin-4-yl)pyridazin-3-amine;
2-(6-(2,2,6,6-tetra methylpiperidin-4-ylamino)-pyridazin-3-yl)phenol;
2-(6-(methyl-(2,2,6,6-tetra-methylpiperidin-4-yl)amino)pyridazin-3-yl)benzo[b]-thiophene-5-carbonitrile;
6-(quinolin-3-yl)-N-(2,2,6,6-tetramethyl-piperidin-4-yl)pyridazin-3-amine;
3-(benzo[b]-thiophen-2-yl)-6-(2,2,6,6-tetra-methylpiperidin-4-yloxy)pyridazine;
2-(6-(methyl-(2,2,6,6-tetra-methylpiperidin-4-yl)amino)-pyridazin-3-yl)phenol;
6-(6-(methyl-(2,2,6,6-tetra-methylpiperidin-4-yl)amino)-pyridazin-3-yl)naphthalen-2-ol;
6-(benzo[b]-thiophen-2-yl)-N-(2,2,6,6-tetra-methylpiperidin-4-yl)pyridazin-3-amine;
7-(6-((2,2,6,6-tetramethylpiperidin-4-yl)oxy)pyridazin-3-yl)isoquinoline;
6-(6-((2,2,6,6-tetramethylpiperidin-4-yl)oxy)pyridazin-3-yl)isoquinoline;
N-methyl-6-(quinolin-7-yl)-N-(2,2,6,6-tetramethyl-piperidin-4-yl)pyridazin-3-amine;
N-methyl-6-(quinolin-6-yl)-N-(2,2,6,6-tetramethylpiperidin-4-yl)pyridazin-3-amine;
6-(isoquinolin-7-yl)-N-methyl-N-(2,2,6,6-tetramethylpiperidin-4-yl)pyridazin-3-amine;
6-(isoquinolin-6-yl)-N-methyl-N-(2,2,6,6-tetramethylpiperidin-4-yl)pyridazin-3-amine;
6-(imidazo[1,2-a]pyridin-6-yl-pyridazin-3-yl)-methyl-(2,2,6,6-tetramethyl-piperidin-4-yl)-amine methyl-[6-(6-phenyl-pyridin-3-yl)-pyridazin-3-yl]-(2,2,6,6-tetramethyl-piperidin-4-yl)-amine;
methyl-[6-(6-pyrrol-1-yl-pyridin-3-yl)-pyridazin-3-yl]-(2,2,6,6-tetramethyl-piperidin-4-yl)-amine;
methyl-(6-quinoxalin-2-yl-pyridazin-3-yl)-(2,2,6,6-tetramethyl-piperidin-4-yl)-amine;
methyl-(6-quinolin-3-yl-pyridazin-3-yl)-(2,2,6,6-tetramethyl-piperidin-4-yl)-amine;
N-methyl-6-(phthalazin-6-yl)-N-(2,2,6,6-tetramethylpiperidin-4-yl)pyridazin-3-amine;
6-(benzo[c][1,2,5]oxa-diazol-5-yl)-N-(2,2,6,6-tetramethyl-piperidin-4-yl)pyridazin-3-amine;
6-(benzo[d]thiazol-5-yl)-N-(2,2,6,6-tetramethyl-piperidin-4-yl)pyridazin-3-amine;
6-(2-methylbenzo-[d]oxazol-6-yl)-N-(2,2,6,6-tetramethyl-piperidin-4-yl)pyridazin-3-amine;
3-(6-(methyl(2,2,6,6-tetramethylpiperidin-4-yl)amino)pyridazin-3-yl)naphthalen-2-ol;
5-chloro-2-(6-(methyl(1,2,2,6,6-pentamethylpiperidin-4-yl)amino)pyridazin-3-yl)phenol;
3-(6-(2,2,6,6-tetramethylpiperidin-4-ylamino)pyridazin-3-yl)naphthalen-2-ol;

5-chloro-2-(6-(1,2,2,6,6-pentamethylpiperidin-4-ylamino)pyridazin-3-yl)phenol;
4-hydroxy-3-(6-(methyl(2,2,6,6-tetramethylpiperidin-4-yl)amino)pyridazin-3-yl)benzonitrile;
3-[6-(2,2,6,6-tetramethyl-piperidin-4-yloxy)-pyridazin-3-yl]-naphthalen-2-ol;
2-{6-[methyl-(2,2,6,6-tetramethyl-piperidin-4-yl)-amino]-pyridazin-3-yl}-4-trifluoromethylphenol;
2-fluoro-6-{6-[methyl-(2,2,6,6-tetramethyl-piperidin-4-yl)-amino]-pyridazin-3-yl}-phenol;
3,5-dimethoxy-2-{6-[methyl-(2,2,6,6-tetramethyl-piperidin-4-yl)-amino]-pyridazin-3-yl}-phenol;
4,5-dimethoxy-2-{6-[methyl-(2,2,6,6-tetramethyl-piperidin-4-yl)-amino]-pyridazin-3-yl}-phenol;
5-methoxy-2-{6-[methyl-(2,2,6,6-tetramethyl-piperidin-4-yl)-amino]-pyridazin-3-yl}-phenol;
4,5-difluoro-2-{6-[methyl-(2,2,6,6-tetramethyl-piperidin-4-yl)-amino]-pyridazin-3-yl}-phenol;
5-fluoro-2-{6-[methyl-(2,2,6,6-tetramethyl-piperidin-4-yl)-amino]-pyridazin-3-yl}-phenol;
3-hydroxy-4-(6-(methyl(2,2,6,6-tetramethylpiperidin-4-yl)amino)pyridazin-3-yl)benzonitrile;
1-allyl-6-(6-(methyl(2,2,6,6-tetramethylpiperidin-4-yl)amino)pyridazin-3-yl)naphthalen-2-ol;
6-(benzo[b]thiophen-2-yl)-N-(1,2,2,6,6-pentamethylpiperidin-4-yl)pyridazin-3-amine;
N-allyl-3-hydroxy-4-(6-(methyl(2,2,6,6-tetramethylpiperidin-4-yl)amino)pyridazin-3-yl)benzamide;
2-(6-(methyl(2,2,6,6-tetramethylpiperidin-4-yl)amino)pyridazin-3-yl)-5-(1H-pyrazol-1-yl)phenol;
5-(5-methyl-oxazol-2-yl)-2-{6-[methyl-(2,2,6,6-tetramethyl-piperidin-4-yl)-amino]-pyridazin-3-yl}-phenol;
5-(4-hydroxymethyl)-1H-pyrazole-1-yl)-2-(6-(methyl(2,2,6,6-tetramethylpiperidin-4 yl)amino)pyridazin-3-yl)phenol;
5-(1H-imidazole-1-yl)-2-(6-(methyl(2,2,6,6-tetramethyl-piperidin-4-yl)amino)pyridazin-3-yl)phenol;
5-(4-amino-1H-pyrazole-1-yl)-2-(6-(methyl(2,2,6,6-tetramethylpiperidin-4-yl)amino)pyridazin-3-yl)phenol;
5-(4-amino-1H-pyrazol-1-yl)-2-(6-(methyl(2,2,6,6-tetramethylpiperidin-4-yl)amino)pyridazin-3-yl)phenol;
5-(3-amino-pyrazol-1-yl)-2-{6-[methyl-(2,2,6,6-tetra methyl-piperidin-4-yl)-amino]-pyridazin-3-yl}-phenol;
2-(6-(methyl-(2,2,6,6-tetramethylpiperidin-4-yl)amino)pyridazin-3-yl)-5-(1-(2-morpholinoethyl)-1H-pyrazol-4-yl)phenol;
2-(6-(methyl-(2,2,6,6-tetramethylpiperidin-4-yl)amino)pyridazin-3-yl)-5-(1-methyl-1H-pyrazol-4-yl)phenol;
5-(5-amino-1H-pyrazol-1-yl)-2-(6-(methyl-(2,2,6,6-tetramethyl-piperidin-4-yl)amino)pyridazin-3-yl)phenol;
2-(6-(methyl-(2,2,6,6-tetramethylpiperidin-4-yl)amino)pyridazin-3-yl)-4-(1H-pyrazol-1-yl)phenol;
2-{6-[(2-hydroxy-ethyl)-(2,2,6,6-tetramethyl-piperidn-4-yl)-amino]-pyridazin-3-yl}-5-pyrazol-1-yl-phenol;
2-(6-(piperidin-4-yloxy)pyridazin-3-yl)-5-(1H-pyrazol-1-yl)phenol;
2-(6-(((2S,4R,6R)-2,6-dimethylpiperidin-4-yl)oxy)pyridazin-3-yl)-5-(1H-pyrazol-1-yl)phenol;
5 2-(6-((2, 6-di-methylpiperidin-4-yl)oxy)pyridazin-3-yl)-5-(1H-pyrazol-1-yl)phenol;
2-(6-((2, 6-di-methylpiperidin-4-yl)oxy)pyridazin-3-yl)-5-(1H-pyrazol-1-yl)phenol;
5-(1H-pyrazol-1-yl)-2-(6-(pyrrolidin-3-yloxy)pyridazin-3-yl)phenol;
2-(6-((-2-methylpiperidin-4-yl)oxy)pyridazin-3-yl)-5-(1H-pyrazol-1-yl)phenol;
(S)-5-(1H-pyrazol-1-yl)-2-(6-(pyrrolidin-3-ylmethoxy)pyridazin-3-yl)phenol;
(R)-5-(1H-pyrazol-1-yl)-2-(6-(pyrrolidin-3-yl methoxy)pyridazin-3-yl)phenol;
2-(6-((3-fluoropiperidin-4-yl)oxy)pyridazin-3-yl)-5-(1H-pyrazol-1-yl)-phenol;
2-[6-(1,2,2,6,6-pentamethyl-piperidin-4-yloxy)-pyridazin-3-yl]-5-pyrazol-1-yl-phenol;
5-pyrazol-1-yl-2-[6-((2,2,6,6-tetramethylpiperidin-4-yloxy)-pyridazin-3-yl]-phenol;
5-(1H-pyrazol-4-yl)-2-(6-((2,2,6,6-tetramethylpiperidin-4-yl)oxy)pyridazin-3-yl)phenol;
2-(6-piperazin-1-yl-pyridazin-3-yl)-5-pyrazol-1-yl-phenol;
3-[6-(azetidin-3-yl-amino)-pyridazin-3-yl]-naphthalen-2-ol;
2-[6-(azetidin-3-ylamino)-pyridazin-3-yl]-5-pyrazol-1-yl-phenol;
2-[6-(3, 5-di methyl-piperazin-1-yl)-pyridazin-3-yl]-5-pyrazol-1-yl-phenol;
2-[6-(7-methyl-2,7-diaza-spiro[4.4]non-2-yl)-pyridazin-3-yl]-5-pyrazol-1-yl-phenol;
2-(6-[1,4]diazepan-1-yl-pyridazin-3-yl)-5-pyrazol-1-yl-phenol;
2-{6-[4-(2-hydroxy-ethyl)-piperazin-1-yl]-pyridazin-3-yl}-5-pyrazol-1-yl-phenol;
2-[6-(3, 6-diaza-bicyclo[3.2.1]oct-3-yl)-pyridazin-3-yl]-5-pyrazol-1-yl-phenol;
2-[6-(2, 7-diaza-spiro[3.5]non-7-yl)-pyridazin-3-yl]-5-pyrazol-1-yl-phenol;
2-[6-(3-hydroxy-methyl-piperazin-1-yl)-pyridazin-3-yl]-5-pyrazol-1-yl-phenol;
2-[6-(1,7-diaza-spiro[4.4]non-7-yl)-pyridazin-3-yl]-5-pyrazol-1-yl-phenol;
2-[6-(4-amino-4-methyl-piperidin-1-yl)-pyridazin-3-yl]-5-pyrazol-1-yl-phenol;
2-[6-(3-dimethyl-amino-piperidin-1-yl)-pyridazin-3-yl]-5-pyrazol-1-yl-phenol;
2-[6-(12,2,6,6-pentamethyl-piperidin-4-ylamino)-pyridazin-3-yl]-5-pyrazol-1-yl-phenol;
2-[6-(3,3-dimethyl-piperazin-1-yl)-pyridazin-3-yl]-5-pyrazol-1-yl-phenol;
2-(6-(7-(2-hydroxyethyl)-2,7-diazaspiro[4.4]-nonan-2-yl)pyridazin-3-yl)-5-(1H-pyrazol-1-yl)phenol;
2-(6-((3aR,6aS)-hexahydropyrrolo[3,4-c]pyrrol-2(1H)-yl)pyridazin-3-yl)-5-(1H-pyrazol-1-yl)phenol;
3-(6-(piperazin-1-yl)pyridazin-3-yl)naphthalene-2,7-diol;
5-pyrazol-1-yl-2-[6-(1,2,3,6-tetrahydro-pyridin-4-yl)-pyridazin-3-yl]-phenol;

2-(6-piperidin-4-yl-pyridazin-3-yl)-5-pyrazol-1-yl-phenol;
3-(6-(1,2,3,6-tetra-hydropyridin-4-yl)pyridazin-3-yl)naphthalen-2-ol;
3-(6-(1,2,3,6-tetrahydropyridin-4-yl)pyridazin-3-yl)naphthalene-2,7-diol;
3-(6-(2,2,6,6-tetramethyl-1,2,3,6-tetrahydropyridin-4-yl)pyridazin-3-yl)naphthalene-2,7-diol;
3-(6-(1-methyl-1,2,3,6-tetrahydropyridin-4-yl)pyridazin-3-yl)naphthalene-2,7-diol;
3-(6-(piperidin-4-yl)pyridazin-3-yl)naphthalene-2,7-diol;
3-(6-((2,2,6,6-tetramethylpiperidin-4-yl)oxy)pyridazin-3-yl)naphthalene-2,7-diol;
3-(6-(methyl-(2,2,6,6-tetramethylpiperidin-4-yl)amino)pyridazin-3-yl)naphthalene-2,7-diol;
3-(6-((2,2,6,6-tetramethylpiperidin-4-yl)amino)pyridazin-3-yl)naphthalene-2,7-diol;
[3-(7-hydroxy-6-{6-[methyl-(2,2,6,6-tetramethyl-piperidin-4-yl)-amino]-pyridazin-3-yl}-naphthalen-2-yloxy)-propyl]-carbamic acid tert-butyl ester;
7-(3-amino-propoxy)-3-{6-[methyl-(2,2,6,6-tetramethyl-piperidin-4-yl)-amino]-pyridazin-3-yl}naphthalen-2-ol;
N-[3-(7-hydroxy-6-{6-[methyl-(2,2,6,6-tetramethyl-piperidin-4-yl)-amino]-pyridazin-3-yl}naphthalen-2-yloxy)-propyl]-acetamide;
7-(3-hydroxypropoxy)-3-(6-(methyl(2,2,6,6-tetramethylpiperidin-4-yl)amino)pyridazin-3-yl)naphthalen-2-ol;
7-(3-methoxypropoxy)-3-(6-(methyl(2,2,6,6-tetramethylpiperidin-4-yl)amino)pyridazin-3-yl)naphthalen-2-ol;
7-(2-morpholinoethoxy)-3-(6-((2,2,6,6-tetramethylpiperidin-4-yl)oxy)pyridazin-3-yl)naphthalen-2-ol;
3-(6-(piperidin-4-ylmethyl)pyridazin-3-yl)naphthalen-2-ol;
5-(1H-pyrazol-1-yl)-2-(6-((2,2,6,6-tetramethylpiperidin-4-yl)methyl)pyridazin-3-yl)phenol;
3-methoxy-2-(6-(methyl(2,2,6-trimethylpiperidin-4-yl)amino)pyridazin-3-yl)-5-(5-methyloxazol-2-yl)phenol;
2-(6-((6S)-6-((S)-1-hydroxyethyl)-2,2-dimethylpiperidin-4-yloxy)pyridazin-3-yl)-5-(1H pyrazol-1-yl)phenol;
7-hydroxy-6-(6-(methyl(2,2,6,6-tetramethylpiperidin-4-yl)amino)pyridazin-3-yl)-2-naphthonitrile;
3-(6-(methyl(2,2,6,6-tetramethylpiperidin-4-yl)amino)pyridazin-3-yl)-7-(piperidin-1-ylmethyl)naphthalen-2-ol;
3-(6-(methyl(2,2,6,6-tetramethylpiperidin-4-yl)amino)pyridazin-3-yl)-7-(pyrrolidin-1-ylmethyl)naphthalen-2-ol;
1-bromo-6-(6-(methyl(2,2,6,6-tetramethylpiperidin-4-yl)amino)pyridazin-3-yl)naphthalene-2,7-diol;
1-chloro-6-(6-(methyl(2,2,6,6-tetramethylpiperidin-4-yl)amino)pyridazin-3-yl)naphthalene- 2,7-diol;
7-methoxy-3-(6-(methyl(2,2,6,6-tetramethylpiperidin-4-yl)amino)pyridazin-3-yl)naphthalen-2-ol;
7-methoxy-3-(6-(methyl(1,2,2,6,6-pentamethylpiperidin-4-yl)amino)pyridazin-3-yl)naphthalen-2-ol;
7-(3,6-dihydro-2H-pyran-4-yl)-3-(6-(methyl(2,2,6,6-tetramethylpiperidin-4-yl)amino)pyridazin-3-yl)naphthalen-2-ol;
3-(6-(methyl-(2,2,6,6-tetramethylpiperidin-4-yl)amino)pyridazin-3-yl)-7-(tetrahydro-2H-pyran-4-yl)naphthalen-2-ol;
7-(difluoromethyl)-3-(6-(methyl-(2,2,6,6-tetramethylpiperidin-4-yl)amino)pyridazin-3-yl)naphthalen-2-ol;
7-((4-hydroxy-2-methylbutan-2-yl)oxy)-3-(6-(methyl(2,2,6,6-tetramethylpiperidin-4-yl)amino)pyridazin-3-yl)naphthalen-2-ol;
7-(3-hydroxy-3-methylbutoxy)-3-(6-(methyl-(2,2,6,6-tetramethylpiperidin-4-yl)amino)pyridazin-3-yl)naphthalen-2-ol;
2-(6-(methyl(2,2,6,6-tetramethylpiperidin-4-yl)amino)pyridazin-3-yl)-5-(1H-pyrazol-4-yl)benzene-1,3-diol;
3-methoxy-2-(6-(methyl-(2,2,6,6-tetramethylpiperidin-4-yl)amino)pyridazin-3-yl)-5-(1H pyrazol-4-yl)phenol;
5-(1H-pyrazol-4-yl)-2-(6-((2,2,6,6-tetramethylpiperidin-4-yl)amino)pyridazin-3-yl)-3-(trifluoromethoxy)phenol;
2-(6-(methyl(2,2,6,6-tetramethylpiperidin-4-yl)amino)pyridazin-3-yl)-5-(1-methyl-1H-pyrazol-4-yl)-3-(trifluoromethoxy)phenol;
2-(6-(methyl(2,2,6,6-tetramethylpiperidin-4-yl)amino)pyridazin-3-yl)-5-(1H-pyrazol-4-yl)-3-(trifluoromethoxy)phenol;
4-(3-hydroxy-4-(6-(methyl-(2,2,6,6-tetramethylpiperidin-4-yl)amino)pyridazin-3-yl)-5-(trifluoromethoxy)phenyl)-1-methylpyridin-2(1H)-one;
3-methoxy-2-(6-(methyl-(2,2,6,6-tetramethylpiperidin-4-yl)amino)pyridazin-3-yl)-5-(1-methyl-1H-pyrazol-4-yl)phenol;
3-methoxy-2-(6-(methyl-(2,2,6,6-tetramethylpiperidin-4-yl)amino)pyridazin-3-yl)-5-(5,6,7,8-tetrahydroimidazo[1,2-a]pyridin-3-yl)phenol;
3-methoxy-2-(6-(methyl-(2,2,6,6-tetramethylpiperidin-4-yl)amino)pyridazn-3-yl)-5-(pyridin-3-yl)phenol;
5-(1-cyclopentyl-1H-pyrazol-4-yl)-3-methoxy-2-(6-(methyl(2,2,6,6-tetra methylpiperidin-4-yl)amino)pyridazin-3-yl)phenol;
3'5-dimethoxy-4-(6-(methyl-(2,2,6,6-tetramethylpiperidin-4-yl)amino)pyridazin-3-yl)-[1,1'-biphenyl]-3-ol;
3-(benzyloxy)-2-(6-(methyl-(2,2,6,6-tetramethylpiperidin-4-yl)amino)pyridazin-3-yl)-5-(5-methyloxazol-2-yl)phenol;
3-ethoxy-2-(6-(methyl-(2,2,6,6-tetramethylpiperidin-4-yl)amino)pyridazin-3-yl)-5-(5-methyloxazol-2-yl)phenol;
5 3-(cyclopropylmethoxy)-2-(6-(methyl-(2,2,6,6-tetramethylpiperidin-4-yl)amino)-pyridazin-3-yl)-5-(5-methyloxazol-2-yl)phenol;
2-methyl-5-(6-(methyl-(2,2,6,6-tetramethylpiperidin-4-yl)amino)pyridazin-3-yl)-1H benzo[d]imidazol-6-ol;
5-chloro-2-(6-(methyl-(2,2,6,6-tetramethylpiperidin-4-yl)amino)pyridazin-3-yl)phenol;

5-(1H-pyrazol-1-yl)-2-(6-((2,2,6,6-tetramethylpiperidin-4-yl)amino)pyridazin-3-yl)phenol;
3-hydroxy-4-(6-((2,2,6,6-tetramethylpiperidin-4-yl)amino)pyridazin-3-yl)benzonitrile;
2-(6-((2,2-dimethylpiperidin-4-yl)oxy)pyridazin-3-yl)-5-(1H-pyrazol-1-yl)phenol;
2-(6-(methyl-(2,2,6,6-tetramethylpiperidin-4-yl)amino)pyridazin-3-yl)-4-(1H-pyrazol-4-yl)phenol;
2-(6-(methyl-(2,2,6,6-tetramethylpiperidin-4-yl)amino)pyridazin-3-yl)-4-(4,5,6,7-tetrahydropyrazolo[1,5-a]pyridin-3-yl)phenol;
2-(6-(methyl-(2,2,6,6-tetramethylpiperidin-4-yl)amino)pyridazin-3-yl)-4-(4,5,6,7-tetrahydropyrazolo[1,5-a] pyrazin-3-yl)phenol;
4-(1H-indol-2-yl)-2-(6-(methyl-(2,2,6,6-tetramethylpiperidin-4-yl)amino)pyridazin-3-yl)phenol;
4-(cyclopent-1-en-1-yl)-2-(6-(methyl(2,2,6,6-tetramethylpiperidin-4-yl)amino)pyridazin-3-yl)phenol;
2-(6-(methyl-(2,2,6,6-tetramethylpiperidin-4-yl)amino)pyridazin-3-yl)-4-(1H-pyrazol-3-yl)phenol;
4-(4-hydroxy-3-(6-(methyl-(2,2,6,6-tetramethylpiperidin-4-yl)amino)pyridazin-3 yl)phenyl)pyridin-2-ol;
4-(4-hydroxy-3-(6-((2,2,6,6-tetramethylpiperidin-4-yl)oxy)pyridazin-3-yl)phenyl)-1-methylpyridin-2-(1H)-one;
4-(4-hydroxy-3-(6-((2,2,6,6-tetramethylpiperidin-4-yl)oxy)pyridazin-3-yl)phenyl)pyridin-2-ol;
5-(1H-indazol-7-yl)-2-(6-(methyl-(2,2,6,6-tetramethylpiperidin-4-yl)amino)pyridazin-3-yl)phenol;
4-chloro-2-(6-(methyl-(2,2,6,6-tetramethylpiperidin-4-yl)amino)pyridazin-3-yl)-5-(1H-pyrazol-4-yl)phenol;
4-fluoro-2-(6-(methyl-(2,2,6,6-tetramethylpiperidin-4-yl)amino)pyridazin-3-yl)-5-(1H-pyrazol-4-yl)phenol;
5-fluoro-4-(1H-imidazol-4-yl)-2-(6-(methyl-(2,2,6,6-tetramethylpiperidin-4-yl)amino)pyridazin-3-yl)phenol;
5-fluoro-2-(6-(methyl-(2,2,6,6-tetramethylpiperidin-4-yl)amino)pyridazin-3-yl)-4-(1H-pyrazol-4-yl)phenol;
5-fluoro-2-(6-(methyl-(2,2,6,6-tetramethylpiperidin-4-yl)amino)pyridazin-3-yl)-4-(1H-pyrazol-5-yl)phenol;
5,6-hydroxy-5-(6-(methyl-(2,2,6,6-tetramethylpiperidin-4-yl)amino)pyridazin-3-yl)-2,3-dihydro-1H-inden-1-one;
6-(6-(methyl-(2,2,6,6-tetramethylpiperidin-4-yl)amino)pyridazin-3-yl)-1,4-dihydroindeno[1,2-c]pyrazol-7-ol;
6-hydroxy-5-(6-(methyl-(2,2,6,6-tetramethylpiperidin-4-yl)amino)pyridazin-3-yl)-2,3-dihydro-1H-inden-1-one oxime hydrochloride salt;
5-(6-(methyl-(2,2,6,6-tetramethylpiperidin-4-yl)amino)pyridazin-3-yl)-2,3-dihydro-1H-indene-1,6-diol;
2-amino-6-(6-(methyl-(2,2,6,6-tetramethylpiperidin-4-yl)amino)pyridazin-3-yl)-8H-indeno[1,2-d]thiazol-5-ol hydrochloride salt;
15 9-(6-(methyl-(2,2,6,6-tetramethylpiperidin-4-yl)amino)pyridazin-3-yl)-5,6-dihydroimidazo[5, 1-a]isoquinolin-8-ol hydrochloride salt;
4-hydroxy-3-(6-(methyl-(2,2,6,6-tetramethylpiperidin-4-yl)amino)pyridazin-3-yl)-N-((1-methyl-1H-pyrazol-4-yl)methyl)benzamide;
4-(4-(hydroxymethyl)-1H-pyrazol-1-yl)-2-(6-(methyl(2,2,6,6-tetramethylpiperidin-4-yl)amino)pyridazin-3-yl)phenol;
5-(1H-pyrazol-4-yl)-2-(6-((2,2,6,6-tetramethylpiperidin-4-yl)methyl)pyridazin-3-yl)phenol;
6-(3-(benzyloxy)isoquinolin-6-yl)-N-methyl-N-(2,2,6,6-tetramethylpiperidin-4-yl)pyridazin-3-amine;
6-(1-(benzyloxy)isoquinolin-7-yl)-N-methyl-N-(2,2,6,6-tetramethylpiperidin-4-yl)pyridazin-3-amine;
3-fluoro-5-(2-methoxypyridin-4-yl)-2-(6-(methyl-(2,2,6,6-tetramethylpiperidin-4-yl)amino)pyridazin-3-yl)phenol hydrochloride salt;
4-(3-fluoro-5-hydroxy-4-(6-(methyl-(2,2,6,6-tetramethylpiperidin-4-yl)amino)pyridazin-3-yl)phenyl)pyridin-2(1H)-one hydrochloride salt;
30 4-(3-fluoro-5-hydroxy-4-(6-(methyl-(2,2,6,6-tetramethylpiperidin-4-yl)amino)pyridazin-3-yl)phenyl)-1-methylpyridin-2(1H)-one hydrochloride salt;
5-(3-fluoro-5-hydroxy-4-(6-(methyl-(2,2,6,6-tetramethylpiperidin-4-yl)amino)pyridazin-3-yl)phenyl)-1-methylpyridin-2(1H)-one hydrochloride salt;
3-fluoro-5-(1H-pyrazol-4-yl)-2-(6-((2,2,6,6-tetramethylpiperidin-4-yl)oxy)pyridazin-3-yl)phenol hydrochloride salt;
5-chloro-3-fluoro-2-(6-(methyl-(2,2,6,6-tetramethylpiperidin-4-yl)amino)pyridazin-3-yl)phenol hydrochloride salt;
3-fluoro-2-(6-(methyl-(2,2,6,6-tetramethylpiperidin-4-yl)amino)pyridazin-3-yl)-5-(1H-pyrazol-4-yl)phenol hydrochloride salt;
3-fluoro-2-(6-(methyl-(2,2,6,6-tetramethylpiperidin-4-yl)amino)pyridazin-3-yl)-5-(1-methyl-1H pyrazol-4-yl)phenol hydrochloride salt;
5-(5-methoxypyridin-3-yl)-2-(6-(methyl-(2,2,6,6-tetramethylpiperidin-4-yl)amino)pyridazin-3-yl)phenol;
5-(3-hydroxy-4-(6-methyl-(2,2,6,6-tetramethylpiperidin-4-yl)amino)pyridazin-3-yl) phenyl)pyridin-2-ol;
4-(3-hydroxy-4-(6-methyl-(2,2,6,6-tetramethylpiperidin-4-yl)amino)pyridazin-3-yl)phenyl)pyridin-2-ol;
5-(6-methoxypyridin-3-yl)-2-(6-(methyl-(2,2,6,6-tetramethylpiperidin-4-yl)amino)pyridazin-3-yl)phenol;
5-(3-hydroxy-4-(6-(methyl-(2,2,6,6-tetramethylpiperidin-4-yl)amino)pyridazin-3-yl)phenyl)-3-(trifluoromethyl)pyridin-2-ol;
5-(3-hydroxy-4-(6-(methyl-(2,2,6,6-tetramethylpiperidin-4-yl)amino)pyridazin-3-yl)phenyl)-1-methylpyridin-2(1H)-one;
4-(3-hydroxy-4-(6-(methyl-(2,2,6,6-tetramethylpiperidin-4-yl)amino)pyridazin-3-yl)phenyl)-1-methylpyridin-2(1H)-one;
5-(2-methoxypyridin-4-yl)-2-(6-(methyl-(2,2,6,6-tetramethylpiperidin-4-yl)amino)pyridazin-3-yl)phenol;
4-(3-hydroxy-4-(6-((2,2,6,6-tetramethylpiperidin-4-yl)oxy)pyridazin-3-yl)phenyl)pyridin-2-ol;
5-(6-(dimethylamino)pyridin-3-yl)-2-(6-(methyl(2,2,6,6-tetramethylpiperidin-4-yl)amino)pyridazin-3-yl)phenol;
4-(3-hydroxy-4-(6-((2,2,6,6-tetramethylpiperidin-4-yl)oxy)pyridazin-3-yl)phenyl)-1-methylpyridin-2(1H)-one;
2-(6-(methyl-(2,2,6,6-tetramethylpiperidin-4-yl)amino)pyridazin-3-yl)-5-(pyrimidin-5-yl)phenol;
5-(3-hydroxy-4-(6-(methyl-(2,2,6,6-tetramethylpiperidin-4-yl)amino)pyridazin-3-yl) phenyl)pyridin-3-ol;
1-cyclopropyl-4-(3-hydroxy-4-(6-(methyl(2,2,6,6-tetra methylpiperidin-4-yl)amino)pyridazin-3-yl)phenyl)pyridin-2(1H)-one;
2-(6-(methyl-(2,2,6,6-tetramethylpiperidin-4-yl)amino)pyridazin-3-yl)-5-(1,2,3,6-tetrahydropyridin-4-yl)phenol;
5-(cyclopent-1-en-1-yl)-2-(6-(methyl(2,2,6,6-tetramethylpiperidin-4-yl)amino)pyridazin-3-yl)phenol;
5-(3,6-dihydro-2H-pyran-4-yl)-2-(6-(methyl(2,2,6,6-tetramethylpiperidin-4-yl)amino)pyridazin-3-yl)phenol;
5-(imidazo[1,5-a]pyridin-7-yl)-2-(6-(methyl(2,2,6,6-tetramethylpiperidin-4-yl)amino)pyridazin-3-yl)phenol;
5-(imidazo[1,2-a]pyridin-7-yl)-2-(6-(methyl(2,2,6,6-tetramethylpiperidin-4-yl)amino)pyridazin-3-yl)phenol;
2-(6-(methyl(2,2,6,6-tetramethylpiperidin-4-yl)amino)pyridazin-3-yl)-5-(2-methylpyridin-4-yl)phenol;
5-(1H-imidazol-2-yl)-2-(6-(methyl-(2,2,6,6-tetramethylpiperidin-4-yl)amino)pyridazin-3-yl)phenol;
5-(1H-imidazol-4-yl)-2-(6-(methyl-(2,2,6,6-tetramethylpiperidin-4-yl)amino)pyridazin-3-yl)phenol;
5-(imidazo[1,2-a]pyrazin-3-yl)-2-(6-(methyl(2,2,6,6-tetramethylpiperidin-4-yl)amino)pyridazin-3-yl)phenol;
2-(6-(methyl-(2,2,6,6-tetramethylpiperidin-4-yl)amino)pyridazin-3-yl)-5-(5,6,7,8-tetrahydroimidazo[1,2-a]pyrazin-3-yl)phenol;
2-(6-(methyl-(2,2,6,6-tetramethylpiperidin-4-yl)amino)pyridazin-3-yl)-5-(4-methyl-1H-imidazol-2-yl)phenol;
2-(6-(methyl-(2,2,6,6-tetramethylpiperidin-4-yl)amino)pyridazin-3-yl)-5-(1-methyl-1H-imidazol-4-yl)phenol;
2-(6-(methyl-(2,2,6,6-tetramethylpiperidin-4-yl)amino)pyridazin-3-yl)-5-(1-methyl-1Himidazol-5-yl)phenol;
2-(6-(methyl-(2,2,6,6-tetramethylpiperidin-4-yl)amino)pyridazin-3-yl)-5-(4-nitro-1H-imidazol-2-yl)phenol;
2-(6-(methyl-(2,2,6,6-tetramethylpiperidin-4-yl)amino)pyridazin-3-yl)-5-(2-methyl-1Himidazol-4-yl)phenol;
5-(1,2-dimethyl-1H-imidazol-4-yl)-2-(6-(methyl(2,2,6,6-tetramethylpiperidin-4-yl)amino)pyridazin-3-yl)phenol;
1-(3-hydroxy-4-(6-(methyl-(2,2,6,6-tetramethylpiperidin-4-yl)amino)pyridazin-3-yl)phenyl)-1H-pyrazole-4-carboxamide;
2-(6-((3aR,6aS)-5-(2-hydroxyethyl)hexahydropyrrolo[3,4-c]pyrrol-2(1H)-yl)pyridazin-3-yl)-5-(1H-pyrazol-4-yl)phenol;
2-(6-((3aR,6aS)-hexahydropyrrolo[3,4-c]pyrrol-2(1H)-yl)pyridazin-3-yl)-5-(1H-pyrazol-4-yl)phenol;
2-(6-((3aR,6aS)-5-methylhexahydropyrrolo[3,4-c]pyrrol-2(1H)-yl)pyridazin-3-yl)-5-(1 Hpyrazol-4-yl)phenol;
4-(3-hydroxy-4-(6-(5-methylhexahydropyrrolo[3,4-c]pyrrol-2(1H)-yl)pyridazin-3-yl)phenyl)-1-methylpyridin-2(1H)-one;
4-(3-hydroxy-4-(6-((3aR,6aR)-1-methylhexahydropyrrolo[3,4-b]pyrrol-5(1H)-yl)pyridazin-3-yl)phenyl)-1-methylpyridin-2(1H)-one;
2-(6-(2,7-diazaspiro[4.5]decan-2-yl)pyridazin-3-yl)-5-(1H-pyrazol-4-yl)phenol; and 4-(4-(6-(2,7-diazaspiro[4.5]decan-2-yl)pyridazin-3-yl)-3-hydroxyphenyl)-1-methylpyridin-2(1H)-one;

or a pharmaceutically acceptable salt thereof;
for use in the treatment, prevention and/or delay of progression of cancer.

In a particular embodiment, the present invention relates to a FoxM1 gene splicing modifier as described herein selected from the group consisting of:

5-(2-Methoxy-4-(1H-pyrazol-1-yl)phenyl)-N-methyl-N-(2,2,6,6-tetramethylpiperidin-4-yl)-1,3,4-thiadiazol-2-amine;
6-(5-(Methyl(2,2,6,6-tetramethylpiperidin-4-yl)amino)-1,3,4-thiadiazol-2-yl)naphthalen-2-ol;
5-(2-Methoxyquinolin-3-yl)-N-methyl-N-(2,2,6,6-tetramethylpiperidin-4-yl)-1,3,4-thiadiazol-2-amine;
5-(3-Methoxynaphthalen-2-yl)-N-methyl-N-(2,2,6,6-tetramethylpiperidin-4-yl)-1,3,4-thiadiazol-2-amine;
5-(2-Methoxy-4-(1H-pyrazol-1-yl)phenyl)-N-(1,2,2,6,6-pentamethylpiperidin-4-yl)-1,3,4-thiadiazol-2-amine;
5-(2-Methoxy-4-(1-methyl-1H-pyrazol-4-yl)phenyl)-N-methyl-N-(2,2,6,6-tetramethylpiperidin-4-yl)-1,3,4-thiadiazol-2-amine;
5-(2-Methoxy-4-(1H-pyrazol-4-yl)phenyl)-N-methyl-N-(2,2,6,6-tetramethylpiperidin-4-yl)-1,3,4-thiadiazol-2-amine;
4-(3-Methoxy-4-(5-(methyl(2,2,6,6-tetramethylpiperidin-4-yl)amino)-1,3,4-thiadiazol-2-yl)phenyl)-1-methylpyridin-2(1H)-one;
5-(3-Methoxy-4-(5-(methyl(2,2,6,6-tetramethylpiperidin-4-yl)amino)-1,3,4-thiadiazol-2-yl)phenyl)pyridin-2-ol;
5-(3-Methoxy-4-(5-(methyl(2,2,6,6-tetramethylpiperidin-4-yl)amino)-1,3,4-thiadiazol-2-yl)phenyl)-1-methylpyridin-2(1H)-one;
N-Methyl-5-(2-methyl-4-(1-methyl-1H-pyrazol-4-yl)phenyl)-N-(2,2,6,6-tetramethylpiperidin-4-yl)-1,3,4-thiadiazol-2-amine;
1-Methyl-4-(4-(5-(methyl(2,2,6,6-tetramethylpiperidin-4-yl)amino)-1,3,4-thiadiazol-2-yl)-3-(trifluoromethoxy)phenyl)pyridin-2(1H)-one;
5-(4-(3,5-Dimethyl-1H-pyrazol-4-yl)-2-methoxyphenyl)-N-methyl-N-(2,2,6,6-tetramethylpiperidin-4-yl)-1,3,4-thiadiazol-2-amine;
5-(2-Methoxy-4-(1-methyl-1H-pyrazol-4-yl)phenyl)-N-methyl-N-(2,2,6,6-tetramethylpiperidin-4-yl)-1,3,4-thiadiazol-2-amine;
2-(5-(Methyl(2,2,6,6-tetramethylpiperidin-4-yl)amino)-1,3,4-thiadiazol-2-yl)-5-(1-methyl-1H-pyrazol-4-yl)phenol;
2-(5-(Methyl(2,2,6,6-tetramethylpiperidin-4-yl)amino)-1,3,4-thiadiazol-2-yl)-5-(1H-pyrazol-1-yl)phenol;
5-(3-Hydroxy-4-(5-(methyl(2,2,6,6-tetramethylpiperidin-4-yl)amino)-1,3,4-thiadiazol-2-yl)phenyl)-1-methylpyridin-2(1H)-one;
4-(3-Hydroxy-4-(5-(methyl(2,2,6,6-tetramethylpiperidin-4-yl)amino)-1,3,4-thiadiazol-2-yl)phenyl)-1-methylpyridin-2(1H)-one;
5-(3-Hydroxy-4-(5-(methyl(2,2,6,6-tetramethylpiperidin-4-yl)amino)-1,3,4-thiadiazol-2-yl)phenyl)pyridin-2-ol;
3-(5-(Methyl(2,2,6,6-tetramethylpiperidin-4-yl)amino)-1,3,4-thiadiazol-2-yl)naphthalene-2,7-diol;
3-(5-((3aR,6aS)-Hexahydropyrrolo[3,4-c]pyrrol-2(1H)-yl)-1,3,4-thiadiazol-2-yl)naphthalene-2,7-diol;
3-(5-(Methyl(2,2,6,6-tetramethylpiperidin-4-yl)amino)-1,3,4-thiadiazol-2-yl)naphthalen-2-ol hydrobromide salt;
3-(5-(Methyl(2,2,6,6-tetramethylpiperidin-4-yl)amino)-1,3,4-thiadiazol-2-yl)quinolin-2-ol;
2-(5-(Methyl(2,2,6,6-tetramethylpiperidin-4-yl)amino)-1,3,4-thiadiazol-2-yl)-4-(1H-pyrazol-1-yl)phenol;
5-(2-Chloro-4-(1-methyl-1H-pyrazol-4-yl)phenyl)-N-methyl-N-(2,2,6,6-tetramethylpiperidin-4-yl)-1,3,4-thiadiazol-2-amine;
3-Chloro-2-(5-(methyl(2,2,6,6-tetramethylpiperidin-4-yl)amino)-1,3,4-thiadiazol-2-yl)-5-(1-methyl-1H-pyrazol-4-yl)phenol;
5-(2-Chloro-4-(1-methyl-1H-pyrazol-4-yl)phenyl)-N-(2,2,6,6-tetramethylpiperidin-4-yl)-1,3,4-thiadiazol-2-amine;
3-Methoxy-2-(5-(methyl(2,2,6,6-tetramethylpiperidin-4-yl)amino)-1,3,4-thiadiazol-2-yl)-5-(5-methyloxazol-2-yl)phenol;
2-(2-Methoxy-4-(1H-pyrazol-1-yl)phenyl)-5-(1,2,3,6-tetrahydropyridin-4-yl)-1,3,4-thiadiazole; 2-(5-(piperazin-1-yl)-1,3,4-thiadiazol-2-yl)-5-(1H-pyrazol-1-yl)phenol;
5-(7-Methoxyquinolin-6-yl)-N-methyl-N-(2,2,6,6-tetramethylpiperidin-4-yl)-1,3,4-thiadiazol-2-amine;
6-(5-(Methyl(2,2,6,6-tetramethylpiperidin-4-yl)amino)-1,3,4-thiadiazol-2-yl)quinolin-7-ol; 3-methoxy-4-(5-(methyl(2,2,6,6-tetramethylpiperidin-4-yl)amino)-1,3,4-thiadiazol-2-yl)benzonitrile;
3-fluoro-4-(5-(methyl(2,2,6,6-tetramethylpiperidin-4-yl)amino)-1,3,4-thiadiazol-2-yl)benzonitrile;
methyl 3-fluoro-4-(5-(methyl(2,2,6,6-tetramethylpiperidin-4-yl)amino)-1,3,4-thiadiazol-2-yl)benzoate;
5-(2-methoxy-4-(3-(methylamino)-1H-pyrazol-1-yl)phenyl)-N-methyl-N-(2,2,6,6-tetramethylpiperidin-4-yl)-1,3,4-thiadiazol-2-amine;
7-methoxy-6-(5-(methyl(2,2,6,6-tetramethylpiperidin-4-yl)amino)-1,3,4-thiadiazol-2-yl)quinoline-2-carbonitrile;
4-(3-methoxy-4-(5-((2,2,6,6-tetramethylpiperidin-4-yl)oxy)-1,3,4-thiadiazol-2-yl)phenyl)-1-methylpyridin-2(1H)-one;
4-(3-chloro-4-(5-(methyl(2,2,6,6-tetramethylpiperidin-4-yl)amino)-1,3,4-thiadiazol-2-yl)phenyl)-1-methylpyridin-2(1H)-one;
5-(2-Chloro-4-(1H-pyrazol-4-yl)phenyl)-N-methyl-N-(2,2,6,6-tetramethylpiperidin-4-yl)-1,3,4-thiadiazol-2-amine;
5-(2-Chloro-4-(4,5,6,7-tetrahydropyrazolo[1,5-a]pyridin-3-yl)phenyl)-N-methyl-N-(2,2,6,6-tetramethylpiperidin-4-yl)-1,3,4-thiadiazol-2-amine;
N-methyl-5-(5-(1-methyl-1H-pyrazol-4-yl)pyridin-2-yl)-N-(2,2,6,6-tetramethylpiperidin-4-yl)-1,3,4-thiadiazol-2-amine Hydrochloride salt;
2-(2-chloro-4-(1-methyl-1H-pyrazol-4-yl)phenyl)-5-((2,2,6,6-tetramethylpiperidin-4-yl)oxy)-1,3,4-thiadiazole;
5-(2-chloro-4-(6-methoxypyridin-3-yl)phenyl)-N-methyl-N-(2,2,6,6-tetramethylpiperidin-4-yl)-1,3,4-thiadiazol-2-amine;
5-(4-(6-aminopyridin-3-yl)-2-fluorophenyl)-N-methyl-N-(2,2,6,6-tetramethylpiperidin-4-yl)-1,3,4-thiadiazol-2-amine;
5-(2-fluoro-4-(3-methyl-1H-pyrazol-5-yl)phenyl)-N-methyl-N-(2,2,6,6-tetramethylpiperidin-4-yl)-1,3,4-thiadiazol-2-amine;
5-(2-fluoro-4-(1H-pyrazol-5-yl)phenyl)-N-methyl-N-(2,2,6,6-tetramethylpiperidin-4-yl)-1,3,4-thiadiazol-2-amine;
5-(2,3-difluoro-4-(1H-pyrazol-4-yl)phenyl)-N-methyl-N-(2,2,6,6-tetramethylpiperidin-4-yl)-1,3,4-thiadiazol-2-amine;
5-(2,3-difluoro-4-(1H-pyrazol-5-yl)phenyl)-N-methyl-N-(2,2,6,6-tetramethylpiperidin-4-yl)-1,3,4-thiadiazol-2-amine;
5-(2,5-difluoro-4-(1H-pyrazol-4-yl)phenyl)-N-methyl-N-(2,2,6,6-tetramethylpiperidin-4-yl)-1,3,4-thiadiazol-2-amine;
5-(2,5-difluoro-4-(1H-pyrazol-5-yl)phenyl)-N-methyl-N-(2,2,6,6-tetramethylpiperidin-4-yl)-1,3,4-thiadiazol-2-amine;
5-(2,6-difluoro-4-(1H-pyrazol-4-yl)phenyl)-N-methyl-N-(2,2,6,6-tetramethylpiperidin-4-yl)-1,3,4-thiadiazol-2-amine;
2-(2,5-difluoro-4-(1H-pyrazol-4-yl)phenyl)-5-((3aR,6aS)-hexahydropyrrolo[3,4-c]pyrrol-2(1H)-yl)-1,3,4-thiadiazole;

5-(2-chloro-5-fluoro-4-(1H-pyrazol-4-yl)phenyl)-N-methyl-N-(2,2,6,6-tetramethylpiperidin-4-yl)-1,3,4-thiadiazol-2-amine;
5-(3-fluoro-5-(1H-pyrazol-4-yl)pyridin-2-yl)-N-methyl-N-(2,2,6,6-tetramethylpiperidin-4-yl)-1,3,4-thiadiazol-2-amine;
5-(4-(2-aminopyrimidin-4-yl)-2-chlorophenyl)-N-methyl-N-(2,2,6,6-tetramethylpiperidin-4-yl)-1,3,4-thiadiazol-2-amine;
5-(5-(2-aminopyrimidin-4-yl)-2-chlorophenyl)-N-methyl-N-(2,2,6,6-tetramethylpiperidin-4-yl)-1,3,4-thiadiazol-2-amine;
5-(4-(2,4-dimethylthiazol-5-yl)-2,5-difluorophenyl)-N-methyl-N-(2,2,6,6-tetramethylpiperidin-4-yl)-1,3,4-thiadiazol-2-amine;
5-(4-(2,4-dimethylthiazol-5-yl)-2,3-difluorophenyl)-N-methyl-N-(2,2,6,6-tetramethylpiperidin-4-yl)-1,3,4-thiadiazol-2-amine;
4-(3-hydroxy-4-(5-(methyl(2,2,6,6-tetramethylpiperidin-4-yl)amino)-1,3,4-thiadiazol-2-yl)-5-(trifluoromethoxy)phenyl)-1-methylpyridin-2(1H)-one;
5-(2-fluoro-6-methoxy-4-(1H-pyrazol-4-yl)phenyl)-N-methyl-N-(2,2,6,6-tetramethylpiperidin-4-yl)-1,3,4-thiadiazol-2-amine;
2-(2-fluoro-6-methoxy-4-(1H-pyrazol-4-yl)phenyl)-5-((3aR,6aS)-5-methylhexahydropyrrolo[3,4-c]pyrrol-2(1H)-yl)-1,3,4-thiadiazole;
5-(2,3-difluoro-6-methoxy-4-(1H-pyrazol-4-yl)phenyl)-N-methyl-N-(2,2,6,6-tetramethylpiperidin-4-yl)-1,3,4-thiadiazol-2-amine;
6-methoxy-2-(5-(methyl(2,2,6,6-tetramethylpiperidin-4-yl)amino)-1,3,4-thiadiazol-2-yl)-3,4-dihydroisoquinolin-1(2H)-one;
5-(2-Chloro-4-(1H-pyrazol-1-yl)phenyl)-N-methyl-N-(2,2,6,6-tetramethylpiperidin-4-yl)-1,3,4-thiadiazol-2-amine;
5-(2-Chloro-4-(1H-1,2,3-triazol-1-yl)phenyl)-N-methyl-N-(2,2,6,6-tetramethylpiperidin-4-yl)-1,3,4-thiadiazol-2-amine;
5-(2-chloro-4-(2H-1,2,3-triazol-2-yl)phenyl)-N-methyl-N-(2,2,6,6-tetramethylpiperidin-4-yl)-1,3,4-thiadiazol-2-amine;
5-(2-chloro-4-(1H-12,4-triazol-1-yl)phenyl)-N-methyl-N-(2,2,6,6-tetramethylpiperidin-4-yl)-1,3,4-thiadiazol-2-amine;
5-(4-(3-amino-1H-pyrazol-1-yl)-2-chlorophenyl)-N-methyl-N-(2,2,6,6-tetramethylpiperidin-4-yl)-1,3,4-thiadiazol-2-amine;
2-(2-chloro-4-(1H-imidazol-1-yl)phenyl)-5-((3aR,6aS)-5-methylhexahydropyrrolo[3,4-c]pyrrol-2(1H)-yl)-1,3,4-thiadiazole;
5-(2-Chloro-4-(1H-imidazol-1-yl)phenyl)-N-methyl-N-(2,2,6,6-tetramethylpiperidin-4-yl)-1,3,4-thiadiazol-2-amine;
5-(2-fluoro-4-(1H-imidazol-1-yl)phenyl)-N-methyl-N-(2,2,6,6-tetramethylpiperidin-4-yl)-1,3,4-thiadiazol-2-amine;
5-(2-methoxy-4-(1H-pyrazol-5-yl)phenyl)-N-methyl-N-(2,2,6,6-tetramethylpiperidin-4-yl)-1,3,4-thiadiazol-2-amine;
5-(4-(2,4-dimethylthiazol-5-yl)-2-methoxyphenyl)-N-methyl-N-(2,2,6,6-tetramethylpiperidin-4-yl)-1,3,4-thiadiazol-2-amine;
5-(2-methoxy-4-(pyridin-3-yl)phenyl)-N-methyl-N-(2,2,6,6-tetramethylpiperidin-4-yl)-1,3,4-thiadiazol-2-amine;
5-(2-fluoro-4-(1H-pyrazol-4-yl)phenyl)-N-methyl-N-(2,2,6,6-tetramethylpiperidin-4-yl)-1,3,4-thiadiazol-2-amine;
5-(2-methoxy-4-(2-methoxypyridin-4-yl)phenyl)-N-methyl-N-(2,2,6,6-tetramethylpiperidin-4-yl)-1,3,4-thiadiazol-2-amine;
5-(2-methoxy-4-(6-methoxypyridin-3-yl)phenyl)-N-methyl-N-(2,2,6,6-tetramethylpiperidin-4-yl)-1,3,4-thiadiazol-2-amine;
2-(2-Chloro-4-(1-methyl-1H-pyrazol-4-yl)phenyl)-5-((3aR,6aS)-5-methylhexahydropyrrolo[3,4-c]pyrrol-2(1H)-yl)-1,3,4-thiadiazole;
2-(2-chloro-4-(1H-pyrazol-4-yl)phenyl)-5-((3aR,6aS)-5-methylhexahydropyrrolo[3,4-c]pyrrol-2(1H)-yl)-1,3,4-thiadiazole;
2-(2-Chloro-4-(1H-pyrazol-4-yl)phenyl)-5-((3aR,6aR)-1-methylhexahydropyrrolo[3,4-b]pyrrol-5(1H)-yl)-1,3,4-thiadiazole;
1-(4-(5-(2-chloro-4-(1H-pyrazol-4-yl)phenyl)-1,3,4-thiadiazol-2-yl)morpholin-2-yl)-N,N-dimethylmethanamine;
2-(2-chloro-4-(1H-pyrazol-4-yl)phenyl)-5-(2-methyl-2,7-diazaspiro[4.5]decan-7-yl)-1,3,4-thiadiazole;
2-(2-fluoro-4-(1H-pyrazol-4-yl)phenyl)-5-((3aR,6aS)-5-methylhexahydropyrrolo[3,4-c]pyrrol-2(1H)-yl)-1,3,4-thiadiazole;
2-(2-methoxy-4-(1-methyl-1H-pyrazol-4-yl)phenyl)-5-(2,6-diazaspiro[3.5]nonan-2-yl)-1,3,4-thiadiazole;
2-(2-methoxy-4-(1-methyl-1H-pyrazol-4-yl)phenyl)-5-(2,7-diazaspiro[3.5]nonan-2-yl)-1,3,4-thiadiazole;
2-(5-(Methyl(2,2,6,6-tetramethylpiperidin-4-yl)amino)-1,3,4-thiadiazol-2-yl)-5-(1H-pyrazol-1-yl)phenol;
5-(3-chloro-4-(5-(methyl(2,2,6,6-tetramethylpiperidin-4-yl)amino)-1,3,4-thiadiazol-2-yl)phenyl)pyridin-2(1H)-one;

2-(5-(methyl(2,2,6,6-tetramethylpiperidin-4-yl)amino)-1,3,4-thiadiazol-2-yl)-5-(3-(methylamino)-1H-pyrazol-1-yl)phenol;
3-fluoro-2-(5-(methyl(2,2,6,6-tetramethylpiperidin-4-yl)amino)-1,3,4-thiadiazol-2-yl)-5-(1H-pyrazol-4-yl)phenol;
3,4-difluoro-2-(5-(methyl(2,2,6,6-tetramethylpiperidin-4-yl)amino)-1,3,4-thiadiazol-2-yl)-5-(1H-pyrazol-4-yl)phenol;
6-hydroxy-5-(5-(methyl(2,2,6,6-tetramethylpiperidin-4-yl)amino)-1,3,4-thiadiazol-2-yl)-2,3-dihydro-1H-inden-1-one;
2-(5-(methyl(2,2,6,6-tetramethylpiperidin-4-yl)amino)-1,3,4-thiadiazol-2-yl)-5-(1H-pyrazol-4-yl)phenol;
2-(5-(2,6-diazaspiro[3.5]nonan-2-yl)-1,3,4-thiadiazol-2-yl)-5-(1-methyl-1H-pyrazol-4-yl)phenol;
2-(5-(2,7-diazaspiro[3.5]nonan-2-yl)-1,3,4-thiadiazol-2-yl)-5-(1-methyl-1H-pyrazol-4-yl)phenol;
3-fluoro-2-(5-((3aR,6aS)-hexahydropyrrolo[3,4-c]pyrrol-2(1H)-yl)-1,3,4-thiadiazol-2-yl)-5-(1H-pyrazol-4-yl)phenol Di-hydrochloride salt;
3-Chloro-2-(5-(methyl(2,2,6,6-tetramethylpiperidin-4-yl)amino)-1,3,4-thiadiazol-2-yl)-5-(1H-pyrazol-4-yl)phenol;
2-(2-methoxy-4-(1H-pyrazol-1-yl)phenyl)-5-((2,2,6,6-tetramethylpiperidin-4-yl)methyl)-1,3,4-thiadiazole;
2-(2,3-difluoro-4-(1H-pyrazol-4-yl)phenyl)-5-(2,7-diazaspiro[3.5]nonan-2-yl)-1,3,4-thiadiazole;
2-(5-(2,7-diazaspiro[3.5]nonan-2-yl)-1,3,4-thiadiazol-2-yl)-3-fluoro-5-(1H-pyrazol-4-yl)phenol;
4-methoxy-1-methyl-3-(5-(methyl(2,2,6,6-tetramethylpiperidin-4-yl)amino)-1,3,4-thiadiazol-2-yl)quinolin-2(1H)-one;
4-hydroxy-1-methyl-3-(5-(methyl(2,2,6,6-tetramethylpiperidin-4-yl)amino)-1,3,4-thiadiazol-2-yl)quinolin-2(1H)-one;
3-(5-(methyl(2,2,6,6-tetramethylpiperidin-4-yl)amino)-1,3,4-thiadiazol-2-yl)quinolin-2(1H)-one;
1-methyl-3-(5-(methyl(2,2,6,6-tetramethylpiperidin-4-yl)amino)-1,3,4-thiadiazol-2-yl)quinolin-2(1H)-one;
2-(2-Chloro-4-(1H-pyrazol-4-yl)phenyl)-5-((3aR,6aS)-hexahydropyrrolo[3,4-c]pyrrol-2(1H)yl)-1,3,4-thiadiazole Hydrochloride Salt;
2-(2-Chloro-4-(1H-pyrazol-4-yl)phenyl)-5-(2,7-diazaspiro[4.5]decan-2-yl)-1,3,4-thiadiazole Hydrochloride Salt;
(R)-(4-(5-(2-chloro-4-(1H-pyrazol-4-yl)phenyl)-1,3,4-thiadiazol-2-yl)piperazin-2-yl)methanol Hydrochloride Salt;
2-(5-(methyl(2,2,6,6-tetramethylpiperidin-4-yl)amino)-1,3,4-thiadiazol-2-yl)benzo[b]thiophene-5-carbonitrile; and
5-(3-chlorobenzo[b]thiophen-2-yl)-N-methyl-N-(2,2,6,6-tetramethylpiperidin-4-yl)-1,3,4-thiadiazol-2-amine;

or a pharmaceutically acceptable salt thereof;
for use in the treatment, prevention and/or delay of progression of cancer.

In a particular embodiment, the present invention relates to a FoxM1 gene splicing modifier selected from the group consisting of:

3-(6-(methyl(2,2,6,6-tetramethylpiperidin-4-yl)amino)pyridazin-3-yl)naphthalen-2-ol;
3-hydroxy-4-(6-(methyl(2,2,6,6-tetramethylpiperidin-4-yl)amino)pyridazin-3-yl)benzonitrile;
5-(1H-pyrazol-4-yl)-2-(6-(methyl(2,2,6,6-tetramethylpiperidin-4-yl)amino)pyridazin-3-yl)phenol
2-(6-(methyl-(2,2,6,6-tetramethylpiperidin-4-yl)amino)pyridazin-3-yl)-5-(1-methyl-1H-pyrazol-4-yl)phenol;
5-pyrazol-1-yl-2-[6-((2,2,6,6-tetramethylpiperidin-4-yloxy)-pyridazin-3-yl]-phenol;
5-(1H-pyrazol-4-yl)-2-(6-((2,2,6,6-tetramethylpiperidin-4-yl)oxy)pyridazin-3-yl)phenol;
3-(6-((2,2,6,6-tetramethylpiperidin-4-yl)oxy)pyridazin-3-yl)naphthalene-2,7-diol;
3-(6-(methyl-(2,2,6,6-tetramethylpiperidin-4-yl)amino)pyridazin-3-yl)naphthalene-2,7-diol;
7-methoxy-3-(6-(methyl(2,2,6,6-tetramethylpiperidin-4-yl)amino)pyridazin-3-yl)naphthalen-2-ol;
5-(3-hydroxy-4-(6-(methyl-(2,2,6,6-tetramethylpiperidin-4-yl)amino)pyridazin-3-yl)phenyl)-1-methylpyridin-2(1H)-one;
4-(3-hydroxy-4-(6-(methyl-(2,2,6,6-tetramethylpiperidin-4-yl)amino)pyridazin-3-yl)phenyl)-1-methylpyridin-2(1H)-one;
4-(3-chloro-4-(5-(methyl(2,2,6,6-tetramethylpiperidin-4-yl)amino)-1,3,4-thiadiazol-2-yl)phenyl)-1-methylpyridin-2(1H)-one;
5-(2-Chloro-4-(1H-pyrazol-4-yl)phenyl)-N-methyl-N-(2,2,6,6-tetramethylpiperidin-4-yl)-1,3,4-thiadiazol-2-amine;
5-(2,5-difluoro-4-(1H-pyrazol-5-yl)phenyl)-N-methyl-N-(2,2,6,6-tetramethylpiperidin-4-yl)-1,3,4-thiadiazol-2-amine;

or a pharmaceutically acceptable salt thereof;
for use in the treatment, prevention and/or delay of progression of cancer.

In a particular embodiment, the present invention relates to the use of a FoxM1 gene splicing modifier as described herein for the preparation of a medicament for the treatment, prevention and/or delay of progression of cancer.

In a particular embodiment, the present invention relates to the use of a FoxM1 gene splicing modifier as described herein for the treatment, prevention and/or delay of progression of cancer.

In a particular embodiment, the present invention relates to a method for the treatment, prevention and/or delay of progression of cancer comprising administering an effective amount of a FoxM1 gene splicing modifier as described

herein to a subject, in particular to a mammal.

In a particular embodiment, the present invention relates to a pharmaceutical composition comprising a FoxM1 gene splicing modifier as described herein for use in the treatment, prevention and/or delay of progression of cancer.

In a particular embodiment, the present invention relates to a combination comprising a therapeutically effective amount of a FoxM1 gene splicing modifier as described herein or a pharmaceutically acceptable salt thereof and one or more therapeutically active co-agents.

## MANUFACTURING PROCESSES

Compounds of formula (I) can be prepared according to methods as disclosed in WO 2014/028459 (A1), which is herewith incorporated by reference. General synthetic processes are described therein on pages 34 to 37 and specific preparations of working examples on pages 37 to 188.

Compounds of formula (VI) can be prepared according to methods as disclosed in WO 2014/116845 (A1), which is herewith incorporated by reference. General synthetic processes are described therein on pages 38 to 41 and specific preparations of working examples on pages 41 to 123.

## PHARMACEUTICAL COMPOSITIONS, ADMINISTRATION AND USES

Another embodiment provides pharmaceutical compositions or medicaments containing the compounds of the invention and a therapeutically inert carrier, diluent or excipient, as well as methods of using the compounds of the invention to prepare such compositions and medicaments. In one example, compounds of formula I may be formulated by mixing at ambient temperature at the appropriate pH, and at the desired degree of purity, with physiologically acceptable carriers, i.e., carriers that are non-toxic to recipients at the dosages and concentrations employed into a galenical administration form. The pH of the formulation depends mainly on the particular use and the concentration of compound, but preferably ranges anywhere from about 3 to about 8. In one example, a compound of formula I is formulated in an acetate buffer, at pH 5. In another embodiment, the compounds of formula I are sterile. The compound may be stored, for example, as a solid or amorphous composition, as a lyophilized composition or as an aqueous solution.

Compositions are formulated, dosed, and administered in a fashion consistent with good medical practice. Factors for consideration in this context include the particular disorder being treated, the particular mammal being treated, the clinical condition of the individual patient, the cause of the disorder, the site of delivery of the agent, the method of administration, the scheduling of administration, and other factors known to medical practitioners. The "effective amount" of the compound to be administered will be governed by such considerations, and is the minimum amount necessary to modify FoxM1 gene splicing. For example, such amount may be below the amount that is toxic to normal cells, or the mammal as a whole.

The compounds of the invention may be administered by any suitable means, including oral, topical(including buccal and sublingual), rectal, vaginal, transdermal, parenteral, subcutaneous, intraperitoneal, intrapulmonary, intradermal, intrathecal and epidural and intranasal, and, if desired for local treatment, intralesional administration. Parenteral infusions include intramuscular, intravenous, intraarterial, intraperitoneal, or subcutaneous administration.

The compounds of the present invention may be administered in any convenient administrative form, e.g., tablets, powders, capsules, solutions, dispersions, suspensions, syrups, sprays, suppositories, gels, emulsions, patches, etc. Such compositions may contain components conventional in pharmaceutical preparations, e.g., diluents, carriers, pH modifiers, sweeteners, bulking agents, and further active agents.

A typical composition is prepared by mixing a compound of the present invention and a carrier or excipient. Suitable carriers and excipients are well known to those skilled in the art and are described in detail in, e.g., Ansel, Howard C., et al., Ansel's Pharmaceutical Dosage Forms and Drug Delivery Systems. Philadelphia: Lippincott, Williams & Wilkins, 2004; Gennaro, Alfonso R., et al. Remington: The Science and Practice of Pharmacy. Philadelphia: Lippincott, Williams & Wilkins, 2000; and Rowe, Raymond C. Handbook of Pharmaceutical Excipients. Chicago, Pharmaceutical Press, 2005. The compositions may also include one or more buffers, stabilizing agents, surfactants, wetting agents, lubricating agents, emulsifiers, suspending agents, preservatives, antioxidants, opaquing agents, glidants, processing aids, colorants, sweeteners, perfuming agents, flavoring agents, diluents and other known additives to provide an elegant presentation of the drug (i.e., a compound of the present invention or pharmaceutical composition thereof) or aidin the manufacturing of the pharmaceutical product (i.e., medicament).

An example of a suitable oral dosage form is a tablet containing about 25mg, 50mg, 100mg, 250mg, or 500mg of the compound of the invention compounded with about 90-30 mg anhydrous lactose, about 5-40 mg sodium croscarmellose, about 5-30mg polyvinylpyrrolidone (PVP) K30, and about 1-10 mg magnesium stearate. The powdered ingredients are first mixed together and then mixed with a solution of the PVP. The resulting composition can be dried, granulated, mixed with the magnesium stearate and compressed to tablet form using conventional equipment. An example of an aerosol composition can be prepared by dissolving the compound, for example 5-400 mg, of the invention

in a suitable buffer solution, e.g. a phosphate buffer, adding a tonicifier, e.g. a salt such sodium chloride, if desired. The solution may be filtered, e.g., using a 0.2 micron filter, to remove impurities and contaminants.

In a particular embodiment, the present invention relates to a pharmaceutical composition comprising a FoxM1 gene splicing modifier as described herein or pharmaceutically acceptable salt thereof.

In a particular embodiment, the present invention relates to a pharmaceutical composition comprising a FoxM1 gene splicing modifier as described herein or pharmaceutically acceptable salt thereof together with one or more pharmaceutically acceptable excipients.

In a particular embodiment, the present invention relates to a pharmaceutical composition comprising a therapeutically effective amount of a FoxM1 gene splicing modifier as described herein or pharmaceutically acceptable salt thereof together with one or more pharmaceutically acceptable excipients.

In a particular embodiment, the present invention relates to a combination comprising a therapeutically effective amount of a FoxM1 gene splicing modifier as described herein or pharmaceutically acceptable salt thereof and one or more other therapeutically active pharmaceutical ingredients.

In specific embodiments, the cancer treated by the compounds of the present invention is leukemia, acute myeloid leukemia, colon cancer, gastric cancer, macular degeneration, acute monocytic leukemia, breast cancer, hepatocellular carcinoma, cone-rod dystrophy, alveolar soft part sarcoma, myeloma, skin melanoma, prostatitis, pancreatitis, pancreatic cancer, retinitis, adenocarcinoma, adenoiditis, adenoid cystic carcinoma, cataract, retinal degeneration, gastrointestinal stromal tumor, Wegener's granulomatosis, sarcoma, myopathy, prostate adenocarcinoma, Hodgkin's lymphoma, ovarian cancer, non-Hodgkin's lymphoma, multiple myeloma, chronic myeloid leukemia, acute lymphoblastic leukemia, renal cell carcinoma, transitional cell carcinoma, colorectal cancer, chronic lymphocytic leukemia, anaplastic large cell lymphoma, kidney cancer, breast cancer, cervical cancer.

In specific embodiments, the cancer prevented and/or treated in accordance with the present invention is basal cell carcinoma, goblet cell metaplasia, or a malignant glioma, cancer of the liver, breast, lung, prostate, cervix, uterus, colon, pancreas, kidney, stomach, bladder, ovary, or brain.

In specific embodiments, the cancer prevented and/or treated in accordance with the present invention include, but are not limited to, cancer of the head, neck, eye, mouth, throat, esophagus, esophagus, chest, bone, lung, kidney, colon, rectum or other gastrointestinal tract organs, stomach, spleen, skeletal muscle, subcutaneous tissue, prostate, breast, ovaries, testicles or other reproductive organs, skin, thyroid, blood, lymph nodes, kidney, liver, pancreas, and brain or central nervous system.

Specific examples of cancers that can be prevented and/or treated in accordance with present invention include, but are not limited to, the following: renal cancer, kidney cancer, glioblastoma multiforme, metastatic breast cancer; breast carcinoma; breast sarcoma; neurofibroma; neurofibromatosis; pediatric tumors; neuroblastoma; malignant melanoma; carcinomas of the epidermis; leukemias such as but not limited to, acute leukemia, acute lymphocytic leukemia, acute myelocytic leukemias such as myeloblastic, promyelocytic, myelomonocytic, monocytic, erythroleukemia leukemias and myclodysplastic syndrome, chronic leukemias such as but not limited to, chronic myelocytic (granulocytic) leukemia, chronic lymphocytic leukemia, hairy cell leukemia; polycythemia vera; lymphomas such as but not limited to Hodgkin's disease, non-Hodgkin's disease; multiple myelomas such as but not limited to smoldering multiple myeloma, nonsecretory myeloma, osteosclerotic myeloma, plasma cell leukemia, solitary plasmacytoma and extramedullary plasmacytoma; Waldenstrom's macroglobulinemia; monoclonal gammopathy of undetermined significance; benign monoclonal gammopathy; heavy chain disease; bone cancer and connective tissue sarcomas such as but not limited to bone sarcoma, myeloma bone disease, multiple myeloma, cholesteatoma-induced bone osteosarcoma, Paget's disease of bone, osteosarcoma, chondrosarcoma, Ewing's sarcoma, malignant giant cell tumor, fibrosarcoma ofbone, chordoma, periosteal sarcoma, soft-tissue sarcomas, angiosarcoma (hemangiosarcoma), fibrosarcoma, Kaposi's sarcoma, leiomyosarcoma, liposarcoma, lymphangiosarcoma, neurilemmoma, rhabdomyosarcoma, and synovial sarcoma; brain tumors such as but not limited to, glioma, astrocytoma, brain stem glioma, ependymoma, oligodendroglioma, nonglial tumor, acoustic neurinoma, craniopharyngioma, medulloblastoma, meningioma, pineocytoma, pineoblastoma, and primary brain lymphoma; breast cancer including but not limited to adenocarcinoma, lobular (small cell) carcinoma, intraductal carcinoma, medullary breast cancer, mucinous breast cancer, tubular breast cancer, papillary breast cancer, Paget's disease (including juvenile Paget's disease) and inflammatory breast cancer; adrenal cancer such as but not limited to pheochromocytom and adrenocortical carcinoma; thyroid cancer such as but not limited to papillary or follicular thyroid cancer, medullary thyroid cancer and anaplastic thyroid cancer; pancreatic cancer such as but not limited to, insulinoma, gastrinoma, glucagonoma, vipoma, somatostatin-secreting tumor, and carcinoid or islet cell tumor; pituitary cancers such as but limited to Cushing's disease, prolactin-secreting tumor, acromegaly, and diabetes insipius; eye cancers such as but not limited to ocular melanoma such as iris melanoma, choroidal melanoma, and cilliary body melanoma, and retinoblastoma; vaginal cancers such as squamous cell carcinoma, adenocarcinoma, and melanoma; vulvar cancer such as squamous cell carcinoma, melanoma, adenocarcinoma, basal cell carcinoma, sarcoma, and Paget's disease; cervical cancers such as but not limited to, squamous cell carcinoma, and adenocarcinoma; uterine cancers such as but not limited to endometrial carcinoma and uterine sarcoma; ovarian cancers such as but not limited

to, ovarian epithelial carcinoma, borderline tumor, germ cell tumor, and stromal tumor; cervical carcinoma; esophageal cancers such as but not limited to, squamous cancer, adenocarcinoma, adenoid cyctic carcinoma, mucoepidermoid carcinoma, adenosquamous carcinoma, sarcoma, melanoma, plasmacytoma, verrucous carcinoma, and oat cell(small cell) carcinoma; stomach cancers such as but not limited to, adenocarcinoma, fungating (polypoid), ulcerating, superficial spreading, diffusely spreading, malignant lymphoma, liposarcoma, fibrosarcoma, and carcinosarcoma; colon cancers; KRAS mutated colorectal cancer; colon carcinoma; rectal cancers; liver cancers such as but not limited to hepatocellular carcinoma and hepatoblastoma, gallbladder cancers such as adenocarcinoma; cholangiocarcinomas such as but not limited to pappillary, nodular, and diffuse; lung cancers such as KRAS-mutated non-small cell lung cancer, non-small cell lung cancer, squamous cell carcinoma (epidermoid carcinoma), adenocarcinoma, large-cell carcinoma and small-cell lung cancer; lung carcinoma; testicular cancers such as but not limited to germinal tumor, seminoma, anaplastic, classic (typical), spermatocytic, nonseminoma, embryonal carcinoma, teratoma carcinoma, choriocarcinoma (yolk-sac tumor), prostate cancers such as but not limited to, androgen-independent prostate cancer, androgen-dependent prostate cancer, adenocarcinoma, leiomyosarcoma, and rhabdomyosarcoma; penal cancers; oral cancers such as but not limited to squamous cell carcinoma; basal cancers; salivary gland cancers such as but not limited to adenocarcinoma, mucoepidermoid carcinoma, and adenoidcystic carcinoma; pharynx cancers such as but not limited to squamous cell cancer, and verrucous; skin cancers such as but not limited to, basal cell carcinoma, squamous cell carcinoma and melanoma, superficial spreading melanoma, nodular melanoma, lentigo malignant melanoma, acrallentiginous melanoma; kidney cancers such as but not limited to renal cell cancer, adenocarcinoma, hypernephroma, fibrosarcoma, transitional cell cancer (renal pelvis and/or uterer); renal carcinoma; Wilms' tumor; bladder cancers such as but not limited to transitional cell carcinoma, squamous cell cancer, adenocarcinoma, carcinosarcoma. In addition, cancers include myxosarcoma, osteogenic sarcoma, endotheliosarcoma, lymphangioendotheliosarcoma, mesothelioma, synovioma, hemangioblastoma, epithelial carcinoma, cystadenocarcinoma, bronchogenic carcinoma, sweat gland carcinoma, sebaceous gland carcinoma, papillary carcinoma and papillary adenocarcinomas.

In certain embodiments cancers that can be prevented and/or treated in accordance with the present invention include, the following: pediatric solid tumor, Ewing's sarcoma, Wilms tumor, neuroblastoma, neurofibroma, carcinoma of the epidermis, malignant melanoma, cervical carcinoma, colon carcinoma, lung carcinoma, renal carcinoma, breast carcinoma, breast sarcoma, metastatic breast cancer, HIV-related Kaposi's sarcoma, prostate cancer, androgen-independent prostate cancer, androgen-dependent prostate cancer, neurofibromatosis, lung cancer, non-small cell lung cancer, KRAS-mutated non-small cell lung cancer, malignant melanoma, melanoma, colon cancer, KRAS-mutated colorectal cancer, glioblastoma multiforme, renal cancer, kidney cancer, bladder cancer, ovarian cancer, hepatocellular carcinoma, thyroid carcinoma, rhabdomyosarcoma, acute myeloid leukemia, and multiple myeloma.

In certain embodiments, cancers and conditions associated therewith that are prevented and/or treated in accordance with the present invention are breast carcinomas, lung carcinomas, gastric carcinomas, esophageal carcinomas, colorectal carcinomas, liver carcinomas, ovarian carcinomas, thecomas, arrhenoblastomas, cervical carcinomas, endometrial carcinoma, endometrial hyperplasia, endometriosis, fibrosarcomas, choriocarcinoma, head and neck cancer, nasopharyngeal carcinoma, laryngeal carcinomas, hepatoblastoma, Kaposi's sarcoma, melanoma, skin carcinomas, hemangioma, cavernous hemangioma, hemangioblastoma, pancreas carcinomas, retinoblastoma, astrocytoma, glioblastoma, Schwannoma, oligodendroglioma, medulloblastoma, neuroblastomas, rhabdomyosarcoma, osteogenic sarcoma, leiomyosarcomas, urinary tract carcinomas, thyroid carcinomas, Wilm's tumor, renal cell carcinoma, prostate carcinoma, abnormal vascular proliferation associated with phakomatoses, edema (such as that associated with brain tumors), or Meigs' syndrome. In specific embodiment, the cancer an astrocytoma, an oligodendroglioma, a mixture of oligodendroglioma and an astrocytoma elements, an ependymoma, a meningioma, a pituitary adenoma, a primitive neuroectodermal tumor, a medullblastoma, a primary central nervous system (CNS) lymphoma, or a CNS germ cell tumor.

In specific embodiments, the cancer treated in accordance with the present invention is an acoustic neuroma, an anaplastic astrocytoma, a glioblastoma multiforme, or a meningioma.

In other specific embodiments, the cancer treated in accordance with the present invention is a brain stem glioma, a craniopharyngioma, an ependyoma, a juvenile pilocytic astrocytoma, a medulloblastoma, an optic nerve glioma, primitive neuroectodermal tumor, or a rhabdoid tumor.

## EXAMPLES

All compounds being the subject matter of the present application are disclosed and characterized in WO 2014/028459 (A1) or WO 2014/116845 (A1). WO2014/028459 (A1) and WO 2014/116845 (A1) disclose methods for the preparation of the compounds being the subject matter of the present application. WO2014/028459 (A1) and WO 2014/116845 (A1) are hereby incorporated by reference.

**Compound 1**

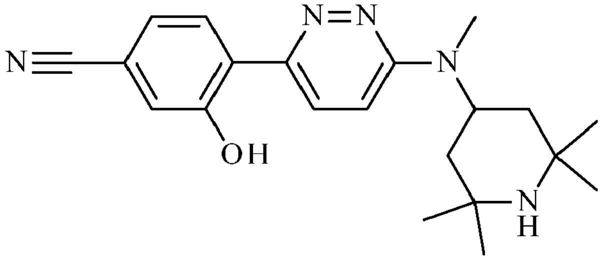

3-hydroxy-4-(6-(methyl(2,2,6,6-tetramethylpiperidin-4-yl)amino)pyridazin-3-yl)benzonitrile was prepared as described in WO2014/028459 (A1) on pages 59-60 for Example 5-1.

**Compound 2**

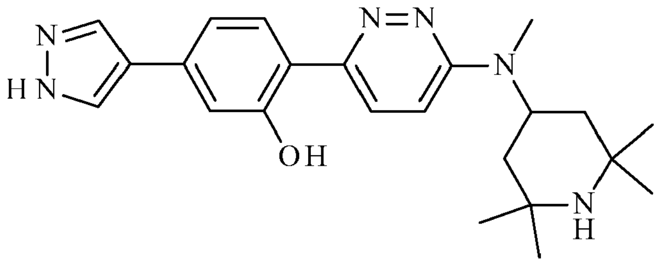

5-(1H-pyrazol-4-yl)-2-(6-(methyl(2,2,6,6-tetramethylpiperidin-4-yl)amino)pyridazin-3-yl)phenol was prepared as described in WO2014/028459 (A1) on pages 69-71 for Example 14-1.

**Compound 3**

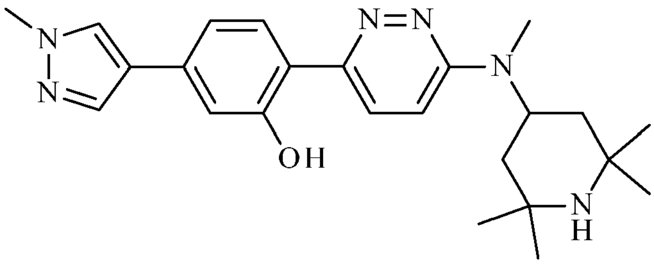

2-(6-(methyl-(2,2,6,6-tetramethylpiperidin-4-yl)amino)pyridazin-3-yl)-5-(1-methyl-1H-pyrazol-4-yl)phenol was prepared as described in WO2014/028459 (A1) on page 74 for Example 16-2.

**Compound 4**

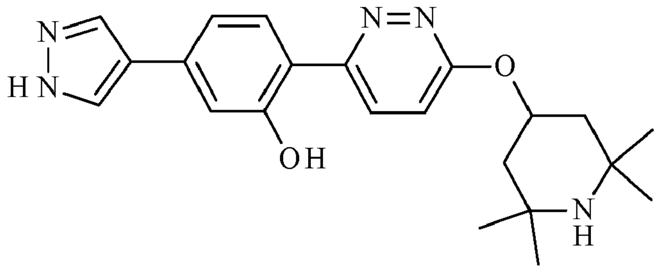

5-(1H-pyrazol-4-yl)-2-(6-((2,2,6,6-tetramethylpiperidin-4-yl)oxy)pyridazin-3-yl)phenol was prepared as described in WO2014/028459 (A1) on pages 81-83 for Example 17-13.

**Compound 5**

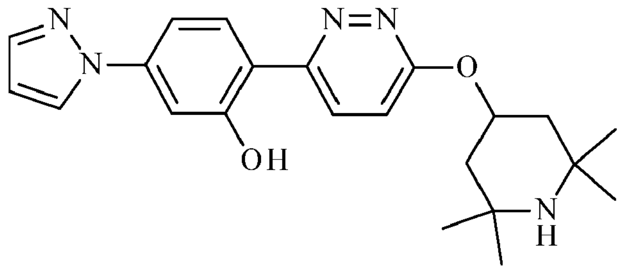

5-pyrazol-1-yl-2-[6-((2,2,6,6-tetramethylpiperidin-4-yl)oxy)-pyridazin-3-yl]-phenol was prepared as described in WO2014/028459 (A1) on page 81 for Example 17-12.

**Compound 6**

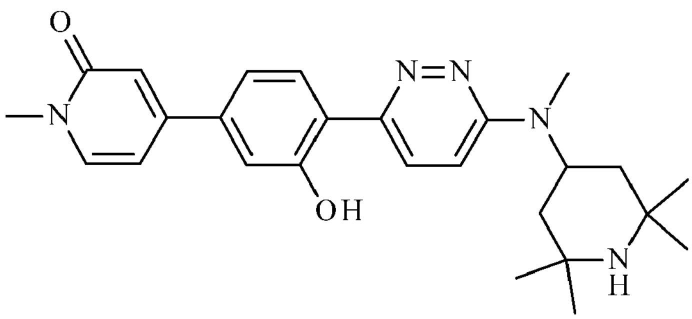

4-(3-hydroxy-4-(6-(methyl-(2,2,6,6-tetramethylpiperidin-4-yl)amino)pyridazin-3-yl)phenyl)-1-methylpyridin-2(1H)-one was prepared as described in WO2014/028459 (A1) on pages 168-169 for Example 41-7.

**Compound 7**

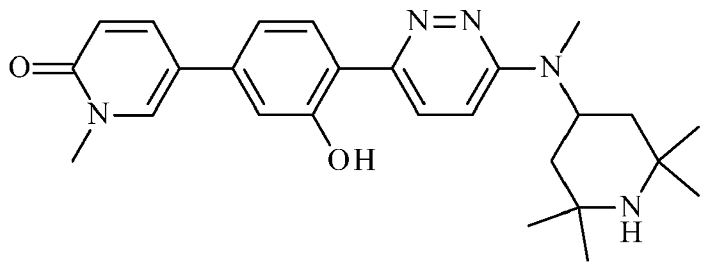

5-(3-hydroxy-4-(6-(methyl-(2,2,6,6-tetramethylpiperidin-4-yl)amino)pyridazin-3-yl)phenyl)-1-methylpyridin-2(1H)-one was prepared as described in WO2014/028459 (A1) on page 168 for Example 14-6.

**Compound 8**

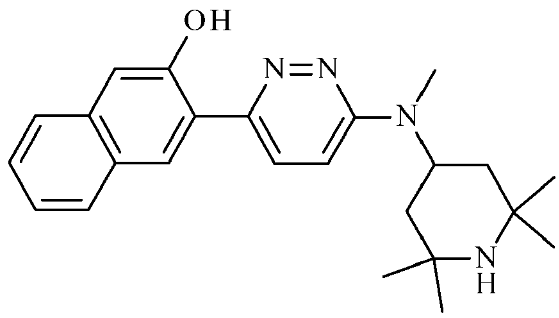

3-(6-(methyl(2,2,6,6-tetramethylpiperidin-4-yl)amino)pyridazin-3-yl)naphthalen-2-ol was prepared as described in WO2014/028459 (A1) on page 55 for Example 3-1.

**Compound 9**

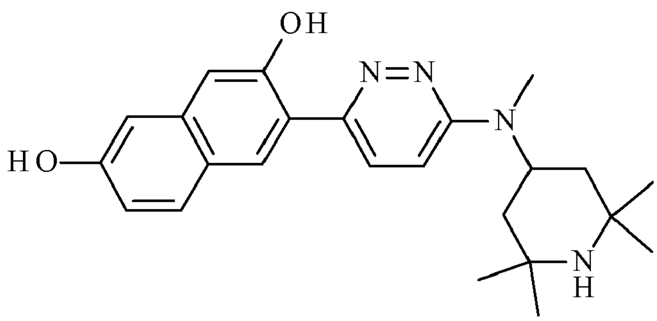

3-(6-(methyl-(2,2,6,6-tetramethylpiperidin-4-yl)amino)pyridazin-3-yl)naphthalene-2,7-diol was prepared as described in WO2014/028459 (A1) on pages 92-93 for Example 20-2.

**Compound 10**

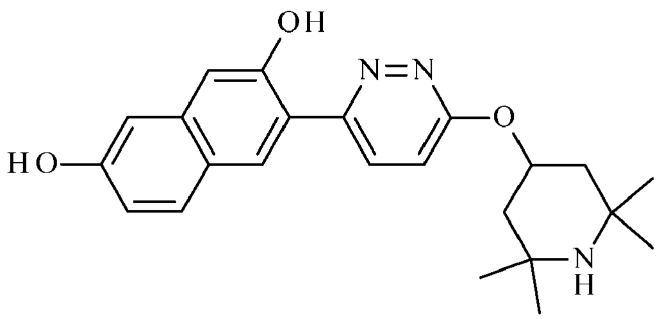

3-(6-((2,2,6,6-tetramethylpiperidin-4-yl)oxy)pyridazin-3-yl)naphthalene-2,7-diol was prepared as described in WO2014/028459 (A1) on page 92 for Example 20-1.

**Compound 11**

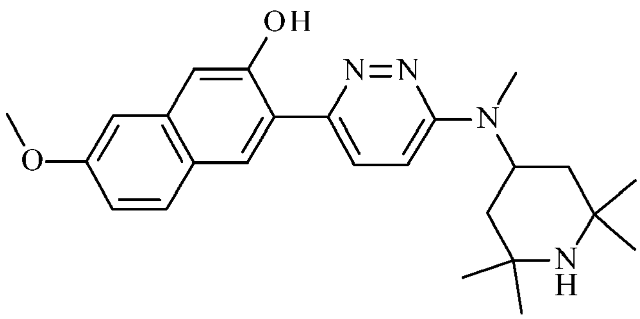

7-methoxy-3-(6-(methyl(2,2,6,6-tetramethylpiperidin-4-yl)amino)pyridazin-3-yl)naphthalen-2-ol was prepared as described in WO2014/028459 (A1) on page 118 for Example 24-6.

**Compound 12**

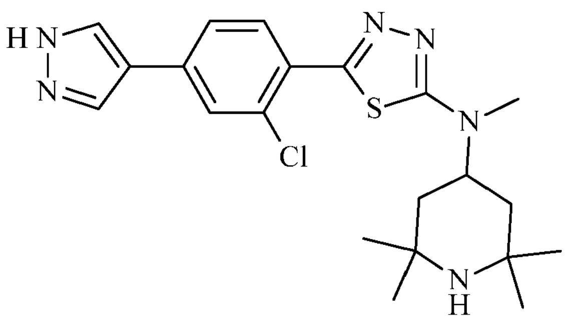

5-(2-Chloro-4-(1H-pyrazol-4-yl)phenyl)-N-methyl-N-(2,2,6,6-tetramethylpiperidin-4-yl)-1,3,4-thiadiazol-2-amine was prepared as described in WO2014/116845 (A1) on page 74 for Example 40.

**Compound 13**

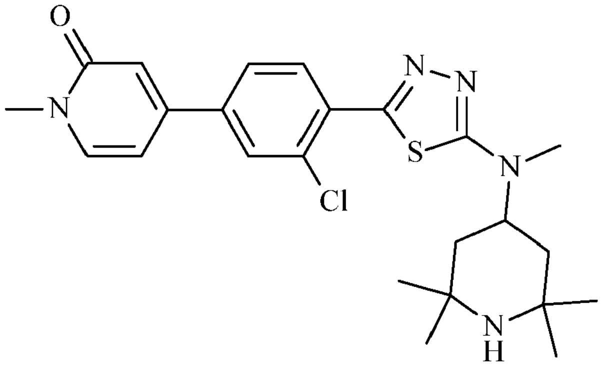

4-(3-chloro-4-(5-(methyl(2,2,6,6-tetramethylpiperidin-4-yl)amino)-1,3,4-thiadiazol-2-yl)phenyl)-1-methylpyridin-2(1H)-one was prepared as described in WO2014/116845 (A1) on page 74 for Example 39.

**Compound 14**

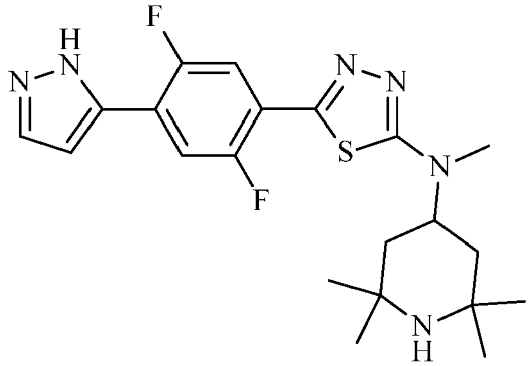

5-(2,5-difluoro-4-(1H-pyrazol-5-yl)phenyl)-N-methyl-N-(2,2,6,6-tetramethylpiperidin-4-yl)-1,3,4-thiadiazol-2-amine was prepared as described in WO2014/116845 (A1) on page 78 for Example 51.

**Example 1**

**Monitoring expression levels of FoxM1 splice variants using real-time quantitative PCR**

Human fibroblasts were plated at 10,000 cells/well in 200 μl DMEM with GlutaMAX and 10% FBS in 96-well plates in a cell culture incubator (37°C, 5% CO2, 100% relative humidity). Cells were then treated with compounds at different concentrations (0.1-300 nM, each in 0.5% DMSO) in triplicate for 24 hours. RNA extraction was performed as per instructions mentioned in the Ambion® Cells-to-CT™ Kits from Applied Biosystems®. RNA samples were frozen at - 20°C until further analysis. Relative expression levels of full-length FoxM1 (FoxM1_FL) or FoxM1 lacking exon VIIa (FoxM1_ΔVIIa) with GAPDH for internal control, was measured using one-step multiplex reverse transcription-polymerase chain reaction (RT-PCR). TaqMan® FAM probes were used for relative quantitation of FoxM1_FL or FoxM1_ΔVIIa expression levels and TaqMan® VIC probes were used for relative quantitation of human GAPDH levels. The fidelity of the amplification methods was determined using the ΔΔCt relative quantification method for quantitative PCR.

**Compounds induce alternative splicing of FoxM1 towards full-length FoxM1**

To investigate an effect on splicing of FoxM1, human fibroblasts were treated for 24 hours with compounds of the present invention in dose response, and analysed by RT-qPCR for presence of mRNA including (FoxM1_FL) or excluding the exon VIIa (FoxM1-ΔVIIa). Figure 1 shows that all compounds increased expression of the FoxM1_FL mRNA. Correspondingly, the mRNAs for FoxM1_ΔVIIa declined. The data demonstrate that upregulation of FoxM1_FL with downregulation of FoxM1_ΔVIIa by treatment with compounds of present invention are directly correlated, indicating an effect of the compounds on alternative splicing of FoxM1. The resulting concentration dependence curves of the

FoxM1_ΔVIIa splice variant were fitted to a Hill binding equation to yield IC50 values that are described in Table 1. The data demonstrate that all compounds affect FoxM1 splicing with various potencies, ranging from IC50 values from 0.7 to 345 nM. Taken together, the data underline a splicing modifying activity in the FoxM1 gene. This may result in arrest of cell cycle and induction of apoptosis, as the FoxM1_FL variant created by compound treatment is functionally inactive, and therefore will antagonize the pro-proliferating effect of functional FoxM1 (H. Ye, T.F. Kelly, U. Samadani, L. Lim, S. Rubio, D.G. Overdier, K.A. Roebuck, R.H. Costa, Mol. Cell Biol. 17 (1997) 1626-1641).

Table 1. Half-maximal effects for the FoxM1_ΔVIIa splice variant and for the SMN protein. FoxM1_ΔVIIa IC50 values were calculated from concentration dependence curves in Figure 1 using a Hill binding equation. SMN protein EC50 values were taken from Activity Tables on pages 189 to 192 of WO 2014/028459 (A1) and on pages 124 to 141 of WO 2014/116845 (A1).

| Compound | FoxM1_ΔVIIa IC50 | SMN Protein EC50 |
|---|---|---|
| **1** | 41 nM | 54 nM |
| **2** | 0.9 nM | 4 nM |
| **3** | 9.8 nM | 15 nM |
| **4** | 17 nM | 17 nM |
| **5** | 278 nM | 31 nM |
| **6** | 1.5 nM | 7 nM |
| **7** | 167 nM | 10 nM |
| **8** | 3.7 nM | 10 nM |
| **9** | 0.7 nM | 4 nM |
| **10** | - | 18 nM |
| **11** | 0.8 nM | 6 nM |
| **12** | 265 nM | 34 nM |
| **13** | 345 nM | 50 nM |
| **14** | 144 nM | 85 nM |

**Example 2**

**Monitoring expression levels of SMN splice variants using real-time quantitative PCR**

Spinal muscular atrophy (SMA) is a neuromuscular disorder due to mutations in the Survival of Motor Neuron (SMN) gene. Loss of SMN is deleterious to motor neurons and results in neuromuscular insufficiency, a hallmark of the disease. From a genetic point of view, SMA is an autosomal recessive condition, caused by disruption of SMN1 gene, located in 5q13 (Lefebvre S. et al. (1995) Cell 80: 155-165). More than 98% of patients with spinal muscular atrophy have a homozygous disruption of SMN1 by deletion, rearrangement, or mutation. All these patients, however, retain at least one copy of the related SMN2 gene.

Compounds of present invention have been described in WO 2014/028459 (A1) and WO 2014/116845 (A1) as being effective splicing modifiers of the SMN2 gene and thus suitable for upregulation of SMN protein and thus for the treatment of SMA.

The potency of the compounds of present invention regarding SMN2 splicing modulation as assessed by the half-maximal effects (EC50) of SMN protein is evident from Activity Table on pages 189 to 192 of WO2014028459A1 and from Activity Table on pages 124 to 141 of WO2014116845A1.

The method of cellular SMN ELISA to measure the effects of compounds on SMN protein elevation is described on page 189 of WO2014028459A1 and on page 123 of WO2014116845A1.

**Potency for splicing modulation of FoxM1 gene is linearly correlated to potency of splicing modulation of SMN2 gene**

It has surprisingly been found that the potency of all compounds investigated to modulate splicing of SMN2

gene is linearly related to the potency to modulate splicing of FoxM1 gene.

Figure 2 shows a graph wherein the half-maximal effects for the FoxM1_ΔVIIa splice variant (IC50) have been plotted versus the half-maximal effects for the SMN protein (EC50).

A regression analysis of the values has resulted in a linear correlation according to the Equation 1:

$$Y = 0.102 * X + 15.2 \qquad \text{(Equation 1)}$$

With Y = log(FoxM1_ΔVIIa IC50) and X = log(SMN protein EC50), this linear correlation allows the calculation of FoxM1_ΔVIIa IC50 values from SMN protein EC50 values according to Equation 2:

$$\text{FoxM1_}\Delta\text{VIIa IC50} = 10^{(0.102 * \log(\text{SMN protein EC50}) + 15.2)} \qquad \text{(Equation 2)}$$

The slope of the linear correlation of Equation 1 is 0.102. Thereby the slope of the linear correlation suggests that on average, a 10-fold higher concentration (1/0.102 = 9.8) of each compound is needed to achieve 50% of splicing correction in the FoxM1 gene as compared to the SMN2 gene.

## Claims

**1.** A FoxM1 gene splicing modifier selected from a compound of formula (I) or a compound of formula (VI)

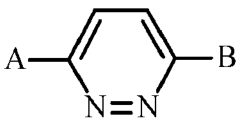
(I)

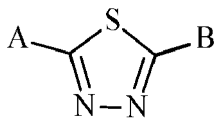
(VI),

wherein

A is 2-hydroxy-phenyl which is substituted with:

0, 1,2, or 3 substituents independently selected from $C_{1-4}$ alkyl, oxo, oxime, hydroxy, halo-$C_{1-4}$, alkyl, dihalo-$C_{1-4}$ alkyl, trihalo-$C_{1-4}$ alkyl, $C_{1-4}$, alkoxy, $C_{1-4}$, alkoxy-$C_{3-7}$ cycloalkyl, halo-$C_{1-4}$ alkoxy, dihalo-$C_{1-4}$ alkoxy, trihalo-$C_{1-4}$ alkoxy, hydroxy, cyano, halogen, amino, mono-$C_{1-4}$ alkylamino, di-$C_{1-4}$ alkylamino, heteroaryl, $C_{1-4}$ alkyl substituted with hydroxy, $C_{1-4}$ alkoxy substituted with aryl, amino, -C(O)NH-$C_{1-4}$alkyl-heteroaryl, -NHC(O)-$C_{1-4}$ alkylheteroaryl, $C_{1-4}$ alkyl-C(O)NH-heteroaryl, $C_{1-4}$alkyl-NHC(O)-heteroaryl, $C_{3-7}$ cycloalkyl, 5-7 membered cycloalkenyl, or 5, 6 or 9 membered heterocycle containing 1 or 2 heteroatoms independently, selected from S, O and N,

wherein two $C_{1-4}$ alkyl groups can combine with the atoms to which they are bound to form a 5-6 membered ring;

wherein heteroaryl has 5, 6 or 9 ring atoms, 1, 2 or 3 ring heteroatoms selected from N, O and S, and is substituted with 0, 1, or 2 substituents independently selected from oxo, hydroxy, nitro, halogen, $C_{1-4}$ alkyl, $C_{1-4}$ alkenyl, $C_{1-4}$ alkoxy, $C_{3-7}$ cycloalkyl, $C_{1-4}$ alkyl-OH, trihalo-$C_{1-4}$ alkyl, mono-$C_{1-4}$ alkylamino, di-$C_{1-4}$ alkylamino, -C(O)$NH_2$, -NH2, $NO_2$, hydroxy-$C_{1-4}$alkylamino, hydroxy-$C_{1-4}$ alkyl, 4-7 membered heterocycle-$C_{1-4}$ alkyl, amino-$C_{1-4}$ alkyl, mono-$C_{1-4}$alkylamino-$C_{1-4}$ alkyl, and di-$C_{1-4}$alkylamino-$C_{1-4}$ alkyl; or

A is 2-naphthyl optionally substituted at the 3 position with hydroxy and additionally substituted with 0, 1, or 2 substituents selected from hydroxy, cyano, halogen, $C_{1-4}$ alkyl, $C_{2-4}$ alkenyl, $C_{1-5}$ alkoxy, wherein the alkoxy is unsubstituted or substituted with hydroxy, $C_{1-4}$ alkoxy, amino, -NHC(O)-$C_{1-4}$ alkyl, -NHC(O)O-$C_{1-4}$ alkyl, $C_{1-4}$ alkylene-4-7 membered heterocycle, 4-7 membered heterocycle, mono-$C_{1-4}$ alkylamino, and di- $C_{1-4}$ alkylamino; or

A is 6 membered heteroaryl having 1-3 ring nitrogen atoms and which is substituted by phenyl or a heteroaryl having 5 or 6 ring atoms, 1 or 2 ring heteroatoms independently selected from N, O and S and is substituted with 0, 1, or 2 substituents independently selected from $C_{1-4}$ alkyl, mono-$C_{1-4}$ alkylamino, di-$C_{1-4}$alkylamino, hydroxy-$C_{1-4}$ alkylamino, hydroxy-$C_{1-4}$ alkyl, amino-$C_{1-4}$ alkyl and mono-$C_{1-4}$ alkylamino-$C_{1-4}$ alkyl, and di-$C_{1-4}$alkylamino-$C_{1-4}$ alkyl; or

A is bicyclic heteroaryl having 9 to 10 ring atoms, 1, 2, or 3 ring heteroatoms independently selected from N, O or S, and which is substituted with 0, 1, or 2 substituents independently selected from cyano, oxime, halogen, hydroxy, $C_{1-4}$ alkyl, $C_{2-4}$ alkenyl, $C_{2-4}$ alkynyl, $C_{1-4}$ alkoxy, $C_{1-4}$ alkoxy substituted with hydroxy, amino, mono-$C_{1-4}$ alkylamino, and di-$C_{1-4}$ alkylamino; or

A is tricyclic heteroaryl having 12 or 13 ring atoms, 1, 2, or 3 ring heteroatoms independently selected from N, O or S, and which is substituted with 0, 1, or 2 substituents independently selected from cyano, halogen, hydroxy, $C_{1-4}$ alkyl, $C_{1-4}$ alkenyl, $C_{2-4}$ alkynyl, $C_{1-4}$ alkoxy, $C_{1-4}$ alkoxy substituted with hydroxy, amino, mono-$C_{1-4}$ alkylamino, di-$C_{1-4}$ alkylamino, and heteroaryl having 5, 6 or 9 ring atoms, 1, 2 or 3 ring heteroatoms selected from N, O and S, and which is substituted with 0, 1, or 2 substituents independently selected from oxo, hydroxy, nitro, halogen, $C_{1-4}$ alkyl, $C_{1-4}$ alkenyl, $C_{1-4}$ alkoxy, $C_{3-7}$ cycloalkyl, $C_{1-4}$ alkyl-OH, trihalo-$C_{1-4}$ alkyl, mono-$C_{1-4}$ alkylamino, di-$C_{1-4}$ alkylamino, -C(O)$NH_2$, -$NH_2$, - $NO_2$, hydroxy-$C_{1-4}$ alkylamino, hydroxy-$C_{1-4}$ alkyl, 4-7 mem-

bered heterocycle-$C_{1-4}$ alkyl, amino-$C_{1-4}$ alkyl, mono-$C_{1-4}$ alkylamino-$C_{1-4}$ alkyl and di-$C_{1-4}$ alkylamino-$C_{1-4}$ alkyl; or

A is phenyl which is substituted with 0, 1,2, or 3 substituents independently selected from $C_{1-4}$ alkyl, oxo, oxime, hydroxy, halo-$C_{1-4}$ alkyl, dihalo-$C_{1-4}$ alkyl, trihalo-$C_{1-4}$ alkyl, $C_{1-4}$ alkoxy, $C_{1-4}$ alkoxy-$C_{3-7}$ cycloalkyl, halo-$C_{1-4}$ alkoxy, dihalo-$C_{1-4}$ alkoxy, trihalo-$C_{1-4}$ alkoxy, cyano, halogen, amino, mono-$C_{1-4}$ alkylamino, di-$C_{1-4}$ alkylamino, heteroaryl, $C_{1-4}$ alkyl substituted with hydroxy, $C_{1-4}$ alkoxy substituted with aryl, -C(O)NH-$C_{1-4}$ alkyl- heteroaryl, -NHC(O)-$C_{1-4}$ alkylheteroaryl, $C_{1-4}$ alkyl-C(O)NH-heteroaryl, $C_{1-4}$ alkyl-NHC(O)-heteroaryl, $C_{3-7}$ cycloalkyl, 5-7 membered cycloalkenyl or 5, 6 or 9 membered heterocycle containing 1 or 2 heteroatoms, independently, selected from S, 0 and N;

wherein two $C_{1-4}$ alkyl groups can combine with the atoms to which they are bound to form a 5-6 membered ring;

wherein heteroaryl has 5, 6 or 9 ring atoms, 1, 2 or 3 ring heteroatoms selected from N, 0 and S and is substituted with 0, 1, or 2 substituents independently selected from oxo, hydroxy, nitro, halogen, $C_{1-4}$ alkyl, $C_{1-4}$ alkenyl, $C_{1-4}$ alkoxy, $C_{3-7}$ cycloalkyl, $C_{1-4}$ alkyl-OH, trihalo-$C_{1-4}$ alkyl, mono-$C_{1-4}$ alkylamino, di-$C_{1-4}$ alkylamino, -C(O)$NH_2$, -$NH_2$,-$NO_2$ hydroxy-$C_{1-4}$ alkylamino, hydroxy-$C_{1-4}$ alkyl, 4-7 memberered heterocycle-$C_{1-4}$ alkyl, amino-$C_{1-4}$ alkyl, mono-$C_{1-4}$ alkylamino-$C_{1-4}$ alkyl, and di-$C_{1-4}$ alkylamino-$C_{1-4}$ alkyl;

B is a group of the formula

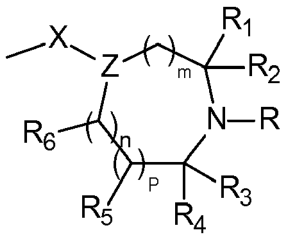

,

wherein

m, n and p are independently selected from 0 or 1;

R, $R_1$, $R_2$, $R_3$, and $R_4$ are independently selected from the group consisting of hydrogen and $C_{1-4}$ alkyl, wherein alkyl is optionally substituted with hydroxy, amino, mono-$C_{1-4}$ akylamino or di-$C_{1-4}$ akylamino;

$R_5$ and $R_6$ are independently selected from hydrogen and fluorine; or

R and $R_3$, taken in combination form a fused 5 or 6 membered heterocyclic ring having 0 or 1 additional ring heteroatoms selected from N, O or S; or

$R_1$ and $R_3$, taken in combination form a $C_{1-3}$ alkylene group; or

$R_1$ and $R_5$, taken in combination form a $C_{1-3}$ alkylene group; or

$R_3$ and $R_4$, taken in combination with the carbon atom to which they attach, form a spirocyclic $C_{3-6}$ cycloalkyl;

X is $CR_AR_B$, O, $NR_7$ or a bond;

$R_7$ is hydrogen, or $C_{1-4}$ alkyl;

$R_A$ and $R_B$ are independently selected from hydrogen and $C_{1-4}$ alkyl, or $R_A$ and $R_B$, taken in combination, form a divalent $C_{2-5}$ alkylene group;

Z is $CR_8$ or N; with the proviso that when Z is N, X is a bond;

$R_8$ is hydrogen or taken in combination with $R_6$ form a double bond; or

B is a group of the formula

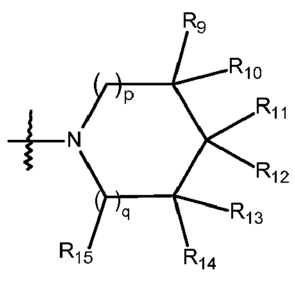

,

wherein

p and q are independently selected from the group consisting of 0, 1, and 2;
$R_9$ and $R_{13}$ are independently selected from hydrogen and $C_{1-4}$ alkyl;
$R_{10}$ and $R_{14}$ are independently selected from hydrogen, amino, mono-$C_{1-4}$ alkylamino, di-$C_{1-4}$ alkylamino, and $C_{1-4}$ alkyl optionally substituted with hydroxy, amino, mono-$C_{1-4}$ alkylamino or di-$C_{1-4}$ alkylamino;
$R_{11}$ is hydrogen, $C_{1-4}$ alkyl, amino, mono-$C_{1-4}$ alkylamino, or di-$C_{1-4}$ alkylamino;
$R_{12}$ is hydrogen or $C_{1-4}$ alkyl; or
$R_9$ and $R_{11}$ taken in combination form a saturated azacycle having 4 to 7 ring atoms which is optionally substituted with one to three $C_{1-4}$ alkyl groups; or
$R_{11}$ and $R_{12}$, taken in combination form a saturated azacycle having 4 to 7 ring atoms which is optionally substituted with one to three $C_{1-4}$ alkyl groups.

or a pharmaceutically acceptable salt thereof;
for use in the treatment, prevention and/or delay of progression of cancer.

**2.** The FoxM1 gene splicing modifier according to claim 1, wherein

A is 2-hydroxy-phenyl which is substituted with:

0, 1,2, or 3 substituents independently selected from $C_{1-4}$ alkyl, oxo, oxime, hydroxy, halo-$C_{1-4}$ alkyl, dihalo-$C_{1-4}$ alkyl, trihalo-$C_{1-4}$ alkyl, $C_{1-4}$ alkoxy, $C_{1-4}$ alkoxy-$C_{3-7}$ cycloalkyl, halo-$C_{1-4}$ alkoxy, dihalo-$C_{1-4}$ alkoxy, trihalo-$C_{1-4}$ alkoxy, hydroxy, cyano, halogen, amino, mono-$C_{1-4}$ alkylamino, di-$C_{1-4}$ alkylamino, heteroaryl, $C_{1-4}$ alkyl substituted with hydroxy, $C_{1-4}$ alkoxy substituted with aryl, amino, -C(O)NH-$C_{1-4}$alkyl-heteroaryl, -NHC(O)-$C_{1-4}$ alkylheteroaryl, $C_{1-4}$ alkyl-C(O)NH-heteroaryl, $C_{1-4}$alkyl-NHC(O)-heteroaryl, $C_{3-7}$ cycloalkyl, 5-7 membered cycloalkenyl, or 5, 6 or 9 membered heterocycle containing 1 or 2 heteroatoms independently, selected from S, O and N,
wherein two $C_{1-4}$ alkyl groups can combine with the atoms to which they are bound to form a 5-6 membered ring;
wherein heteroaryl has 5, 6 or 9 ring atoms, 1, 2 or 3 ring heteroatoms selected from N, O and S, and is substituted with 0, 1, or 2 substituents independently selected from oxo, hydroxy, nitro, halogen, $C_{1-4}$ alkyl, $C_{1-4}$ alkenyl, $C_{1-4}$ alkoxy, $C_{3-7}$ cycloalkyl, $C_{1-4}$ alkyl-OH, trihalo-$C_{1-4}$ alkyl, mono-$C_{1-4}$ alkylamino, di-$C_{1-4}$ alkylamino, -C(O)$NH_2$, -$NH_2$, $NO_2$, hydroxy-$C_{1-4}$alkylamino, hydroxy-$C_{1-4}$ alkyl, 4-7 membered heterocycle-$C_{1-4}$ alkyl, amino-$C_{1-4}$ alkyl, mono-$C_{1-4}$alkylamino-$C_{1-4}$ alkyl, and di-$C_{1-4}$alkylamino-$C_{1-4}$ alkyl; or

A is 2-naphthyl optionally substituted at the 3 position with hydroxy and additionally substituted with 0, 1, or 2 substituents selected from hydroxy, cyano, halogen, $C_{1-4}$ alkyl, $C_{2-4}$ alkenyl, $C_{1-5}$ alkoxy, wherein the alkoxy is unsubstituted or substituted with hydroxy, $C_{1-4}$ alkoxy, amino, -NHC(O)-$C_{1-4}$ alkyl, -NHC(O)O-$C_{1-4}$ alkyl, $C_{1-4}$ alkylene-4-7 membered heterocycle, 4-7 membered heterocycle, mono-$C_{1-4}$ alkylamino, and di- $C_{1-4}$ alkylamino; or
A is phenyl which is substituted with 0, 1,2, or 3 substituents independently selected from $C_{1-4}$ alkyl, oxo, oxime, hydroxy, halo-$C_{1-4}$ alkyl, dihalo-$C_{1-4}$ alkyl, trihalo-$C_{1-4}$ alkyl, $C_{1-4}$ alkoxy, $C_{1-4}$ alkoxy-$C_{3-7}$ cycloalkyl, halo-$C_{1-4}$ alkoxy, dihalo-$C_{1-4}$ alkoxy, trihalo-$C_{1-4}$ alkoxy, cyano, halogen, amino, mono-$C_{1-4}$ alkylamino, di-$C_{1-4}$ alkylamino, heteroaryl, $C_{1-4}$ alkyl substituted with hydroxy, $C_{1-4}$ alkoxy substituted with aryl, -C(O)NH-$C_{1-4}$ alkyl- heteroaryl, -NHC(O)-$C_{1-4}$ alkylheteroaryl, $C_{1-4}$ alkyl-C(O)NH-heteroaryl, $C_{1-4}$ alkyl-NHC(O)-heteroaryl, $C_{3-7}$ cycloalkyl, 5-7 membered cycloalkenyl or 5, 6 or 9 membered heterocycle containing 1 or 2 heteroatoms, independently, selected from S, 0 and N;
wherein two $C_{1-4}$ alkyl groups can combine with the atoms to which they are bound to form a 5-6 membered ring;
wherein heteroaryl has 5, 6 or 9 ring atoms, 1, 2 or 3 ring heteroatoms selected from N, 0 and S and is substituted with 0, 1, or 2 substituents independently selected from oxo, hydroxy, nitro, halogen, $C_{1-4}$ alkyl, $C_{1-4}$ alkenyl, $C_{1-4}$ alkoxy, $C_{3-7}$ cycloalkyl, $C_{1-4}$ alkyl-OH, trihalo-$C_{1-4}$ alkyl, mono-$C_{1-4}$ alkylamino, di-$C_{1-4}$ alkylamino, -C(O)$NH_2$, -$NH_2$,-$NO_2$, hydroxy-$C_{1-4}$ alkylamino, hydroxy-$C_{1-4}$ alkyl, 4-7 memberered heterocycle-$C_{1-4}$ alkyl, amino-$C_{1-4}$ alkyl, mono-$C_{1-4}$ alkylamino-$C_{1-4}$ alkyl, and di-$C_{1-4}$ alkylamino-$C_{1-4}$ alkyl;

or a pharmaceutically acceptable salt thereof;
for use in the treatment, prevention and/or delay of progression of cancer.

**3.** The FoxM1 gene splicing modifier according to any of claims 1 or 2, wherein

A is 2-hydroxy-phenyl substituted with one additional substituent selected from cyano and heteroaryl; or
A is 2-naphthyl optionally substituted at the 3 position with hydroxy and additionally substituted with 0 or 1 substituents selected from hydroxy and $C_{1-4}$ alkoxy; or
A is phenyl which is substituted with two or three substituents independently selected from halogen and heteroaryl;
wherein heteroaryl has 5 or 6 ring atoms of which 1 or 2 are nitrogen and is substituted with 0, 1, or 2 substituents independently selected from $C_{1-4}$ alkyl and hydroxy;
or a pharmaceutically acceptable salt thereof;
for use in the treatment, prevention and/or delay of progression of cancer.

**4.** A FoxM1 gene splicing modifier selected from a compound of formula (II):

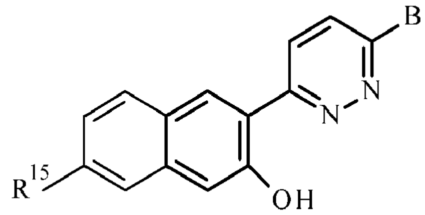

(II),

wherein $R^{15}$ is hydrogen, hydroxy, or $C_{1-4}$ alkoxy, wherein alkoxy is optionally substituted with hydroxy, methoxy, amino, mono-methylamino, di-methylamino or morpholine; or a pharmaceutically acceptable salt thereof; for use in the treatment, prevention and/or delay of progression of cancer.

**5.** A FoxM1 gene splicing modifier selected from a compound of formula (III):

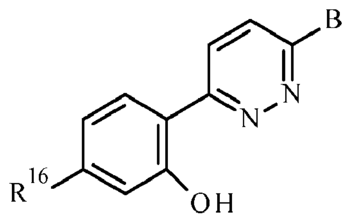

(III),

wherein $R^{16}$ is cyano, 5-membered heteroaryl having two ring nitrogen atoms, or 6-membered heteroaryl having one ring nitrogen atom; wherein the 5-membered heteroaryl is optionally substituted with $C_{1-4}$ alkyl; wherein the 6-membered heteroaryl is optionally substituted with one or two substituents selected from $C_{1-4}$ alkyl and hydroxy; or a pharmaceutically acceptable salt thereof; for use in the treatment, prevention and/or delay of progression of cancer.

**6.** A FoxM1 gene splicing modifier selected from a compound of formula (VII):

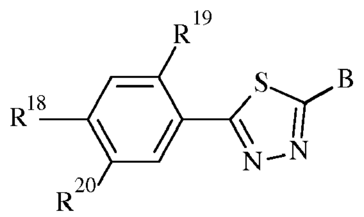

(VII),

wherein $R^{18}$ is 5-membered heteroaryl having two ring nitrogen atoms or 6-membered heteroaryl having one ring nitrogen atom; wherein the 5-membered heteroaryl is optionally substituted with $C_{1-4}$ alkyl; wherein the 6-membered heteroaryl is optionally substituted with one or two substituents selected from $C_{1-4}$ alkyl and hydroxy; $R^{19}$ is hydrogen or halogen; and $R^{20}$ is hydrogen or halogen; or a pharmaceutically acceptable salt thereof; for use in the treatment, prevention and/or delay of progression of cancer.

**7.** The FoxM1 gene splicing modifier according to any of claims 1 to 6, wherein B is selected from the group consisting of

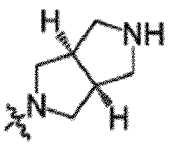 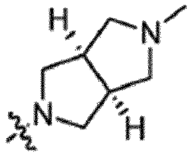 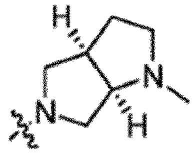 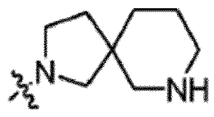

, , , ,

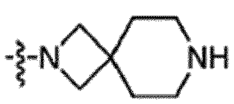 , 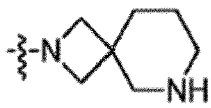 , 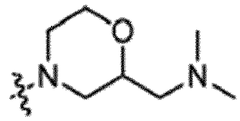 ,

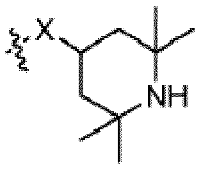 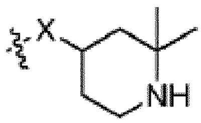 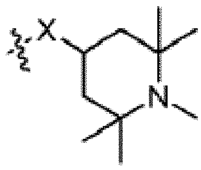 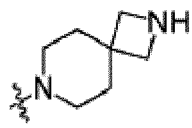

, , , ,

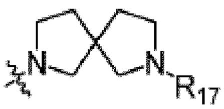 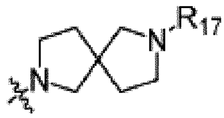 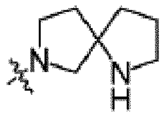 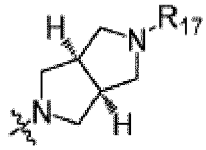

, , , ,

and

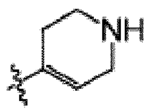

wherein X is O or -N($CH_3$)-; and $R_{17}$ is hydrogen or methyl;
or a pharmaceutically acceptable salt thereof;
for use in the treatment, prevention and/or delay of progression of cancer.

**8.** The FoxM1 gene splicing modifier according to any of claims 1 to 7, wherein B is

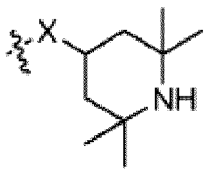

and X is O or -N($CH_3$)-; or a pharmaceutically acceptable salt thereof; for use in the treatment, prevention and/or delay of progression of cancer.

**9.** The FoxM1 gene splicing modifier according to any of claims 1 to 8, wherein X is O; or a pharmaceutically acceptable salt thereof;
for use in the treatment, prevention and/or delay of progression of cancer.

**10.** The FoxM1 gene splicing modifier according to any of claims 1 to 8, wherein X is -N($CH_3$)-; or a pharmaceutically acceptable salt thereof;
for use in the treatment, prevention and/or delay of progression of cancer

**11.** The FoxM1 gene splicing modifier according to any of claims 1 to 8, wherein B is

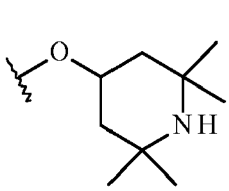

or

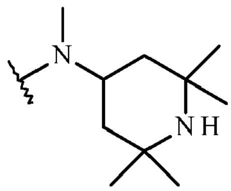

;

or a pharmaceutically acceptable salt thereof; for use in the treatment, prevention and/or delay of progression of cancer.

**12.** A FoxM1 gene splicing modifier selected from a compound of formula (IV) or of formula (V) or of formula (VIII):

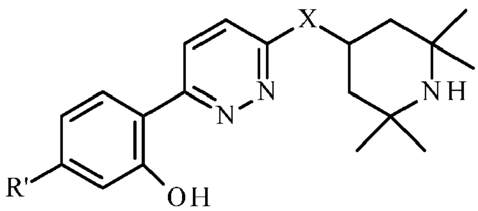

(IV)

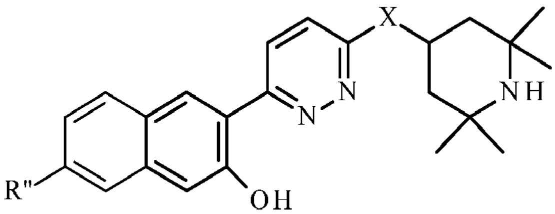

(V)

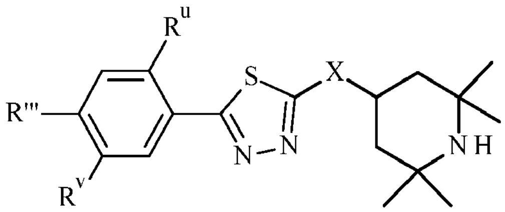

(VIII),

wherein
X is -O- or -N($CH_3$)-; R' is cyano, pyrazolyl optionally substituted with methyl, or pyridinyl substituted with methyl and hydroxy; R" is hydrogen, methyl or methoxy; R"' is pyrazolyl optionally substituted with methyl, or pyridinyl substituted with methyl and hydroxy; $R^u$ is hydrogen, chloro or fluoro; $R^v$ is hydrogen, chloro or fluoro; or a pharmaceutically acceptable salt thereof; for use in the treatment, prevention and/or delay of progression of cancer.

**13.** A FoxM1 gene splicing modifier according to any of claims 1 to 12 selected from the group consisting of:

6-(naphthalen-2-yl)-N-(2,2,6,6-tetramethylpiperidin-4-yl)pyridazin-3-amine;
6-(benzo[b]thio-phen-2-yl)-N-methyl-N-(2,2,6,6-tetra-methylpiperidin-4-yl)pyridazin-3-amine;
2-(6-(2,2,6,6-tetra methylpiperidin-4-ylamino)-pyridazin-3-yl)phenol;

2-(6-(methyl-(2,2,6,6-tetra-methylpiperidin-4-yl)amino)pyridazin-3-yl)benzo[b]-thiophene-5-carbonitrile;
6-(quinolin-3-yl)-N-(2,2,6,6-tetramethyl-piperidin-4-yl)pyridazin-3-amine;
3-(benzo[b]-thiophen-2-yl)-6-(2,2,6,6-tetra-methylpiperidin-4-yloxy)pyridazine;
2-(6-(methyl-(2,2,6,6-tetra-methylpiperidin-4-yl)amino)-pyridazin-3-yl)phenol;
6-(6-(methyl-(2,2,6,6-tetra-methylpiperidin-4-yl)amino)-pyridazin-3-yl)naphthalen-2-ol;
6-(benzo[b]-thiophen-2-yl)-N-(2,2,6,6-tetra-methylpiperidin-4-yl)pyridazin-3-amine;
7-(6-((2,2,6,6-tetramethylpiperidin-4-yl)oxy)pyridazin-3-yl)isoquinoline;
6-(6-((2,2,6,6-tetramethylpiperidin-4-yl)oxy)pyridazin-3-yl)isoquinoline;
N-methyl-6-(quinolin-7-yl)-N-(2,2,6,6-tetramethyl-piperidin-4-yl)pyridazin-3-amine;
N-methyl-6-(quinolin-6-yl)-N-(2,2,6,6-tetramethylpiperidin-4-yl)pyridazin-3-amine;
6-(isoquinolin-7-yl)-N-methyl-N-(2,2,6,6-tetramethylpiperidin-4-yl)pyridazin-3-amine;
6-(isoquinolin-6-yl)-N-methyl-N-(2,2,6,6-tetramethylpiperidin-4-yl)pyridazin-3-amine;
6-(imidazo[1,2-a]pyridin-6-yl-pyridazin-3-yl)-methyl-(2,2,6,6-tetramethyl-piperidin-4-yl)-amine
methyl-[6-(6-phenyl-pyridin-3-yl)-pyridazin-3-yl]-(2,2,6,6-tetramethyl-piperidin-4-yl)-amine;
methyl-[6-(6-pyrrol-1-yl-pyridin-3-yl)-pyridazin-3-yl]-(2,2,6,6-tetra methyl-piperidin-4-yl)-amine;
methyl-(6-quinoxalin-2-yl-pyridazin-3-yl)-(2,2,6,6-tetramethyl-piperidin-4-yl)-amine;
methyl-(6-quinolin-3-yl-pyridazin-3-yl)-(2,2,6,6-tetramethyl-piperidin-4-yl)-amine;
N-methyl-6-(phthalazin-6-yl)-N-(2,2,6,6-tetramethylpiperidin-4-yl)pyridazin-3-amine;
6-(benzo[c][1,2,5]oxa-diazol-5-yl)-N-(2,2,6,6-tetramethyl-piperidin-4-yl)pyridazin-3-amine;
6-(benzo[d]thiazol-5-yl)-N-(2,2,6,6-tetramethyl-piperidin-4-yl)pyridazin-3-amine;
6-(2-methylbenzo-[d]oxazol-6-yl)-N-(2,2,6,6-tetramethyl-piperidin-4-yl)pyridazin-3-amine;
3-(6-(methyl(2,2,6,6-tetramethylpiperidin-4-yl)amino)pyridazin-3-yl)naphthalen-2-ol;
5-chloro-2-(6-(methyl(1 ,2,2,6,6-pentamethylpiperidin-4-yl)amino)pyridazin-3-yl)phenol;
3-(6-(2,2,6,6-tetramethylpiperidin-4-ylamino)pyridazin-3-yl)naphthalen-2-ol;
5-chloro-2-(6-(1,2,2,6,6-pentamethylpiperidin-4-ylamino)pyridazin-3-yl)phenol;
4-hydroxy-3-(6-(methyl(2,2,6,6-tetramethylpiperidin-4-yl)amino)pyridazin-3-yl)benzonitrile;
3-[6-(2,2,6,6-tetramethyl-piperidin-4-yloxy)-pyridazin-3-yl]-naphthalen-2-ol;
2-{6-[methyl-(2,2,6,6-tetramethyl-piperidin-4-yl)-amino]-pyridazin-3-yl}-4-trifluoromethylphenol;
2-fluoro-6-{6-[methyl-(2,2,6,6-tetramethyl-piperidin-4-yl)-amino]-pyridazin-3-yl}-phenol;
3,5-dimethoxy-2-{6-[methyl-(2,2,6,6-tetramethyl-piperidin-4-yl)-amino]-pyridazin-3-yl}-phenol;
4,5-dimethoxy-2-{6-[methyl-(2,2,6,6-tetramethyl-piperidin-4-yl)-amino]-pyridazin-3-yl}-phenol;
5-methoxy-2-{6-[methyl-(2,2,6,6-tetramethyl-piperidin-4-yl)-amino]-pyridazin-3-yl}-phenol;
4,5-difluoro-2-{6-[methyl-(2,2,6,6-tetramethyl-piperidin-4-yl)-amino]-pyridazin-3-yl}-phenol;
5-fluoro-2-{6-[methyl-(2,2,6,6-tetramethyl-piperidin-4-yl)-amino]-pyridazin-3-yl}-phenol;
3-hydroxy-4-(6-(methyl(2,2,6,6-tetramethylpiperidin-4-yl)amino)pyridazin-3-yl)benzonitrile;
1-allyl-6-(6-(methyl(2,2,6,6-tetramethylpiperidin-4-yl)amino)pyridazin-3-yl)naphthalen-2-ol;
6-(benzo[b]thiophen-2-yl)-N-(1 ,2,2,6,6-pentamethylpiperidin-4-yl)pyridazin-3-amine;
N-allyl-3-hydroxy-4-(6-(methyl(2,2,6,6-tetramethylpiperidin-4-yl)amino)pyridazin-3-yl)benzamide;
2-(6-(methyl(2,2,6,6-tetramethylpiperidin-4-yl)amino)pyridazin-3-yl)-5-(1H-pyrazol-1-yl)phenol;
5-(5-methyl-oxazol-2-yl)-2-{6-[methyl-(2,2,6,6-tetramethyl-piperidin-4-yl)-amino]-pyridazin-3-yl}-phenol;
5-(4-hydroxymethyl)-1H-pyrazole-1-yl)-2-(6-(methyl(2,2,6,6-tetramethylpiperidin-4 yl)amino)pyridazin-3-yl)phenol;
5-(1H-imidazole-1-yl)-2-(6-(methyl(2,2,6,6-tetramethyl-piperidin-4-yl)amino)pyridazin-3-yl)phenol;
5-(4-amino-1H-pyrazole-1-yl)-2-(6-(methyl(2,2,6,6-tetramethylpiperidin-4-yl)amino)pyridazin-3-yl)phenol;
5-(4-amino-1H-pyrazol-1-yl)-2-(6-(methyl(2,2,6,6-tetramethylpiperidin-4-yl)amino)pyridazin-3-yl)phenol;
5-(3-amino-pyrazol-1-yl)-2-{6-[methyl-(2,2,6,6-tetra methyl-piperidin-4-yl)-amino]-pyridazin-3-yl}-phenol;
2-(6-(methyl-(2,2,6,6-tetramethylpiperidin-4-yl)amino)pyridazin-3-yl)-5-(1-(2-morpholinoethyl)-1H-pyrazol-4-yl)phenol;
2-(6-(methyl-(2,2,6,6-tetramethylpiperidin-4-yl)amino)pyridazin-3-yl)-5-(1-methyl-1H-pyrazol-4-yl)phenol;
5-(5-amino-1H-pyrazol-1-yl)-2-(6-(methyl-(2,2,6,6-tetramethyl-piperidin-4-yl) amino)pyridazin-3-yl)phenol;
2-(6-(methyl-(2,2,6,6-tetramethylpiperidin-4-yl)amino)pyridazin-3-yl)-4-(1H-pyrazol-1-yl)phenol;
2-{6-[(2-hydroxy-ethyl)-(2,2,6,6-tetra methyl-piperidn-4-yl)-amino]-pyridazin-3-yl}-5-pyrazol-1-yl-phenol;
2-(6-(piperidin-4-yloxy)pyridazin-3-yl)-5-(1H-pyrazol-1-yl)phenol;
2-(6-(((2S,4R, 6R)-2,6-dimethylpiperidin-4-yl)oxy)pyridazin-3-yl)-5-(1H-pyrazol-1-yl)phenol;
5 2-(6-((2,6-di-methylpiperidin-4-yl)oxy)pyridazin-3-yl)-5-(1H-pyrazol-1-yl)phenol;
2-(6-((2,6-di-methylpiperidin-4-yl)oxy)pyridazin-3-yl)-5-(1H-pyrazol-1-yl)phenol;
5-(1H-pyrazol-1-yl)-2-(6-(pyrrolidin-3-yloxy)pyridazin-3-yl)phenol;
2-(6-((-2-methylpiperidin-4-yl)oxy)pyridazin-3-yl)-5-(1H-pyrazol-1-yl)phenol;

(S)-5-(1H-pyrazol-1-yl)-2-(6-(pyrrolidin-3-ylmethoxy)pyridazin-3-yl)phenol;
(R)-5-(1H-pyrazol-1-yl)-2-(6-(pyrrolidin-3-ylmethoxy)pyridazin-3-yl)phenol;
2-(6-((3-fluoropiperidin-4-yl)oxy)pyridazin-3-yl)-5-(1H-pyrazol-1-yl)-phenol;
2-[6-(1,2,2,6,6-pentamethyl-piperidin-4-yloxy)-pyridazin-3-yl]-5-pyrazol-1-yl-phenol;
5-pyrazol-1-yl-2-[6-((2,2,6,6-tetramethylpiperidin-4-yloxy)-pyridazin-3-yl]-phenol;
5-(1H-pyrazol-4-yl)-2-(6-((2,2,6,6-tetramethylpiperidin-4-yl)oxy)pyridazin-3-yl)phenol;
2-(6-piperazin-1-yl-pyridazin-3-yl)-5-pyrazol-1-yl-phenol;
3-[6-(azetidin-3-yl-amino)-pyridazin-3-yl]-naphthalen-2-ol;
2-[6-(azetidin-3-ylamino)-pyridazin-3-yl]-5-pyrazol-1-yl-phenol;
2-[6-(3, 5-dimethyl-piperazin-1-yl)-pyridazin-3-yl]-5-pyrazol-1-yl-phenol;
2-[6-(7-methyl-2,7-diaza-spiro[4.4]non-2-yl)-pyridazin-3-yl]-5-pyrazol-1-yl-phenol;
-(6-[1,4]diazepan-1-yl-pyridazin-3-yl)-5-pyrazol-1-yl-phenol;
2-{6-[4-(2-hydroxy-ethyl)-piperazin-1-yl]-pyridazin-3-yl}-5-pyrazol-1-yl-phenol;
2-[6-(3,6-diaza-bicyclo[3.2.1]oct-3-yl)-pyridazin-3-yl]-5-pyrazol-1-yl-phenol;
2-[6-(2, 7-diaza-spiro[3.5]non-7-yl)-pyridazin-3-yl]-5-pyrazol-1-yl-phenol;
2-[6-(3-hydroxy-methyl-piperazin-1-yl)-pyridazin-3-yl]-5-pyrazol-1-yl-phenol;
2-[6-(1,7-diaza-spiro[4.4]non-7-yl)-pyridazin-3-yl]-5-pyrazol-1-yl-phenol;
2-[6-(4-amino-4-methyl-piperidin-1-yl)-pyridazin-3-yl]-5-pyrazol-1-yl-phenol;
2-[6-(3-dimethyl-amino-piperidin-1-yl)-pyridazin-3-yl]-5-pyrazol-1-yl-phenol;
2-[6-(1,2,2,6,6-pentamethyl-piperidin-4-ylamino)-pyridazin-3-yl]-5-pyrazol-1-yl-phenol;
2-[6-(3, 3-dimethyl-piperazin-1-yl)-pyridazin-3-yl]-5-pyrazol-1-yl-phenol;
2-(6-(7-(2-hydroxyethyl)-2,7-diazaspiro[4.4]-nonan-2-yl)pyridazin-3-yl)-5-(1H-pyrazol-1-yl)phenol;
2-(6-((3aR,6aS)-hexahydropyrrolo[3,4-c]pyrrol-2(1H)-yl)pyridazin-3-yl)-5-(1H-pyrazol-1-yl)phenol;
3-(6-(piperazin-1-yl)pyridazin-3-yl)naphthalene-2,7-diol;
5-pyrazol-1-yl-2-[6-(1,2,3,6-tetrahydro-pyridin-4-yl)-pyridazin-3-yl]-phenol;
2-(6-piperidin-4-yl-pyridazin-3-yl)-5-pyrazol-1-yl-phenol;
3-(6-(1,2,3,6-tetra-hydropyridin-4-yl)pyridazin-3-yl)naphthalen-2-ol;
3-(6-3(1 ,2, 3, 6-tetrahydropyridin-4-yl)pyridazin-3-yl)naphthalene-2,7-diol;
3-(6-(2,2,6,6-tetramethyl-1,2,3,6-tetrahydropyridin-4-yl)pyridazin-3-yl)naphthalene-2,7-diol;
3-(6-(1-methyl-1 ,2,3,6-tetrahydropyridin-4-yl)pyridazin-3-yl)naphthalene-2,7-diol;
3-(6-(piperidin-4-yl)pyridazin-3-yl)naphthalene-2,7-diol;
3-(6-((2,2,6,6-tetramethylpiperidin-4-yl)oxy)pyridazin-3-yl)naphthalene-2,7-diol;
3-(6-(methyl-(2,2,6,6-tetramethylpiperidin-4-yl)amino)pyridazin-3-yl)naphthalene-2,7-diol;
3-(6-((2,2,6,6-tetramethylpiperidin-4-yl)amino)pyridazin-3-yl)naphthalene-2,7-diol;
[3-(7-hydroxy-6-{6-[methyl-(2,2,6,6-tetramethyl-piperidin-4-yl)-amino]-pyridazin-3-yl}-naphthalen-2-yloxy)-propyl]-carbamic acid tert-butyl ester;
7-(3-amino-propoxy)-3-{6-[methyl-(2,2,6,6-tetramethyl-piperidin-4-yl)-amino]-pyridazin-3-yl}naphthalen-2-ol;
N-[3-(7-hydroxy-6-{6-[methyl-(2,2,6,6-tetramethyl-piperidin-4-yl)-amino]-pyridazin-3-yl}naphthalen-2-yloxy)-propyl]-acetamide;
7-(3-hydroxypropoxy)-3-(6-(methyl(2,2,6,6-tetramethylpiperidin-4-yl)amino)pyridazin-3-yl)naphthalen-2-ol;
7-(3-methoxypropoxy)-3-(6-(methyl(2,2,6,6-tetramethylpiperidin-4-yl)amino)pyridazin-3-yl)naphthalen-2-ol;
7-(2-morpholinoethoxy)-3-(6-((2,2,6,6-tetramethylpiperidin-4-yl)oxy)pyridazin-3-yl)naphthalen-2-ol;
3-(6-(piperidin-4-ylmethyl)pyridazin-3-yl)naphthalen-2-ol;
5-(1H-pyrazol-1-yl)-2-(6-((2,2,6,6-tetramethylpiperidin-4-yl)methyl)pyridazin-3-yl)phenol;
3-methoxy-2-(6-(methyl(2,2,6-trimethylpiperidin-4-yl)amino)pyridazin-3-yl)-5-(5-methyloxazol-2-yl)phenol;
2-(6-((6S)-6-((S)-1-hydroxyethyl)-2,2-dimethylpiperidin-4-yloxy)pyridazin-3-yl)-5-(1H pyrazol-1-yl)phenol;
7-hydroxy-6-(6-(methyl(2,2,6,6-tetramethylpiperidin-4-yl)amino)pyridazin-3-yl)-2-naphthonitrile;
3-(6-(methyl(2,2,6,6-tetramethylpiperidin-4-yl)amino)pyridazin-3-yl)-7-(piperidin-1-ylmethyl)naphthalen-2-ol;
3-(6-(methyl(2,2,6,6-tetramethylpiperidin-4-yl)amino)pyridazin-3-yl)-7-(pyrrolidin-1-ylmethyl)naphthalen-2-ol;
1-bromo-6-(6-(methyl(2,2,6,6-tetramethylpiperidin-4-yl)amino)pyridazin-3-yl)naphthalene-2,7-diol;
1-chloro-6-(6-(methyl(2,2,6,6-tetramethylpiperidin-4-yl)amino)pyridazin-3-yl)naphthalene- 2,7-diol;
7-methoxy-3-(6-(methyl(2,2,6,6-tetramethylpiperidin-4-yl)amino)pyridazin-3-yl)naphthalen-2-ol;
7-methoxy-3-(6-(methyl(1,2,2,6,6-pentamethylpiperidin-4-yl)amino)pyridazin-3-yl)naphthalen-2-ol;
7-(3,6-dihydro-2H-pyran-4-yl)-3-(6-(methyl(2,2,6,6-tetramethylpiperidin-4-yl)amino)pyridazin-3-yl)naphthalen-2-ol;
3-(6-(methyl-(2,2,6,6-tetramethylpiperidin-4-yl)amino)pyridazin-3-yl)-7-(tetrahydro-2H-pyran-4-yl)naphthalen-2-ol;
7-(difluoromethyl)-3-(6-(methyl-(2,2,6,6-tetramethylpiperidin-4-yl)amino)pyridazin-3-yl)naphthalen-2-ol;

7-((4-hydroxy-2 -methylbutan-2-yl)oxy)-3-(6-(methyl(2,2,6,6-tetramethylpiperidin-4-yl)amino)pyridazin-3-yl)naphthalen-2-ol;
7-(3-hydroxy-3-methylbutoxy)-3-(6-(methyl-(2,2,6,6-tetramethylpiperidin-4-yl)amino)pyridazin-3-yl)naphthalen-2-ol;
2-(6-(methyl(2,2,6,6-tetramethylpiperidin-4-yl)amino)pyridazin-3-yl)-5-(1H-pyrazol-4-yl)benzene-1,3-diol;
3-methoxy-2-(6-(methyl-(2,2,6,6-tetramethylpiperidin-4-yl)amino)pyridazin-3-yl)-5-(1H pyrazol-4-yl)phenol;
5-(1H-pyrazol-4-yl)-2-(6-((2,2,6,6-tetramethylpiperidin-4-yl)amino)pyridazin-3-yl)-3-(trifluoromethoxy)phenol;
2-(6-(methyl(2,2,6,6-tetramethylpiperidin-4-yl)amino)pyridazin-3-yl)-5-(1-methyl-1H-pyrazol-4-yl)-3-(trifluoromethoxy)phenol;
2-(6-(methyl(2,2,6,6-tetramethylpiperidin-4-yl)amino)pyridazin-3-yl)-5-(1H-pyrazol-4-yl)-3-(trifluoromethoxy)phenol;
4-(3-hydroxy-4-(6-(methyl-(2,2,6,6-tetramethylpiperidin-4-yl)amino)pyridazin-3-yl)-5-(trifluoromethoxy)phenyl)-1-methylpyridin-2(1H)-one;
3-methoxy-2-(6-(methyl-(2,2,6,6-tetramethylpiperidin-4-yl)amino)pyridazin-3-yl)-5-(1-methyl-1H-pyrazol-4-yl)phenol;
3-methoxy-2-(6-(methyl-(2,2,6,6-tetramethylpiperidin-4-yl)amino)pyridazin-3-yl)-5-(5,6,7,8-tetrahydroimidazo[1,2-a]pyridin-3-yl)phenol;
3-methoxy-2-(6-(methyl-(2,2,6,6-tetramethylpiperidin-4-yl)amino)pyridazn-3-yl)-5-(pyridin-3-yl)phenol;
5-(1-cyclopentyl-1 H -pyrazol-4-yl)-3-meth oxy-2 -(6-(methyl( 2,2,6,6-tetra methylpiperidin-4-yl)amino)pyridazin-3-yl)phenol;
3' 5-dimethoxy-4-(6-(methyl-(2,2,6,6-tetramethylpiperidin-4-yl)amino)pyridazin-3-yl)-[1,1'-biphenyl]-3-ol;
3-(benzyloxy)-2-(6-(methyl-(2,2,6,6-tetramethylpiperidin-4-yl)amino)pyridazin-3-yl)-5-(5-methyloxazol-2-yl)phenol;
3-ethoxy-2-(6-(methyl-(2,2,6,6-tetramethylpiperidin-4-yl)amino)pyridazin-3-yl)-5-(5-methyloxazol-2-yl)phenol;
5 3-(cyclopropylmethoxy)-2-(6-(methyl-(2,2,6,6-tetramethylpiperidin-4-yl)amino)-pyridazin-3-yl)-5-(5-methyloxazol-2-yl)phenol;
2-methyl-5-(6-(methyl-(2,2,6,6-tetramethylpiperidin-4-yl)amino)pyridazin-3-yl)-1H benzo[d]imidazol-6-ol;
5-chloro-2-(6-(methyl-(2,2,6,6-tetramethylpiperidin-4-yl)amino)pyridazin-3-yl)phenol;
5-(1H-pyrazol-1-yl)-2-(6-((2,2,6,6-tetramethylpiperidin-4-yl)amino)pyridazin-3-yl)phenol;
3-hydroxy-4-(6-((2,2,6,6-tetramethylpiperidin-4-yl)amino)pyridazin-3-yl)benzonitrile;
2-(6-((2,2-dimethylpiperidin-4-yl)oxy)pyridazin-3-yl)-5-(1H-pyrazol-1-yl)phenol;
2-(6-(methyl-(2,2,6,6-tetramethylpiperidin-4-yl)amino)pyridazin-3-yl)-4-(1H-pyrazol-4-yl)phenol;
2-(6-(methyl-(2,2,6,6-tetramethylpiperidin-4-yl)amino)pyridazin-3-yl)-4-(4,5,6, 7-tetrahydropyrazolo[1,5-a]pyridin-3-yl)phenol;
2-(6-(methyl-(2,2,6,6-tetramethylpiperidin-4-yl)amino)pyridazin-3-yl)-4-(4,5,6,7-tetrahydropyrazolo[1,5-a] pyrazin-3-yl)phenol;
4-(1 H-indol-2-yl)-2-(6-(methyl-(2,2,6,6-tetramethylpiperidin-4-yl)amino)pyridazin-3-yl)phenol;
4-(cyclopent-1-en-1-yl)-2-(6-(methyl(2,2,6,6-tetramethylpiperidin-4-yl)amino)pyridazin-3-yl)phenol;
2-(6-(methyl-(2,2,6,6-tetramethylpiperidin-4-yl)amino)pyridazin-3-yl)-4-(1H-pyrazol-3-yl)phenol;
4-(4-hydroxy-3-(6-(methyl-(2,2,6,6-tetramethylpiperidin-4-yl)amino)pyridazin-3 yl)phenyl)pyridin-2-ol;
4-(4-hydroxy-3-(6-((2,2,6,6-tetramethylpiperidin-4-yl)oxy)pyridazin-3-yl)phenyl)-1-methylpyridin-2-(1H)-one;
4-(4-hydroxy-3-(6-((2,2,6,6-tetramethylpiperidin-4-yl)oxy)pyridazin-3-yl)phenyl)pyridin-2-ol;
5-(1H-indazol-7-yl)-2-(6-(methyl-(2,2,6,6-tetramethylpiperidin-4-yl)amino)pyridazin-3-yl)phenol;
4-chloro-2-(6-(methyl-(2,2,6,6-tetramethylpiperidin-4-yl)amino)pyridazin-3-yl)-5-(1H-pyrazol-4-yl)phenol;
4-fluoro-2-(6-(methyl-(2,2,6,6-tetramethylpiperidin-4-yl)amino)pyridazin-3-yl)-5-(1H-pyrazol-4-yl)phenol;
5-fluoro-4-(1 H-imidazol-4-yl)-2-(6-(methyl-(2,2,6,6-tetramethylpiperidin-4-yl)amino)pyridazin-3-yl)phenol;
5-fluoro-2-(6-(methyl-(2,2,6,6-tetramethylpiperidin-4-yl)amino)pyridazin-3-yl)-4-(1H-pyrazol-4-yl)phenol;
5-fluoro-2-(6-(methyl-(2,2,6,6-tetramethylpiperidin-4-yl)amino)pyridazin-3-yl)-4-(1H-pyrazol-5-yl)phenol;
5,6-hydroxy-5-(6-(methyl-(2,2,6,6-tetramethylpiperidin-4-yl)amino)pyridazin-3-yl)-2,3-dihydro-1H-inden-1-one;
6-(6-(methyl-(2,2,6,6-tetramethylpiperidin-4-yl)amino)pyridazin-3-yl)-1,4-dihydroindeno[1,2-c]pyrazol-7-ol;
6-hydroxy-5-(6-(methyl-(2,2,6,6-tetramethylpiperidin-4-yl)amino)pyridazin-3-yl)-2,3-dihydro-1H-inden-1-one oxime hydrochloride salt;
5-(6-(methyl-(2,2,6,6-tetramethylpiperidin-4-yl)amino)pyridazin-3-yl)-2,3-dihydro-1H-indene-1,6-diol;
2-amino-6-(6-(methyl-(2,2,6,6-tetramethylpiperidin-4-yl)amino)pyridazin-3-yl)-8H-indeno[1,2-d]thiazol-5-ol hydrochloride salt;
15 9-(6-(methyl-(2,2,6,6-tetramethylpiperidin-4-yl)amino)pyridazin-3-yl)-5,6-dihydroimidazo[5, 1-a]isoquinolin-8-ol hydrochloride salt;
4-hydroxy-3-(6-(methyl-(2,2,6,6-tetramethylpiperidin-4-yl)amino)pyridazin-3-yl)-N-((1-methyl-1H-pyrazol-4-yl)methyl)benzamide;
4-(4-(hydroxymethyl)-1H-pyrazol-1-yl)-2-(6-(methyl(2,2,6,6-tetramethylpiperidin-4-yl)amino)pyridazin-3-yl)phenol;
5-(1H-pyrazol-4-yl)-2-(6-((2,2,6,6-tetramethylpiperidin-4-yl)methyl)pyridazin-3-yl)phenol;
6-(3-(benzyloxy)isoquinolin-6-yl)-N-methyl-N-(2,2,6,6-tetramethylpiperidin-4-yl)pyridazin-3-amine;
6-(1-(benzyloxy)isoquinolin-7-yl)-N-methyl-N-(2,2,6,6-tetramethylpiperidin-4-yl)pyridazin-3-amine;
3-fluoro-5-(2-methoxypyridin-4-yl)-2-(6-(methyl-(2,2,6,6-tetramethylpiperidin-4-yl)amino)pyridazin-3-yl)phenol hydrochloride salt;
4-(3-fluoro-5-hydroxy-4-(6-(methyl-(2,2,6,6-tetramethylpiperidin-4-yl)amino)pyridazin-3-yl)phenyl)pyridin-2(1H)-one hydrochloride salt;
4-(3-fluoro-5-hydroxy-4-(6-(methyl-(2,2,6,6-tetramethylpiperidin-4-yl)amino)pyridazin-3-yl)phenyl)-1-methylpyridin-2(1H)-one hydrochloride salt;
5-(3-fluoro-5-hydroxy-4-(6-(methyl-(2,2,6,6-tetramethylpiperidin-4-yl)amino)pyridazin-3-yl)phenyl)-1-methylpyridin-2(1H)-one hydrochloride salt;
3-fluoro-5-(1H-pyrazol-4-yl)-2-(6-((2,2,6,6-tetramethylpiperidin-4-yl)oxy)pyridazin-3-yl)phenol hydrochloride salt;
5-chloro-3-fluoro-2-(6-(methyl-(2,2,6,6-tetramethylpiperidin-4-yl)amino)pyridazin-3-yl)phenol hydrochloride salt;
3-fluoro-2-(6-(methyl-(2,2,6,6-tetramethylpiperidin-4-yl)amino)pyridazin-3-yl)-5-(1H-pyrazol-4-yl)phenol hydrochloride salt;
3-fluoro-2-(6-(methyl-(2,2,6,6-tetramethylpiperidin-4-yl)amino)pyridazin-3-yl)-5-(1-methyl-1 H pyrazol-4-yl)phenol hydrochloride salt;
5-(5-methoxypyridin-3-yl)-2-(6-(methyl-(2,2,6,6-tetramethylpiperidin-4-yl)amino)pyridazin-3-yl)phenol;
5-(3-hydroxy-4-(6-methyl-(2,2,6,6-tetramethylpiperidin-4-yl)amino)pyridazin-3-yl) phenyl)pyridin-2-ol;
4-(3-hydroxy-4-(6-methyl-(2,2,6,6-tetramethylpiperidin-4-yl)amino)pyridazin-3-yl)phenyl)pyridin-2-ol;
5-(6-methoxypyridin-3-yl)-2-(6-(methyl-(2,2,6,6-tetramethylpiperidin-4-yl)amino)pyridazin-3-yl)phenol;
15 5-(3-hydroxy-4-(6-(methyl-(2,2,6,6-tetramethylpiperidin-4-yl)amino)pyridazin-3-yl)phenyl)-3-(trifluoromethyl)pyridin-2-ol;
5-(3-hydroxy-4-(6-(methyl-(2,2,6,6-tetramethylpiperidin-4-yl)amino)pyridazin-3-yl)phenyl)-1-methylpyridin-2(1H)-one;
4-(3-hydroxy-4-(6-(methyl-(2,2,6,6-tetramethylpiperidin-4-yl)amino)pyridazin-3-yl)phenyl)-1-methylpyridin-2(1H)-one;
5-(2-methoxypyridin-4-yl)-2-(6-(methyl-(2,2,6,6-tetramethylpiperidin-4-yl)amino)pyridazin-3-yl)phenol;
4-(3-hydroxy-4-(6-((2,2,6,6-tetramethylpiperidin-4-yl)oxy)pyridazin-3-yl)phenyl)pyridin-2-ol;
5-(6-(dimethylamino)pyridin-3-yl)-2-(6-(methyl(2,2,6,6-tetramethylpiperidin-4-yl)amino)pyridazin-3-yl)phenol;
4-(3-hydroxy-4-(6-((2,2,6,6-tetramethylpiperidin-4-yl)oxy)pyridazin-3-yl)phenyl)-1-methylpyridin-2(1H)-one;
2-(6-(methyl-(2,2,6,6-tetramethylpiperidin-4-yl)amino)pyridazin-3-yl)-5-(pyrimidin-5-yl)phenol;
5-(3-hydroxy-4-(6-(methyl-(2,2,6,6-tetramethylpiperidin-4-yl)amino)pyridazin-3-yl) phenyl)pyridin-3-ol;
1-cyclopropyl-4-(3-hyd roxy-4-(6-(methyl(2,2,6,6-tetra methylpiperidin-4-yl)amino)pyridazin-3-yl)phenyl)pyridin-2(1H)-one;
2-(6-(methyl-(2,2,6,6-tetramethylpiperidin-4-yl)amino)pyridazin-3-yl)-5-(1,2,3,6-tetrahydropyridin-4-yl)phenol;
5-(cyclopent-1-en-1-yl)-2-(6-(methyl(2,2,6,6-tetramethylpiperidin-4-yl)amino)pyridazin-3-yl)phenol;
5-(3,6-dihydro-2H-pyran-4-yl)-2-(6-(methyl(2,2,6,6-tetramethylpiperidin-4-yl)amino)pyridazin-3-yl)phenol;
5-(imidazo[1,5-a]pyridin-7-yl)-2-(6-(methyl(2,2,6,6-tetramethylpiperidin-4-yl)amino)pyridazin-3-yl)phenol;
5-(imidazo[1,2-a]pyridin-7-yl)-2-(6-(methyl(2,2,6,6-tetramethylpiperidin-4-yl)amino)pyridazin-3-yl)phenol;
2-(6-(methyl(2,2,6,6-tetramethylpiperidin-4-yl)amino)pyridazin-3-yl)-5-(2-methylpyridin-4-yl)phenol;
5-(1H-imidazol-2-yl)-2-(6-(methyl-(2,2,6,6-tetramethylpiperidin-4-yl)amino)pyridazin-3-yl)phenol;
5-(1H-imidazol-4-yl)-2-(6-(methyl-(2,2,6,6-tetramethylpiperidin-4-yl)amino)pyridazin-3-yl)phenol;
5-(imidazo[1,2-a]pyrazin-3-yl)-2-(6-(methyl(2,2,6,6-tetramethylpiperidin-4-yl)amino)pyridazin-3-yl)phenol;
2-(6-(methyl-(2,2,6,6-tetramethylpiperidin-4-yl)amino)pyridazin-3-yl)-5-(5,6,7,8-tetrahydroimidazo[1,2-a]pyrazin-3-yl)phenol;
2-(6-(methyl-(2,2,6,6-tetramethylpiperidin-4-yl)amino)pyridazin-3-yl)-5-(4-methyl-1H-imidazol-2-yl)phenol;
2-(6-(methyl-(2,2,6,6-tetramethylpiperidin-4-yl)amino)pyridazin-3-yl)-5-(1-methyl-1H-imidazol-4-yl)phenol;
2-(6-(methyl-(2,2,6,6-tetramethylpiperidin-4-yl)amino)pyridazin-3-yl)-5-(1-methyl-1H imidazol-5-yl)phenol;
2-(6-(methyl-(2,2,6,6-tetramethylpiperidin-4-yl)amino)pyridazin-3-yl)-5-(4-nitro-1H-imidazol-2-yl)phenol;
25 2-(6-(methyl-(2,2,6,6-tetramethylpiperidin-4-yl)amino)pyridazin-3-yl)-5-(2-methyl-1H imidazol-4-yl)phenol;
5-(1,2-dimethyl-1H-imidazol-4-yl)-2-(6-(methyl(2,2,6,6-tetramethylpiperidin-4-yl)amino)pyridazin-3-yl)phenol;
1-(3-hydroxy-4-(6-(methyl-(2,2,6,6-tetramethylpiperidin-4-yl)amino)pyridazin-3-yl)phenyl)-1H-pyrazole-4-carboxamide;
2-( 6-((3aR,6aS)-5-(2-hydroxyethyl)hexahydropyrrolo[3,4-c]pyrrol-2 (1H)-yl)pyridazin-3-yl)-5-(1H-pyrazol-4-yl)phenol ;
2-(6-((3aR,6aS)-hexahydropyrrolo[3,4-c]pyrrol-2(1H)-yl)pyridazin-3-yl)-5-(1H-pyrazol-4-yl)phenol;
2-(6-((3aR,6aS)-5-methylhexahydropyrrolo[3,4-c]pyrrol-2(1H)-yl)pyridazin-3-yl)-5-(1 Hpyrazol-4-yl)phenol;
4-(3-hydroxy-4-(6-(5-methylhexahydropyrrolo[3,4-c]pyrrol-2(1H)-yl)pyridazin-3-yl)phenyl)-1-methylpyridin-2(1H)-one;
4-(3-hydroxy-4-(6-((3aR, 6aR)-1-methylhexahydropyrrolo[3,4-b]pyrrol-5(1H)-yl)pyridazin-3-yl)phenyl)-1-methylpyridin-2(1H)-one;
2-(6-(2, 7-diazaspiro[4.5]decan-2-yl)pyridazin-3-yl)-5-(1H-pyrazol-4-yl)phenol; and
4-(4-(6-(2,7-diazaspiro[4.5]decan-2-yl)pyridazin-3-yl)-3-hydroxyphenyl)-1-methylpyridin-2(1H)-one;
5-(2-Methoxy-4-(1H-pyrazol-1-yl)phenyl)-N-methyl-N-(2,2,6,6-tetramethylpiperidin-4-yl)-1,3,4-thiadiazol-2-amine;
6-(5-(Methyl(2,2,6,6-tetramethylpiperidin-4-yl)amino)-1,3,4-thiadiazol-2-yl)naphthalen-2-ol;
5-(2-Methoxyquinolin-3-yl)-N-methyl-N-(2,2,6,6-tetramethylpiperidin-4-yl)-1,3,4-thiadiazol-2-amine;
5-(3-Methoxynaphthalen-2-yl)-N-methyl-N-(2,2,6,6-tetramethylpiperidin-4-yl)-1,3,4-thiadiazol-2-amine;
5-(2-Methoxy-4-(1H-pyrazol-1-yl)phenyl)-N-(1,2,2,6,6-pentamethylpiperidin-4-yl)-1,3,4-thiadiazol-2-amine;
5-(2-Methoxy-4-(1-methyl-1H-pyrazol-4-yl)phenyl)-N-methyl-N-(2,2,6,6-tetramethylpiperidin-4-yl)-1,3,4-thiadiazol-2-amine;
5-(2-Methoxy-4-(1H-pyrazol-4-yl)phenyl)-N-methyl-N-(2,2,6,6-tetramethylpiperidin-4-yl)-1,3,4-thiadiazol-2-amine;
4-(3-Methoxy-4-(5-(methyl(2,2,6,6-tetramethylpiperidin-4-yl)amino)-1,3,4-thiadiazol-2-yl)phenyl)-1-methylpyridin-2(1H)-one;
5-(3-Methoxy-4-(5-(methyl(2,2,6,6-tetramethylpiperidin-4-yl)amino)-1,3,4-thiadiazol-2-yl)phenyl)pyridin-2-ol;
5-(3-Methoxy-4-(5-(methyl(2,2,6,6-tetramethylpiperidin-4-yl)amino)-1,3,4-thiadiazol-2-yl)phenyl)-1-methylpyridin-2(1H)-one;
N-Methyl-5-(2-methyl-4-(1-methyl-1H-pyrazol-4-yl)phenyl)-N-(2,2,6,6-tetramethylpiperidin-4-yl)-1,3,4-thiadiazol-2-amine;
1-Methyl-4-(4-(5-(methyl(2,2,6,6-tetramethylpiperidin-4-yl)amino)-1,3,4-thiadiazol-2-yl)-3-(trifluoromethoxy)phenyl)pyridin-2(1H)-one;
5-(4-(3,5-Dimethyl-1H-pyrazol-4-yl)-2-methoxyphenyl)-N-methyl-N-(2,2,6,6-tetramethylpiperidin-4-yl)-1,3,4-thiadiazol-2-amine;
5-(2-Methoxy-4-(1-methyl-1H-pyrazol-4-yl)phenyl)-N-methyl-N-(2,2,6,6-tetramethylpiperidin-4-yl)-1,3,4-thiadiazol-2-amine;
2-(5-(Methyl(2,2,6,6-tetramethylpiperidin-4-yl)amino)-1,3,4-thiadiazol-2-yl)-5-(1-methyl-1H-pyrazol-4-yl)phenol;
2-(5-(Methyl(2,2,6,6-tetramethylpiperidin-4-yl)amino)-1,3,4-thiadiazol-2-yl)-5-(1H-pyrazol-1-yl)phenol;
5-(3-Hydroxy-4-(5-(methyl(2,2,6,6-tetramethylpiperidin-4-yl)amino)-1,3,4-thiadiazol-2-yl)phenyl)-1-methylpyridin-2(1H)-one;
4-(3-Hydroxy-4-(5-(methyl(2,2,6,6-tetramethylpiperidin-4-yl)amino)-1,3,4-thiadiazol-2-yl)phenyl)-1-methylpyridin-2(1H)-one;
5-(3-Hydroxy-4-(5-(methyl(2,2,6,6-tetramethylpiperidin-4-yl)amino)-1,3,4-thiadiazol-2-yl)phenyl)pyridin-2-ol;
3-(5-(Methyl(2,2,6,6-tetramethylpiperidin-4-yl)amino)-1,3,4-thiadiazol-2-yl)naphthalene-2,7-diol;
3-(5-((3aR,6aS)-Hexahydropyrrolo[3,4-c]pyrrol-2(1H)-yl)-1,3,4-thiadiazol-2-yl)naphthalene-2,7-diol;
3-(5-(Methyl(2,2,6,6-tetramethylpiperidin-4-yl)amino)-1,3,4-thiadiazol-2-yl)naphthalen-2-ol hydrobromide salt;
3-(5-(Methyl(2,2,6,6-tetramethylpiperidin-4-yl)amino)-1,3,4-thiadiazol-2-yl)quinolin-2-ol;
2-(5-(Methyl(2,2,6,6-tetramethylpiperidin-4-yl)amino)-1,3,4-thiadiazol-2-yl)-4-(1H-pyrazol-1-yl)phenol;
5-(2-Chloro-4-(1-methyl-1H-pyrazol-4-yl)phenyl)-N-methyl-N-(2,2,6,6-tetramethylpiperidin-4-yl)-1,3,4-thiadiazol-2-amine;
3-Chloro-2-(5-(methyl(2,2,6,6-tetramethylpiperidin-4-yl)amino)-1,3,4-thiadiazol-2-yl)-5-(1-methyl-1H-pyrazol-4-yl)phenol;
5-(2-Chloro-4-(1-methyl-1H-pyrazol-4-yl)phenyl)-N-(2,2,6,6-tetramethylpiperidin-4-yl)-1,3,4-thiadiazol-2-amine;
3-Methoxy-2-(5-(methyl(2,2,6,6-tetramethylpiperidin-4-yl)amino)-1,3,4-thiadiazol-2-yl)-5-(5-methyloxazol-2-yl)phenol;
2-(2-Methoxy-4-(1H-pyrazol-1-yl)phenyl)-5-(1,2,3,6-tetrahydropyridin-4-yl)-1,3,4-thiadiazole;
2-(5-(piperazin-1-yl)-1,3,4-thiadiazol-2-yl)-5-(1H-pyrazol-1-yl)phenol;
5-(7-Methoxyquinolin-6-yl)-N-methyl-N-(2,2,6,6-tetramethylpiperidin-4-yl)-1,3,4-thiadiazol-2-amine;

6-(5-(Methyl(2,2,6,6-tetramethylpiperidin-4-yl)amino)-1,3,4-thiadiazol-2-yl)quinolin-7-ol;
3-methoxy-4-(5-(methyl(2,2,6,6-tetramethylpiperidin-4-yl)amino)-1,3,4-thiadiazol-2-yl)benzonitrile;
3-fluoro-4-(5-(methyl(2,2,6,6-tetramethylpiperidin-4-yl)amino)-1,3,4-thiadiazol-2-yl)benzonitrile;
methyl 3-fluoro-4-(5-(methyl(2,2,6,6-tetramethylpiperidin-4-yl)amino)-1,3,4-thiadiazol-2-yl)benzoate;
5-(2-methoxy-4-(3-(methylamino)-1H-pyrazol-1-yl)phenyl)-N-methyl-N-(2,2,6,6-tetramethylpiperidin-4-yl)-1,3,4-thiadiazol-2-amine;
7-methoxy-6-(5-(methyl(2,2,6,6-tetramethylpiperidin-4-yl)amino)-1,3,4-thiadiazol-2-yl)quinoline-2-carbonitrile;
4-(3-methoxy-4-(5-((2,2,6,6-tetramethylpiperidin-4-yl)oxy)-1,3,4-thiadiazol-2-yl)phenyl)-1-methylpyridin-2(1H)-one;
4-(3-chloro-4-(5-(methyl(2,2,6,6-tetramethylpiperidin-4-yl)amino)-1,3,4-thiadiazol-2-yl)phenyl)-1-methylpyridin-2(1H)-one;
5-(2-Chloro-4-(1H-pyrazol-4-yl)phenyl)-N-methyl-N-(2,2,6,6-tetramethylpiperidin-4-yl)-1,3,4-thiadiazol-2-amine;
5-(2-Chloro-4-(4,5, 6, 7-tetrahydropyrazolo[1,5-a]pyridin-3-yl)phenyl)-N-methyl-N-(2,2,6,6-tetramethylpiperidin-4-yl)-1,3,4-thiadiazol-2-amine;
N-methyl-5-(5-(1-methyl-1H-pyrazol-4-yl)pyridin-2-yl)-N-(2,2,6,6-tetramethylpiperidin-4-yl)-1,3,4-thiadiazol-2-amine Hydrochloride salt;
2-(2-chloro-4-(1-methyl-1H-pyrazol-4-yl)phenyl)-5-((2,2,6,6-tetramethylpiperidin-4-yl)oxy)-1,3,4-thiadiazole;
5-(2-chloro-4-(6-methoxypyridin-3-yl)phenyl)-N-methyl-N-(2,2,6,6-tetramethylpiperidin-4-yl)-1,3,4-thiadiazol-2-amine;
5-(4-(6-aminopyridin-3-yl)-2-fluorophenyl)-N-methyl-N-(2,2,6,6-tetramethylpiperidin-4-yl)-1,3,4-thiadiazol-2-amine;
5-(2-fluoro-4-(3-methyl-1H-pyrazol-5-yl)phenyl)-N-methyl-N-(2,2,6,6-tetramethylpiperidin-4-yl)-1,3,4-thiadiazol-2-amine;
5-(2-fluoro-4-(1H-pyrazol-5-yl)phenyl)-N-methyl-N-(2,2,6,6-tetramethylpiperidin-4-yl)-1,3,4-thiadiazol-2-amine;
5-(2,3-difluoro-4-(1H-pyrazol-4-yl)phenyl)-N-methyl-N-(2,2,6,6-tetramethylpiperidin-4-yl)-1,3,4-thiadiazol-2-amine;
5-(2,3-difluoro-4-(1H-pyrazol-5-yl)phenyl)-N-methyl-N-(2,2,6,6-tetramethylpiperidin-4-yl)-1,3,4-thiadiazol-2-amine;
5-(2,5-difluoro-4-(1H-pyrazol-4-yl)phenyl)-N-methyl-N-(2,2,6,6-tetramethylpiperidin-4-yl)-1,3,4-thiadiazol-2-amine;
5-(2,5-difluoro-4-(1H-pyrazol-5-yl)phenyl)-N-methyl-N-(2,2,6,6-tetramethylpiperidin-4-yl)-1,3,4-thiadiazol-2-amine;
5-(2,6-difluoro-4-(1H-pyrazol-4-yl)phenyl)-N-methyl-N-(2,2,6,6-tetramethylpiperidin-4-yl)-1,3,4-thiadiazol-2-amine;
2-(2,5-difluoro-4-(1H-pyrazol-4-yl)phenyl)-5-((3aR,6aS)-hexahydropyrrolo[3,4-c]pyrrol-2(1H)-yl)-1,3,4-thiadiazole;
5-(2-chloro-5-fluoro-4-(1H-pyrazol-4-yl)phenyl)-N-methyl-N-(2,2,6,6-tetramethylpiperidin-4-yl)-1,3,4-thiadiazol-2-amine;
5-(3-fluoro-5-(1H-pyrazol-4-yl)pyridin-2-yl)-N-methyl-N-(2,2,6,6-tetramethylpiperidin-4-yl)-1,3,4-thiadiazol-2-amine;
5-(4-(2-aminopyrimidin-4-yl)-2-chlorophenyl)-N-methyl-N-(2,2,6,6-tetramethylpiperidin-4-yl)-1,3,4-thiadiazol-2-amine;
5-(5-(2-aminopyrimidin-4-yl)-2-chlorophenyl)-N-methyl-N-(2,2,6,6-tetramethylpiperidin-4-yl)-1,3,4-thiadiazol-2-amine;
5-(4-(2,4-dimethylthiazol-5-yl)-2,5-difluorophenyl)-N-methyl-N-(2,2,6,6-tetramethylpiperidin-4-yl)-1,3,4-thiadiazol-2-amine;
5-(4-(2,4-dimethylthiazol-5-yl)-2,3-difluorophenyl)-N-methyl-N-(2,2,6,6-tetramethylpiperidin-4-yl)-1,3,4-thiadiazol-2-amine;
4-(3-hydroxy-4-(5-(methyl(2,2,6,6-tetramethylpiperidin-4-yl)amino)-1,3,4-thiadiazol-2-yl)-5-(trifluoromethoxy)phenyl)-1-methylpyridin-2(1H)-one;
5-(2-fluoro-6-methoxy-4-(1H-pyrazol-4-yl)phenyl)-N-methyl-N-(2,2,6,6-tetramethylpiperidin-4-yl)-1,3,4-thiadiazol-2-amine;
2-(2-fluoro-6-methoxy-4-(1H-pyrazol-4-yl)phenyl)-5-((3aR,6aS)-5-methylhexahydropyrrolo[3,4-c]pyrrol-2(1H)-yl)-1,3,4-thiadiazole;
5-(2,3-difluoro-6-methoxy-4-(1H-pyrazol-4-yl)phenyl)-N-methyl-N-(2,2,6,6-tetramethylpiperidin-4-yl)-1,3,4-thiadiazol-2-amine;

6-methoxy-2-(5-(methyl(2,2,6,6-tetramethylpiperidin-4-yl)amino)-1,3,4-thiadiazol-2-yl)-3,4-dihydroisoquinolin-1(2H)-one;
5-(2-Chloro-4-(1H-pyrazol-1-yl)phenyl)-N-methyl-N-(2,2,6,6-tetramethylpiperidin-4-yl)-1,3,4-thiadiazol-2-amine;
5-(2-Chloro-4-(1H-1,2,3-triazol-1-yl)phenyl)-N-methyl-N-(2,2,6,6-tetramethylpiperidin-4-yl)-1,3,4-thiadiazol-2-amine;
5-(2-chloro-4-(2H-1,2,3-triazol-2-yl)phenyl)-N-methyl-N-(2,2,6,6-tetramethylpiperidin-4-yl)-1,3,4-thiadiazol-2-amine;
5-(2-chloro-4-(1 H-1 ,2,4-triazol-1-yl)phenyl)-N-methyl-N-(2,2,6,6-tetramethylpiperidin-4-yl)-1,3,4-thiadiazol-2-amine;
5-(4-(3-amino-1H-pyrazol-1-yl)-2-chlorophenyl)-N-methyl-N-(2,2,6,6-tetramethylpiperidin-4-yl)-1,3,4-thiadiazol-2-amine;
2-(2-chloro-4-(1 H-imidazol-1-yl)phenyl)-5-((3aR,6aS)-5-methylhexahydropyrrolo[3,4-c]pyrrol-2(1H)-yl)-1,3,4-thiadiazole;
5-(2-Chloro-4-(1H-imidazol-1-yl)phenyl)-N-methyl-N-(2,2,6,6-tetramethylpiperidin-4-yl)-1,3,4-thiadiazol-2-amine;
5-(2-fluoro-4-(1H-imidazol-1-yl)phenyl)-N-methyl-N-(2,2,6,6-tetramethylpiperidin-4-yl)-1,3,4-thiadiazol-2-amine;
5-(2-methoxy-4-(1H-pyrazol-5-yl)phenyl)-N-methyl-N-(2,2,6,6-tetramethylpiperidin-4-yl)-1,3,4-thiadiazol-2-amine;
5-(4-(2,4-dimethylthiazol-5-yl)-2-methoxyphenyl)-N-methyl-N-(2,2,6,6-tetramethylpiperidin-4-yl)-1,3,4-thiadiazol-2-amine;
5-(2-methoxy-4-(pyridin-3-yl)phenyl)-N-methyl-N-(2,2,6,6-tetramethylpiperidin-4-yl)-1,3,4-thiadiazol-2-amine;
5-(2-fluoro-4-(1H-pyrazol-4-yl)phenyl)-N-methyl-N-(2,2,6,6-tetramethylpiperidin-4-yl)-1,3,4-thiadiazol-2-amine;
5-(2-methoxy-4-(2-methoxypyridin-4-yl)phenyl)-N-methyl-N-(2,2,6,6-tetramethylpiperidin-4-yl)-1,3,4-thiadiazol-2-amine;
5-(2-methoxy-4-(6-methoxypyridin-3-yl)phenyl)-N-methyl-N-(2,2,6,6-tetramethylpiperidin-4-yl)-1,3,4-thiadiazol-2-amine;
2-(2-Chloro-4-(1-methyl-1H-pyrazol-4-yl)phenyl)-5-((3aR,6aS)-5-methylhexahydropyrrolo[3,4-c]pyrrol-2(1H)-yl)-1,3,4-thiadiazole;
2-(2-chloro-4-(1H-pyrazol-4-yl)phenyl)-5-((3aR,6aS)-5-methylhexahydropyrrolo[3,4-c]pyrrol-2(1H)-yl)-1,3,4-thiadiazole;
2-(2-Chloro-4-(1H-pyrazol-4-yl)phenyl)-5-((3aR, 6a R)-1-methylhexahyd ropyrrolo[3,4-b]pyrrol-5(1H)-yl)-1,3,4-thiadiazole;
1-(4-(5-(2-chloro-4-(1H-pyrazol-4-yl)phenyl)-1,3,4-thiadiazol-2-yl)morpholin-2-yl)-N,N-dimethylmethanamine;
2-(2-chloro-4-(1H-pyrazol-4-yl)phenyl)-5-(2-methyl-2,7-diazaspiro[4.5]decan-7-yl)-1,3,4-thiadiazole;
2-(2-fluoro-4-(1H-pyrazol-4-yl)phenyl)-5-((3aR,6aS)-5-methylhexahydropyrrolo[3,4-c]pyrrol-2(1H)-yl)-1,3,4-thiadiazole;
2-(2-methoxy-4-(1-methyl-1H-pyrazol-4-yl)phenyl)-5-(2,6-diazaspiro[3.5]nonan-2-yl)-1,3,4-thiadiazole;
2-(2-methoxy-4-(1-methyl-1H-pyrazol-4-yl)phenyl)-5-(2, 7-diazaspiro[3.5]nonan-2-yl)-1,3,4-thiadiazole;
2-(5-(Methyl(2,2,6,6-tetramethylpiperidin-4-yl)amino)-1,3,4-thiadiazol-2-yl)-5-(1H-pyrazol-1-yl)phenol;
5-(3-chloro-4-(5-(methyl(2,2,6,6-tetramethylpiperidin-4-yl)amino)-1,3,4-thiadiazol-2-yl)phenyl)pyridin-2(1H)-one;
2-(5-(methyl(2,2,6,6-tetramethylpiperidin-4-yl)amino)-1,3,4-thiadiazol-2-yl)-5-(3-(methylamino)-1H-pyrazol-1-yl)phenol;
3-fluoro-2-(5-(methyl(2,2,6,6-tetramethylpiperidin-4-yl)am ino)-1,3,4-thiadiazol-2-yl)-5-(1H-pyrazol-4-yl)phenol;
3,4-d ifluoro-2-(5-(methyl(2,2,6,6-tetramethylpiperidin-4-yl)amino)-1,3,4-thiadiazol-2-yl)-5-(1H-pyrazol-4-yl)phenol;
6-hydroxy-5-(5-(methyl(2,2,6,6-tetramethylpiperidin-4-yl)amino)-1,3,4-thiadiazol-2-yl)-2,3-dihydro-1H-inden-1-one;
2-(5-(methyl(2,2,6,6-tetramethylpiperidin-4-yl)amino)-1,3,4-thiadiazol-2-yl)-5-(1H-pyrazol-4-yl)phenol;
2-(5-(2,6-diazaspiro[3.5]nonan-2-yl)-1,3,4-thiadiazol-2-yl)-5-(1-methyl-1H-pyrazol-4-yl)phenol;
2-(5-(2,7-diazaspiro[3.5]nonan-2-yl)-1,3,4-thiadiazol-2-yl)-5-(1-methyl-1H-pyrazol-4-yl)phenol;
3-fluoro-2-(5-((3aR,6aS)-hexahydropyrrolo[3,4-c]pyrrol-2(1H)-yl)-1,3,4-thiadiazol-2-yl)-5-(1H-pyrazol-4-yl)phenol Di-hydrochloride salt;
3-Chloro-2-(5-(methyl(2,2,6,6-tetramethylpiperidin-4-yl)am ino )-1,3,4-thiadiazol-2-yl)-5-(1H-pyrazol-4-yl)phenol;
2-(2-methoxy-4-(1H-pyrazol-1-yl)phenyl)-5-((2,2,6,6-tetramethylpiperidin-4-yl)methyl)-1,3,4-thiadiazole;
2-(2,3-difluoro-4-(1H-pyrazol-4-yl)phenyl)-5-(2, 7-diazaspiro[3.5]nonan-2-yl)-1,3,4-thiadiazole;
2-(5-(2,7-diazaspiro[3.5]nonan-2-yl)-1,3,4-thiadiazol-2-yl)-3-fluoro-5-(1H-pyrazol-4-yl)phenol;
4-methoxy-1-methyl-3-(5-(methyl(2,2,6,6-tetramethylpiperidin-4-yl)amino)-1,3,4-thiadiazol-2-yl)quinolin-2(1H)-one;
4-hydroxy-1-methyl-3-(5-(methyl(2,2,6,6-tetramethylpiperidin-4-yl)amino)-1,3,4-thiadiazol-2-yl)quinolin-2(1H)-one;
3-(5-(methyl(2,2,6,6-tetramethylpiperidin-4-yl)amino)-1,3,4-thiadiazol-2-yl)quinolin-2(1H)-one;
1-methyl-3-(5-(methyl(2,2,6,6-tetramethylpiperidin-4-yl)amino)-1,3,4-thiadiazol-2-yl)quinolin-2(1H)-one;
2-(2-Chloro-4-(1H-pyrazol-4-yl)phenyl)-5-((3aR,6aS)-hexahydropyrrolo[3,4-c]pyrrol-2(1H)yl)-1,3,4-thiadiazole Hydrochloride Salt;
2-(2-Chloro-4-(1H-pyrazol-4-yl)phenyl)-5-(2, 7-diazaspiro[4 .5]decan-2-yl)-1,3,4-thiadiazole Hydrochloride Salt;
(R)-(4-(5-(2-chloro-4-(1H-pyrazol-4-yl)phenyl)-1,3,4-thiadiazol-2-yl)piperazi n-2-yl)methanol Hydrochloride Salt;
2-(5-(methyl(2,2,6,6-tetramethylpiperidin-4-yl)amino)-1,3,4-thiadiazol-2-yl)benzo[b]thiophene-5-carbonitrile; and
5-(3-chlorobenzo[b]thiophen-2-yl)-N-methyl-N-(2,2,6,6-tetramethylpiperidin-4-yl)-1,3,4-thiadiazol-2-amine;

or a pharmaceutically acceptable salt thereof;
for use in the treatment, prevention and/or delay of progression of cancer.

**14.** A FoxM1 gene splicing modifier selected from the group consisting of:

3-(6-(methyl(2,2,6,6-tetramethylpiperidin-4-yl)amino)pyridazin-3-yl)naphthalen-2-ol;
3-hydroxy-4-(6-(methyl(2,2,6,6-tetramethylpiperidin-4-yl)amino)pyridazin-3-yl)benzonitrile;
5-(1H-pyrazol-4-yl)-2-(6-(methyl(2,2,6,6-tetramethylpiperidin-4-yl)amino)pyridazin-3-yl)phenol
2-(6-(methyl-(2,2,6,6-tetramethylpiperidin-4-yl)amino)pyridazin-3-yl)-5-(1-methyl-1H-pyrazol-4-yl)phenol;
5-pyrazol-1-yl-2-[6-((2,2,6,6-tetramethylpiperidin-4-yloxy)-pyridazin-3-yl]-phenol;
5-(1H-pyrazol-4-yl)-2-(6-((2,2,6,6-tetramethylpiperidin-4-yl)oxy)pyridazin-3-yl)phenol;
3-(6-((2,2,6,6-tetramethylpiperidin-4-yl)oxy)pyridazin-3-yl)naphthalene-2,7-diol;
3-(6-(methyl-(2,2,6,6-tetramethylpiperidin-4-yl)amino)pyridazin-3-yl)naphthalene-2,7-diol;
7-methoxy-3-(6-(methyl(2,2,6,6-tetramethylpiperidin-4-yl)amino)pyridazin-3-yl)naphthalen-2-ol;
5-(3-hydroxy-4-(6-(methyl-(2,2,6,6-tetramethylpiperidin-4-yl)amino)pyridazin-3-yl)phenyl)-1-methylpyridin-2(1H)-one;
4-(3-hydroxy-4-(6-(methyl-(2,2,6,6-tetramethylpiperidin-4-yl)amino)pyridazin-3-yl)phenyl)-1-methylpyridin-2(1H)-one;
4-(3-chloro-4-(5-(methyl(2,2,6,6-tetramethylpiperidin-4-yl)amino)-1,3,4-thiadiazol-2-yl)phenyl)-1-methylpyridin-2(1H)-one;
5-(2-Chloro-4-(1H-pyrazol-4-yl)phenyl)-N-methyl-N-(2,2,6,6-tetramethylpiperidin-4-yl)-1,3,4-thiadiazol-2-amine;
5-(2,5-difluoro-4-(1H-pyrazol-5-yl)phenyl)-N-methyl-N-(2,2,6,6-tetramethylpiperidin-4-yl)-1,3,4-thiadiazol-2-amine;

or a pharmaceutically acceptable salt thereof;
for use in the treatment, prevention and/or delay of progression of cancer.

**15.** The FoxM1 gene splicing modifier according to any of claims 1 to 14 or a pharmaceutically acceptable salt thereof for use in the treatment, prevention and/or delay of progression of cancer, wherein the FoxM1 gene splicing modifier induces a transcriptionally inactive FoxM1 variant.

**16.** The FoxM1 gene splicing modifier according to any of claims 1 to 15 or a pharmaceutically acceptable salt thereof for use in the treatment, prevention and/or delay of progression of cancer, wherein the FoxM1 gene splicing modifier induces the transcriptionally inactive FoxM1 variant FoxM1A.

**17.** The FoxM1 gene splicing modifier according to any of claims 1 to 16 or a pharmaceutically acceptable salt thereof for use in the treatment, prevention and/or delay of progression of cancer, wherein the FoxM1 gene is the human

FoxM1 gene.

**18.** The FoxM1 gene splicing modifier according to any of claims 1 to 17 or a pharmaceutically acceptable salt thereof for use in the treatment, prevention and/or delay of progression of cancer, wherein the cancer is selected from the group consisting of cancer of the liver, prostate, brain, breast, lung, colon, pancreas, skin, cervix, ovary, mouth, blood and nervous system.

**19.** The FoxM1 gene splicing modifier according to any of claims 1 to 18 or a pharmaceutically acceptable salt thereof for use in the treatment, prevention and/or delay of progression of cancer, wherein the cancer is selected from the group consisting of leukemia, acute myeloid leukemia, colon cancer, gastric cancer, macular degeneration, acute monocytic leukemia, breast cancer, hepatocellular carcinoma, cone-rod dystrophy, alveolar soft part sarcoma, myeloma, skin melanoma, prostatitis, pancreatitis, pancreatic cancer, retinitis, adenocarcinoma, adenoiditis, adenoid cystic carcinoma, cataract, retinal degeneration, gastrointestinal stromal tumor, Wegener's granulomatosis, sarcoma, myopathy, prostate adenocarcinoma, Hodgkin's lymphoma, ovarian cancer, non-Hodgkin's lymphoma, multiple myeloma, chronic myeloid leukemia, acute lymphoblastic leukemia, renal cell carcinoma, transitional cell carcinoma, colorectal cancer, chronic lymphocytic leukemia, anaplastic large cell lymphoma, kidney cancer, breast cancer, and cervical cancer.

**20.** Use of a FoxM1 gene splicing modifier according to any of claims 1 to 19 for the preparation of a medicament for the treatment, prevention and/or delay of progression of cancer.

**21.** Use of a FoxM1 gene splicing modifier according to any of claims 1 to 19 for the treatment, prevention and/or delay of progression of cancer.

**22.** A method for the treatment, prevention and/or delay of progression of cancer comprising administering an effective amount of a FoxM1 gene splicing modifier according to any of claims 1 to 19 to a subject.

**23.** A pharmaceutical composition comprising a FoxM1 gene splicing modifier according to any of claims 1 to 19 for use in the treatment, prevention and/or delay of progression of cancer.

**24.** A combination comprising a therapeutically effective amount of a FoxM1 gene splicing modifier according to any of claims 1 to 19 or a pharmaceutically acceptable salt thereof and one or more therapeutically active co-agents.

**25.** The invention as described herein before.

Fig. 1A

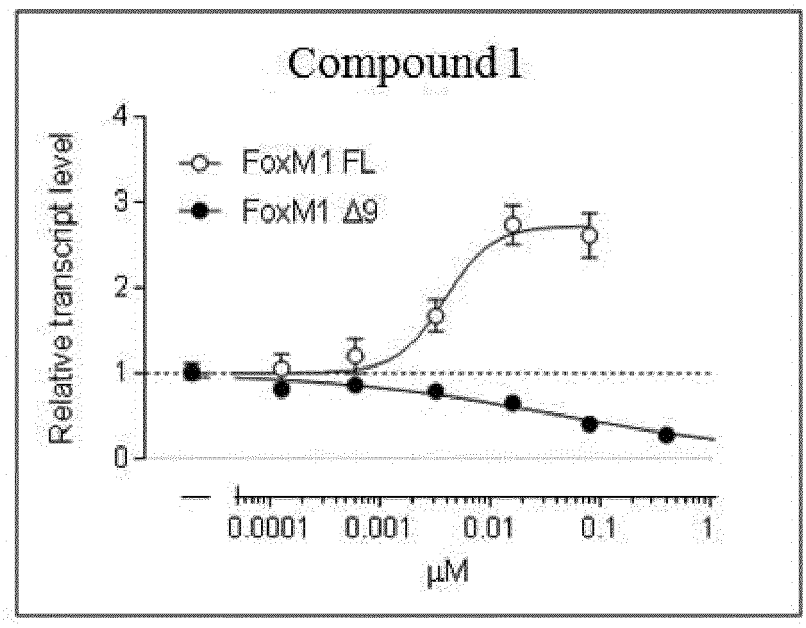
Compound 1
Relative transcript level
4
3
2
1
0
FoxM1 FL
FoxM1 Δ9
—
0.0001
0.001
0.01
0.1
1
μM

Fig. 1B

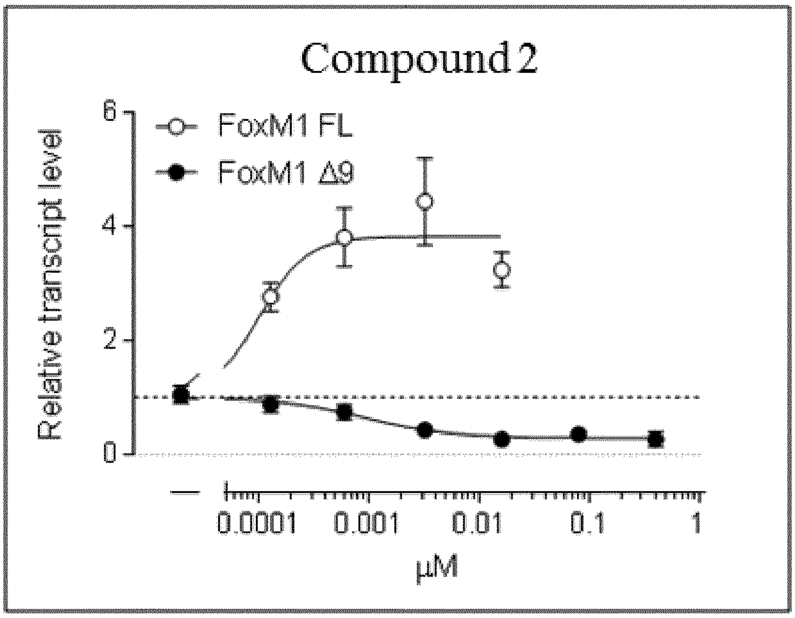
Compound 2
Relative transcript level
6
4
2
0
FoxM1 FL
FoxM1 Δ9
—
0.0001
0.001
0.01
0.1
1
μM

Fig. 1C

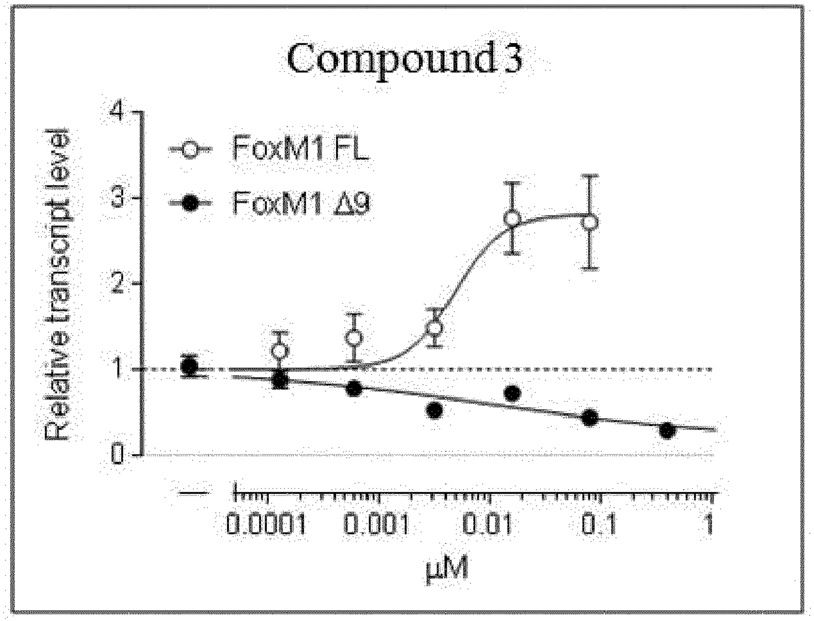
Compound 3
FoxM1 FL
FoxM1 Δ9
Relative transcript level
4
3
2
1
0
0.0001
0.001
0.01
0.1
1
µM

Fig. 1D

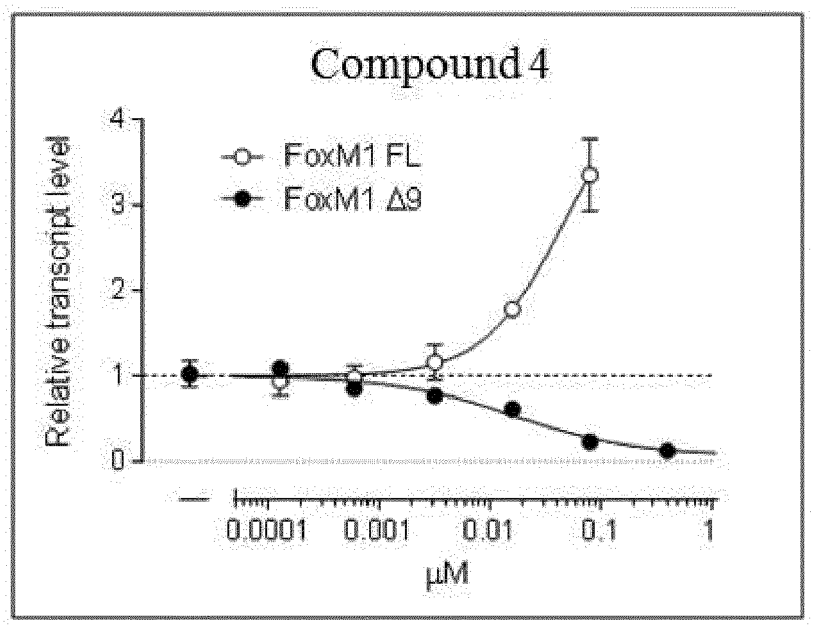
Compound 4
FoxM1 FL
FoxM1 Δ9
Relative transcript level
4
3
2
1
0
0.0001
0.001
0.01
0.1
1
µM

Fig. 1E

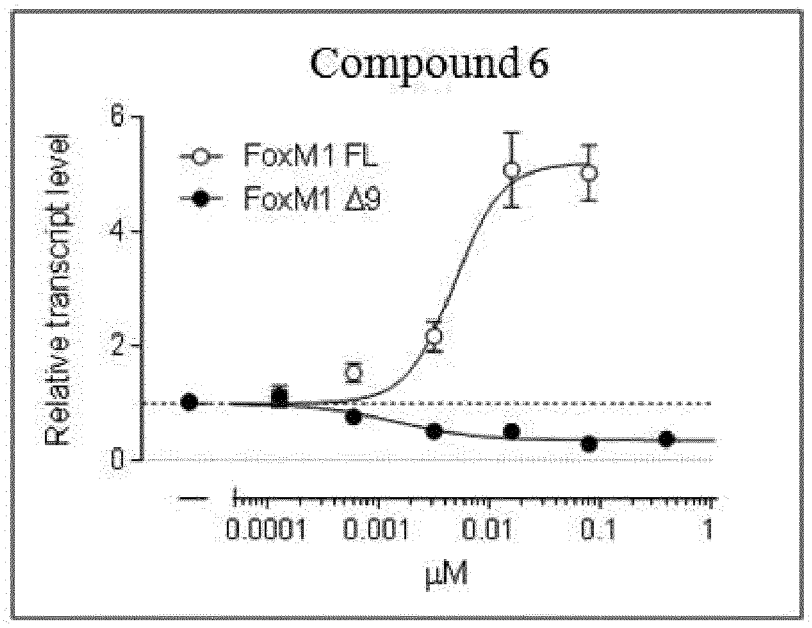
Compound 6
Relative transcript level
6
4
2
0
FoxM1 FL
FoxM1 Δ9
0.0001
0.001
0.01
0.1
1
μM

Fig. 1F

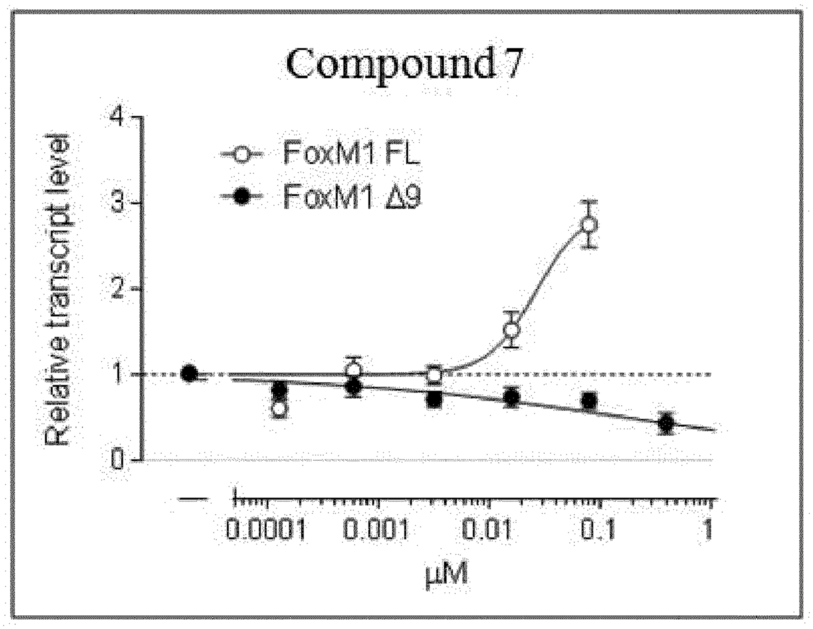
Compound 7
Relative transcript level
4
3
2
1
0
FoxM1 FL
FoxM1 Δ9
0.0001
0.001
0.01
0.1
1
μM

Fig. 1G

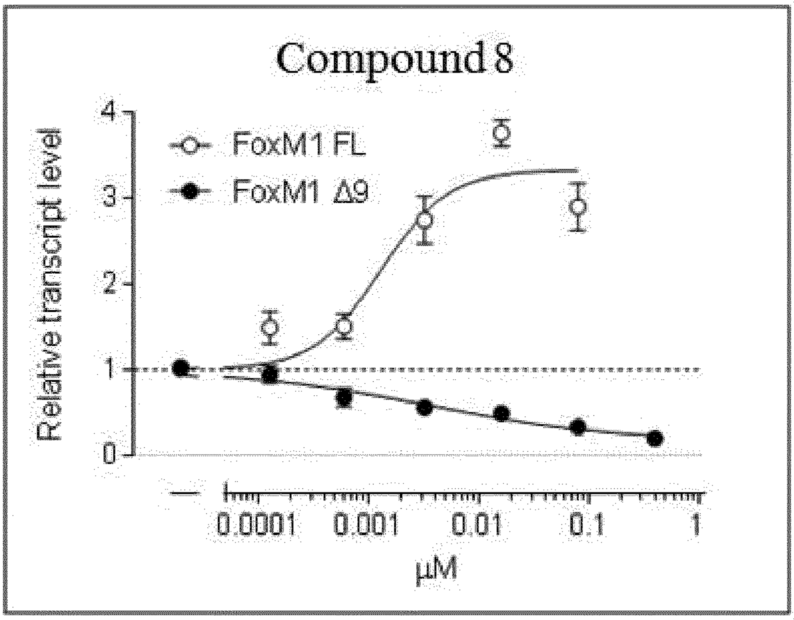
Compound 8
FoxM1 FL
FoxM1 Δ9
Relative transcript level
4
3
2
1
0
0.0001
0.001
0.01
0.1
1
µM

Fig. 1H

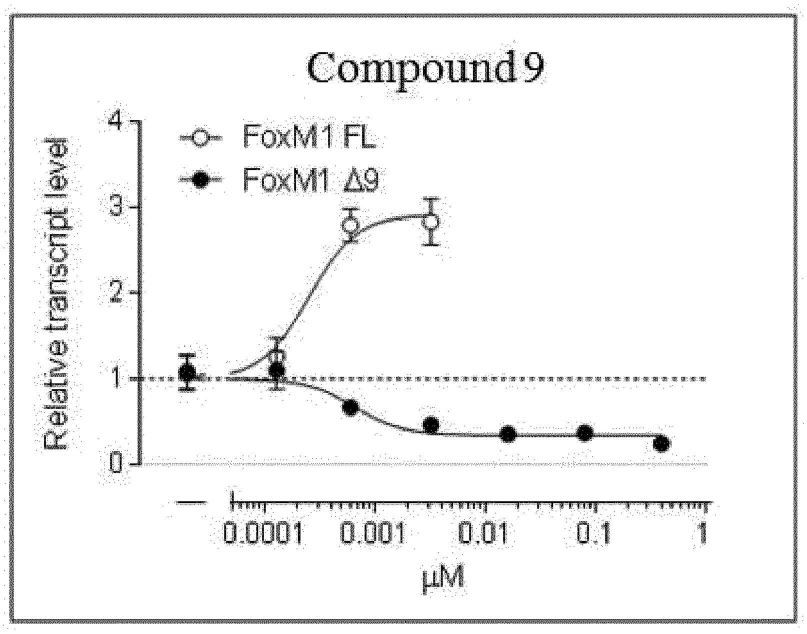
Compound 9
FoxM1 FL
FoxM1 Δ9
Relative transcript level
4
3
2
1
0
0.0001
0.001
0.01
0.1
1
µM

Fig. 1I

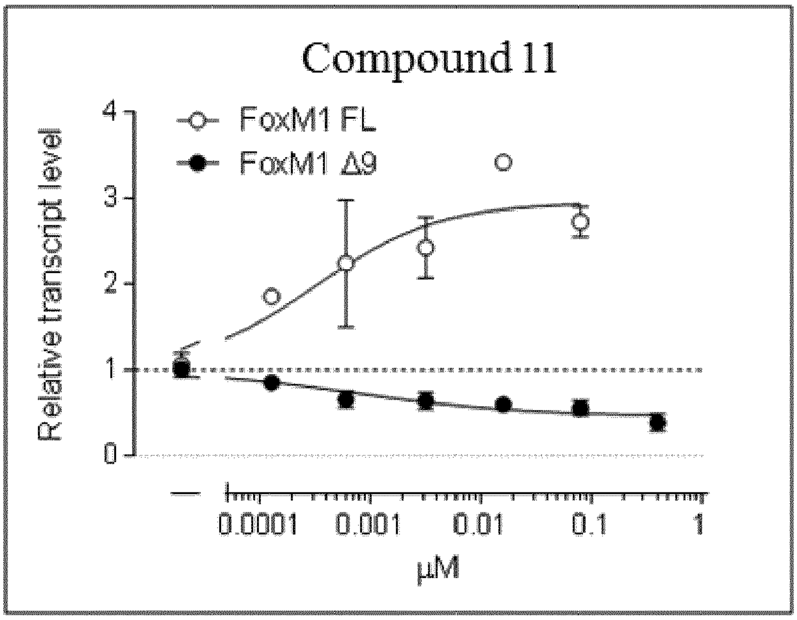
Compound 11
FoxM1 FL
FoxM1 Δ9
Relative transcript level
4
3
2
1
0
0.0001
0.001
0.01
0.1
1
μM

Fig. 2

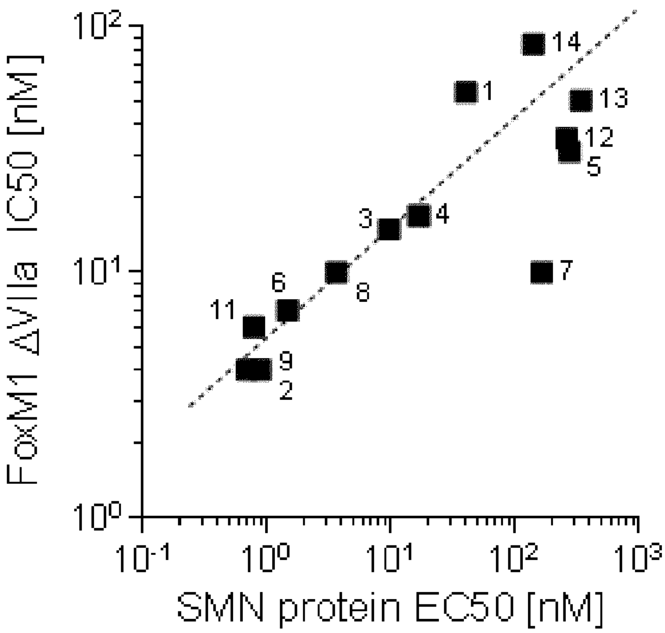
FoxM1 ΔVIIa IC50 [nM]
SMN protein EC50 [nM]
10²
10¹
10⁰
10⁻¹
10³
14
1
13
12
5
3
4
7
6
8
11
9
2

Europäisches Patentamt
European Patent Office
Office européen des brevets

# EUROPEAN SEARCH REPORT

Application Number
EP 15 15 4342

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2007/003604 A2 (NOVO NORDISK AS [DK]; HOHLWEG ROLF [DK]; ANDERSEN KNUD ERIK [DK]; SOER) 11 January 2007 (2007-01-11)<br>* abstract *<br>* page 42, lines 25-30 *<br>* page 73; example 14 *<br>* page 95; example 55 *<br>* page 111; example 79 *<br>----- | 1,2, 15-25 | INV.<br>A61K31/433<br>A61K31/435<br>A61K31/4353<br>A61K31/437<br>A61K31/438<br>A61K31/4436<br>A61K31/454<br>A61K31/4545<br>A61K31/4709<br>A61K31/506<br>A61K31/5377<br>A61P35/00<br>A61K45/06<br>A61K31/501<br>A61K31/502<br>A61K31/551 |
| X | WO 2011/082732 A1 (MERCK PATENT GMBH [DE]; STIEBER FRANK [DE]; WIENKE DIRK [DE]) 14 July 2011 (2011-07-14)<br>* abstract *<br>* page 25, line 5 *<br>* page 34, line 20 *<br>* page 36, line 5 *<br>* page 263, line 30 *<br>* claims 7,8 *<br>----- | 1,2, 15-25 | |
| X | WO 2007/133561 A2 (NEUROGEN CORP [US]; XU YUELIAN [US]; CALDWELL TIMOTHY M [US]; XIE LING) 22 November 2007 (2007-11-22)<br>* abstract *<br>* page 96; example 350 *<br>* page 41, lines 9-10 *<br>----- | 1,15-25 | TECHNICAL FIELDS SEARCHED (IPC)<br>A61K<br>A61P |
| X | EP 2 014 656 A2 (TRANSTECH PHARMA [US]) 14 January 2009 (2009-01-14)<br>* page 33; examples 19,20 *<br>* paragraph [0083] *<br>----- | 1,15-25 | |
| X | WO 03/074500 A2 (SANOFI SYNTHELABO [FR]; ARANYI PETER [HU]; BALAZS LASZLO [HU]; BATA IM) 12 September 2003 (2003-09-12)<br>* page 41; compound 9.28 *<br>* page 1, line 31 *<br>-----<br>-/-- | 1,15-25 | |

The present search report has been drawn up for all claims

1

| Place of search | Date of completion of the search | Examiner |
|---|---|---|
| Munich | 15 January 2016 | Garabatos-Perera, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 15 15 4342

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2008/145681 A2 (BOEHRINGER INGELHEIM INT [DE]; EBEL HEINER [DE]; PETERS STEFAN [DE]; H) 4 December 2008 (2008-12-04)<br>* page 41; compound 53 *<br>* malignant diseases;<br>page 50 *<br>* combinations;<br>page 56 *<br>----- | 1-3, 15-25 | |
| A,D | WO 2014/116845 A1 (NOVARTIS AG [CH]; AXFORD JAKE [US]; DALES NATALIE [US]; SUNG MOO JE [U) 31 July 2014 (2014-07-31)<br>* the whole document *<br>----- | 1-25 | |
| A,D | WO 2014/028459 A1 (NOVARTIS AG [CH]; CHEUNG ATWOOD [US]; CHIN DONOVAN NOEL [US]; DALES NA)<br>20 February 2014 (2014-02-20)<br>* the whole document *<br>----- | 1-25 | |
| A | WO 2015/017589 A1 (NOVARTIS AG [CH]; CHEUNG ATWOOD [US]; DALES NATALIE [US]; HURLEY TIMOT) 5 February 2015 (2015-02-05)<br>* the whole document *<br>----- | 1-25 | TECHNICAL FIELDS SEARCHED (IPC) |
| A | CN 103 965 169 A (PENG ZHENGZHONG)<br>6 August 2014 (2014-08-06)<br>* abstract *<br>* claims *<br>----- | 1-25 | |
| A,D | WO 2011/127297 A1 (UNIV ILLINOIS [US]; RAYCHAUDHURI PRADIP [US]; CARR JANAI [US])<br>13 October 2011 (2011-10-13)<br>* abstract *<br>* claims *<br>-----<br>-/-- | 1-25 | |

The present search report has been drawn up for all claims

1

| Place of search | Date of completion of the search | Examiner |
|---|---|---|
| Munich | 15 January 2016 | Garabatos-Perera, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 15 15 4342

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A,D | EP 2 298 896 A1 (TRUSTEES OF THE UNIVERSITY OF ILLINOIS BOARD OF [US]) 23 March 2011 (2011-03-23) * abstract * * claims * ----- | 1-25 | |

TECHNICAL FIELDS SEARCHED (IPC)

The present search report has been drawn up for all claims

1

| Place of search | Date of completion of the search | Examiner |
|---|---|---|
| Munich | 15 January 2016 | Garabatos-Perera, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 15 15 4342

This annex lists the patent family members relating to the patent documents cited in the above-mentioned European search report. The members are as contained in the European Patent Office EDP file on
The European Patent Office is in no way liable for these particulars which are merely given for the purpose of information.

15-01-2016

| Patent document cited in search report | Publication date | Patent family member(s) | Publication date |
|---|---|---|---|
| WO 2007003604 A2 | 11-01-2007 | AT 536344 T | 15-12-2011 |
| | | AU 2006264966 A1 | 11-01-2007 |
| | | BR PI0613564 A2 | 18-01-2011 |
| | | CA 2614116 A1 | 11-01-2007 |
| | | CN 103110635 A | 22-05-2013 |
| | | EP 1902028 A2 | 26-03-2008 |
| | | EP 2233470 A1 | 29-09-2010 |
| | | EP 2386554 A1 | 16-11-2011 |
| | | ES 2375929 T3 | 07-03-2012 |
| | | JP 5121707 B2 | 16-01-2013 |
| | | JP 2009500372 A | 08-01-2009 |
| | | KR 20080032069 A | 14-04-2008 |
| | | RU 2011142654 A | 27-04-2013 |
| | | US 2009312309 A1 | 17-12-2009 |
| | | US 2012232078 A1 | 13-09-2012 |
| | | WO 2007003604 A2 | 11-01-2007 |
| WO 2011082732 A1 | 14-07-2011 | AR 079483 A1 | 25-01-2012 |
| | | AU 2010341229 A1 | 02-08-2012 |
| | | CA 2784647 A1 | 14-07-2011 |
| | | CN 102639513 A | 15-08-2012 |
| | | EA 201200875 A1 | 30-01-2013 |
| | | EP 2513066 A1 | 24-10-2012 |
| | | JP 2013514287 A | 25-04-2013 |
| | | KR 20120096076 A | 29-08-2012 |
| | | SG 181643 A1 | 30-07-2012 |
| | | US 2012252815 A1 | 04-10-2012 |
| | | WO 2011082732 A1 | 14-07-2011 |
| WO 2007133561 A2 | 22-11-2007 | AU 2007249925 A1 | 22-11-2007 |
| | | CA 2651654 A1 | 22-11-2007 |
| | | EP 2021004 A2 | 11-02-2009 |
| | | JP 2009536651 A | 15-10-2009 |
| | | KR 20090015956 A | 12-02-2009 |
| | | US 2008247964 A1 | 09-10-2008 |
| | | WO 2007133561 A2 | 22-11-2007 |
| EP 2014656 A2 | 14-01-2009 | AR 066946 A1 | 23-09-2009 |
| | | CA 2690486 A1 | 18-12-2008 |
| | | CN 101795567 A | 04-08-2010 |
| | | EP 2014656 A2 | 14-01-2009 |
| | | EP 2166850 A1 | 31-03-2010 |
| | | JP 2010529200 A | 26-08-2010 |
| | | TW 200918054 A | 01-05-2009 |
| | | US 2010267721 A1 | 21-10-2010 |
| | | WO 2008154126 A1 | 18-12-2008 |

EPO FORM P0459

For more details about this annex : see Official Journal of the European Patent Office, No. 12/82

# ANNEX TO THE EUROPEAN SEARCH REPORT ON EUROPEAN PATENT APPLICATION NO. EP 15 15 4342

This annex lists the patent family members relating to the patent documents cited in the above-mentioned European search report. The members are as contained in the European Patent Office EDP file on
The European Patent Office is in no way liable for these particulars which are merely given for the purpose of information.

15-01-2016

| Patent document cited in search report | Publication date | Patent family member(s) | Publication date |
|---|---|---|---|
| WO 03074500 A2 | 12-09-2003 | AR 038868 A1 | 02-02-2005 |
| | | AT 443054 T | 15-10-2009 |
| | | AU 2003209514 A1 | 16-09-2003 |
| | | BR 0307960 A | 15-02-2005 |
| | | CA 2475312 A1 | 12-09-2003 |
| | | CN 1639159 A | 13-07-2005 |
| | | CN 1990486 A | 04-07-2007 |
| | | EC SP045274 A | 26-10-2004 |
| | | EP 1487807 A2 | 22-12-2004 |
| | | HR P20040910 A2 | 28-02-2006 |
| | | HU 0200849 A2 | 30-08-2004 |
| | | IL 163286 A | 03-06-2007 |
| | | IS 7434 A | 02-09-2004 |
| | | JP 4559737 B2 | 13-10-2010 |
| | | JP 2005529078 A | 29-09-2005 |
| | | KR 20040091102 A | 27-10-2004 |
| | | MA 27105 A1 | 20-12-2004 |
| | | MX PA04008613 A | 08-06-2005 |
| | | NZ 535662 A | 31-05-2007 |
| | | RS 79004 A | 15-12-2006 |
| | | TW I250978 B | 11-03-2006 |
| | | UA 78291 C2 | 15-03-2007 |
| | | US 2005130981 A1 | 16-06-2005 |
| | | US 2008161310 A1 | 03-07-2008 |
| | | US 2010210838 A1 | 19-08-2010 |
| | | WO 03074500 A2 | 12-09-2003 |
| | | ZA 200406467 A | 22-06-2005 |
| WO 2008145681 A2 | 04-12-2008 | AR 066799 A1 | 16-09-2009 |
| | | CA 2687931 A1 | 04-12-2008 |
| | | EP 2155689 A2 | 24-02-2010 |
| | | JP 5603770 B2 | 08-10-2014 |
| | | JP 2010528089 A | 19-08-2010 |
| | | TW 200904434 A | 01-02-2009 |
| | | US 2010204209 A1 | 12-08-2010 |
| | | WO 2008145681 A2 | 04-12-2008 |
| WO 2014116845 A1 | 31-07-2014 | AU 2014209315 A1 | 23-07-2015 |
| | | CA 2896875 A1 | 31-07-2014 |
| | | CN 104936955 A | 23-09-2015 |
| | | EA 201591363 A1 | 30-11-2015 |
| | | EP 2948448 A1 | 02-12-2015 |
| | | KR 20150107871 A | 23-09-2015 |
| | | TW 201443046 A | 16-11-2014 |
| | | US 2014206661 A1 | 24-07-2014 |

EPO FORM P0459

For more details about this annex : see Official Journal of the European Patent Office, No. 12/82

# ANNEX TO THE EUROPEAN SEARCH REPORT ON EUROPEAN PATENT APPLICATION NO. EP 15 15 4342

This annex lists the patent family members relating to the patent documents cited in the above-mentioned European search report. The members are as contained in the European Patent Office EDP file on
The European Patent Office is in no way liable for these particulars which are merely given for the purpose of information.

15-01-2016

| Patent document cited in search report | Publication date | Patent family member(s) | Publication date |
|---|---|---|---|
| | | US 2015218175 A1 | 06-08-2015 |
| | | UY 35273 A | 29-08-2014 |
| | | WO 2014116845 A1 | 31-07-2014 |
| WO 2014028459 A1 | 20-02-2014 | AR 092108 A1 | 25-03-2015 |
| | | AU 2013302859 A1 | 19-02-2015 |
| | | CA 2880273 A1 | 20-02-2014 |
| | | CN 104583196 A | 29-04-2015 |
| | | CR 20150078 A | 29-05-2015 |
| | | CU 20150014 A7 | 30-07-2015 |
| | | EA 201590371 A1 | 30-06-2015 |
| | | EP 2885288 A1 | 24-06-2015 |
| | | JP 2015524842 A | 27-08-2015 |
| | | KR 20150041655 A | 16-04-2015 |
| | | PH 12015500236 A1 | 30-03-2015 |
| | | US 2014051672 A1 | 20-02-2014 |
| | | US 2014213570 A1 | 31-07-2014 |
| | | UY 34974 A | 31-03-2014 |
| | | WO 2014028459 A1 | 20-02-2014 |
| WO 2015017589 A1 | 05-02-2015 | NONE | |
| CN 103965169 A | 06-08-2014 | NONE | |
| WO 2011127297 A1 | 13-10-2011 | US 2013142784 A1 | 06-06-2013 |
| | | WO 2011127297 A1 | 13-10-2011 |
| EP 2298896 A1 | 23-03-2011 | AT 501252 T | 15-03-2011 |
| | | EP 1758999 A1 | 07-03-2007 |
| | | EP 2298896 A1 | 23-03-2011 |
| | | JP 4852539 B2 | 11-01-2012 |
| | | JP 2008503234 A | 07-02-2008 |
| | | US 2009075376 A1 | 19-03-2009 |
| | | WO 2006009575 A1 | 26-01-2006 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 2298896 A1
- WO 2011127297 A1
- WO 2014028459 A1
- WO 2014116845 A1


### Non-patent literature cited in the description


- **W. KORVER ; J. ROOSE ; H. CLEVERS.** *Nucleic Acids Res.,* 1997, vol. 25, 1715-1719
- **H. YE ; T.F. KELLY ; U. SAMADANI ; L. LIM ; S. RUBIO ; D.G. OVERDIER ; K.A. ROEBUCK ; R.H. COSTA.** *Mol. Cell Biol.,* 1997, vol. 17, 1626-1641
- **NAKAMURA et al.** *Oncogene,* 2004, vol. 23, 2385-400
- **PILARSKY et al.** *Neoplasia .Q,* 2004, 744-50
- **LIU et al.** *Cancer Res,* 2006, vol. 66, 3593-602
- **WANG et al.** *Proc Nat. Acad Sci,* 2002, vol. 99, 16881-6
- **TEH et al.** *Cancer Res.,* 2002, vol. 62, 4773-80
- McGraw-Hill Dictionary of Chemical Terms. McGraw-Hill Book Company, 1984
- **ELIEL, E. ; WILEN, S.** Stereochemistry of Organic Compounds. John Wiley & Sons, Inc, 1994
- **CAHN et al.** *Angew. Chem. Inter. Edit.,* 1966, vol. 5, 385
- IUPAC - Compendium of Chemical Terminology. Blackwell Scientific Publications, 1997
- **HARLOW, E. ; LANE, D.** Antibodies: A Laboratory Manual. Cold Spring Harbor Laboratory Press, 1988
- **MICHAEL R. GREEN ; JOSEPH SAMBROOK ; PETER MACCALLUM.** Molecular Cloning: A Laboratory Manual. 2012, vol. 2, 028
- **ANSEL, HOWARD C. et al.** Ansel's Pharmaceutical Dosage Forms and Drug Delivery Systems. Lippincott, Williams & Wilkins, 2004
- **GENNARO, ALFONSO R. et al.** Remington: The Science and Practice of Pharmacy. Lippincott, Williams & Wilkins, 2000
- **ROWE, RAYMOND C.** Handbook of Pharmaceutical Excipients. Pharmaceutical Press, 2005
- **LEFEBVRE S. et al.** *Cell,* 1995, vol. 80, 155-165